(19) Europäisches Patentamt / European Patent Office / Office européen des brevets

(11) EP 1 379 686 B1

(12) **EUROPEAN PATENT SPECIFICATION**

(45) Date of publication and mention of the grant of the patent:
**05.09.2012 Bulletin 2012/36**

(21) Application number: **01991929.9**

(22) Date of filing: **09.11.2001**

(51) Int Cl.:
*C12Q 1/68* (2006.01)

(86) International application number:
**PCT/US2001/043035**

(87) International publication number:
**WO 2002/044413 (06.06.2002 Gazette 2002/23)**

(54) **METHOD OF DETERMINING EPIDERMAL GROWTH FACTOR RECEPTOR AND HER2-NEU GENE EXPRESSION AND CORRELATION OF LEVELS THEREOF WITH SURVIVAL RATES**

METHODE ZUR BESTIMMUNG DER EXPRESSION VON EPIDERMALEM WACHSTUMSFAKTOR REZEPTOR UND DES HER2-NEU GENS UND KORRELATION DER RESULTIERENDEN WERTE MIT ÜBERLEBENSRATEN

PROCEDE PERMETTANT DE MESURER L'EXPRESSION DU RECEPTEUR DU FACTEUR DE CROISSANCE EPIDERMIQUE ET DU GENE HER2-NEU ET CORRELATION DE CES NIVEAUX D'EXPRESSION AVEC LES TAUX DE SURVIE

(84) Designated Contracting States:
**AT BE CH CY DE DK ES FI FR GB GR IE IT LI LU MC NL PT SE TR**

(30) Priority: **01.12.2000 US 250122 P**
**04.12.2000 US 250469 P**
**11.06.2001 US 877177**

(43) Date of publication of application:
**14.01.2004 Bulletin 2004/03**

(60) Divisional application:
**10012188.8 / 2 361 990**

(73) Proprietor: **Response Genetics, Inc.**
**Los Angeles, CA 90033 (US)**

(72) Inventor: **DANENBERG, Kathleen, D.**
**Altadena, CA 91001 (US)**

(74) Representative: **O'Brien, Simon Warwick et al**
**D Young & Co LLP**
**120 Holborn**
**London EC1N 2DY (GB)**

(56) References cited:
**EP-A- 0 856 579**
**WO-A-02/44423**
**WO-A-95/17507**

- **HENGSTLER J G ET AL: "Contribution of c-erbB-2 and topoisomerase IIalpha to chemoresistance in ovarian cancer." CANCER RESEARCH. UNITED STATES 1 JUL 1999, vol. 59, no. 13, 1 July 1999 (1999-07-01), pages 3206-3214, XP002246248 ISSN: 0008-5472**
- **KE L D ET AL: "Differential expression of epidermal growth factor receptor in human head and neck cancers." HEAD & NECK. UNITED STATES JUL 1998, vol. 20, no. 4, July 1998 (1998-07), pages 320-327, XP009012684 ISSN: 1043-3074**
- **LEI W ET AL: "ENHANCEMENT OF CHEMOSENSITIVITY AND PROGRAMMED CELL DEATH BY TYROSINE KINASE INHIBITORS CORRELATES WITH EGFR EXPRESSION IN NON-SMALL CELL LUNG CANCER CELLS" ANTICANCER RESEARCH, HELENIC ANTICANCER INSTITUTE, ATHENS,, GR, vol. 19, no. 1A, 1999, pages 221-228, XP000929677 ISSN: 0250-7005**
- **MÉNARD S ET AL: "Role of HER2 gene overexpression in breast carcinoma." JOURNAL OF CELLULAR PHYSIOLOGY. UNITED STATES FEB 2000, vol. 182, no. 2, February 2000 (2000-02), pages 150-162, XP009012973 ISSN: 0021-9541**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- NEWBY J C ET AL: "Expression of epidermal growth factor receptor and c-erbB2 during the development of tamoxifen resistance in human breast cancer." CLINICAL CANCER RESEARCH: AN OFFICIAL JOURNAL OF THE AMERICAN ASSOCIATION FOR CANCER RESEARCH. UNITED STATES SEP 1997, vol. 3, no. 9, September 1997 (1997-09), pages 1643-1651, XP001147667 ISSN: 1078-0432
- KOOPMANS M ET AL: "OPTIMIZATION OF EXTRACTION AND PCR AMPLIFICATION OF RNA EXTRACTS FROM PARAFFIN-EMBEDDED TISSUE IN DIFFERENT FIXATIVES" JOURNAL OF VIROLOGICAL METHODS, AMSTERDAM, NL, vol. 43, no. 2, 1993, pages 189-204, XP000997425 ISSN: 0166-0934
- ETIENNE M C ET AL: "Response to fluorouracil therapy in cancer patients: the role of tumoral dihydropyrimidine dehydrogenase activity." JOURNAL OF CLINICAL ONCOLOGY: OFFICIAL JOURNAL OF THE AMERICAN SOCIETY OF CLINICAL ONCOLOGY. UNITED STATES JUL 1995, vol. 13, no. 7, July 1995 (1995-07), pages 1663-1670, XP009012683 ISSN: 0732-183X
- FLINKE J ET AL: "AN IMPROVED STRATEGY AND A USEFUL HOUSEKEEPING GENE FOR RNA ANALYSIS FROM FORMALIN-FIXED PARAFFIN-EMBEDDED TISSUES BY PCR" BIOTECHNIQUES, EATON PUBLISHING, NATICK, US, vol. 14, no. 3, 1993, pages 448-453, XP000877191 ISSN: 0736-6205
- HORTOBAGYI G N: "Developments in chemotherapy of breast cancer." CANCER. UNITED STATES 15 JUN 2000, vol. 88, no. 12 Suppl, 15 June 2000 (2000-06-15), pages 3073-3079, XP001153076 ISSN: 0008-543X
- FRY D W ET AL: "Specific, irreversible inactivation of the epidermal growth factor receptor and erbB2, by a new class of tyrosine kinase inhibitor." PROCEEDINGS OF THE NATIONAL ACADEMY OF SCIENCES OF THE UNITED STATES OF AMERICA. UNITED STATES 29 SEP 1998, vol. 95, no. 20, 29 September 1998 (1998-09-29), pages 12022-12027, XP002246246 ISSN: 0027-8424
- NESKOVIC-KONSTANTINOVIC Z ET AL: "Expression of epidermal growth factor receptor in breast cancer, from early stages to advanced disease." JOURNAL OF EXPERIMENTAL & CLINICAL CANCER RESEARCH: CR. ITALY SEP 1999, vol. 18, no. 3, September 1999 (1999-09), pages 347-355, XP009012808 ISSN: 0392-9078
- NICHOLSON R I ET AL: "Relationship between EGF-R, c-erbB-2 protein expression and Ki67 immunostaining in breast cancer and hormone sensitivity." EUROPEAN JOURNAL OF CANCER (OXFORD, ENGLAND: 1990) ENGLAND 1993, vol. 29A, no. 7, 1993, pages 1018-1023, XP001153037 ISSN: 0959-8049
- EADS CINDY A ET AL: "CpG island hypermethylation in human colorectal tumors is not associated with DNA methyltransferase overexpression." CANCER RESEARCH, vol. 59, no. 10, 15 May 1999 (1999-05-15), pages 2302-2306, XP002170143 ISSN: 0008-5472 cited in the application

SEQUENCE LISTING

<110> K. Danenberg et al.

<120> Method of determining Epidermal Growth Factor Receptor and HER2-New Gene Expression

<130> 11220/120

<140> To be Assigned
<141> 2001-03-12

<160> 11

<170> FastSEQ for Windows Version 4.0

<210> 1
<211> 18
<212> DNA
<213> Artificial Sequence

<220>
<223> Primer

<400> 1
tgcgtctctt gccggaat 18

<210> 2
<211> 20
<212> DNA
<213> Artificial Sequence

<220>
<223> Primer

<400> 2
ggctcaccct ccagaagctt 20

<210> 3
<211> 21
<212> DNA
<213> Artificial Sequence

<220>
<223> Primer

<400> 3
acgcattccc tgcctcggct g 21

<210> 4
<211> 23
<212> DNA
<213> Artificial Sequence

<220>

<223> Primer

<400> 4
ctgaactggt gtatgcagat tgc 23

<210> 5
<211> 17
<212> DNA
<213> Artificial Sequence

<220>
<223> Primer

<400> 5
ttccgagcgg ccaagtc 17

<210> 6
<211> 24
<212> DNA
<213> Artificial Sequence

<220>
<223> Primer

<400> 6
tgtgtacgag ccgcacatcc tcca 24

<210> 7
<211> 18
<212> DNA
<213> Artificial Sequence

<220>
<223> Primer

<400> 7
accaccacgg ccgagcgg 18

<210> 8
<211> 18
<212> DNA
<213> Artificial Sequence

<220>
<223> Primer

<400> 8
tgagcgcggc tacagctt 18

<210> 9
<211> 22
<212> DNA
<213> Artificial Sequence

<220>
<223> Primer

<400> 9

tccttaatgt cacgcacgat tt 22

<210> 10
<211> 197496
<212> DNA
<213> Homo sapiens

<400> 10

```
ttctttttag cacagaataa caatccattg tccacatgta ccatggttta tttatccact 60
catccacatg aagacatctt agttgattct aagttaggga agttatgaat aaagctgtta 120
taaatattca tgagcagatt tatgtggaca acagtgttca actcatttgg gtaagtatca 180
aggagagaaa tcattggatc atatggtaag agtatgtaca cttttatagg aaactgctaa 240
```

gctgcattcc taagtggctg taccattgtg ccttcccatc agcaatgaat gagacttcct 300
attgttccac atcctcatca gaatttggtg ttgtcactga tctgaatttt ttccattgta 360
acagatgtgt agtggtatct cactgttgtt ttaatttgca atttcctaat gacatatgat 420
gttgaacatc tttttatatg cttacttgcc atcagtgtat cctctgatga ggtgtttgtg 480
tagggctttg gcccattttt aaatcaggtt atttatcctc ttattattaa cttttaagag 540
tttagttctt tgcatatttt ggataacaat cctttatcac atatttcttt tgcaaatttt 600
tctccagtat atggcttgtc ttcttctcct ggcattgtcc ttctcagagc agaagttttt 660
aattttaata aactccagct tataaattat ttatttcatg gattgtgcct ttggttttgt 720
acttaaaaag tcattgtcat acctaaggtc atctaggttt tctcctacgt tatctcctag 780
gtgttttata gttttgcatt ttacatttat atgtatgatc agttttgagt taatttttat 840
gaagtgtgta aggtttgtat ctacattcat ttttgcatg tggatgtcca tttgttccag 900
caatatttgt tgaaaagact atacttgctc tattgtattg tgtttctttt ttgtcaaaga 960
tcaattgact aaatttatgt gcgtcagttt ctgatctctc tggtccattg atatatttgt 1020
ctattctttc accaatacca catagtctag actactgtag ttgtatatgt cttgaagtca 1080
ggtagtgttg atcctccaat tttgttctcc aatattgagt tggctattgt gggtcttttg 1140
cttgcccata gaatcaatta gtcaatattt acaaaataac ttgctcgaac tttgactggg 1200
attaatctat aaatcaagtt gggaataagt gacattttga cattatggag tctttctgac 1260
catgaacaca aactattgat ccatttattt agttatttga tatctttcac cagagttttt 1320
ttgttttttt cttatagatc ttatacatat tttcttatat tcatacctca gtattccatt 1380
tcagggtgtt aatgtaaatg gtaatgtgtt tttaatttca aattccctta gttctttgct 1440
ggtatatagg aaagtgattg gcttttgtat gttaacgttg tatcctgctc acttgctata 1500
actgcttatt agttccagga gctttttat tgtttctttt ggattttcta agagacaatt 1560
acattatcag tgaacaaaca cgatttattt cttccctccc aatcagtatt catttttattt 1620
atttattgtg tgttattgca ttagctagga cttctaatac aatgttgaaa agcattggtg 1680
aaaggaaaca tccttgcttt gttcctgatt ttagctatag gtttttgtag ctgttcttta 1740
ttaagttgag gatatccttc tctattctta gtttgctgag aatttttatc atgaataggt 1800
gtaggatttt gcctaatgtt ttcttctgta tctattgata tgatcatgta atttttcttt 1860
atctattgat atgatctgtt gatgtgatga actacattaa ttgagttttc aaatgttgaa 1920
ccagtcttgc atatctggaa taaatcggag ttggtcatca tgtataactt tgttacactt 1980
tgttgcattt gattttgtaa tattttcttg agaattttta catctatgtt cataaaagat 2040
atcggtctac agttttcttt cctttcttgt aatatctctg tctggttttg ctattaaggt 2100
aattctggct tcacaaaatt aattatggag tcttccctct acttctagtt tctggaagag 2160
attgtagaga atggatgtaa tttctttctt aaatgtttga cgaaaatcag cactgatctc 2220
atctgggctt ggtgctttct gttttggaag gttattaatt atttattcaa tttctatagt 2280
ggatataggc ctatattgat tggcaatttc ttcttgtatg acttttggta cattctattt 2340
caaggaattg gttcatttca tgtaggttgt taaattcgtg ggtatagctg ttcataatat 2400
tcatttatta tcctttcaat gttcatgaga tcagtagtga tgttccttct ttcatttctg 2460
atattcataa tttgtgtatt ctctctttgt ttcttagcct ggtgagaggc ttataaattt 2520
tattgatttt ttgaagaatc actttttggt tttgctgatt ttcttgctgg gattataggc 2580
gtgagccacc acactcttgc tcattttttc tatttttgtt ttccacactt tttctgcctc 2640
tgtggatttt acacagcatt ttgtataatt cgatttcctc tttttagcat agcaattatt 2700
ttagtcttta actttttaaa atcagttgcc ctagattttt ttctcccaac attttgggag 2760
gccaagggga gaggatcact tcaagccagg agtttgagac cagcctgagc aacatagcaa 2820
ggcactatct atacaaacat aattttaaaa agtccaggca ttagctagga ctgtgcctgt 2880
agtcccagct actcaggagg ctgagatgag aagatcaccg gagcctagaa atttgaggct 2940
gcagtgggct gtgatcatgt cacttcactc ctgcctagaa gacagttaga ccctgcctct 3000
aaaataaaca agcaaataaa taaaaagaaa ggaagaaaag aagagcaagg gcagcaaata 3060
gaaaatagta ataaatatgg tagctattaa tccaactatg tcaataatta ccttaaatgt 3120
tagtggtcta aatatactgc aatggactga atgtttatgt ctcctcaaaa tgtatatgat 3180
gaaatgtaag ctcccaaaat gatgttatca ggggagagtg tttttgggag gggattatgt 3240
catgagggtg gaggccttac aaatggggtt agtgctataa aagagaccac agagagctgc 3300
cttggtcctt ctgccatgtg agggcactgt gaaattatgg ccatctatga agaagtgggc 3360
ccttattaga catcaaatct gcaaatacct tgatcttgaa tttcccagcc tccagaacta 3420
tgggaaataa atttctgttg tttacaagta aatcatttta tgttattttg ttacagaagc 3480
ccaaaaagat gaagacatac accagatcac tccattctct tcttgcttgc atggtttctg 3540
aggatacgtt ggatgtaatt ctaatattct ctataggtat tttctttatg gctcctctac 3600
aggtaaggtg ttttttccct ttggcttcat ttaagaattt ttcgttaacc tttgattttc 3660
tgaagtgtga atatgttatg cctaggtatc atttgtttgt ttgggttttc ttggcatata 3720
tcttcctgat gttctctgaa cttccagaat ctgtgatttg ttgtctgaca ttaatttgga 3780
ggaattttg gtattattgc tttaaatatt gcttctgctc ctttttctct ttctttgact 3840
tacagtattc ccattacatg taattatctc acagttcttg aaagttttgt tttgttcttt 3900
ttgttagtcg tttttctttc tgtttttcag ttttggcagt ttctgtttac gtatcttcaa 3960
tctcagaaat tctttcctca agcatgttca gcccactaat gtgtacttca aaagtattct 4020

acatttcttt tacagtgttt ttgatctcta gaatttttaa attctttctt aaaactttca 4080
tctctcagga attcaagact agcctgggca acatagtgaa actctatctc tacaaaacat 4140
tagccaggta tggtgatgca tgcctgtagt cagagctact caggaggcta aggtgggagg 4200
atcacctgag cctgggaagt tgaggttgca gtgagccaag gtcacgccac tgcactctgg 4260
attgggcaac agagccagac cctgtctcaa aaaaaagaaa aattccatgg ctctgcttac 4320
attatccatc tgatcttaca tgttgcctat tttttccatt aaaactccta gcctattaat 4380
catagttttt ttataattaa tactccgatg tgataatgtc ttagtccaat tactgtggtt 4440
ataacagaat gccacaaact gggtgattta taaacaaaag aagctgattt aggctgattt 4500
agaggctggg gagtccaaga gcttggtgct agcatctgat gagtgtcttc ttgcttcatc 4560
ataacatggg agagggcatc acgtgtgaag agagcttact cttataacat agccactccc 4620
acaagaatta acccaccgcc atgagagcca tgtgaattca ttcatgagga cagcgggtta 4680
agtttccaat atatggactt ttcggggaca cattcaaacc acagcagtta gttgtaacgt 4740
tcgtgtcatg tctcattctg gttctgatgc ttgtgcagtc tcttcaaact gcgtctttgc 4800
cttttagtgt gccttgcaat gtggaaatga tatactgggt aagaggagct gtagtaaaga 4860
ggcttctagt gacgtagtga caagctgtgg ggagagggag tgttgcacag tcctgccgca 4920
tgtcacagtc ttccagtgag cctgtgtccc tggactgtga acttcatgct tgcttctcag 4980
cttccccagc cccttagatg gtacagaact gttggagggg ggtggagttg tatatttccc 5040
ttgctctggg taggtcaccc tctgataaaa caccaggtta ggcctctggt gaaataattt 5100
ctcctgaggg cagaccttct attaataata gaatgttcca acctatttca aaatggttcc 5160
tcttctcctt ccactgccag aagcataatg agattttccc cctaatattc gtggtaagga 5220
cctagcagag ctccaggagg taacactctc aagtgtctca tactaccctg caccatgact 5280
gggctctgct ggagttctta atttgcagaa ctgcccacac tgagcctccc gcaatttctc 5340
aattacaggg caaactttcc cagccggcac tgggtccttg gaggtttctg tctgctggtt 5400
tcttcctctg gaggttgtgc ttctgtgttt gcctgtctct ccaatttggg gggcagtggt 5460
ttgcccaatg acctcaattc tctgaaagag ctaagaagag gtgttaattt ttcggtttgc 5520
tcagctttct acttgttgct agaatggagc gccaatagtg cctcctatag tgacatgtaa 5580
ccctcaactc tagagatgat gaagcatact aatgacaaag gagaaatgct tcagcagttt 5640
tctgtcagca cattacccct tgaaaaagct gcttcttcca cattctgcaa gagatgggtc 5700
tcaactcaga gctcaaggca aatgacttcc ttcaaggaga aggaataaac agtctcagaa 5760
accatgaaag cctgccccca ggagtgtccc tgaacctcag caggggccac acttaccttg 5820
cagaaatagg tgaggcatgc tcctggtaca aaatcccaat ggtacagaag gacaaattga 5880
aaaacaagtc tccctctaaa cccctgaccc cgagctacct agttctcctc cctagaggca 5940
aagctgttac cagattcttg tatctcctta acatatatcc ttagaagagc tgtcaagtga 6000
acacatgttt aagtgaaaac ctattttaga agtgcatttt cttaaggaac tttagggttg 6060
gaaggaacct gtgtcagtcc ttaattcaca acctccatta gtacttattg ttcttgcaca 6120
aaaatctttc tcaaaaaagc cctttccact ctgacatagc ttattctact tttacttagc 6180
tccaataact tataaaacat atttttgaaa gtctaaaatc tgccactatg tttttttttc 6240
ctaatcaatc tttactttga cctctaagcc agagaaaaca ggtggtcaaa tgccttttgc 6300
ctaagatgga acttagaata tttgaagacc tcagatcttc accctgccaa ataacgtgtt 6360
tctcctcccc tttcacagag catttggttt taggaaattc agagccacat tccttataga 6420
caagactaaa ctcttattca acatactcag aaacttcttc taagaggata accactcatc 6480
agaggaaaaa agtttctcat gtacagctgg caaagggatg gaaccatctg tgttattaaa 6540
attgacagac gcttatgaga tttattaagg gaaatactag agtcttagta catacttgct 6600
aatatagcat acatgaaggc tttatctata atttttttgg ccaagcagaa attttggtat 6660
tactcaccct aacaaatttc caagacatta tgaaatagaa ttttaggtcc tgacatcacc 6720
atttgtctca ggttttgaag cgttgctgga caagaggggt aaaacacggc tctgccttgg 6780
attcaaagtt ggcctctcat actagcaagt ataccttggt atcctggtca cttctcccgg 6840
ccacagcatc acattgctat aaaaggcaga tacaagtatt aaccagctca caggttatca 6900
gataagctta gtctgaccaa tgcttaacac agcaactggg ccactattgt cattcctgtg 6960
gtggtggcac acacacccag cctctgtccg ggccatggtc taggaccacc ctccacagag 7020
gctgtgagct agagccctaa ctgtgcaggg ccctaactat gccaggctac ttatctctct 7080
taagaggact tcattagtgc ctgctcggcc atacagtttt ttacttacca agtaacacag 7140
ttatcagcac actccaggta ctagccaagg actacaaaat caacgtgaat gtcagctttt 7200
gtatcaaaag ctcaaaggag aaactcaaac tttacataga tgtcccatga agatgttcag 7260
caaacccatt cttctctgtt ccctggaatc catcccagta ttgtgctatg tgtgtgtcta 7320
gtaattcttt acaaaaagct ctgtttcttg tgatgctatc agatcacatt gaagaatata 7380
caagccgtac tatgaaggct gttgtctcat atagtcctaa cgtagtgaga actgatgttc 7440
ttacatgctg tctttttggg cactcaaaga aattcctgta cagtcttaca aatcagttgt 7500
agcttaaatt gatttgtgtt gtgacttgta cacacaggtc acattccctt gacagaaaat 7560
atagtttaaa accaaatttg cagcccttgt taagtgaatg cacaggactt tattgtattc 7620
aggtctttta ttgtaagact cactcctgtc ttcattttat gttccactgt tgtgcttccc 7680
atttgccttt ctctagtttt gttttctgtg tttctacgga ctgctctcag cccaggtgtg 7740
caggaagcac acacatgcct gcagagcctt catggcctct gcattcaggg catgacttca 7800

acgcacagtg gctgtactga tttgttaaaa caaaggaaca gattacttct cctaattcac 7860
agggaagttc caggttgtgc gggcagtgag cagacctgtg tctgtctgcg cttgccctgg 7920
tgaaaaaccc caccgttcag gctgcagggt gcgagaccca ggcacaaaca ttttgctgga 7980
tgaggaggaa agatgtaagg ttgctcccct tcagagacag caaagggcag gtctgtagct 8040
tcacttactt caggattgtg atttttgaca gagccgagag atcagggttg ttgaaccagg 8100
cctgaaggtc ctagtgaatc tcgtgaagag aggaggggtc tggctgtaac atggacctag 8160
aggacatttt tactgcagga gaaggaacag tggggatggg gtggacttgc caaaggaata 8220
tagctcaagt tcctgcagcc caaaaaagct cagtttcttt tggccaaagc ttccgcgagt 8280
ttccctggca tttctcctgc gggagctaca ggggcagtgg gacacttagc ctctctaaaa 8340
gcacctccac ggctgtttgt gtcaagcctt tattccaaga gcttcacttt tgcgaagtaa 8400
tgtgcttcac acattggctt caaagtaccc atggctggtt gcaataaaca ttaaggaggc 8460
ctgtctctgc acccggagtt gggtgccctc atttcagatg atttcgaggg tgcttgacaa 8520
gatctgaagg accctcggac tttagagcac cacctcggac gcctggcacc cctgccgcgc 8580
gggcacggcg acctcctcag ctgccaggcc agcctctgat ccccgagagg gtcccgtagt 8640
gctgcagggg aggtggggac ccgaataaag gagcagtttc cccgtcggtg ccattatccg 8700
acgctggctc taaggctcgg ccagtctgtc taaagctggt acaagtttgc tttgtaaaac 8760
aaaagaaggg aaagggggaa ggggaccctg gcacagattt ggctcgacct ggacataggc 8820
tgggcctgca agtccgcggg gaccgggtcc agaggggcag tgctgggaac gcccctctcg 8880
gaaattaact cctcagggca cccgctcccc tcccatgcgc cgccccactc ccgccggaga 8940
ctaggtcccg cgggggccac cgctgtccac cgcctccggc ggccgctggc cttgggtccc 9000
cgctgctggt tctcctccct cctcctcgca ttctcctcct cctctgctcc tcccgatccc 9060
tcctccgccg cctggtccct cctcctcccg ccctgcctcc ccgcgcctcg gcccgcgcga 9120
gctagacgtc cgggcagccc ccggcgcagc gcggccgcag cagcctccgc cccccgcacg 9180
gtgtgagcgc ccgacgcggc cgaggcggcc ggagtcccga gctagccccg gcggccgccg 9240
ccgcccagac cggacgacag gccacctcgt cggcgtccgc ccgagtcccc gcctcgccgc 9300
caacgccaca accaccgcgc acggcccccct gactccgtcc agtattgatc gggagagccg 9360
gagcgagctc ttcggggagc agcgatgcga ccctccggga cggccggggc agcgctcctg 9420
gcgctgctgg ctgcgctctg cccggcgagt cgggctctgg aggaaaagaa aggtaagggc 9480
gtgtctcgcc ggctcccgcg ccgcccccgg atcgcgcccc ggaccccgca gcccgcccaa 9540
ccgcgcaccg gcgcaccggc tcggcgcccg cgcccccgcc cgtcctttcc tgtttccttg 9600
agatcagctg cgccgccgac cgggaccgcg ggaggaacgg gacgtttcgt tcttcggccg 9660
ggagagtctg gggcgggcgg aggaggagac gcgtgggaca ccgggctgca ggccaggcgg 9720
ggaacggccg ccgggacctc cggcgccccg aaccgctccc aactttcttc cctcactttc 9780
cccgcccagc tgcgcaggat cggcgtcagt gggcgaaagc cgggtgctgg tgggcgcctg 9840
gggccggggt cccgcacgtg cgccccgcgc tgtcttccca gggcgcgacg gggtcctggc 9900
gcgcacccga ggggcgggcg ctgcccaccc gccgagactg cactgtttag ggaagctgag 9960
gaaggaaccc aaaaatacag cctcccctcg gaccccgcgg gacaggcggc tttctgagag 10020
gacctccccg cctccgccct ccgcgcaggt ctcaaactga agccggcgcc cgccagcctg 10080
gccccggccc ctctccaggt ccccgcgatc ctcgttcccc agtgtggagt cgcagcctcg 10140
acctgggagc tgggagaact cgtctaccac cacctgcggc tcccggggag gggtggtgct 10200
ggcggcggtt agtttcctcg ttggcaaaag gcaggtgggg tccgacccgc cccttgggcg 10260
cagaccccgg ccgctcgcct cgcccggtgc gccctcgtct tgcctatcca agagtgcccc 10320
ccacctcccg gggaccccag ctccctcctg ggcgcccgcg ccgaaagccc caggctctcc 10380
ttcgatggcc gcctcgcgga gacgtccggg tctgctccac ctgcagccct tcggtcgcgc 10440
ctgggcttcg cggtggagcg ggacgcggct gtccggccac tgcagggggg gatcgcggga 10500
ctcttgagcg gaagccccgg aagcagagct catcctggcc aacaccatgg tgtttcaaaa 10560
tggggctcac agcaaacttc tcctcaaaac ccggagactt tctttcttgg atgtctcttt 10620
ttgctgtttg aagaatttga gccaaccaaa atattaaacc tgtcttacac acacacacac 10680
acacacacac acacacacac cggattgctg tccctggttc aagtgtgcca agtgtgcaga 10740
cagaacatga gcgagtctgg cttcgtgact accgaccata aacccacttg acaggggaaa 10800
catgccttgg aaggtttaat tgcacaattc caaccttgag ctgcgcgggt tccaagagcc 10860
aggcccgtac ttgctgttga tgtcattggc ttggggagtt ggggtttggt gcccagcgcg 10920
gtcgttgggg gaggggcaag gcatagaaca gtggttccca gaccttgctg cacattggaa 10980
ttacctggga ttaaaaaaaa aaaaatcaaa acaaaaacca gtgtctggct cccgccccca 11040
gacattctga tttaattggc atggggcaag acctggactt gggatttttt ttaatgctct 11100
tcatgtgatc tgttgggcag ccagatttgg ggatcactag acggaagaag gattgttaaa 11160
gtctccggag atgttacttg ccaatgctaa gagctctttg aggacatctg gaattgttac 11220
aatattgcca aatataggaa agagggaaaa ggtagagtgt gattccaata ataaaggatt 11280
ccgcttttca ttgaaggaac tggtggaaag gtttcttctc tgctgagcct gcaggcccgt 11340
cctgcctgcc tggggtgccc gggagacgcg ggcctgctcc ggagactgct gactgccggt 11400
cctgttagtc aggtgtcagc cctgtctctg ccgaagagac tcttctcttt atttaaatt 11460
aaaccctcag agcaccacca aagcatcact tttctccctc cattggtgtt ctcattcttt 11520
gatgttactt gtttgaacac cactattagt agttggagat ttgttcctga gaaaaatata 11580

aataccactt aatttgcctg tttgtcccgc attcactcaa aacagaatgc tcctgaagac 11640
aagagagaga gtaggagaac agacgctatt ccattacagt aacataaaag actggatttt 11700
caggggcaaa ttattaaaat aggagatgag ctcttttaac agaaatttgt ttaaggcctg 11760
tgtctatcaa attcagtgga ttttattcaa gatgcacttt gtttagtggg agttttgttt 11820
ggttctggga catgctaact tctagacttg ctgctcttag aggtaatgac tgccagacac 11880
catttcatga gtcctaatcc ccacattaag cataagaggt gcacactctc ctcctatggg 11940
ggaaactgag gtacgaagaa ctaaagtgac tttcccacag ctggtgggag gcagacggga 12000
aattcacacc aggggcttcc aactccagat ccctctctca acttccaaac tccactgcct 12060
tgtccgagtt ctggtttcag gagatccaaa tcaggtgtgt gcaaatgtct aatgtcagag 12120
ctggcaaggg gaaagggccc agggagccgg ctcatgacga tgagcctgtc tgaagcttca 12180
acgcgggctg tccggcagtc tgcattcctg ccgagttcct cagccctctg ttgggtcacc 12240
ttccatagag gcagcttagt cctcagttca gtgagcatgg agtggagact gcttgagggg 12300
tgctgagcaa agccctgcct cttacaggat gaaggtgctc tccagaaggg acactggaaa 12360
gtattccaag gcgagtcgaa ttcccaactg agggagcttt gtggaaataa gcccgcccag 12420
ccccacttct ggagacgttc ccattcagta ggtccgagct gtcttaaaga gaaaccaaag 12480
tggggatatt aatggtatcc aaagtgagat ctaccccacc ctccctcctc aaaggaggtc 12540
agatcaagaa agcccaagcc cggcctggca attgggacct ttcttctcac tccagcccag 12600
ggtgaaggtg gacaagtcac tttgaccctt caggcttctg agctgttgtt tctgaattca 12660
gtgaatattt actgagtgca tagaatatgc tagatattct gggctaaagg ttgaaggggg 12720
ggtgagtttt aagggtttct gctcttgctt ccagattgct ttcaaatctg gaaaggacac 12780
cagtggtttg tgtgttagac ccacactgcc gtagcacaga atacaagaaa ctggctgaga 12840
gctccaatag gcttttaaca gtaatttctg gcttcacgta tttagtttca taactcatga 12900
tttttcaaaa acttctggtt tgaagacacc gattgccgaa agtccattgt gctgcataat 12960
tacacttggt ccacgtgaca gcactaacat gttctgaaat gtttttagaa gtagtctcag 13020
caaagatgaa ggattcctcc ctgtttgaaa agaaaatatt ctttgttttt tctttgatct 13080
aagctctaag actagcagct agcatctgaa actttttga cgagagtgac aaaccaactc 13140
taatattaaa ggcaattgat gattatgggc actgaaggga aggtaacccc aggctggtgc 13200
cccggaatag ggatgggtca caatgttgag gacatttcgc ctgttgcaga acccacctgc 13260
aacacagtgt ggcccttgcc atgtgacttg tgtgtgtgcc tgtgtgtctg tgtgtgcgtg 13320
ttttaatttt gacttcataa gtactctagt tatgagctta tttaacattg ggttttacta 13380
ataggggtat gtgttgagaa aatttcaaag ttttagaata tggttcaccc acatgttgct 13440
tccctgtaaa tataattttt aaaaccagat tctgggccgg gcatggtggc tcacctctat 13500
aatcccaaaa cgttgggagg ccgaggcagg cgaatcatga agccaggagt ttgagaccag 13560
gctgaccaac acggtgaaac ccagtctcta ctaaaaatac aaaaaaaatt agctgggcgt 13620
ggtggcaggt gcctgtaatc ccagctactc aggaggctga ggcaggagaa ttgcttgaac 13680
ccaggaggca gaggttgcag tgagccaaga tcgcaccatt gcactccagc ccgcgcgaca 13740
gtgtgagact ccatctcaaa aaaaaaaaaa aaaaaacaga ttctgttcct cagatccatt 13800
ccatttttgt tttcctttat cacttatgga catttgaaat tatggtaata aacattgtta 13860
gtctcagtta attattactg gtttattctt gaaccactaa tccatagaga atagagtgta 13920
aatcttaact tgttcctgta ggccatcccc attaaacatc atagtgtttt ctcattcgtt 13980
ctttttcgtt ttcctcctac aggaatgaat tttctaagaa aattccagca gttggctctt 14040
tggacgacat ctctagattg tcctccattg ggcccatagg cacaagctgg ccagtttgaa 14100
tttgggcaag aatccaggca ttggaactta ttcaaataac tagtttgcct gtaattttca 14160
ctttttcaga gtcatctgat aaagctttct tgctacacat ttagatagat acactcaatc 14220
cagttgtcta gaaagttccc tgagccagct gggagcagga ggggtagttg gggccaggaa 14280
tattggggt gtgtttactg agcccctaga aagtaagtgc tagatttgac atttcaatcc 14340
ctgaaggccc tgaagttcag tatcaaatga ctggtcctgt ggactgagca tctgtgaatt 14400
gcatatgctt agagtaaatt ttactcctac cagtttcagc agcttgcttt agcaagcagt 14460
atggaaacac taacatgggg gagtagaatt tctctctctg atccaagttt tatctcattc 14520
tggtgggttt tcaaggagag actcggagtc caagtgtcct ttctgaatat atctggaact 14580
tctcattaac aaaagactca agttataatt taggggacaa ggcacccaat gagaatgcct 14640
tgcaggcagc cctaagtaca cctgcaatta caccattact agcgcggcag cacacatggc 14700
cctgacttag tttaaataat tacgtaagtc aaccatgatt gtttgccctt tgcatagaag 14760
ggcaagtatt ggtacctgtt acaacttagg ctttttttc tttatgtttg agccatgatg 14820
agtgatttac actgttgcat ccatatgttg agatgtaaga ataaattaga cttggtaatt 14880
gcccttaagt gtctggaagt caactgggga aagagagcta gagataataa gtgtgaaaca 14940
atgtcacaga atcaatgacg gaactcttcc caggacaaag gatgactttt gagttcagtc 15000
tttgccttta attctacatg gggaggagag cacgtttagc cacaaatgga agggattact 15060
catttgagct atttggttat atgattattt ccccagagaa taggatgtgc agggcattac 15120
acaagcagtg ccaatagcag caaagttctt gagagtgcta gtaattcaaa tggcaggaag 15180
agaaggaata aatggtaagg ctacctacag ttcacagaga gctccatcct cactgtggct 15240
ttggattttg tcctgtgtga aagagaagtg actgtgaact gacatgctgt gtttggtgtt 15300
ttagaaagat ggctgcagca gcggtttggg gaatggactg caggagtggc attggaaaca 15360

ggaaggttca tgactattgc cagagacaga ggatgaagca ggagcaagga agattcagga 15420
caggggactc cggggctgat caggaggcag aactggttga taagtatatg tagcagcata 15480
agaaagaaag aatcccagat tgacacccag gcttctcact tggaagcctg gatagatact 15540
gaatgcaatc acaaaggctg ggaagtcaat gggactgcag ggaagggaag ggaagggagg 15600
agaagaggaa gggcaggagg gtccaatatc aatattcagc ttttagatgt gttgagcttg 15660
aagtgctcag atggagaagt ccaggaggca gtagaatacg gtggtccaga gcacaggaga 15720
gcaatgtggc ttgagttgtc atttgctcac atatttccgt gtcagttact tgtcttagat 15780
cacagaacaa gttctcctct cacagtttcc tggctccacc tgtctcatgc tcaccgtcag 15840
catcgaaatt gagccacacc aggggttctg gataccagct tctctctagg tgaggctgct 15900
atagtcagca gctgattagt tgcagttatc agcaactggt aatataatat attgtgcata 15960
taagtgtacc agaagtcatg tttatatatt gctgcaaata ctcggaatgg ggatctcttg 16020
ttccctgctt aagaccacat cacattactt ggttttgtac gctagtggct gaaccaaaaa 16080
aagtaggaga tgattttttt tcttttttct taaagcagta gcttttgaac cttgaccatg 16140
ctttctaacc agctgagggg cttttgaaaa agagggtgcc ttactgtgcc ccagaccagg 16200
acaatcagta tttctgggga atggagcctg gcacacacac atttcttaaa gctcccttgg 16260
caattctgag gagtggatta catgttgtat gtagctcgta acgaaagaaa tcttgtcttt 16320
gctctcagac ccccatttct tactcatctc atgagctcct tcgagatcca gaaacagttg 16380
catatttcat tagtaaatca gttccagagt cacattttat ttcacaagtt agtccattaa 16440
aagtttcctg cagtgaggaa atagccagaa agaacactcc acccctcctc cttttttataa 16500
ctatagggtc tggctcgaca gagcaggagc atcgccatct tggacaagcc cctcattcta 16560
aagttcacct taataaaaaa ctgcctaaat tcaaactgca tcagcctaat ggctaaggtc 16620
agcatgacca taaaccacaa ataacatctc caaccggaaa cattcgaaac tcctcctcga 16680
ccagagacat gctagtcccg agataacccc cctccagcag ggaagatgcc agtctcggga 16740
taacctctct ctggccggaa agatgcctgc cccaagataa acttgcctcc tcccagagat 16800
attccaaccc tgccataaaa cttctccctc aaacaggaac attccaaaat tctgataatc 16860
tccctcaccc taaaaccaat atatactcct agtctgtaag agaaagcgct cttgaccaaa 16920
attcaccagg agtgcctccc aggttttaac taaagaaaac ctctctttaa ctgccaaaaa 16980
aaaaaaggga aaaaaaaaag ctttctgcag tggctttcag cgggcccagc atggcagcag 17040
cacctgagaa cctgttggag atgcacactc ttggacccca ccctggcctc tgagtaagac 17100
actggaaggg caggccccgg tctgtgcaca caagtcctca gggagattct gactgatgca 17160
tgccagattt tgagaactgc tgatatactc caggcacatc gcatgctggg atctagatac 17220
accaagggaa caaaataact gcacttgtcc tctgaggacc gacttacctt ttggaagggc 17280
tgagaaagag agacacatac aagatcactc cctgtaatgc aatgttttat aacagatgtg 17340
atttgggatt tcagtgggag cccaaaagag ggactgacta attcagcctc tgtgacaagg 17400
ggagtttctc agaaacagaa tgcttagctg ggcctccagg cacagggaca ggaatgagga 17460
aatacttgta ggccctgtgc tccttcagca aaaccctcag tttcttgtta tttttataaa 17520
tgcaaacatc ttattaaagt agatgctaag gcattagaat ttcctgcttt atttttctaa 17580
atgaccatga ggaaacctgg aatgtcaaag ataaagtgca acacattctg catttaaaaa 17640
ttaaaatgat cctttttaaa agtagcaacc agatgtgaaa aattggactg gagtccaggt 17700
tatagttgat agctttaact ttctccccaa cagcaacagc acaatttcc ctaaaatgtg 17760
ttatgaataa gtaaaatgac tacttcacat cctttaactc ttcctacaga aatctaagag 17820
agaaatgaaa caaaagtttg cacagttcta gacacgataa atacatgtga aatcacacaa 17880
ctcagaaaat gtcccttaaa ttaattgagc cattggtact tgtgaattag aagagacatc 17940
tatgttctga tccactgttg aaagctgtac aatgttacct atttatttgc agacatcctt 18000
tggaaacaaa taggtagatt tgcaacaaat aaagagtgga gtacagctgc tgacattacc 18060
ttgtatattc atgcctttat gtaaaaaaaa aaaaaaaaat atatatatat atatatatat 18120
atatatatat atacacacac acacacatat ggaggtaaag accactgctt gctttgcagt 18180
tgttttaaga gcattcatga aggattttat tttataagca gaaatgtgat atctgacgat 18240
tttaccacta catgcttgca ggccagtgca cagcagatga cgtcatgatt gttttagcag 18300
tcctatcgtt ttacttatga tgtcattaca accctttgct aaaatttctt tcctttactc 18360
caggttttgg ataaaattga tgcattgcac atagtctctc tgataagaca aactggcatt 18420
tgtatgtgaa aaactgtgca tgttttagtg tctctgctga tactcaaatt atccattatt 18480
ttagtgctgg aataaaaaca aaccacttag tgaatttgtg caggtcctta aggacaggca 18540
aaggtgtcct gagattttct gatcattgta taccaaattt tagaaacttt ttcaaaaaca 18600
tttttttaat ttcaaaaacc tggttttgtt tatttaccag caatcattga atacctgaaa 18660
gctttcagga gattttatta caatggtttc tattcactta caaaattatc tcctagttca 18720
ttctcataca ctgtaagcca ttgtaaatgc ttcaaattgt gccgaacaag ataaactaga 18780
caaactattt taagtttgtt ctagtgctaa cttgcaagat ctaatggctc caactagatt 18840
tttaaaataa agtatatttt aatatattat tagaaagtta agcaattatc tgtttatagg 18900
taacaaaaac cctggaaccc caatgtcaga tgtcatccac ttttgattaa gtccaaacat 18960
atgacagata aacaaaagat ggttggctgg gctcagtggc tcatgcctgt aatctcagca 19020
ctttcagagg ccgaggcggg cggatcacaa ggtcaggagt ttgagacttg cctgaccaac 19080
atggtgaaac ccgcctctac taaaaataca aaaaaaacag ctgggtgcgg tggcacgtgc 19140

ctgtagtccc agctactcag gaggctgagg caggagaatc acttaaacct ggaaggcagg 19200
ggttgcagtg agctgagatc acaccactac actccagcct aggcgacaga gcaagactca 19260
gtcaaaaaac aaaaaaaaag tggtcattgg agaattattg tgtcacctgt tgttttttaa 19320
tgtactaatt ttgagaggct tttaaataga gtgcactata gaactttttc ttggcttcaa 19380
tttgctacaa tgttaataga gaatcagaaa ccttatcctt atagatgttt cttgattttt 19440
ttaatttctg gtgacattta tgagtgagaa tagtgtattg ccctgttttc tttcttactc 19500
ccctttcttc ttccttcctt gctttctttc ttcttccctt ccttctttct cttcctcgct 19560
ccttcttttt tacaagctgt tatgaattag ccttcacaga gaaagaaaaa tttttataaa 19620
taactggaaa tgaaactttg caaaggactg cagatgaaaa actttgtcaa atgactgtaa 19680
aaatatacta tataattttc aaaagttaga aagtaccaaa cacactcagt attcatggtt 19740
atacaagtat gcatacacat gtattgctcc ctgaaaagtg gtgttgttaa gggagttttt 19800
cttagtacgc ggcttaacat atttttttct gtaatttgtt gttagttata atggggagag 19860
aaaacaggtt agagtctccc ctctcagttt caccttccat aaaacagcta aactagacga 19920
tcgtcagact ccttccagct gaaaacatct gtaaaattaa aaacaaatct aaatgtatgc 19980
aagatatgta tttaaacatg ctggtaataa gtgtgctgtc cctataattt agatgctaaa 20040
acattgatgt cataataata acaacacctc gcatttgtac agcacctcat agtttacaca 20100
atgccttaac attcttctct ctcagcctcc tacaacccca caggattggg atagctttcc 20160
agattgggag gtgagggacc caggctcaga gcgattctgc tgttgtccgt aatcaccagg 20220
ctggtgatca gtgggcactg ggtgctctcc tgctacacag cactgtctct caacatgcag 20280
gtcaaggtta cttattcctc cttcaagacg tcattgggtt ttttagctat ggatgcccca 20340
tcacttttag ttctatttgt gaatcaaagg ctaaataaag tattcctcaa aatttgttat 20400
acttctgtta ctaatgctta atgtccctca caatttctgt atatttctgt gtatttctgc 20460
tctgttttgg ttcctttccc aggtttcttt tttgttatga agtagttttt agactcaagt 20520
ctcttctgta tgtgttataa ctgcccattc cataagatac agggcagtga atttgtgagc 20580
cttgaaaata tttactttag aaatgagaag tatgactttt caacgttgtg tcatcaactt 20640
ctgtaaattt tccagaccta taaatacttg cagaaaaaaa atgaaaggag aaggcaactt 20700
gatttagcag ttgggtcagt tagcaatgcc tatggcaagc tgtagtaatt ccccttacata 20760
gatttgtaag actcatttct atgatttaaa tgaaggcata cacttaacct ctttagggtg 20820
tgaaacagct tttacaaaaa gagacaaact taagaaacag tgtggccctc caagagtgtt 20880
cattttccat atcataccat ttgtaataag ctattctggc tgggatttac ttgcaagcat 20940
tggcttttaa gaagagatgg tttcacacat caaattattc acttggaggc actttctggg 21000
ttgaaggaat ggaatggaga gtgcggcagt gagtagatct ctcagtgacg gtgatgtgcc 21060
tctcccagaa gaaatttcaa aatgcagtgt tcattttcct ccacaagaaa ggaagaaact 21120
gttttgttat tgtttattcc taacatagtg gaaacttttc agtactctgg cagaaatttc 21180
ccaaaagcaa ttttctattt catgattata aagtagcaaa ggaaaaagtc ctgcactcca 21240
gctgagcaat ggatctccag ttgttatcta ggtgctgcag gtttagagag gattgccagg 21300
agaacacatc gatttttcag gcctgtgatg acgtatctct tgttgaataa gtaaaccctt 21360
ccagtaaaca gacagttagt atattgattt cagggtggct ttagccactg aacctgtaag 21420
tcttgcaaag gttacttggg caaaagcatc attattttac cttcagtcaa caaaaatcta 21480
cctggccaag gcagaacaga aagttcagca atttgatgaa gtgggacaac atgaagaatc 21540
aggtgagttg cctacttttt cacttcactt tccaccttta gagattcttg tttagatgca 21600
gagtagtgac gtgcctggtg tcagggagag agttgaatga gaaaagtccc agaagggcag 21660
aagacttggg tgattatctg agtccatctt tccttatcac atgacagagt tcttgaagtc 21720
ttggctagga attctaggct tttagattct ttgggcaatg gctactaaat gttcataatg 21780
ttgctcagtt gcaaaaacaa gacattcaaa ctatagccag ggagataagt agtcacgaac 21840
tcaaggccta aattctgctg atggagccga tgagaattgg gtgctaaggc aaagagagtt 21900
gccaatatta tattcttcgg ggtttttgt ttttattcgc attttggaaa aggaaaatat 21960
tagcattcct ctgacttaat attgagaaga cattgggcac tctttttcct cccacacttg 22020
tcttctttca ctaggtgaca agggaagagg tagcatgagg tggtggtcac aggtgagagg 22080
ggctgttgtg agcacaggca tgttgactgc acattggtca cctagtagaa gttttgcagg 22140
cttggtgact tctgaacact gttttcaagg ttgattttta gttgagagaa cctctaggta 22200
ccacgtaatg ttattaacag tagtactgat ctcacaatcg ccctatgtcc cattcacaag 22260
atgttctgcc aagccataaa aggcccagtt aagtttaaga gaagtctcaa aagtaacaga 22320
tgataactaa ttaataccca gtgattttga aatgtagaca tcaaacatac caattcagtg 22380
gtatcatcct tagaggcaga cagaggatga ttaaatcatt cagcccatct ctgtctgagg 22440
acgcagctta gcacagcatg gtggaggcta aatgggcctt aagggaaaaa atgatatctg 22500
aagatgcaat ttatttcaaa aagagtttgc tcccgtgaat tttcactctc tatgtagaac 22560
ggcaccagca cacacttttc ctgagccttt gcatgtgtgg caggcagcgg cctggcatcc 22620
tggggaactg aatgaggacg cagatgaccc ggacgtgttc acagtttgac acatctgact 22680
cccagatcag ggacagctag ctttgctggc tggttaagtt gatgattcca tctttgcctg 22740
gttctctgac tgtctcatgc tttctgttat tactattttg cagcagatat ttctgctcat 22800
ttttcaatca tatatgcatc ctggatggca tagagttgat tctcctaaca aatcagtgtc 22860
cctttgtatt tttttctggc cataagatag aatatatatg tcatttatta aaaatggaga 22920

aaatgttcag gagtttcttg actcagagag ggaaaaggga tactcagggc actttttcag 22980
ccaggaattt actacctttg cagggtaaag gggactcacc acgctggaag tcaaaataag 23040
ccaccagtgc caagtgttca aagcccttag aatcacaatg ctcttaaagc aaagtcttca 23100
acaatgcttg aaaacttcca ctggttctca gtatgtccaa aattgtcatg tctatgaatg 23160
attttctcaa tctgaaaatt tttatagcag gctaaagaat gagataggtc agtgtgattc 23220
tagaactaat cattaacatt caatagatga ctattttatt ctagaaaaag cagcaacttt 23280
ctatttactc tctattttga gggtaaattc tctgtaagta gaaaaagcaa aatgtggaca 23340
tgggactaac atatgaatat acaaagcaaa tgtaccgaaa aaatcttaag acctgccttg 23400
tggtgttttt tgttttgttt tgttttcatt aaagtgactt gttagcctct tgctccctgt 23460
gaagcacagg gaggtgacgt gatgtgcaca gggcagactc tgccatatgc cctggccttg 23520
aactcagggc cccctgggga ctgcagggga tgctggccat gctgagcaat gcctgtgggt 23580
gtcagtttcc tcatctgcag aatgagggta ggcctggtgc ttatttcata gggtcgcaga 23640
ggggattcag tgacagggtg gtgtagaggc tggagcgtgc cccatgtgtg cacgacagcc 23700
ttccaactag gggaggcggg cctgggctct caccagagag cctgtgttct ccatggctac 23760
atgactttgc cccagacgtc cttcccgtgg tctggaccct gggaagtcgc caagagccag 23820
acaggagaaa ggctccactt ggctctcctc tttggtgacc atcccttgcc tccatggcgg 23880
gactctcagg tgacatccca ccaaccctca ctttgcttcc ctggtgggtc tcactttccc 23940
tcaagagtgt tgcttttttg tttcctgcat agtcctgggc cagttttgat aaccctcttc 24000
atttcacttc agaaaccctg atgatttctt cctgtgctct ttttacctta ggacttttac 24060
tatgacgact gtgactggcc catttcttgt tttttttctc ttgctctgct ttctccccca 24120
tcatcactaa agcagacatg gcaatgatgg ccatgcacac tttccaaggg tccagctgta 24180
gatcttcatg gttccccagg tgcctggacc atcttgtgag gagggaggca aacacaccct 24240
gcctggagca cttggccctt tcggcaatgt tttggcttcc tcaagtgaga aaagaatgga 24300
tttgtattcc ccctctgcat tattgttttt gttttgtttg tttgttttgt tttgtattga 24360
gacagagtct cactttttttc cccaggctgg agtgcagtgg cccgacctcg gctcactgca 24420
acctccacct tccgggttca agtgattctc ctgtctcagc cccctgagta gctgggacta 24480
caggtgcccg ccaccacacc tgactaattt ttgtatgttt tgtagagaca gggtttcacc 24540
atgttggcca ggtgcccatt attatttgat ctggaattaa ctgagctact gcaggaattg 24600
cttgattcac tgatgactgg tgttgagcca gtacacaccc acacccaagg actgtgactg 24660
tcttctgagg tccatcctca gaaattcctg tctcttcacc tagtgtgtaa taaggcctgc 24720
gcgtgttata tggaactgta aaaaatgcgc caaccatctg tccttcctct ttatctgatt 24780
acttatcatt gttctctaag ttgcaagtta atagactgat cataaattaa tgcatgctgg 24840
agacttgctg tttcctacta gcagcatata aaagttattt ttaaagttgt tttaaatctg 24900
tgagtaaaaa taaattgctt tgctgcaaga aacaccaaac atggaaaagc taacggttca 24960
aagttaataa tttatcttat ggacatcact agtggcatag ttgctttaaa cagtgagagg 25020
atttaataga tatttgattt gcaagtggga tgaagggtgg tctaaccttt gtcctgtgtt 25080
taccttccat gagatcctag aggttgtaca gcacagtagt ggcatgtgac acacttgaga 25140
gtgcctgttc tgtttggaaa cctggaaact atgaagggaa gtggccttcg agcttaacac 25200
ataagacttg ggaggcaaaa ccttttattc tctttaaata ttcactttag gataagcatt 25260
tttttaggtg ttaggaacag ggaaaactgt gtggttagga aggaagaaag aagaaagtta 25320
actgttgtac attccctagg taatgttttt aagcattgtt attcactttc aaaacacatt 25380
ttatttattt ggacttaata ttttgatctt attttttcaa tttcttttaa tttaacagac 25440
aggatgagtt tttttatagt tgtattactt agaaattata ctaaaaatgg ccgagtgtgg 25500
tggctcacac ctgtaatccc agcactttgg gaggccaagg caggtggatc acttggatca 25560
cttgaggttg ggagttcaag accagcctgg ccaacatagc aaaaccccgt cttcactaaa 25620
aaaaaaaaac aaaaaaaaaa ctagccacgc atggtggcag gtgtgcctgt aaccctatct 25680
actagggaga ctgagacata agaatcactt gaatccagga agcagaggtt gcagtgagca 25740
gagattgcac cactgcactt cagcctgggt gacagagcaa gactctgtct cggaaaaaaa 25800
aaaaaaaaag gataaagaaa tcatactaaa aacaaaacag aatgctgacc accttataga 25860
aatagaaata gtggtttgct gtgatagcaa attttcttgt taacttttta tttttaaaga 25920
attgcacatt cacaggaagt tgcaaaaaat ctactgggag gtcctatccc ccttccccca 25980
acctcctcca gtagtaacat cttagtagca aagttttgta tatttatttt gatatcatta 26040
tctaagtttg acatcattat ctaatattaa cctaagccaa aagcccacta ttttaattat 26100
ctagtgatgc agtgttatag aactcatagc ctttcacagc attatttgga agttaatttt 26160
cttaagtgaa atgtttttgg tctttaaggt ttggaggcca tggaggcatg aggagaaatg 26220
ggatgaggga gagagagcta agatagataa agacagagat ggggagatcc actgattcgt 26280
tgaacaaacc agatacttcc ttatagtttt tggattaact tacatgagct aagtttatat 26340
tctgttcaga tcacaagtgg tcaagtttgt gtgtgtgtgg gggggggggg gtgggtgtgt 26400
gtgtgtacca ctctacccat cctatattta ttgtcctgta tttggtctgt tctgccttct 26460
ttattttcag gataggtgtc ctaaatgagg gtctttggaa agctggtgag gccatgttgc 26520
ccgtttcagg tgttccgtgc tcaaatgtat tcatttcttg aaaaattcag ggagtgcaca 26580
cttttgtaca ttttcctatg tgtatatgat accattatat aaatcttaaa aatatatatg 26640
gttcacctga atccccagcc atttggtaga gaagatagaa aacctacaga ggaggctaag 26700

attttattag aaaattcagc ttctcgacgg aggtattggc tttaaagtca aggcaatgca 26760
tctattcttt cttttgatat aactagctaa aagatctctt aaattcaaag tggccctcat 26820
cttactgtta ctgcaattta ctcttaatta caaattatat aaaaataggt tttgaaatac 26880
tgtagcgaca aagtaacata cctctgctcc attacacaga taaaacctct aaggaacacc 26940
tcctctctta acaggcatta accaactgca gaaactgcag aaggacaggg ctatttggga 27000
ataacacagc tcccttcctt gtctgttccc tcccattgtc aggcttctgt ggagccatat 27060
tcagagcaac atagggaggg ggaagagaaa atcaacccct tggtgaagga aagctcccaa 27120
ttcacagagc aaacatgggt actcttgttt gtgggagctc ccagggcctc ccagctcacc 27180
gagcattctg agccctgatc cttacactaa ttgtattatg caaccataaa tgatgtctgc 27240
tgtaccagcg gggacagttt attttaatag attggtataa cttggcagaa tcttatctgc 27300
atgtttcatc ttggattttt agctcaattc aactcaatag gcatgtgtca aatgtctact 27360
gcagactgag cactgaaaag ctgctgggta cagggttaca tggatagaaa acgtagcctc 27420
tgacccctaa ggagcctgta atccagatcc ccattctttc catcccattc tcccaagcaa 27480
gaatttacct aatgtggttt gcgagaattt aagagctgga aaggtggtca cgagaagccg 27540
gaatgggttc gctaaaatgt gtctatatga ttaagcataa cgtagctttg cagcactctt 27600
cacagcttcc tcagagcctt ccgcacgcgg tgtctcattt gaatacttgt gtgaggatag 27660
cctcataccc ctcagtgagc tcttcatgga gtgatgcagt agacagcaag cctcacactt 27720
ctatgctcac ggaagaccaa atttgccttg aaaaatcttt atagtctctt cacatttcta 27780
agttgacatc aaaaatcggt taccataaaa tcctaatagt tgaagagatg taatttcaat 27840
tatttggtaa acctgacctt cattgtcaaa gcaattagtc aactcagatt tactttctcc 27900
cagataatag attctgactt cttttttttct gattaaaaaa cttaacacct tcctcaggag 27960
atctatctca gttctgaatg ctgattctaa ctaagaagga tatttggcta catgctggga 28020
agaggggtac tgaggcacgc cgcgattcca ctccagcatt tccagttagt cgggtgcctc 28080
tgcactcccg gtgttccggc gcccagttag ttgtgtactc tgggctgtcc ctatactgga 28140
gtcctaaaac acttacgact gcagataggg ggaggttttt caaaaccttg gtctgaaaag 28200
ccatagaagg gagataggaa agcggggggg tggagccaca gtacattcag gtggatccgt 28260
ttttggaaat agtacaaact ggaggtgaaa ccctggaaat tgatctgtcg ttcacatgct 28320
tcatgccgag tccttgtgga cccacagaga cacactcgcc ccagtttgaa ggctgctaac 28380
ttgattctga ggacaccagt gaggtggtag tgtgcaaatg atgtgtgagg aaactttgga 28440
ggagtctcac cctgcctgga gcacgtggcc cctaaaacag cgcagcctcc caaagacaga 28500
agatgtggac tagtgagaag ccaggtatgg tgactgctgc tggatgaagc ttgtcccacc 28560
agaggctcgc ttgtttcatt gagcacctac tgtgtgcttg tgggatgcaa acacacgtgt 28620
ggtccctgcc ctcaggttaa taggcagggg tggaacagtt atgaaactgc tctaaagtca 28680
tttctcaaa ctgggagtga caaatgtatc cacttggaaa agattgagaa ttttataaga 28740
tttttaaatt tttgtttatt cacattgagg agaatctaaa ttcttttgaa cttatgtata 28800
gatttcacca ttttatagta ataaatcagt cctcctgtgt gtgtgtgtgt atgtgtgtgt 28860
gtgtgtatgt aaacctcacc ttgcaatatt attattttaa atagccactt gcatcttaag 28920
gaaattaaga ggacaaaaga aaagctgctg ttttgtatgt atccacatat ttaccagctg 28980
cttccctgcc ggcaggtgct ctggttctgc actgcctgtt gtcccttgcc tgaaaatggt 29040
tgcctccaat attttgctca gttttctgat tgtttacagt ggcagaggag ggtagatctg 29100
gtaccagtta gtaattgcca gaggtggaag tctgtggatg aaatttgtat aacatggaac 29160
gttagttcca cagttaatgc tactcaattg gaacccatgg aaattatttt ttggtgaaaa 29220
gggcccatgc gttatgaaat ttgagatcca tcactttaag tgaatgtagg ccctggatac 29280
agtgggagct cagaagagca aatcagttgg tcaccttgct caacgtattt tactaagggc 29340
atcagtaagg ctttctatga cctgctcctt caatgcttgg ttgacatttg gggagcaaag 29400
ataaactaag gattctaagt tctgtcctgt gatgctgtaa ggggaatctc aaacctctag 29460
gtggaggagt gcagagatga ccaggatggt ggaagcctgc aggagagctg aacacctgaa 29520
gacacccagt gggaagacca ggacctttaa cgcccatatc tgctgctcaa gactggcaga 29580
gagaagaggg tttgtgatga gaaaaggtgg tgaaaggcac aaggaggcac agagcatgtc 29640
aggtcccata tcccaaaagg aatgtgcttg ggtgagggag agctcctcca tggctggagg 29700
cattcagaga ccaggcagtc gcttgtgggt ttgtgattag agtgaggttc ttttataaag 29760
ggagtgagaa gagaaggtct gtggatactt gagtgtatcg gtaattaaga aataaattgt 29820
gtacatccca tttctttcca cattttcctg ggctgtcaca gtggctgcaa agaaagcagt 29880
ccgtgaactg aactgtgatc ccagacaggc aagcacacca ggaatctctt ctcagctgtt 29940
gataatgagg gagcgctggg gagagaaatg gggtcctctt tgagtttcct ctgtgccgat 30000
acctttctct ttgttaaaac agctaattaa acactgaagc agtatagctc tcttactata 30060
cactggtagt catagttctc ttactgttct cttcactgac agttctctta ctatacactg 30120
atggtgacgc agaaattcag aattccccgc atgtgtcccg gtttgaaagc cactgtgctt 30180
tgctgtggat taggatcaga cagttgagtc ttgttccaac aaggaaagtt gcttattgga 30240
aagttttgct gcagggagcc ttgagttctg catcaggctt ggaagtgggc tctgtggagg 30300
tcagaaggag gatcccccac ccgcagcctc aagaaaaata tgaaaagtgg attatgcctc 30360
tgtagctata ttgcctataa actttctgca gaatgacagt attcatatcc tacatttttt 30420
caaagcgata ttaatcctga gacctgcagc taaagtcaag tagaatttag ggataattaa 30480

taggaggaag gtggggttgg aagatctgca tgattatagt cctctgatat aactggaaaa 30540
ttctttccat tagcaaggag ctttggttaa tataaaatgg acagattaaa cctaggcaat 30600
ttattttact cattgctgta tttttatttc agagctggtt gaaaatatta caaagtaata 30660
ttttaaagtg cttatctaaa ctcttactct gcattttatc attgggttat gaaatgactg 30720
gggaaagact tttcttgctt ttatttctca gtgtctactt ataaacatgt tttttgaact 30780
actgtttttg tgacaacatg ccttttttccc agaaaatctc aggttaacat taaataggca 30840
ctggatgttt atctgatctt gtttatagaa acacaagaaa attttaacct tgtatatact 30900
ttactcaatt aactaggtaa gaggtcattg aaacatttag aattccactc tacatttcaa 30960
taattatcag gtgaaagcta ctgcatctac atcagaagat gtttgtaatt tatttaagaa 31020
taaaattagc tatgcaagaa atagtatgtg gagtcctatg tggaaatcac agaaaccctg 31080
acaacttgat gatctttccg caagctaaaa atatcactct ggatcacagc agtagaggac 31140
tctgtaaatt taatctgtgt gtctcctgta aataagtgca ttagcagtac acaggtggtg 31200
tcagagtcag tgatgatgga tagaaattct acataaaatc caggctcagt ggctcatgcc 31260
tttaatccca gcactttggg agtctgaggc gggtggatca cctgaggtca ggagttcgag 31320
accagcctgg ccaacatggc aaaacctcgt ctctactaaa aatacaaaaa ttagctggat 31380
gatggcacat gcctgtaatc ccagctattc gggaggcgga ggcaggagaa tctcttgaac 31440
ctgggaggta gaggttgcag tgagccgaga tcacgccatt gcactccagc ctgggcaaaa 31500
gagcgacact ccatcgcaaa aaaaaaagaa gtaagaagtt ttacataaaa acgtggagtg 31560
agcccaaggt gccatttatc cagcccatac acatcgtacc atgtacagag tggacaccag 31620
ataaatacat tgactgcatg ccacaaacat atatatgtag gcaccgttgc attcaaatac 31680
acatctgcag ccctaacaca tctttatttg ctaacgagca tcaatgtatt taaaaacaaa 31740
catgtttaaa ctagtgaatg attagattat aatgatctta attcataagt tttctcattg 31800
gccttttgta tacttcaatt gtaataccta gaaaaacagt tatgtccaaa ggagtgaata 31860
ggccttatct gaaacaggtg agcgtgacaa gtgttttctt acttatttta cttttcagat 31920
aattcatcct taaagtacat tagtttaaaa gtactgttta aggaaacagt acttggatta 31980
aaacttgaat cattgttaag gaaaactata ccttaacttc atgtaatcac aattaaacct 32040
cttcatatag aaggatctaa gaattttctg cagcattcac cagcaccaaa aagctcagag 32100
acatatattt ctttctctgt atatgtattt taaattcaag ttagtataaa ttgacaggca 32160
ggtcagagta atatatgatc ttctgagtcc ccttagtaat taaaagaaat gattattttt 32220
gcatgaaata tgataaagtg attttaagtg cctgataaaa agtcttaacc atgacaacca 32280
ttaaagatta catcaaagaa aaataagttt gactttcatt taccttggaa acagctatta 32340
actggtaacc tcaagaaaca ccatgaagag tcagtttgct ccacacatgt cttgtaaaag 32400
tcaaataact ggtggttatc cagtaatgac aagaggtaga agttacatcc ttgctgtctg 32460
attgaacctt cccagagctg gcacaaggct gggaagacca taggtgctaa atgaggaact 32520
acttaaagaa agaaaatgga atttcacgga caagaaaatc catgtccatt tggttctgtg 32580
acccacatcc tttgtatcct atgctttttt acacttggta catggttgca agattgcccc 32640
tgttttctac ttatagttcc atgcagcatg gatgtgggaa aaagtctcct ctgcaaaggg 32700
ggttaatgca ggtcactcta cgtatgtgca cgaggtcgtt ataaagctcg aaaatatggg 32760
ctcaccaacc aggtgatttt tttaattatc caaccagaag acataacata taggggaatc 32820
aaaagaaatc tctgagtaaa ataatgataa caggtcaaac tttgcggtcc cacgtgaggc 32880
tggagatgcg tattgtcttg actttgcatc tacaagttta acaaatgatg ctttctcagt 32940
ttacctctgg aaatggaaat tagcattgca aatgacttca tgaggaggta gaagctatct 33000
gtgaatttcc tttcgctgtg tttacgatag actctcacgt ctagatgtgt catgtattat 33060
gttaaattgg tatgtcttga agttataaag cacagccctc tataagtata tatattccac 33120
ctctttcaaa tcggatggta cctatccttc aaactgctat ttaatgactg tctgctatgt 33180
tcaaggcact gctctcaatg ttaatacttg atgagatcgg gcgcgttcaa ggtggcatgg 33240
ccgtagactc aatgttagta tctgaaatat ggcctacgag ctgagttgtg aatcaagtta 33300
atagattttc ggaatgttaa ggtctaaacc agtagctctt aactgagaca atcctgtcct 33360
catctcacct gggagacatc tggcaatgtt tggagaacct tttggttgtc acactggggc 33420
atctagtgag tagaggtcag ggatggtggt aaacaagttt ttttgtttgt ttgttttgtt 33480
tttgagacag agtctcactt tgtcacccag gctggagtgc agtggtgtga tctcagctca 33540
ctgcaacctc tgcctcctag gttcaagcaa ttcttatgcc tcagcctccc aagtagtagc 33600
tgggattaca ggtgtgcacc actacactca gctaattttt gcatttttag tagagacggg 33660
gttttgccat gttggctagg ttggtctcga actcctggcc tcaagagaac cgccccccttc 33720
ttggcctccc aatatgccgg gattacaggt gtgagccacc gtgcccaggc taacattctt 33780
taatgcatag gacagccccc accatacaga ggaatcccca gcccagaatg ttaatagttc 33840
taaggttgag aaacccaagg ttaagccaag tcaacttatc tatcttcttt aaaattgcat 33900
aagaatgcag tcctgttctt cattcctctt gctttgcagt taatgatcct ttgcctggac 33960
tttctaagtg cccagaagag caacagccag catgcaggat ggcattcctg accagttgca 34020
cttggcctag cattccaacc tcacctgcct cagcttgttc aacctgaaaa cctaccaagt 34080
gaaagcaaga gccacgtgaa gacgccttag ttatatgcac ccacccagac acttgctcag 34140
aaaggaatca gtggggccct ggccttagaa actggctcct tcactgctgt agaaacaaca 34200
taaatttaac ataaaacacg tgcttttctt ttttcttctt acttttttcct gtcttggcaa 34260

```
tgcaaggatg ccattaggta aagaaatcct tcaccacact aatcctgcag agccagaaga 34320
gaaaccagct tgttctaacc cagctttgtc atggagagaa ggcagctgct ccagtctgaa 34380
ctattctttc ttttggtagc agcctgccca agggtgaaag tgtgtttaat agtttgaatt 34440
acacaagtga acagtaaatg tatgcctgtt tctgctttat gggactttga aataatgttg 34500
tttgtgccaa ggttttagat tactatacct aacaacctag aaaaagaaat gaaaaggaag 34560
ccttctgcca ggcagaggtc actacgggcc tggagctggg cacctgactc agcagctgcc 34620
cagatcccca gagctgagaa gtcaccatgc atttgtggtg cttcgagcga gttaccagag 34680
tcctggaaca gagcagcaca cctgcggggt gtccccttgg catttgggca gggcaggtga 34740
ccaagggtct tgttggaact gaagtccagc ttgaaaagca aatctggttg tgagctagag 34800
tccagtaaca cttgtttccc gccgcccccc gcataactcg tgtgtcctaa aatacaataa 34860
tttcttgaac ttcagtcact tatgcctata agcgggcata caacaggggc acaataaatg 34920
tttgttaagt gaatgaattc tttcagaact agatgggatc ttagtccaac tctcttattt 34980
aacgaggtcc acagaggttc tgcgattgtc taagaaagaa ggctgtgttc atggcctttg 35040
ttgtttacgt ggccctgtga ttctcttggc tccgtgaaag tcctgatgca gacattccgg 35100
ccatctagaa aggcatgcag acaagccatc cagctggcat gatcctgagt ccagctttct 35160
ttaaaagagc ttccaaaact gcttaagctt tgactgcaca aaacctgcat cacctccagt 35220
tgagaaactc aagagaataa gtaagttatg gagttggaga ccccagctta actactagtt 35280
ttaaaatagt gaaatcaaca ttttcaaatc tttgacttca ctaagattta ataaagttta 35340
ttaatcatat attatgagtt attgctctct ctttatgtct gtaatgcagt tgctcctctc 35400
tgtataaatt aataagtttt agagatccaa aatgagaatt ttaaaataaa ttacgtatat 35460
tttaatcaag tttaatttga ctatatccag ctaaacaatt gattgaactt cacttgcttt 35520
tctatgacag gtttttttgtt cttagtaaaa gaccccagtt ttctcacttg tgaacagaag 35580
gggttagact tcatgacagc taaggttcct tccgtctcta acaaaagtgg cctgaagaga 35640
ggcttctaga ctatactcac ggtgggttct tgggacctca gagtcagctc catcacttaa 35700
gtggctgtgt gattgagtgg agacacctca atctctttgt gcctcagttt cctcacctgt 35760
cgagtgtcaa catgatggca cctaaagctg ttgagacttc agaaaggtaa tgtgtgaaaa 35820
gtgaaaagtg cctggcatcc aggaagtact caataaatac caactatttt attgctgcag 35880
ctgttcttat agatgtgatt tctagaacat tgccttctaa tagggtagcc atgggccaca 35940
attgttggct gttcggtgtt tcacatatgg ttagtccaaa ctaagatgtg ttgtgagtct 36000
caaatacaca ctggattgtg aagacttagg acaaggaaaa caatgttaat aaaatctcat 36060
tgataacttt taaattaatt acatgttgaa atgaaaatat ttgggacata ttgagttaaa 36120
taaaacagga gattaatttc ttctgtttct ttctactttt tttattagtg tggctactca 36180
aaaatgtgac attatgtatg catctcgtat tacatttcta ttggacagca gcgctctaga 36240
cagtactatg ggtagtatct gtggggaggt tctcagaaac atgtcgcatg ctcttttaga 36300
accttaaagt attcctagtc tcctctactt ccagcccttg gctcttgggc ctcagtcttt 36360
ttacttttgc ggctgtgttt ctctgaaggc ttggcattag tagattgaaa agaataacca 36420
tctagggaaa tgtgaattca gtttctttct gacattctgc tctctacaag gggatattat 36480
gtacacataa acctacttcc aaaataatga agtgaggcct aattccttac tcttcagaga 36540
gcccactgtg gaagtgtcac tgaccttgtg tatgggctgc ccttcatggc tctgggagtc 36600
attataaagg gcagcatttg gcgtggtgcg tcctaagcca gtgtttctcg gctctgttcc 36660
ttagacatgt gttagtgtta atagatgttc ttggaaaaaa aaaaaaaaaa cagcattctg 36720
aggtcaaaca tgctcagaaa gcttggaatc tgcactacgc ttctcgtaca catttcatat 36780
taaagatttt ggaaagtcct gcaatacaga gccctgtcta atattgccac aacccacaat 36840
tgctcaaatg taaatagatt tgagtttatt cacattcaga tcacctctta aggcccccacc 36900
tcccaatgct gtcacaatgg caattagatt tccacatgag ttttggaagg gacattcaga 36960
ccacagcagg ggaaagcagg gtacttgctg ctttgcaagt gtgtccacat ctaattaata 37020
gtacagttct tactcttggt gtgtccggtg atattaaaaa ttaatgtgcc ttatttagat 37080
aagtaacata aaaatcacaa aatgtatgcc ttagatttat atgtatttat aactagtcta 37140
tttcctgaaa acagttgaga caccttgtaa aagttaccgg tacgataggg ccattccaac 37200
aaagctgtaa agtggtgata acacagtcat aaagaagagg agatagctct gggagaaaag 37260
gtggcccaga aaccagctct gagcctcatg gctgcaggca aggtctgcag gttcctggtc 37320
ctgattgcag gccatttgct gccttgagtg gtggttacac aaggccagcc ctgggggtat 37380
cacccagaac acctagtaca cgaatttcag tttagaggac gaagcattac tggagtattg 37440
ttatgcagga aaactttttc ctaaaaatgc cctgaaaaga gagtagccta atgcattcaa 37500
tcaaaatgtt tttaagtgga aaacatattg tgtgtacttg atctggcctg ctgcttttaa 37560
aagattaaaa ctgggactgg gcatggtggc tcacacctgt aatcccagca ctttgggagg 37620
cagaggcagg tggatcacct gaggtcaaga gttggagacc agcctgacca agatggtgaa 37680
accccatgcc tactaaaaat gcaaaaagtt agccaggctt ggtggcgcat gccggtaatc 37740
ccagctagtt gaggggctga ggcaggggaa tcacttgaac ctgggagccg gaggttgcag 37800
tgagctgaga tcgcatcatt gtactccagc ctgggcaaca agagtgaaac tccatctcga 37860
aaacaaacaa acaaacaaaa aaacactggg gccaaagaac tctgtgtgct gtatcaccta 37920
accacatttc atgacacggc tagagaagaa tcatgcaaat aaaaatttcc aacatgttcg 37980
taaactggga aagtatttca ctggggagtg agcagaaaag taatactata acctctatat 38040
```

ctagacaaat gtgaattcag tttcacatat aaatatataa gtgaaaaaat atataaatat 38100
aaataatatg aaataatggt tatctcacca ctttctacat cttttgtgaa tattttatag 38160
tgctcaaata tattagtgca ctagtatatg tacattacat taaataacta atcatttatt 38220
aggaggatgt gcttgttttt tgctaataaa gatgataata aaaaaatcct tagaccccccc 38280
ctcggtttgt tttcagttag gaattaggga tatttataag aatatcttta aatgacacat 38340
gccttgctct gggacgaggc atctgcatgg gtgacacata tgtgttgtgt gtacaggctc 38400
ccagcatttc cagggccctg ctcagaatgt aggccttact gattcttaca gagttacaag 38460
cgctggtgag gttggcgaag tttaggtaaa cacagctggg aatgccccat ggcctctggg 38520
tgactttgga catcactgaa ctttaccctt agagatgcat acctgcatct tttttaccct 38580
gatagggcct tccatgatgc tttcaaagtg tttttgtctg cttttcggtt aatagacttt 38640
cacagtagcc aattgaatat attggttaaa tgcatctctt tatacacaga ctggattcaa 38700
actgaggttg tgtctctccc tggctgtgtg acgttgggta tgatccaagt gtcagattac 38760
tcaacttcaa aatgaggaca gagcctttcc cttctagggc tgccaggaac attgaatgag 38820
agagtgctgg cagcttagta caggtgttca ttgctcttgt atggtactgt ctgtggcacg 38880
gctagataaa atacagtagc cactgattca aatttcaact gaggagtaaa ataaactgaa 38940
taacttagaa aagttttctt cttttgaatg actctaagaa tttaaggagc atgtgagtgt 39000
tgatggctct aaaagggtaa cagagcccaa ctagctcagt tctcagcatg aaaatagtca 39060
tatggcacag actcagtgga gtgggtgcac ttcaataact ggaagcacag atgccctaca 39120
gcagcatcaa agatggcact ctaaactact ttcaatcctt taaaataaat ggaaacgcac 39180
atttagtatg catatgacaa cacgaaggac ttcgattttg ctgatgcaat acagttttac 39240
aggatttttt atactcaaat tagtaaaatt ctgtattgca tccaaattat aaattataat 39300
atcatctaga ttggacatag gaataacgac cactggtatc tgcccagaaa gctctaccgc 39360
ctgtttataa gctcctgcag gagacacaaa aagaagagaa tttgaatata acttgaaatg 39420
accgtaatct cctgccccaa ctcatttcat taccaaaccg cctctttctt cattatttct 39480
cctgaagcac aaatctatag agaactcagc tgccagtctc tcccactgca ctcagcagtg 39540
aaagggttag gcctaggctt ttcaaacaga ccagtgcttg tatcagccct taaacatctc 39600
tggagaagga aatgggatcc ttctttggta attcattttt gacagttggg gattaggtgt 39660
tctgtatctg gggggccttg ctgtcttctc tcctcctcct cccactgcag accctctcct 39720
cccctcccct ctccagctct ctgatgactg cttcatgctc cttccacctg aggactgcca 39780
gcacagccta ttgcaggaac agccaatgag gggctggctg tgctctttta tttataaaat 39840
tataaactca agcaaaatct agactatgtg tccccaagat cagaggagca caaatcccttt 39900
gcttacagat tgcatggggg gcacattctt taaaattggt ccctgatcta gactctagcc 39960
tgagaatcat ctttaagttc agaatttcca ctcatgacct cacatctgtg ggctcccaca 40020
ttgtcttcca aaacacacat ggcatctggc atcaccttca cccccaccct cagagcctca 40080
tctccctgca ggtagatagt caaggcaacc tcttcactct tctgccaagc ctcctctcct 40140
cagctcttcc cttcctctct ctttttgaaa atatttttaa ttgtggcaaa atatacacaa 40200
cataaaattt accatcttaa tcatgtataa aagtggagtt cagtggcatt aaatacattc 40260
acgttgttct atagccataa acaccattca tctccagagc tcctttcatc ttgcaaagct 40320
gaaactctgt ccccattaag caatggctct gttttcctcc gttcccccag cccctggcca 40380
ccatcctcag ttttctgtct ctgtgagttt gattactcta agcacctctt ataagtggat 40440
catacaatgt atctgtcttt ttgtgactgg cttgtttcac tttccataat gtcttcaagg 40500
ttcatccacg ttgcagcata tggcagaaca tctgtccatt tccaggctga atggtactct 40560
tttgtacgtg tggaccacat ttcatttatc cattcatcca cgggagggca cttgggttgc 40620
ttctgctttt tagctattgt gaataacgct gctatgaaca tagctgtatg cctttgtctt 40680
ttaaagccca aatctgatca agtcactccc cagcttaaaa ccttccactg ctccccagca 40740
gtgggataaa ggccagtctc ccctgtaggt ctctcccgcc agccctgctc agtcttcttg 40800
cttgtcatcc ttggctaggc cttgcattgc catagccctc tgcctctgtt cacgctctct 40860
catcttggag catgagcctt ccatcatctc taccagatga actctcattt cttctttcaa 40920
aaaataaaaa acccaaaaaa cccagagatc ccaactgtcc tggtgtctgc atagtctgca 40980
gcacacgccc cctccatggc ccttcctcca taagcagaat cactcctcac tgttcctgca 41040
gcacctcctg tgtgcccaca cagctgtcct gcggtgggct gtgtgtgtga gtgtgccccc 41100
tctaggacct gagctccttc tggagggtgg gcacagcatc cattcattct gggaatcctg 41160
gtcggcacca tgctagaact tctgcaagtg agtgcctttg gtgctggccc atgggagagc 41220
tgttggtaag gcatactttt gcagattcca gttgctgctg aggttgttgc tctttgcaca 41280
agtttcttct agtcaccagt gaagtgacat gtgtggcagg catggcccag ggaggctttt 41340
tcataaagaa gaggttgaat ctttggggct gtggtttgaa tatgtccctc aagcttatgt 41400
gttggaaact taatcccaaa tgcaatagtg ttaggaggtg gggcctaatc acaggtgatt 41460
aggtcataag gctctgccct catggatggc ttaacatgtt tagtgaggca gtgggttagc 41520
tattgtgaga gtgggcttgt tagaaaattg agtgcagccc cctcttgctt gctggctacc 41580
atgctctctt gcttttctgc cttctgccgt ggggtgacac agcaagaaga ccctccccag 41640
atgctggcac catgccctgg gactttccag ccttcagaac cacgagccag acaaatttct 41700
tttctttata aattacccag tctgtggtat tctgttatag aaacacaaaa tggactaaga 41760
caatcttctt tcatcaagtt agggtaccaa cctttaaaga ctgccagtcc aaggttaaag 41820

gaaacttttc aagagcagtc caaacatgat ctggccctca gctactctcc agggtcatgc 41880
caccctatca cccactggct cacacagacg ctgaccactg cttagtttct caaactgaag 41940
ttttcctcct cagagctttt gcaaaacctt ttctttgcct ggaaaactcc ccccacaaat 42000
ctttagttgt aggttccttc tcatcttgca gaattattag tttgctcttc aaatagtctc 42060
tccagctaga ctatcaactc caggagggca gagttcttct tcgcttcctt cacccatgtg 42120
cccactgagt ccagaactgt atagcagttt gattgaaaaa atccacaggg tggaggatga 42180
gaggaccctg gatcccagcc tcacagcctc ttacttcacc tgtgtgattt tggtcaagtc 42240
ctttattctt cctgggcttt agttttccct tatctaaaat atgagaaaag ttcccctctc 42300
ctgggtattc tgggagactc atgtaaaagg cactgagcca gtgcagcaca tctatgacca 42360
ggaagggtca gcttcctgcc ttgcatgaga cacacattcc cttcttcatg cacagttatt 42420
catgagttaa atatgtattg agaagtgggt tctcaggaga tgatgcatcc acagcattgt 42480
ttgtatgcct ctgtctttga tgtccctgcc tgagtcgccc actttagagc ccttctgttc 42540
ttcagaaacc agacttttct ttcaatagtt tcagtaatca atcgatcaat caatcaacca 42600
atcaacagtg ataataatca tgagtgagcc cctgcccgtg ctggctgtgt cctgctgaag 42660
gcacactaag tgctgccctt cccagaagcc tcaggaagct tgcgaagctc aggtgcatgg 42720
atgcctggtg gaatgaggaa gggatgcagc caggtagaga aatgccctgc catcacttgc 42780
atcagcatct gtgaagagct ggccaggctt ttgctcacag tggttgacac agtcaaggag 42840
caagggcccc gtaggagagg ggagtcaagg gctccgggtg ggaatggagc tgggggctga 42900
tgctggcttc tggagcactg taatgtgact gagaaaggtg aaggagccgt tctgaaaaag 42960
aagaaggcag gagctcgcac agctcttgac tcatcttgac ttcttttccc tgcttcatcc 43020
aagcaggtcg actctctcgt gatctcagag acagagtgaa gtcatgagtg ggaggggagc 43080
acagaaaata agaccttgat tcccagcatt gggagactcc ctgctcccct gagtctcgga 43140
aaatagcacc cttcaaatgt tttagggatc cagatttgat gaagagatgt tattttggct 43200
tttagattct taggagagat ttgtctttct caggtcagga agaaaatgct gcccgctgca 43260
cattcttcgg gacagactct tttaattatt actagtttaa tgtatgtttt gcttagttaa 43320
ggaaaacccc tgtggtttct tgacgtgctt cagtattcta actcacagct gattcagttc 43380
aggggggctgg ggagatgtcc tcgacctctg gaaaggaggg tgcatctcta gaaataaggc 43440
taagtatgcc actgacactg tctgcataaa cgtgtgtgat ctcaggtcca aaggatgggg 43500
cctggtctaa gccagggacg tgggaaatca ttttcctgtg gcaacttgtg aagaccattc 43560
tgtgaccttg gtgtctctgg gccttctctt agattttcta agttggctag tcagtggagc 43620
tgccatccct cctttgccca tgttctactc ccagagttcc tccaagaaat tgcggagcaa 43680
tgcctgtttc atgagagctg agtttgctgt gtcttccact tagaaacaac actgtggacc 43740
aggaggacac acagctccca gggccatcac cacacaaagt gaaggctggt gaatccgagg 43800
cttctagccc ttgccgggcc aggcccgcag cactccgctc cccaacccag ccgctgcttt 43860
gtcgcaggaa cctcagcagg gcagggtgtt tcctaggagg acatccgatt cccagccatt 43920
cctttcagtg aatcacctga gctcacattc tttttcttt tatttttgaa gctcttagcc 43980
aatctgcttc gcgatgaacc agttttgctt gaagcagaca aacccgattg tcaggagaca 44040
gtgatgattt cttcagtctc tgaggaagag ttttcatttt ccccaattcg caaaaaaagt 44100
caggtccctc cctccctccc tctccgtaga atattttcca tgtgtgttaa caatggctga 44160
gcgtggtaga tgccaggaat ttctgtcaac cctcaaagag gaaagccctg cctaatggtc 44220
tgcccgttct tgttcactcc ctgccccagg ctccccaccc gccttctttc tggaaggtat 44280
aaaggctcct gcttatacct ggcactgcac gcttcgctcc ctctgatctc ctgactgtca 44340
tgcccagtgt ctcagcctat cattctacct ctaactcgac cttgagtgac cttgagcaag 44400
tttctcagga ttccacctcc aagtcactct ccctttggga tatgcagcac taagttaagc 44460
ttgcctggaa aacatcactt gaagctggaa aaccactttt aacacagcgg gaaaagctat 44520
ttgttcagac aggagtgggg tgggtctggg cagagcactg ctctaacttg gccatgccgt 44580
ggcagcagct cctttaatgc cactttttcc tggcgcgccc gcggggcctg gagctcagaa 44640
agaggggaac gctccctcgt ctctcaacag ttgctccaga caggtcagca aacatggaat 44700
tcagaatgtt cattaaacac tggctgtgtc ttttgtgttc aaaagcaaga cactctctct 44760
gaaccatggc cccacagaga gtgcagaatg tgtgaaacct gccgggaagg tctggacccc 44820
ttgcggggca gtgggcagca ccgtgcctcc gttcacacca ctcacatggc tgtgcctctg 44880
cttccttctg gcatggctgc ttcttcctca ggtctcaacc atctccctca gatgctcttt 44940
cccatgtttg tggctacagg tccccgtgac ctgcagaggc agagcactca ccagcagccc 45000
agcctcgttg cgcacccatg tttgcatttg caggccctag aaccactcca agctccgtgt 45060
ggcgagatgc accctcctgc ccttcactgg ggagctgccc tcctgttcac agcggcacct 45120
gagtcacaca tctggagcca tcctggactg cctcatttcc ccgatggggg gtttccctga 45180
cttcatccat cctgtctttt gggtccccat aataactgac atgggtcggc ccgtaccagc 45240
ccctgtgaga agggctttaa ctgccttccc acccccctgct catcttagag tctctctata 45300
gtgctgctga aagaatctct aaatcagtgg ttctcaacct cagccgcaca ttgagaatca 45360
cctgggaccc ttaaaaaaat cttaactctt ggtccaagaa ttctattaca atcggtctgg 45420
gatggggccc tacaggtatt tttttaaagc tctccagttg gtaatgcata gctagagttg 45480
agtatcgctg ttctaacgtg cagatctggt catgttacca gccttttagg tggtcttctt 45540
tggctttctc tatctaaagt tcaaaaccga acatgtgcgc attcagtgca cccattttca 45600

actgtgcatt aacacattca gcccaccagc aagatttatg aaccattttc tgctgttgta 45660
tataacatat catatgcata atggcatagg ttattgtttt cttcaaaata tatgagatgt 45720
gagtccttct acgaactgac tcacactgat tgcccaactt cctctctcga ggtctcatcc 45780
tctttccctg cagccgtctc cctcttgcac gcacacacac acacacacac cacacacaca 45840
cacacaccac acacaccagg gtcgatgcca tctaccctgg acttcatctt gaactccttc 45900
gagtgtgagt cattactcct ttgtgcacct ctgctttctc ttctcaagat gttcacctgc 45960
ttgaggtcag ttccttgagc gtcttccact tgccatgttc accacagtgc tcaacatgcc 46020
tgaatgcatg gatggcgact tctcagatcc tcagtctcct catctgggta ataaggcatt 46080
gggttggcgg gtccatctgg tttcttccag ctctgagagt gcatttgctc tgtgattcat 46140
tcgttccaca acacttcacc aattaaagag agggtacaaa aggtgaacat ccttggctcc 46200
cagcagatgc tcctcaaaac ctgaaaaatc agataggtga gggaagattg aatgaaaggc 46260
ctcttatgat tctgcagcaa ttttggtggt ttaagaactc tatggaaaaa tcatcagtat 46320
ttctggaatt gaagtaaaat ggatagtgag cctctgtgta tgtgaaggcc cgcatctgga 46380
acatgaaaga acctgtctga tgtgttctag tcaggaaagc aggtagccaa tactatttat 46440
agaatttaca gaaactgaag attttgtttc tactgatttt caaaatagta ttatgtctga 46500
tttttttcct cagaaatata cttcctgctc ttctcaacaa actcatttga aaatatgatt 46560
agaacatgat agaattttac tcatttgcca actgcggttc ccatttcaca tattgttaga 46620
attctgcatg gtggctttgc cctttaacca ctaactgata aatgatgtag ttagctttta 46680
aatgtgtgga aaaatataat ttcaggttca accataggtc agaagtacac gtgttttgtt 46740
agtctatttg tctctcagtc atctcatgga aaattctcag cttttggtat ggaaataatt 46800
ttcttgaagg caatatttgt tgagtgactg acggaatgaa aaacgccagt tgcgtaagtg 46860
tgaaaaagat ctgggtgttt tcattggatc caaattccac atgagccaac aacagcgtgg 46920
tgtggaggct ggagcacatt aataagaaca gtgtcctaaa ttcaggaggt aatgctctgc 46980
ccatgccctg tgcagctcag acggtgtgtg cagtgcagta tgtaacccag ggcacatttc 47040
aggggcccac agggagctgc agcttgtaag gtggagtgca gccaacagag cagagagtca 47100
gaatccccgc agagtggttg aaggcacaag gatgcgcagc aaggaagaca gacttatagg 47160
tggtgcgact gccatcctct ggtactgaag gtgctatcat ggagggaggg aagtagattg 47220
accctcctgg ctccagagta cggaactcag acaaacggtc agaagcttac agggaggcca 47280
attttggatc aactttaaga agaatttttt aaaagctaga gcaatcctaa aatggaattt 47340
gctctttata aagttgcgaa tgcctcaccc tggaattgct taagcaaagt tgggacgggc 47400
agttgtgagt aatctccttt ccaatccata cccgcaatca ccagaaacgt ggacttccct 47460
gacactgagc acctcttaat taagcatctc ataagtgaac aaaacccagc ccttcaaaga 47520
agtcacttta tttatgtgtg ggtctgcagc ttggatttct tgataatgtt aaataaaact 47580
ccatctactc ttccacaaac acttcaagaa acctaagact tttggccaga gtaacaccga 47640
ggtttgagag aaaggatatg tgtgtgagag gtgtggtttc attagaacat attatttgac 47700
ttcatgttga atcaacactt ttgtgcaaaa tgcagtttta ccagcctctt tccttgtttt 47760
ggtcacataa tttaacttaa cattctcggt acttgatttt ctaacataaa atgggattga 47820
gaggggaatt ttgaagttcc catggtctgt cctctacatt ctgacagctc attatctctg 47880
cggtattgtt ctcacattta agtgaggtta gcggaggcag aggcctctca ggcctgaaga 47940
tagcctctgt tttcagggaa atactagact gtgagatctg tgacactgaa gcactaagtt 48000
catctcacaa aagcaacgtg ctctttttaa atggttgatc aaagttactt tcaaaaggaa 48060
gtgttagttt ttgttattag ccgaaacaag agctgcttta atgtagtata tttaaaatca 48120
tatctcaatt aagatgttat tcaaatacta tttgacccac caatctcatt actggatata 48180
tacccaaagg aatagaaatc attctattat aaaaacacat ggctgggcac agtggctcac 48240
gcctgtaatc ccagcatttt gggaggccga ggcgggtgga tcacgaggtc aggagttcaa 48300
gaccagcctg gccaagatgg tgaaacctca tctctactaa aaatacaaaa attagccagg 48360
cgcggtggca ggcacctgta atcccagcta ctcggaaggc tgaggcagga aaattgcttg 48420
aacgcgggag gcggagtttg cagtgaacag agatgaagcc actgcacttt agcctaggtg 48480
acagagcgag actctgtctc aaaaaaaaaa aaagaaccac ttgcatatac actattcaca 48540
atagcaaaga cgtggaatca acctaaatgc ccatcggtga tagactgcat aaagaaaatg 48600
tggtacatat ataccacgaa atactatgca gccataaaaa agaacaagat catgtccttt 48660
gcggggacat ggatggaact gcaggtcatt atccttagca aacgaataag aaaagaaaac 48720
aaaataccgc atgttatcac ttataagtgg gaggtaaatg atgagaacac aaggatacac 48780
tggggcctac ttgagggtag agggttgaag ggagagaagc agaaaaaata actattgggg 48840
tactaggctt agtaccaggg tgacaaaata atctgtacaa caaactacta tgacacaagt 48900
ttacctgttt aacatacctg cacatgtacc cctgaactta aaaaaatttt taaaaagatg 48960
ctatgcaata aaattctcaa ttaagaattt aacttggtaa atgttcattt aatgatctaa 49020
aaatatgtgt ctggatggct ctagcaaaaa aataaataat aagtttctca gagatggtaa 49080
ggctgaaata aatggggaaa aatctgaatt gtaatccttt ttctgttgga cctggtgttg 49140
gggtttcaca cttgtgggtg aatgtgggcc tcctgtgagc accagcacaa aagactaaac 49200
tgaacaaaag attaaatgtc acctctaaaa ttctgtgcaa caagacttcc agccacagaa 49260
tgtgcaactc agatttccaa gtaaaaacac accaggaagc agatcttaga tctctgttat 49320
ctccttggca ccagctggta ttcatcctca atgctagcta gagttgaaat aaagagtgaa 49380

agaactttct cttttattac ttaataaact tcctttttg agctgtttta ggcttacaga 49440
aaaattgagt ggcagtttca gggagttcca gcacggcccc tgtttctttc tcatggtccc 49500
tgcaggtttc ccctattatt aacgtctgtc attagcatgg cacatttgtt acaattaatg 49560
agccaatatt gatacattat tcactaaagc ccacaggttg cgttaggggt cattcttggt 49620
ggtgtacgtt cttcaggtct ggacaaatct ataatgacat gcattcacca ttactatatc 49680
acgcagagtc gtctcctggc cctacaagtc ccctccttcc ccacctgctc actcctcctt 49740
cccaccctcc ccaaactgtg gcaaccattt aacttttgac tgaatggatt tattcttatt 49800
ctgccttatt gtatgtacac catattttaa taagataaaa taatagtcta tagtagactt 49860
ctgtaaatac tcaatgaata aatacttgca tgaatgcagg aaaaatcaat cagtcttgca 49920
ggatttctta tgcgttacat cgtccttata agaaagcagt cattctcacc gagatgtgct 49980
gagcagatac tggacatgtt ctgacccaga taagggctgg gtggaagtag ggctggagac 50040
acagagaccc agtgccaact tccaggacct cggaagaact gaaggcagag aggtcctctc 50100
agtgtggact gggcctctgc tggcagccac cagcgggcac agagctgatg tgtgttatgc 50160
cacgtgggga aaacctacag acgattctga gaaaggctca cagggacacc ctctgcccct 50220
aaaagaacaa tttaactcta atttatttct gtcactctgc atttctgac ctttcccaag 50280
tgtacagttt tatatgcatt taactgccaa attgtcatgt gagattatat ggttatattt 50340
cattaatata ttctagtttg ttcagctgtt cttactgggt gaatttgtgt ggtttcctga 50400
catttttgtt tttagtagtg cctcagtagt tttatacata attacgtttc ccttctggat 50460
tatttcctta gtatctagtt caagaagtga aatcgctgga ttcttgtggt aaatttttga 50520
atttcacagt ataatgctga ttttctcaaa gtctcacatt ctaagaaagt ataatgaggc 50580
aaaacaaaca acaaacatct taagttgatt ttttcctagc atcttttcct tccatctttg 50640
cttgtagaat ctagactatt tcatgaaccc aagatataat cagtatcctt cttcagtatg 50700
gccaaagtga gtttctcatt attttacctc cccttcagga aatgactttt catcttgtgt 50760
tttgggagcc atagatggtt ctgggcagga aactggcttt ggatagaccc agcatgtaga 50820
tggctatttg gccttgctcc cagtataacg atgcagttcc ctgtgaaagg gtatgagtag 50880
gttttggggc tctggatacc gtgtggcctg aagagacaag ggctcaatgc caactctgcc 50940
tgtttccaac tgtgtaacca tgtgagcgtc aaaaatcatg gacgtgctct ggttaacact 51000
gagtgggagc tcaacaaatt attattttta attgttactt ggacatggcc aagttgacta 51060
cactttatgt tctgctacct gccagtctga aagtgacgcc acagaaggtg aaccgcatgt 51120
tgggagatgc tcctcatctg cttaaatgag gtgcaaacac agcccatgcg cctgctcttc 51180
atgactgtat ctgtaccagc aatatttgta ttggcaaatc acatgcccca gtgggaacta 51240
cttaagggga attcaatgga tttcattcct tttatgtaat tggccactta gtaatagacg 51300
tgtaggtctc ttgtgtggat aaggattctg ccttttatgt aagatatgtg ttgcaattca 51360
gctttcaggt cccagccccg ggaaggctcc aggccttcac aaactggccc acccacgaga 51420
aggaaagcaa ttgtccaaat gtgggtagct tttcttccca ctgttgtcag ctgcttccaa 51480
ttagccccca tatacataat cccagtttgt gtctgtatca gtacaattct cccatgtcaa 51540
tgtgaatttt aagccacaga gggaaagggg acagagaata tgctttcatt cagctctcct 51600
cgtctcacac ctcttgccct gcatgcattt ctttgctctg attaaacgag cattttataa 51660
gccacatttg ctgtgtgaaa ggcaaagtct tccctcccac ggatgacggt ctccagggat 51720
gtgtgtgtgt gtgtgtgtgt gtgtgtgtgt gtgtgtgaga gagagagaga gagagagaga 51780
ctgtaaacat atatctctgt gaaacttcat tttccatatg tgaattttg gaaccgagac 51840
aaatggaact tagctaaaag atgggaaagg tagactgact ctgacttaat ctacttaacc 51900
taccaggcaa tttataactt gatggcctaa tttttgcagc acccagaagc aagcctgttt 51960
cagcacggca aaggctcagc tgctaagtgg gcagcattgt tggaggtgag cagcttaggc 52020
tgactgttca tcaaaggacc aagcgcttga ggttcgctca tcgctggagg ccagagtggg 52080
gagggccatt taactgctca aggccatgga actctactgt cagtttcagg gaaatttggg 52140
accctggagc acaaaccaaa actccaatta accaggagag gaactcgatc cccaggagat 52200
aagtgaagag taagaagtct atctttagaa acaagagatg tccaaggcta gaaagatggg 52260
gaaggagggt ggaactgttc tggaagtggg tctcaatctc agcaccagca gctctcaaga 52320
ctttctagag aaggaaactt catttctgaa ttaaaattag tcttcaatga catggcaggg 52380
atttcggcac actctcttgc gtcataggcc actgtgttgg aggcaggagt gttggctttg 52440
gaggcataga gattaaaatt agagtaacac gtgagcactg aaaaggttaa acagtagaga 52500
catggaggac tcccgacccc catgtacccc tttcttaacc ctttaattaa gatcacagcc 52560
ctagaaatag cttgcaaaat aattaactac tgatcattta taccttagtg cttctgtgag 52620
catgttttct ctttcattgc tgctcatctg catggaaaaa tgtgcatggg tttctgaata 52680
taactccatg gtgcttgctt ccattatatt tgtgccattt ggatcataac tgataagcaa 52740
ccaaagagtc ccatattact gcacgttccc atcgctattt tatgtgaagg tggtcctggg 52800
ggctgttctg aattctcagt ttccttttt cccctcccca gttctttgaa aatatcagaa 52860
acggacttgt ggcatctttg aaaagctact taaaatgtgc tgctgtgctc tgaacttgaa 52920
aatgtgcttt taatacaaag tttgtgcagc ccttgctgct catacgagat gaatcttacc 52980
atgtggtgga tgcccgtctc atgccaggca ctgtgctcta agcccattgg tttatttcag 53040
tgcttgaaat tggctttcga gagaggcacc acggttccct ttttacagga gaggaaacac 53100
cagaggatca gagatggaga gtctttctcc acaaactcac agaccccaaa ggcaagctca 53160

gggttgtcag cttccaaagt ctgcctgctc caggacctca tgttgcatct ccattctctt 53220
cactgagggt caaatggaaa gaacacatgg gggtcaagtt tcagaaaata agagaaatga 53280
agaaatatgt gcccggaagc aagaacgacc gacctcatta aactggctcc cttcacctcc 53340
tctcacatct ttttctgcct tttggccaag ttttctctcc cccgcatttc ctccttgatc 53400
tcgtttgaat cctcttccct ggtgaagtca tttaggttca ggctcttatt ttactttggt 53460
ccataattta gatcgaacca catgtgctga tgtgattgaa acgatgtgga attctctgga 53520
cagagataga attatggagg ggttagtgtg tgtgtttaag attaaaagac caggtgtatg 53580
ggaggaaata taatgaacaa aaaatagtat tttaaatgaa tactaaactt gcactcatgg 53640
aaaaagttct cttcccatga ggttctcgca aagcatttta ccatcagcac acgcagtttt 53700
tctcagtttt ctgagatggg gccatcttga atccaacaga caacacacag catcagccag 53760
actaacacaa aggacgtcat gggcatggac gtaaatactg gtgtcaacac taggtctgca 53820
cctcgagagg agtggagcaa aaggatggag tggcagatga aggtatgctg ttcagaaagg 53880
aggcagaaat gaaaggaaga ccatcagtgc gctccacagc ttgaggaccg tcctggaggg 53940
caaatgccag ctgctcactt ctgaaaagaa aaattccagt gaaatgagta cagtcattct 54000
taggattact cacttgatac tgtgtatgtc tcttcttggc ttctcatctc cacacaaaac 54060
cctcaggtgg taaaaatcta attaaaaaaa ttatataaag tcttgtagat ttattagcct 54120
gaacataata gattttttt aagcacgtta agtcttccat ggactaaaag aaaacttgta 54180
aacctaagag aacctctatt tttgatatac aaaataatac atttccttaa actatgatct 54240
tgatactaga attttaatta aaaaatacct gcagtttata tgcaaagtta tagattaatg 54300
cttaaaaata ggttgtatgt agtatccaca ggtcatgttt gactgtcaaa tagatgtaat 54360
tttaattcat aataattgtg tcgtgttctt ccccactaga agccaattat gcaagcttca 54420
ccattcacac atggaaaata atttaatgga gtactcattg caatttcact tatccagaat 54480
tggctgttgt tctcagagca gcttgtgttg ccttgttaag gagaatatgt tagtatccag 54540
acatccagaa aggatccttt actgtttcag agtccatttt ccccactttt gaaatacaca 54600
cacaaacacc cattcatgca aaccaaacag agattgtaaa gtgattccac tgacatttat 54660
gcacttcttt tttctctttg gttcttcaaa ctctcagtca gtgcgcattt actcttaatt 54720
tagatacggt ttaaacctaa ttagaaacca gaagctcttg tatttccaca aaggattatg 54780
acagccccaa gaaaagatag tgaaaccatt atataacaag ataaaggctt cttaacaata 54840
caaggatgga ttttctcatt gatcttagcc ttctgaattt tagaaattgc catttcaaag 54900
tctaaaacaa aggaaaatca gggaataaaa gaatggtaag tagacacaaa cctactggct 54960
ccatcatttc tgttttagca aataacctgc cacatatacc aatagcccaa gagatgggca 55020
tgtccctgca tttcctggtc aaggtgacaa cactgcgtcc tcctggaaga ggtctgccac 55080
tcaccatacc acaaaccaaa tataataaaa tcagaaggca cactatagtg aatttttag 55140
aggcatgtat tgaaaagcat ctcaaaaagc attctcgaag cttccagaag tcaactcaag 55200
ttatctgaaa agtgacactt ttgatgattg ctcgcttaat actgggagag ccagatgaag 55260
attcctcccc acttcctcag atgtgcaact ctggaatttc ttagtgttac tggagattcc 55320
tgctgcattc tgggccttta atgcataaac actgagatgt tctaaggaaa ttactcccta 55380
gggaggagag gggtggacga ggagtaagct ttgctggtga ctcatgcgct gtgtggaaac 55440
tccctgcaca agtgagctgc gcagggtgag tctaaagggt taatgcactt tcaaaagcct 55500
ctaatttgtt attccagaag agtaatttac tcactagaag tatctgggtg gctactaaca 55560
catttgtgtc tttaaaaaga tcagttttat tttaagatta aaaatataaa gcaagagctg 55620
gaaagtcact aaaaactgac agccagtttc ccattttcaa gagtatttat taaaaggttc 55680
tggttgcaga aggaataaga aatggcttga gatcatgaca cagtgaatca tgttgtaaac 55740
atgttagcta tggctgtgaa ttcaaccagc gatgagttca agcgtcccca gaaggtgttg 55800
ggggaattag ggacatggct gtgtttcccc agagaaaagt ggccatttta ctttccctct 55860
tcactaacat gcttttgaca tgcatggcag agctgaaggc aaggggaagg ggacaacata 55920
gtaagtgact aagtggcttt tttttttttt tttttgccaa gtgaagctga gtcatatggc 55980
ctctgtcatt ccaaaactat tctctacggc tgcattcctt tcgctcttgc cttcctttag 56040
aaccctggag aaggcctcct gaagcctggc cctattatgt atcctgacaa agataaactt 56100
ttccaaaaag ctgcatgttg tttctagcac agtttttcct cgcagtgact acgtgatgaa 56160
agtaccatgc agaggaggtg tctgactgag gcgttcgtgg tgtgtgacag agtcccctgc 56220
acaggacagc cgcactcccc tcttgcgtcc tttcctccca tgtttgcaaa gcctctttcc 56280
ctgtcagcag gggtgttct ggcagttgac atttctgaaa actacagcct acatttttaa 56340
aaaatccagt aagtgaaaac taaaaaatta ataccgtggt cataatagtg tggcatttga 56400
taactaatga ggcactgtcg tgccagctat tattttcaga catttacagt cctttttaa 56460
atacaaagaa atatttggtg tgaaatgttc cccgggagct ggtgcaagca gaggcgacag 56520
ggcaagggag cttgggttgt agcctcgaat tcctccggcc agggctaccg tcagcctgcg 56580
gcacacaagt aaatcaaata taaaaccaaa atttctgtaa gcaaatcagt ttctaactca 56640
ctgtaacgaa ttatctttcg cacatcacag aggcatctct tttcactgtc gagtttggtt 56700
tgcttggtta caaaaagggc agttcaaaag ctttggttgc tattgtgaaa gtcagctgaa 56760
ttccttccac cgtgctgggg tggggtgggg ttcacgcagg ttctcttttg tcaccagggg 56820
tgctgtggat tcacaagtaa gcaagaggct cctcaggtca agcctctggc tgctccctga 56880
ggtcagctgc ctagcttctc ctcctctgag atagacggga acaaagtctt tgatgtgtgc 56940

atttctcaag cttgacaatg atacagctac ataaaaaccc atgatttcat atagatattc 57000
caaaacgtaa aagtaaacca tgcatccaca gagacatgga attacagaac tggatgctga 57060
gctggtcact tgggaggcag gcgtccttgc cattggttta tgcctcagcc ccaccatgca 57120
gtggctggcc aggtgaccta ggccagtcct gcatcctcgg ctcctcacct gcctggtggg 57180
acagtgacat ctctcctgca gcactgctgt cagggtgagg gaggtagggc gcagtttcag 57240
aaaaccattg ggctgcacct gcgtgagcac agctgcagga gcaaaagtca gaaaggtcag 57300
caaaggattt caggagcaaa ggtcagaaga aaccctcaag gtggttgtgt ctgcaggaaa 57360
gtgctgtcgt ctcctgcaat gctttcaaga ctattcagaa gcacagtgtg aagggagagc 57420
cggagcccat ggggaaatga ctccagagtg ttccacgtgt tggaaggcat ctgttggaaa 57480
acggacattc aagcaaatag ttgcctgcat agacaacgca gaatgactgg gaaagcccca 57540
acaagttacc tactggtaaa tgaggtgaga agcttaaagt gagaacccca ttgctgcctc 57600
tttttcactt taaaaacatt taagttttga attatggtaa aatacacgta agatttacta 57660
ctgtaaccat ttttaagtgt acggttcagt agtgttaagt atattcacat tgctaaggaa 57720
ccaatctgct acttttgttt attaattttt tcctgagggg aaatattttt aaattttaaa 57780
atatttaatt gacaaataaa aattgtgtat attcaaggtg tagaacatga tttcatatgc 57840
acgtacattg tatactcatt accacaatca aagaaattaa cacatccaac ccacccatag 57900
ttgccattgt gtgtgcgcgg atgtgcgtgt atgtgtgtgt atgtgtgcac gtgtgcgcct 57960
gtgtgtgtct gtgtgtctct gtgtatacgt gtgtgtacat gtgtgtacgt gtgtgttcct 58020
gtgtatgtgt gtctgcgcac gtgtgtatgc atgtatatgg gtatgtgtgt acgtgtgtac 58080
gtgtgtgtgc atgtgtgtat atgtgtgtct gtgggcacag gtgtgcctgt gtgtatgtgt 58140
atatgtgtat gtgtgtacat gtatgtacgc gtgtgcatac gtgtgtgtgt gtgcacaggt 58200
gtgtatgtgt gtgcctgtgt gtgtgtgtgc atgtgtggtg gggacactaa aaatctctca 58260
tcaccttttt agtcaaaaga acagttgttt tggtttggct cttctgtttt aaaatatcag 58320
aacaataata atttcccaca gacaaaatcc tcaatcctca ccatccttct atttcctata 58380
ttcatcataa acttcatgct tgatgttgaa attgttttct gaaaatagag aatacaaaga 58440
ggagatttta aaatgtcagt ggcagcccca cactcctttt taatcttatt tcctgatatc 58500
ttgagtttac ttggacgtag agttttcctt gactatggtt atttctggta gtagcagctc 58560
cagattaggc aatggttttc ttcagagata gcttagagtg agccccagaa caaggtcaat 58620
gcgaagattg cttgtgtctg cgtgtccagg gcacagtgat cctcatcact agccgggggg 58680
ctccgtgagg atctgctcct ggtcgtttct gttctgtatc ttctctgcag cccttactga 58740
agccgttacc aactggcaca attcaattcc tactgtaccc atcatgcaca gatggctgaa 58800
gtattgagaa cgctccagtg accgggaggc aatagtctgt ccacatctaa gaacacactt 58860
ggaataacct tagagaagag agagagagag agaatgcatg gttagtaggt tatcaaactc 58920
ctatgacttt tcacaggaaa agccctcatc cacaccaact ttaggaatgt gtagaaagaa 58980
gggtcaggga caggggtgag tggtgggcag agcagttgga gggcacaggg aaaaggcatc 59040
tggtcatgta tttggagtag gaggtcttgc tttactattg aattgcaggg acactttggg 59100
aacagtgttc acttcttttt gcaaccattt cttcagagaa aagtcatgat actcaagtct 59160
tcttacaaag cagtttgagg ctttgagtac cagactgatt acagagatga gtatgaagca 59220
ttattgtagt atttttaagt gaaattcact aaatgcaaat aaacctagca aatgctctat 59280
ggttaatttt tttctaaaat tcagataatt aagacaattc attctcctga aactgctgtt 59340
catgtaaaaa ggaattttat cgaggtggcc cttgagtgcc aaacagcctg tcctcagctg 59400
caaaatgagt cgttgatgat cctccagcaa gggatacttt ttagctcgtg tggtgattgc 59460
tgcacacggg atatgtgcag caagtatctg ctgagctaat aataaacagc ctcagacaga 59520
aagacagtgg gcacaaggtc atgcttaaaa agaccccttg ttctactgca tcccagctcc 59580
ccaccatggg gcctcacagg ccctggtgac caagcacatc agacctggtt cttgctcagt 59640
cctgggagcc acagaaccca gcacgtactt tacccccaag accagactcc agcttggctt 59700
ttgtcctcct ctccaggatt ggtgacctcc taggtcgtga agctgtgatg agcaaagaca 59760
cactcctctc cattctccca acttcaggtc cctttgacag tgtcagcagg catttaaata 59820
gcagaccacc cacagcaggg ctggtagatg cagtgaactc aggaagatgc ctgcatagac 59880
tctagtgtta aagacagaat ccttacaagg aacccccata gttacctaac tgctgtctcc 59940
agtggtcata gaagtgtgat aacccactaa tcatcattct ctgtctctct gtctttctca 60000
tacacactta cacacacata cacacaacct tgttgcttaa ttttcagaga gtctactttc 60060
agaaaagcct tcaggaatac atcatgtaca aaactgagaa attacctgaa gtatctttaa 60120
atttagtaaa aagttgcatt gttttttgaa catcacactt gaaaagtaca tgaatacaaa 60180
catacttagg aaaaaaagct ttaattaatt taaaaaggag aacaatgcta tatgctgtat 60240
cccacctttc tctgaatgtt acattttctc ccctatccca ggctgcatct aagaaaactc 60300
agaggaata tgctatctat cttttccgag caatgaaagc tctgggtttt ttccttgctt 60360
ttcagggcac aatacttctc tttcttcctg gttagacagg ataagttctg agtcccctgg 60420
tatcatcagc ttacttcttc tctgttaaat attcacaaaa aatcactaac tttcatgcct 60480
cagcaaacct ccactgccta aaatatagtg aggtcattca tcttcggaca aattgcccca 60540
actacggtgg gaaaagaacc aatgtgttgg actatttatc taatttttgt ttagttcggg 60600
gatacaaata aatgcataga tacatacaaa catgcgtaca taatagcagc agcagcctgt 60660
gaaacattga caagacctgg agttggaaga ggactttgcc atcctccagt ccaacagttg 60720

```
cctgtcacag attagacgac tgggatgtgc gcaggcgatt atttgcaaac ggccctgagt 60780
cccccagttt atgtcttaat tcgcagccag ggctgattgt agaagcaaat ttgcaaacat 60840
gtgcaagaag aaatcacaca tcctagagct tggatttcct cgtttcttgc tatttctatc 60900
cgtagacaga accattgctg agctgttaaa tttgtctcct tcccctatac cagtcttgaa 60960
aaaggaaagg aagtggagca aagaaaaaga aattaataaa gccggcagat cctaggagaa 61020
tcttatttaa tccaagcttt gtaaagtttt gctttattcc atggcaacat gggtatacac 61080
atcccaccgg ctgtttcagt ggctcagagc aggtaaggcc tgtgccaaac gccgctagca 61140
ggaggaacaa cgtggagaca gccccagagg tggaacgttg gcccttctgt ggctccggtg 61200
tctcaggacc tccctaaagc ccagccctga cactgagcaa gtttccacca ctgttaggaa 61260
gaagtagaaa ggaatttgga gggttggtgt tactgttcaa gagctggaag gcttctgccc 61320
ccattcccat tccattaatt gcgtgaggta gagaactcat agaagatagg aacacatatg 61380
ctgatttcca aaattgcctt tgtatatttt cacgtgaaga ctttaggggc aaaagaaaag 61440
aagcaagcat tttgaatatg tgtttcaatt tgccttctgt tatataaaat tgtattttgc 61500
ctattctttt ttcattattc ggaaccttca agaaataaat taagttctct caaaaatgtg 61560
tttttgaaa agaggactaa aacagatggc ctggctgtgt taaacacagg gaccagacca 61620
gcacccacct ctccacctgc cctgccttca ctggcagaat tgtgatccat catgttctct 61680
gttcaatgtc atcatccctt tcagagcatg ggtctcttcc tttctaggca gtcttaccag 61740
gatgcatggg tgtgcctgcg taggcacacg cacagctccc aaggactcta aaaaaagata 61800
tttttctgct tatatactaa taatatgtta gagatttatg tttcaaatta gtacagaatc 61860
acatggttct ctccaaatta tatttgagag agaaagaata gaacaaaatt tattttacaa 61920
aaatactcag tacatttagg gcatatacaa agatgttcca gaatgtagct tatctcttta 61980
aagacaatta acacagtttc tgggcaaggc aaggcaaaat attcagtaac ttagcaacac 62040
caacagaaga cagccaatat tgcagcacat ttttctcttg gattgggtca gagagtactg 62100
cagagaaaat ggagtagaga gacctgaaat actttcgcac acactgtggt cagtgcagcg 62160
tccactgtgt gccacagtaa tactagaaac tccctggtta ggccttggaa tccagctctc 62220
atttcgtatg tgacctgcag ggaagtaagt taaatgcaca cgttttatca agttcaaatg 62280
caaacttaat tttaaatgta tgcaacatca gtttaagcgt tgtagctatt actagcaatt 62340
gtacctatta ctagtctgta ctctgcacaa ctttggagta tactgcctac tcaaggtgga 62400
ttttagagct ctatttgtgg cattatatca cggacaaaag cacgttcatc agagtcagag 62460
gaatgtggtg caaatcccag ctgtcccact taccagctgt gggacttgag taagctcctg 62520
aagcagctgc acctgcattt tctggtgggc accatggagc tgtcagcagt gctttcctca 62580
gagggctgcg ggctggatga ggtttgctgg tgcatgtgaa gtgtcaatca ttgctctcat 62640
gagtggtgat gctgatgccg ttccctttttt tagggaagtg attttccctt acaaagttac 62700
caacagtttc atgttggccc atttttctat taattgtttc cactaatagg accaacagtg 62760
gtagtcccat cattttatta ctgcttgtcg tagcacaagc agttgcttca ttgtgtttag 62820
ataaatattg acggctgctt ttaacagtct gctgttttgt ctccttttga ggtccttaaa 62880
gtaatcctta aaaagatagt gcagatggaa agatgtctgg agtcagtgaa cctgccttct 62940
ttcctgtgtg cttgtcagtt tctaaaatgc catacacaaa ggactttcat gatttctttt 63000
taggtacatg attacagttc aattcacttc actgtctgga aaatttcctt ataatcagga 63060
tgaaatttct catgttagcc tttcacattt cactactttt agataaggaa ttctcaggct 63120
ttgctatatc tgactgctct tggaggctga gcttttggct aactacctga ctactttgtc 63180
gtttctcttc ccttggaatg aagcaaatat ctaacttctc actcattgtt tctgctattt 63240
taccatttag tcatctgtga tttttctaaa tactgaaaga cttccctcaa ttcaaactat 63300
gtgccggatc aaggaaaggg cagttggata ttgcagacag catagtgcaa ttgtgaagag 63360
tgtctgctta ccagccacgc tgccttgcac aagttatcaa gcctctcaac ccacttcctc 63420
aatctgtaaa ataggtatga gtgtaggacc ttcccagggg attttttgt gactatagaa 63480
tgattctcag aagactttca ggcagtatgt gggtgaggca catgctggaa aggcttctgc 63540
aggtgcagtg atcaatgctt ttctcagtgt gtacatccca taatacagac acgttaccag 63600
aaactcccta gccaggactt tgattgcagc tcacattttg tatatggccc atagggaaat 63660
gaagtgtgta ttttttataa agttcaagtg ttaacttaat ttggaattta ctatcaaatc 63720
tcagttgtta tgggcattta tagctattaa tacttcgtcc catgtgtccc atgaggaaac 63780
caaggaacag aaattaaagt tctttctgga gtcccctgaa tctcgttcct gttcttttgc 63840
accctgttaa ttacatagag acattcacag ctcttctgac cttatcagcg ttaaggaaaa 63900
cagaaaacca gcgtgctatt tgttctgtcc cttagtcaag ccttctcaac atatattttt 63960
cttccaagat tttgcatgtg cacagggatg cctatcctct acaagaaaca cattttaggc 64020
aaattataat taaaatgctg tttacatctc ttcaccttta gaatttaaag aatgatcatt 64080
tcttagattg catctcagac acacccttcc cctagtctgg agagggcgag gcccatgggt 64140
actgcaaaca gcctgacgtt gtcaggggcg gtctcaacgg ctcattcacc acatctgcct 64200
cgcgaaggct aagccatgtg ctgttacccc tgctgcgctc tggctcattc taaggtacac 64260
gctattaacc ttgtgagaaa acaaagaggc cagccccacc cttcctgctc actctgagtc 64320
acggtgaaaa tgtttcagga tctcgggttc gaccatgagt cctgtccagg tccaggagga 64380
aattcggaag gaccacatgt tcactctgag atcccacttt catttccctc ctggttgagc 64440
agcattaata ctctggctag atttaaattc tggctttctc cagttagaac tgaaagttat 64500
```

gacaatgtaa tcaaaataga atgtgggttt acagctggcc ccctggcctg gtttgtgaac 64560
ataaaacaga aacagaaagt gtaagtggtg acatcatatt ctctcattca atgtgaaagg 64620
ccaccgaagt ctttccagaa ttatttttga gaataatatg aatttttaaa aaatacctaa 64680
ttattttaaa tatcgtcttg cttgctcccc aaatacctac tgttttcaac ttggatatac 64740
gacatgatta aagaatatct aatatttggg aatgcatact ttaaccttat aaactaccac 64800
tgtaaataga cagactcatt aaagtgaaag gacattttaa atcaattagt aagcaaatca 64860
attaggtggc aaagacaaga ttatttttcc ttatggtagt tgaagaataa tgcttaacct 64920
gtcattctaa ttaccaagca cggtgttctc tttggaagat catttcaaca aaacattatt 64980
ttcatccaga atttgaacct tgagattgca tggtatttta gaaatctatt ttagaaatct 65040
ttggcaaagg ttactattaa aacaatcaca ttcatggaaa atcagtataa gagcaactaa 65100
aataactcac aataccagta aaatcacttt gtcatcttct taagactttt aaagagcatt 65160
tgtaagtaac tgaatagaag gccaaagggt gtgtaggtag cccagaccat cagtgggcag 65220
ccagggccag ggcaggggcc acggttgcag cctgcattct tctaaagggc agagcaaatt 65280
aaagttgaag caggagctaa aaaaaaaaaa aaaaatgttt caaagaattc caccaaccag 65340
aggatactac ctaggacagt ttgggcctaa cttatctgtg aaggcctcca gcttcctcca 65400
caccggtggc cacttttcat tcactctgaa cccttctttg tatggaggtc attttattaa 65460
ttgagctgtg accaacatga cagaatttcc tgttttaggg cttttataat atagatagtt 65520
tatatctaat ttcagaatat attcactggg gaatggactt agcaaccact accacaacaa 65580
tgcaacaatg tgttttggaa caaatttacc aatctgaatt tccccctaga ttaggtcaca 65640
ggaacattgc agctgatgta cagctatgtt cctcctgaaa cttggagaca catcctcttg 65700
agctgggtta taatgggcca cccaaagctc gagttcctgt aatggataca ctcaggcagc 65760
agaacctacc accgtagtga ggacagcacc cagagccctc agaggccatc acaagtgcac 65820
cacagctgcc ttctctggca cgctcagagc tacacagtgt actctgggat tggaactctt 65880
tatttttttt tcagttgatt tgtaaataag attgcacaaa aatccatgca catcaactct 65940
ccaaatcaga atttgctgag ctaaaaagag cattaaatta gatgggctgg ctttcaaggg 66000
gtgggggtgc aatagtggaa ctctgcacaa cagttcttta caaagagaca agcaagcaca 66060
tcgcgtggaa atttccattc aactggaaat gtccaagcct gtttacctca attaattgtc 66120
cttgttcact tgtccagcct agcaattgtc cattagtaat ttgttataaa tgagacattt 66180
ggtattaaag catctctttg ggatactggt atggtttatt ataacattct gttagtagtg 66240
ttgtacaagc ttgagatgta ttaatacgaa atccaagctg catgagggct ttatttttca 66300
agcctacacc ttgctgaaat tctgaattaa aatatgattc tcagtacaaa tgaataaatc 66360
aacagaaatg gtaacgcatg tcaaatattc ttaaaaccca agaaagcctt gtaacttcct 66420
tcaatctaat gggaaatgca ggcaaataca agactgatgt ccttgagttt tattatcaag 66480
actcaagggc accagtaaaa tctagtttca ttggttggaa aaaaaatcct gataagcact 66540
gttaggcata ttaactttaa tgattacaat ttttaggaca ctctgtggcc tagacttaga 66600
aacacaacta atgtccagaa aaagattcct ctttttattc catcatctga taggcctatt 66660
tttacacata cacaccaacc aaaagtagcc aagcaaacaa aacaacatac tcacacccct 66720
tcgcctatta tcatctaggt gattttcaat gctcattgca atgaaaccta cttattgtgc 66780
atggcaccca cccccactga ggaatactgt agtttctttc cctttgaact tcattagtag 66840
agcacatggt tcattcactc ctgaagagtt cttcgtatgt cagaatatat atactacaac 66900
ataatttcca tcagagctct gaccacccgc ttatctattt tcataatgcc tgccactcca 66960
tcattagctg ttgtcatgta ggctatcaat aaatatatga caaataaaac agttagggaa 67020
tgagggaaat tgactagcag ccaaagacct aagccatcct ctgcttggac attagaaaac 67080
tgagttcact acagtcataa gatacacaaa ggcagaatgt aagccataca aaaatccatg 67140
tcaatcccaa tatgtgagta caactattga acaccatgta ctaatggatg agttggtaaa 67200
tcattcaatg tcttcatgag gtcaattaca gattattatt tagaccccaa agattccaaa 67260
gatggtattt cggtcagatc ttcatccttt gtaagcctag cagaaaatat ggcagtttta 67320
ttgactacta ttctttgctg ggtgtggtat ttttaaactg agacatcagt gtgcctagca 67380
cagggcctca agcacacaga aaaattcctt gataataatt aaataaaatt tcagcaaaaa 67440
atatcatctt aaggctgtga aattatcttc ctgtgtggct aaaatagtga ataaaattca 67500
gcgcaatata aatcatagta caatttcatc actaaatttt ctgatcttga tcttgtcatt 67560
ttacattgga agtaaaaatg tgtcctcctt tttttctctg acagtgaaaa gtgtgtgtgt 67620
gttgtgtgcc cttttgcaca ccctgcctca cacttgctgg tctaattcct tccagcatga 67680
ttatgatata attaaatgac agaaatgttt acttccaagt ggaactaagc cagggtaact 67740
cagggtaggg cagctgcttg caccgaaaga ccaagactgc tagagaacta ggaaacaggc 67800
ggtgcaagaa ctccaggctc tcatggaaga gcgggaggct tctatggggc tgcagaaact 67860
ctttggtgct tggggaaaaa atgggttaaa tgctcttaaa aaagaaacct gggagaggta 67920
gtttccagat gcaggcccgt cttttctttt aaacagaggc agctccgaag agctggacat 67980
tgaaccctga gcaggaactg gaggccgtca gcgcagcttt gtttggcgag cggagctttg 68040
caagggtgta atgctgcacc agggagacgc tatctgcagg gaccggtgac gccgtgggtg 68100
tggaggggga ggcagtggct ggccctcttg gggtaaggta cgcccaggaa cagtttagaa 68160
taacgtgcgc gagtcaaagg gaagaagaag ctcctgcaga ccttctgggc actgtgcagg 68220
gtttgctcct gtccaccgtg ccgtgttcct gtcctggggt atttgggtgt gtggcgtgtg 68280

gggaggggag aaggagcaag gcggcaggga ggggatgagg accaccctgt ccatgggaca 68340
ggccctgggc cccgcacaca ccccaagccc cgcgtcccgc gtcctcactg tcctgggaca 68400
ccccccaccc caccccaccg ccacagccca gagcggtgcc aggaagccgc ctcgacgcag 68460
ccgtatcttg aggctccagc cccatcccca gggtaccacg ccacgtagag acactatttt 68520
tcacttcgtg tttgtcactc ctaaagcatg tgtgctagct gcaccaaccc tgggatgcct 68580
cggtgcatag ggtttatgtg cgtcctcctc cttccctctg agctggtccc ccgtggggaa 68640
ctgctgccca gactgacctg cgtccttccg cacgtgcagg aaaatgtcca cgtgcacttg 68700
tcagggtggg ggccacacgg gcaccaccac tgatcatctg tgggatcgag ttactgccca 68760
tgcagatccc acgtgcaggg cccagtcgct ttggtgagag agtggacgct gtggtgactc 68820
cacggtctgt ggctgtgctc aggaggacag agagggggaca tcctgagatg gtttgggcag 68880
cccgcggatc ctgtgcatgt ccccagagcg tccactttct ccatggagca gtggagtggc 68940
gttgctgaga cagaaagttc aggttctcca ctccccatgc agccccccact cccctgtctc 69000
cggccaggca cgcgtctggg gtggagactc ccggtgcccg gggccctcca gacctctttc 69060
cccaccccag ggagcaggcg ggtacttcta ttccgtttgg cttcagaagg gaaaagagaa 69120
cgtaagttca gggagttctc gtccattcct ctcccgtggg ccgggcaggc agcagggaca 69180
gccttcagga gccaggaggg gctcgagctg cgaggccctg gaatgaggca ggcatgggct 69240
gaggctggag ggaaagcccc gctaaggctg ggcgggggcg ggaaaactta ccaccagggg 69300
actcgagatg gggaaggaaa ggtcagaaga ggagaggcca ggcacggggt gtgggcggcc 69360
tgcagagctg gagcaggtgc tccgcccaga gccaggcatg cacactcaga gtaggtggcc 69420
tgtgcagcgg ggaagagggg cgggtcggcg tgctgctgaa gatgcaggag ctgcggcctg 69480
ctctgtgcgt gctgaaggtg tggtgagaag cacttacaaa aagaaatgga ctgtgttagg 69540
attgcacatt ttactttgtt tctcccaaat acgtgttctt tgaatttttt tccttccagg 69600
gccaggactg gagtgatggt tgagacaggc acgcactggg tcttgtctgc atttacattt 69660
tgagattttg ttcagcatgg attttatggc gtttttttgt ttgtttgttt gttcgttttc 69720
aaaatactgc acggtttatc gtgaagacag ggtcctttgc tgccgtctta agttttgggc 69780
ccaagaacgt gccccaccct aggcccgggc ctgctggctt catagctctc atcattccca 69840
cggaacctta agacctgagg acagaaagga aggaaacaag cccagtagtc cgtgaaaatc 69900
cagggtcccg ccactccagg tgtctgcagc agagctgaac acacgtaggc tcttgccagg 69960
aggggcattt gtatgtgctg agcattcctt atattctcaa tatgacgcct ttgaaagatc 70020
tgtggtttgc aaatatttac tctcagtcca taacttatct ttccaacctc ttaccaggct 70080
cttttgctga ataaaagttt taaattttga agtctaatat atttttaatt tttttatttt 70140
atggatcata ctttttgtgt caggtttgag aagtctgcac caaagtatgt cctgtggttt 70200
tcccttaggt catcttcaac aagtttcata gtattttgtt tagatgtaaa tctgtggccc 70260
attttgagtt agtttttgca caagagttga ggtcaaggtt cttttttttgc ctgtgatgtt 70320
cagtggctct ggcaccattt gttgaaaaca tgatagccaa tgtcaagact taatagttat 70380
aataatcagg agcttttgtt tctttttgtt ttgtttttag taactgccag tcactgcttg 70440
tggtatacat acacaatgga atactattca gtcttaaaaa aaaaaaaaga aggaaatcct 70500
gtcatttgca tacctggagg acattatgtt aagtgaaata agccaggcac caaaagaaaa 70560
acattgcatg atctcactcc ttcatggaat ctaaaaaatt gtattcagag aagcagagag 70620
tggaatggtg gttaccaggg gctgggaagg tgtgagcttg gggagatttg gtgaaaggac 70680
atagaatctc agttagacag gaggaataag ttaaagagat ctattgcaca tcatggtaac 70740
tgtagttagt gacaatgtat tgtatacatg aaaattgcta agagagtaga ttttaagtgt 70800
tctcaccaca ccaaaaaaag gtatgtgcag taatacagtc attaattagc ttgatgtagc 70860
cattccacaa tggatacata tatcaaaaca tcatgttgta taccataaat atatactgtc 70920
tctttatgta aatttaaaaa taagataaaa taaatgttat tcacttgtcg tggatgtggt 70980
ggggacaggt gtgggatagc cctccctgta caactaggac ccaggggtga tctagtgaca 71040
ctagccattt atcaggacgt atgggtgcca gtcaggatga taaagcttcc ttttggccac 71100
tatactactt agaaatgccc tgcaaaaggt gcacatcaaa gattgaaagc tcaatcctgg 71160
attttaagtg cttcaaaagt gcacttaatt gccacatttt tgtcaaacat tttcccaggt 71220
agtatttttc ctcatgtaaa acaacagcaa tttaatttga acagaaagca ttttgaaaca 71280
tacttttggc agggttcctt gcagatcaga atggaaatga ttaacagggc aattatcaat 71340
catggacttt tggcggcaga aggaactgta ttgtttggta cagtctgggc cagggccaca 71400
caccgtaacg gagatactct attctgtgga cggttggagg gggctgtgct gagcagggta 71460
actgcatctt ttcctagact gttcacactg ctgccacgaa ggagtcttgt ttagactgga 71520
cctggctttc ttcttcgcaa tgagtgttgc agactcccga caaaggccag gtggtaaagt 71580
gtggtgtctg tgagcgagag cctgagatgc ctgagctgac ctgtcctcag ccacctgcca 71640
tcgtgcagag gtgagagcag cccctgaatt ctgcccctcg gtctctccat agctaaagca 71700
aaaccatcct tccgtgctcc caggacaagc aggctattac caaatcaccc actaaccctg 71760
ggcgaggagg ggccatcact gcacaattca tcagtgtctg tgacaggaag agattgtttt 71820
agactggttt tttttttttt atttgcaagc ttttttctct ctccaaaacg tgctgtcagt 71880
gtgttctaat ttactctgta aggaattctg gagctaatca taggctcaca aaaagcagca 71940
caggaaagtt tcccagataa catctatttc agtggctttc aaacattttt gaccttacca 72000
aagtaagaaa tacattttaa tatcatggca cacatacagc tgtatctaaa ctttcataat 72060

actgccttta cgatatcact ctgatattgt ctattctttt ctgtttattt ttctttttgt 72120
tccttgttat gctggttgtg acccactcca gtgatttcac aatgcaggct gggtggtgtc 72180
ccacagtttg aaatcccaat ctagggcctt cctctcactg tacaaagtag gtaactgggg 72240
acattagtgg atcagtgatc aaaccaaagt tatttgatct taccaagtga tatcaggatg 72300
agaaagctgt tagagtgtca gatatgtgaa ggaacttggg tcattcctga tacctcaaag 72360
agaaaaaagg tagtccttga acacctccta cttgtaaagg atgcacaatc ctacatgccc 72420
ctccctttcc tttcctcccc tctgtacccc acccctgccc acatttttctt cataagcagc 72480
tttggtgttt tggcttgttt gtttcccttg tctcctacct gtgactttat agccttttgg 72540
agactcacag caatagttgt atttaaactc agtgggtggc atccaaggct aaaaaggaga 72600
ttgcctagac acaaaaccac ccaagggaga aagcaggaca gcatcttact atgattgttt 72660
cttgtttctt cctgtctcat aaggattatt acccagggtt ttcatttttt tcatttcatg 72720
gttcattttc gctccagtgt agacatacaa tagaccactc gtccctgtgg ctccgggcag 72780
cagcctcatc tgagaccctc ctgagacatc tcgtgcaggg cagccgtagt gtgtggcttc 72840
cccagggctg ctctaacaga tcaccatcct tgccatggct taagaagctg cagatttatt 72900
tgcttacagc tctggaagcc agaagtccaa aatcaaggtg tcagtagagt ctctctctct 72960
gaaacctgct gaggatgatg cccctggcct ctccccagcc tctggtgttc ccagcagccc 73020
ttggcattcc ttgccttgta gatgcaaaac tccgatctcc acctctatcc tcacagtgag 73080
ttctcctgca tgtctgtctc tgtgccttca cattcctctc tgtgtgtctg tgtttccatc 73140
tccttatgag gacacccatc actgaatcag ggcccactct ataccagtaa gacctcattt 73200
caactccatt acatcttcaa aaaccccatt ctcaaataag gttacttcac aagtgctgga 73260
ggttaggact tgaacatacc ttattgaaca atccaactga tgacacatag taatttatgc 73320
actcgttctt ggagacgttg actttattta gtagcattaa ccatggcaat gtcaccagca 73380
tcgctgacag cctgaagcat atgatctcca gaatgtattt caatcatcat gttcacttcc 73440
ttggtattct ttagacaata actcagcctt gaactccagt aaagggtttc cctgggattt 73500
tcttcttgac tcactccact gtggcctccc tcatccagga ctgtaacaga cgcctgacgt 73560
cagtggtcta gacctctctg ctgaatgtca tctttggtga atgtcttatg agaaaacaca 73620
tggttggtca ctcttagaag ggcatgaaag cctgtctgca gtataaccaa aacaggcaca 73680
tggcgaggca cactgtgcgc atgtgtgtac aattaatatc atggttttaa attattttca 73740
ggccaagggg agatctttgc tgcatctact gaagaaagcg aatctttttc ttcctgaaaa 73800
aaaatggcta cttattagtc gaatttgtgt tttaaaaata tgtgaactaa tataatgcag 73860
acatgcatta atgtttaaat atactggaag tttttggtaa aatgaaaccc attgtctctg 73920
ttgattactt tgatgagtca agaagtaaca tcctgggaat gattggccag tttaaatgag 73980
tgcctcaggt ttttggaata caagaaatca agaggaaggg attagaacat ataggttagc 74040
aagattggga tcctaaaata cagacccaaa tgaatggaac aaaatcaggg aatttattaa 74100
taacagggtc aaggccaaat cagtaacaaa tatcctgagt ggaagaaagg tggtttaaca 74160
aatgccccta tgaaagatag agattggctt accatgatga gatgtaagcc caagttatga 74220
ggttggcaca caaaaccaca aatgtcatag cttaaaacaa cacacacttc ttatctctgt 74280
ttctgtgggt cagggtctgg gttctcaggg actcacaaag tatgttttca tctggagctc 74340
caggtcctct tccaggctca taagggttct tggcagaatt cagtttcttg aggctgtagg 74400
actgaggtcc tggctcctag aggccacccct ctcccataagc agttcttagc atggccgcct 74460
gcttctccag gcccagtggg aaagcatgtg cctccaggag ggctcagtcc attcttcatg 74520
gcttttacct ggttaagtca ggcccactca ggataacttc attttgtatt aaatcaaaac 74580
cagctgattt gggatgttaa ttacatctgc acaacttcaa ctttgccata taacctaacc 74640
atgggactga tatttatcat gcatttgggt caagttgcat taagagatat aataaagctg 74700
gacaagcttc tgttgattag aagagttcag ttacaaggct acacttggga ggaatgttta 74760
caaactggaa tggtcagagg atggggaaga cacttgagaa aagtcaagtg acggatgaag 74820
gcaaatgtgg atatttatct gggagaaaac taagaggagt tataatagct gtcttcaaat 74880
atttaaaggg cttttattag gaagaggaat ttggcatatt ggattttgcc ttcagagaag 74940
tggagtcctg agatgctctt agccattcat tccagcctcc agggctcacc tgctgtcttc 75000
tgtccaggtt ctcggtagca gggcagtaca gccccatccg tgatcttcca tagtcaggca 75060
tattgtcaca ctcagtgagc ggagagtcaa ccgggaggaa ggcacagttt ctctggaatg 75120
acctacggaa tggtacgctc aaatgcaaat tctccttccc ttccccagtc cttgtccttc 75180
agatggtaat ttaggagctg aaggtcaggg caccagcagc ctttggaagc ctacaggaca 75240
acagtcagcc tggctagaaa aaaaaacaat gtcacaggca tgttgtgttt aatcacatga 75300
aggatatttg cattgttttc caactgatgc cagcagacac attgtcagtg gtatcatgcc 75360
tggggtatca gagttgacat tgggttgccc cttctctgag gcattcatgt aaatcctttt 75420
aagtttataa aacctccatg tggctcctgc atgcttcatc atttgcatgt gtctcttttt 75480
ccaggggagg cagcatgggg agcaggatgc tggtgggctc caggtgcaga gagcagggtg 75540
ggcgtcagac cccaggtcca ctgtgcacgc cctcttgtag agcccgttcc gttgtccatg 75600
agatgaggag tgttcttatc tctaaagtat tatcatgaaa acctaacaat gtagaaagac 75660
taaagcacat gggtggtgct tcataaatag tatttctccc actttctgaa aactcctgct 75720
gaagtaactg cacaagaatc cttgaacatt tagaattctg gttttagcca taccataaag 75780
tcagtagtgc gtggtggaat tctgctaacg aaaattgcga aggatcaagg cagagtacag 75840

agctggtgtg tagcgggtac cttctgtctg ctggcactag gtattttaca cattaaatca 75900
gctcgttctc acatcagctc ttttaaaaat aaggaaatga ggagccacag tggcccaact 75960
gatgcagtgg cagaagtaga atttgagctt gtgcagatgt gcctccgtgt tttgtctcct 76020
gagcatgctg ccccaagttt gacaatacca agatttgtac tggaacattc cctcccatcc 76080
ccacccccta gaagcccctc ttcctccctt agatttgaca catagtttga aaccactatt 76140
aactacctta tgagagccac tgtttgtgaa gtgctgacta tgtgccaggt cccgtgccgt 76200
gcaatttttg tgaattatct cgtgtctaca gtgcctcaca atttctctgc tcaatacctc 76260
catgttactg ccgaggaaag ggaagctcag agagagtaag taatttgctc gagttaaaga 76320
gctggccagg acagccaggg gcttgcaccc cggagccttc atccactaca ctgtcagctg 76380
gtatctcaac cagccattac aggctgtaaa aaaattatat aagatagtct atggtaatgc 76440
agaaaagtga ggttattttg ctccctttcc ctttgaagaa aaaagccctg gaaagacata 76500
tcacttgagt atgggaaaaa atgaagctgt ggcttttctg tgagtcaatt ctttcctggc 76560
agcttcttgg aataagacca agtatagcag cagagttttc tgttttaatt tgagctgcag 76620
ggtgactttt tttcttctat gctttcatct ctctgtggct tcttttgcct cgttaatttc 76680
atgccctgcc caggcgggct actgtgctgc ccagtcaccc gggtctgggg cggccaccgc 76740
tggccagcag gcaggccctc cagaggcaga ggtggccacg cttaggtcgc tcccgctgtg 76800
gaggcggcac acttgggtgg cagcacagct gtgatgtggc ggcagctggc agccccatgg 76860
gaaagatgtg tgaagtgtgg ggtttgacga cccatgggag aacagacttt cttcctcttc 76920
ttgttttccc ttcaaagccg tgagtcaacc tcaaattctc tgtctttttt ctccaccccc 76980
tcgtgcctct ctccctcacg ctctgcatct ctcattgcaa gcttgcattt ttttgcacac 77040
aacactatct taatatttct cttttctgca ggcaggaaat gagaagtcat ttttcagggt 77100
cattcaggaa gtcatccaga gttataatgg cccattatct actggtcaga gtttacttag 77160
gctttcacta cttccactgc ccacttgaaa cagggaaaaa tattttcccc ccgcgctgtg 77220
agtgtgctat ttagagctga ccacaagcgg ggggaagaga ggatggctcg gatgctgcat 77280
ttccactgag aacacaaggc tggcaaagct tgtctgctgc ccagcaagca cttcaggctc 77340
acaccatttt aggttcactt taagtagttt ctcaattgtt aaaaaaaaaa caaaaaaaaa 77400
aaaacctgta ctctgaggat atgcttataa tcccatagct aacccagaat ttcttagaga 77460
actgatcaac atcagcagtg gcacttactg aaaatgcaca ttctcaggcc ctgcgtaggg 77520
cctactgagt tagaatatta gagagcaggt ctcagaaaca ttctatccgg cagtcttatt 77580
ctatgcaccc gaagggataa gagccatgct ttcatgaaac atgggttgtg tgtaaaatgt 77640
ttaaaaggta tggcaaaatg tgtttgattg gcaccaagga tttctggttc ctcctagaat 77700
cattaatcaa actttgaagg agaaataaga gagtcggcat tttcttgcac attctttgtg 77760
atgttgtgat gagttggaaa cttcccgatt gggtttatta gagcatgaac acccaggcac 77820
ccagcttcta gccagccctg tcaggcagag tctcctcgaa gatgtggaaa ggactgacca 77880
acagctgagg cctacaggaa cctgagcagg caaggggaga ggcaccccgg aaccaggagc 77940
aatggccttc ccaccctccc tcgtcctctc ctcttctcct tttggagttg caggccacag 78000
aaaggaagtg acatgagtca ctttgggcct tcttaattcc ttcatcaaag gcagcacagg 78060
tgtgtatgtg tgttggtggc taattgaggt aggcccacag aggagataac agatggacat 78120
actatttcct ttcttccatt ctgatataat tcagggtata aacacacaca cacacacaca 78180
cacacattct cacttctttg gcatctacca cacctgcccc agtgcccatt tctctcccac 78240
ctgaataaaa agcccccaca aagcctgagg tacatggaaa ggagcagtgg tctggctccc 78300
aggagtgtga gaagcagcca tgttttcaga ggctgtattc cacttggact tggccctacg 78360
ctgaaggtag gagcggatgg gggaggcccc cttcgcacaa agagccccat gaaagagtgc 78420
acagtccagt ctataaaaca gacgcagaaa atgtgtgtag gacttcttcc tgaaaaagag 78480
cgtggtgcgt ccagtacctc catgttcatg gaacttccca gtctgcagtt taccctttg 78540
tgcaactccc ttttggtaaa gccctggtca cacttctggt tgttcagatt atacagggat 78600
aattccagag tgattttaaa gtcaactgcc aggcatccgc acttgcaaat tagatgcctg 78660
gcacatgctt gtgttaaggt aataattcat tacaatacaa attacagggg agttcctctg 78720
ggcatgcgac ctttcccgtc atttggcttt ccctgtgatt atcaggggag cttccatcgt 78780
gctgctaatg ggaccttaac catgtgtcaa cccatggctg taatgctgac actgttttct 78840
ttctggaatg aaaggccttc gcaattgaaa ccaaaatgtt atccaactca gtcctgtccc 78900
tttgacgatg aaaacatcaa gttctggaga ctggccatcc agcctccctg cctcatctcc 78960
cacgccctcc atcatttttt gtctctactt acttatttat ttggctgtat tttacgtaca 79020
tcatgcaaaa atattcctct ttgtaaaaag tataatgatt tcaggaaatt agagggtaaa 79080
aagcaagaac catgctttca ctccactgtc aagagttgtg gaagaatcct tccagcattt 79140
tttctgtgta ttttacatac atacaaatat atgtacaaat aaaggtcgat catttaggtt 79200
ttgtttatat ttttgtatat atgagcttat gtcattcata catattgttt tgcctcttgc 79260
ttttttttaa cttaatttta ctttgcttga gagctttttg aactgaagta cgtgtaagtc 79320
agcctatgca tgtaatggct ccctcatctt ctgtgaggct gtcactaaaa aggggattta 79380
gcttgttctg ggctttgcag cccgtacact gggcactgtt catacgtact tctctgtgca 79440
cgcaaaggag ggcttgctag ggaggcctgg cagagggtgc cattcaaata ggattttcaa 79500
tggaggaatt tttaaatttt cagttatttg aataagtttt aatatatatc cagaacccca 79560
aatcatcaag tttgttttct tccacatctg tccttccatt tctgaactat tttaaggcca 79620

gtcatgtctc atccaagaaa tcccatcctt tcacacaaca ctatctccgt ttcatggtta 79680
tgaatctcta aaagcatgat ttttaaaaca taatcacaat gctgtcatcg aacttaaaaa 79740
ttagccataa atctcttatg ttacccaaca accagcctac tgacacatct ccagttgtct 79800
caaaaatgtg ttttccattg tggtttgtct gaaacatgat ccaaaagtca gacccacctc 79860
tcacctttcc ctaacctgcc ggagcccatg tttctttcca gccaggcttg gagaccacca 79920
cacgggattt gcttcttggg gcctccctct aaccagctat gcaggatgcc ctctttcctg 79980
tcaatacaag ctgctcaaag gactcattca gttcaaattc acctatgtga gcctaggtga 80040
tgctacttat ttatttattt atttatttat ttatttattt atttatttat tttgagatgg 80100
agtctcactc tgttgcccag gctggagttc agtggcataa tctgggctca ctgcaagctc 80160
tgcctcccgg gttcaagtga ttctcctgcc tcagcctcct cagtagctga gattacaggc 80220
acgtgccacc acgcccagct aatttttata gtttagtag agacagggtt tcaccatgtt 80280
ggtcaggttg gtctcaaact cctgacctcg tgatccaccc acctcggctt cccaaagtgc 80340
ttcatgtttt caggagctgt acgtgcattt ttagttttga tgaccaggtc ctttttctgt 80400
tttttaaaga acttcaaatg atctccaggg tacacagcgc ttgtgtgctg atgaaaaagc 80460
tggcagtaca aaggccacca gccaaggtca cacagccaaa aagcccctga cctcgggccc 80520
cttcccagac cctgggtctt ttgctgccac atgaatcttc ttcaaggtcc tatgtgtaga 80580
ttttcttgac ttggccatat tatttaggat tcagatataa taacaaaata gatgttaaag 80640
cataacatga aggcatttaa aagggtagaa agcacatgat ttactaaaac cataaatctt 80700
atgacctgaa agtttcacct aatctcttaa aaaataccgt actaaaccct gattgaaaat 80760
cagagctcag acatacagcc tgagatgcca aaaaatggcc aggcttgtct gttgagaaag 80820
ccatatgtaa ctaactgttt ggaaattcaa aatatatctt atcattttaa aaacatcttt 80880
cttctaaaga caatcatctt ggcttcagga atgaggctag taaaaagtga aatactccta 80940
cttgtggaag aaatcctcat tttaaccatg aagaactgaa aaatgcattc tgatgttgat 81000
ggacccaacc tatatttggg tattttatga tgtacacaat atacttttgt atatgagatt 81060
gttattaaat gtgactttgc tttttcaaga catacaatgt tcctccgggg gtcaggcact 81120
gtgtttagca ctttgtcctg acctcatctg acttctcagc tgtccctgag aggtaccagt 81180
gtgcaagatc gctgagttgg caagtgatag tgacaatatt ttcaccccaa tttctaattt 81240
aaagaccccg atttctagtt ttgttttgta ttggatttgc acaatttcac gttctgaaag 81300
aggatgccct caactttgca aaatgggcct tttgaatgaa aaggatcagt catgtcagga 81360
aaagcgctac aatgatgaaa tatgataaat aagtcagtct ttcatctgta attatctact 81420
atggggtaaa aagtgatgaa aactaccatc ttgaaaggtt ctggtgatag tggttcctaa 81480
tgcagtgaaa gatgtgtaag tcaaagattt gtaaccagcc agggaatgag aggcgaagcc 81540
atagctggtg gcgggggcca catctgggtg tggggaggcc acagttgggt tgggggtggg 81600
gcctgcagtt atccacaccc ctcccacctc ccttcgacag tacaggcttc ctggttacct 81660
tccagagagt aaggccaggg agagttgaat aagttgagaa atgtcatgtc gaagctattg 81720
gtggaaagag ttccattaat tgacaataca agtccctact acattctaaa atctggtcct 81780
gactagtggc aagccgggcc caggagtagc acttaaacaa tggcaggctt gtgttgctgg 81840
caggatactt cagcctcaga ggagctgtgt gcagctgggg agactcacac tcagaggatt 81900
tcaaagcaga gggcatctcg tagagcaact tatccaaacc ctgacccact gtaaacacac 81960
acacacacac acacacacac acacacacac acacacaccc tgagagagag aaagagagag 82020
agataactaa agagagagaa ctaaagtttg gcaaaataat acatgctcta atgaaggttt 82080
attaatgatt aatctactcc tagcatttcc tagtccactc tatctcctta aaaaaaaatt 82140
ctggttgcag cccactaact tgattgtaca gctgcttaat ggatagcagg ctgtaatttt 82200
cagagaactg tttaatgcgg gctacctctg ttcttccatg ctgcttgtgg ttcctgctct 82260
gctcaggaca gaatggggag gaaaacaggc tctgcggcac aatattggca agtgaaattt 82320
tgtaaaccgg ccctcccttc cttttgcatt tggtctgaaa attcaattag atgctgagtc 82380
ctacaatgta tttgagaagc ccaggagtgc cctagaggat gagactgggt ggctccctgt 82440
caggttgaac atttgcctta attactttgg caagatttgc atcagtggta ttagtccctg 82500
cctcacttgg aggcctgcac ttaagtggcc acattcaggc tccaatttcc tggtgatttc 82560
atagtgtagg gcacttgcaa tcaaaactag gcttaaagcc caaccctctt acattttacc 82620
cacccccaca aatgcagcaa ataaaatgac tctgattttc attccctaga cctcttttct 82680
atatttatta cattattgtt aagacagttt ttgaagaaag ctgttttatt taacaaaata 82740
gctttatgga atcaacttca tatatcttct ccgccagatc aaaacaagct cgtagtatta 82800
gatgtcaccg agcaccatga caggcagatg aacatcatcc ctgtgcccgg ctaatgatag 82860
ctcggcctgc cccggcgtca gccgctcctg gcagggccag cgggcggtgt gggaccggca 82920
ccgtatctcc agcaattcgc agataacaaa tatggttctg atgatgttac taaagatctg 82980
tccctttcaa gattggatta gacattagga atttggaggg cttttattg ctagcatttt 83040
taagaataac caattagagt attgattcta aagtctgaaa gccacatgga cagagttcat 83100
gtaattggct actttatgtg cctcttccta gattgccctg cattttcaaa acaagagcct 83160
ttctatttta atcaaaagaa tccagaatga aatgaggctt tgaaaactca gcctatgttt 83220
gtcttgattt ccttaactga catctagaag aaaatatgag ctcaggggtc cgctgggttc 83280
cttccagcgc ctaagcctgt aagctcttcc tgctggaacc aagctttaaa tgcacttgtc 83340
agtcatgtcc catgagaata gatactgcct tccatgtttt tttgttctga tttccgtgtt 83400

tgaaatgatg aaaatcattt ttctgtgctt tttaaaaatg gaattgcttt tgtgttggga 83460
attgtgctgt tcatttttac tctacctcgt tttggaatca ctaatgtggc caatttatag 83520
ccaaaaatca gtatcgtaga gtgagcaatg aatggcatgg tgactgtgtg agcgaattca 83580
tgccctccct ccccaccgct cgccccgcgt ctcagtcctc agtgatggta aacagaatga 83640
ggaccttctc ccgaccgtga tgcgcctcag ccctacttcc cttgtccttt cctatcataa 83700
aatcttcttt catagaaatg gtcatttctg ttcatatctg tggactgtaa ataacaagga 83760
agtcattttt gaggtgaaaa ctgcacttag actcattcca attttgatgg aaacttttag 83820
ctggtggatg gcattttgtt ttgtcttagt tttgcaagga gttatcttaa tttagggaga 83880
tgaaactagt ctgtgatccg aggtctcact tccatacatt tctctcgggc agtgtggctg 83940
cctgaatcat gcctggatgc cacaggtgct tagccagctg gtcctgtcgt aactgtcact 84000
ggtagctcag ggagtgcaga ggtgccagca gacactatga aattggcctc gtaaagcatc 84060
agttatgttg tgatggtggc aaagctgcag gcgagatggg aagtgcagcc actgagaact 84120
cacagtagag cgtgtgtaac gtaaaaagat gaaacccatt gtacacagct gtgtactgcc 84180
tccttgaagt caaatttccc ccattaccaa ggaaaagttt tttctgaagg gggctgcttg 84240
acaggatgac atctggtgat atcatttatt cctttggaaa tcaatctgtg gaagtgagtt 84300
tccactgact gatgaggaga aaaatgaatt ggcttcaccc agcatccagc ttcttatcct 84360
gggagagata gctcttggtc tgtcatccac gcagctgcct ggtgcaagag ccaagtttgt 84420
gcagcctgca gagcactctt cctgagctgt gggctgccag gtcgggggc agggggggcc 84480
tcactgtgca gcctcctgcc acccactgat catctgggga gactggccta tcctgtcagg 84540
agacgcagtt gcccagacgt tttcaagggc ctaagatgta ggcagttgat ccacagattt 84600
ttggagagtc cttgagttgg agattacagg tgacctcaga ggagggagtg agaacatctg 84660
ggtcatgggt ttctactagg agtccacagt gaaaacaaga agaggaattt acgacaagac 84720
agtccagcaa cttcctttct aacttctcct ttcacatatg ctggatactc caagactttg 84780
catttacatg gacatcacag atccactttg agagaagtag ggtaaaaaga aataaataca 84840
tagtgcttta ggtgtatttc tatacatctt aattgatatg ggattacatt ttcacttgtg 84900
tttactgtac agactctaga cagatcctgc tcttttgcag gtaaaacaaa tatttcttaa 84960
aacctagaaa gacccaaaac aatttaacag aaacattttg gaccattttg gaccttggca 85020
gttaggcccc agtgcagcag cggcaaccat aaacctctcc ataggtgctg aacccaggtg 85080
atccctggca ccggcagcct tatgtcaggg ctctcttatc gctggttttt atttctccta 85140
ataaaagtga ttaaaagatt catcttttaa agaaagcaag gacacagagg tggattctcc 85200
ctgacgctag cacagctcat gcccaagcca ctcctgcagg gctctggtct aagtgcaaaa 85260
gctggaaaag ctgcaggtcc cgcaagacac agagcaaccc tgcaagccag gtcaccttcc 85320
ctcttctctg ctgtccgact ggccctccac catgtgacat tcaaaagctc aagttactta 85380
acctctcaaa actcagcatc cttttctgta cagtggggaa gatactggac tgttgtgagg 85440
attaagtgag gagagtggcc caatgaggtt gacagttatt actgtcattg tcattatttg 85500
ccttctcaca ggcaggcgtg ccacagtcat tttactgaag ctgcttcagt gggtcctgaa 85560
ttaggccctg tcctttggga gagacagtcc tggttcaaca cacagctccc tgcccagggc 85620
agcttgggag tgtgggccag tttcgccttt agaaccacaa ttctctgata tgtgcaatga 85680
gagaattaat tatagactca aaggattgca tgcagacaca cacagataca aacacataca 85740
cacaacacac agagttacac acagacatgc tcacaataca cagaaataca cacagacaca 85800
cgcacacagc acacagagat acacacagac acacacacac acacacacag acatacgcac 85860
agatgggcac acacagagac acactcacag agacacacag atacacacag gcacacacac 85920
agagagacat acacacagcc cacagggata cacacagaca cacagagaca tacctacaac 85980
acacagagat acacacagtc acacacagag agacatacat acaatacaca gagatacaca 86040
cagagacaca gatacagaca cagacagaca tacacacaga cacgggcaca cacagagaca 86100
cacagacaca cacaggcaca cacgtgcaga taaggtaata ttagctagtt caggaggaga 86160
aagagataaa gataaagtaa tattagctag ttcaggagga gtgaaagaag ccttgttttt 86220
ctccactttt tatagaagag aaagtgaaga ttcgatttga ggtgagttca gcacaaaagc 86280
gtatcccagg ccctctggct ccaactgcag ccctttctac ctcattccca gaccccacct 86340
aagccttttc tcttcaaaat cttctcaggc acactgatac acatacctca gatttttaat 86400
tctccggttg tgttcaccag gtgcttggtc atgattaaga attccgtgat gtgtacccca 86460
tgtgtttaaa tttgctgctg agttaacttt gtggcggcct gtggactaga cctctgcaca 86520
tgcaatgcag aacggcaggg ccagatttga aatcctgcta tcttttcggc tgccttgtaa 86580
aaataacatc aggcgatggg gatacgatgc cagaggtcac ctgtgataag ttctgtttat 86640
ggccatttta cttctaggaa gacaggaagt gtcaggatct cagggatcta ggaagccaaa 86700
atgtttttcc actctgaaat aaagtgactg accaggagtt cccggccacg cagccctgtg 86760
ggaactgccg cacggccact tttatgaagt ggacacgtgt tggtcccact gaaaagaaac 86820
tccccaccca tggctccctc acgctgcagc agaggccctg ccacagcacc tgtcagcccc 86880
tgccagcttg caggggcgca ggcgcagagc ggtttgtgcc cttgctggag ccagggaagg 86940
gcacagggtc cctcctggag tcatgggagg tgcagccgag gttctatatt aaaatacaga 87000
ggctagcaca tgtgcttggg gaatgcagct acagtagtgg aatgaaagtg ctgtccgttc 87060
cttacccccc cagctcctca cctgtcctcc acacgcatat ccctggctcc ctttccctag 87120
taaggagact gaattgaaat tgtggcttgc ccgaggctgc atacctgtgc tctttctgaa 87180

gcccaagtca ctggctctag aattctaacc tgtgaggaag ccactgagga tgtttgtcaa 87240
aatacatatt tctgtgcctt gccccagttc cacggcccag gaatctgcag ttttcacaag 87300
cacccccagg tgattctggt ggtgtctttg cacttcttca aggcagtact gcctggaacg 87360
cagaatccca gcctcctcta tcctccttgc ctaatggcct ggatgctctc agatctacag 87420
gggaagggaa ggtcacacag tcatcgcaat agtaacctca gctgataaat cctcccccat 87480
aaaacttatt ccccagtgtt ttttaatagg aaacaataaa actgtaacca gcccaaatat 87540
ccatcaaaga gaaaatggag aagtaaatca tcgcacattc acctggacca gatctattgt 87600
aaagccaata atactgaagc cccttccaag gccctgggag tcctaacagt gcactggcag 87660
tgtctataat ttatattatg aaatttgcat aaggaaaaca ttttgtctca tttgtgcaat 87720
ttctccttct aaatatacgt gtcactttgt acctgatttc tataagaccc aggacctaca 87780
aaccctgtgt ctgcccctgc agccacccag ggaaggactg cacagcagca agacagattg 87840
ccatggagca tgttgtgccc aactagggac agcgcagata gattctgtaa tttgcctaac 87900
aatgtctata ggatgatccc atttgtcaaa aaaaaaaaag aactgggctt tattgatgtc 87960
acctaaatgc acctaaactt ctttttgcc ccatgctctt ctgtactctt gatctttccc 88020
caaattttta aaaacatgac actcattccc ttatttttcc tacttagaaa agtgtagatg 88080
gttttatcat aggaagttca aaaaaattaa aatataatga aaaatactca aatagtgcct 88140
cacaacagta actactgcta acataaataa aatccatatt tcctctcata cagaccccag 88200
agttgctttg cctgacagtg tagttgatgg agaaaataat ctttatcctt agcctccatc 88260
tggttgcaga ccataaagac agggaaaaaa tgagggtgtt ggtagcttcg ttagaaactg 88320
aaagctcact gatttttca aaacctaaat agcctgtgtt tctccaaata actaatttgc 88380
agccttcggc agccaggact ggcagggatg gggctagggg gactggggag aactgctctc 88440
tcctgagggt ggtctgaccc gacagcacgc atgaccttcc cacagtcagg aactgctcag 88500
agacgtgatg gcaactccat agaatgaaat actcttcagc cagtaaaatg tatttttgga 88560
taaatatttg ctttaaaaaa ctttactata tgttgttaaa tgaaaaaaaa accttaaggc 88620
atcagaaatt atgtgcagta aaatctcact tttgtaaata aatatacctg tttactacgt 88680
atgcataaaa agaatcctga gaaatataag tactgtatgc atattgttgt taagtatttt 88740
ttctgtttgc ttatctataa ttctaatttt gcttcaaaga acaagttact ccggcaatat 88800
aaaaataaaa taactaattt gtcttgtcat caaacagata gtaagaacag gcaaacctgg 88860
ccctccacac tgccagcctt ttgtgattca aggcttcagt ttcctccact tgttaaaaag 88920
attcaacaaa gtagttgaaa tagtatgtga accagtaaac cctaaaaggt gtccagtgtt 88980
gtctgtgagc taattaagtg atttgattct gactccccga gtcttctgat ttcgaagcag 89040
tggggagtca gacaggagcc tcaggtggcc tctcctgaga ggccctggaa agtgatgaga 89100
acctggcctc tggcagctct tcataaacgt ccatgttttc cctctactct ctcactcttt 89160
tcccagggcc tcaaacagaa gatgaaaatc aatttctaaa acagccctct gtgtgctctc 89220
tcgtatctct ccttttcaca catcgtggtg gtggctttct ctgtgttcct ctgttgattc 89280
agtctctgga attaacggat caggattcca tgcccagaat gctacaaaga ctgtgcttga 89340
gttctcccac atctcactca attacacaga agtttcagat tatgtaacag atgctgtgct 89400
gggttaggca gagccatctg acttgttttg ctttatttta gaccatgaga tgggtgagtt 89460
tttcttttta atgccacatt cttttaagaa ttaaaaacct ccacttggct gtcagcattg 89520
gaaatcagag tgatggtgca agccctgatg aggacaatgt ccttgtctat gaaaaggtga 89580
aatcattgct tgaaatcgct aagcaggaca tgcagtccca gatggagggg ggaattcggg 89640
agctggttgg aaaagagtat ttggcacttt gcagccttga gaggtgcaga agagacaccg 89700
aggggttcac caccagagcc accattgtca gagaggcgtc cagctgtgtc cacctgggac 89760
tctgccttca gggcttcttg cctggctggg agctgcacag gcagactcct gggacggtgt 89820
gccgacagct ctgggcaccc ccttctagga tctgattcct gaggaatcac aatgtggatt 89880
tcacaatcac ttccagtgtc ttttgccaac ctctgtgaac agatgtgcaa ttaaaaaaaa 89940
aaaaagaaag gggcccaatt ctcaacactg taagtggaaa ctttttaatg gaaaaggata 90000
ggctaatgaa ttgaatttga aatctgagac agaaccgatg catcaaatgt gctggtgttt 90060
acagataata caagggggc tgcatcttat ggtttcaatc ctttttaaa tttttgttct 90120
gagagaccca gccagcagac tgccgccagt cttgtcagag atgtcagtgg tggccactct 90180
gaatggaaag cagcatctct cagcatctct gaggcactgc tcctcagcgg agactgtggt 90240
ggctttgcct ttcagcacgc atcctttcta cgatgcctga cagtgcccag ggaatgggca 90300
gagctgggag ctctgaagcc ctttcaccta aaccaccctg ggtcacctga cctagttttc 90360
ctcccaattt taattatgtc aggcacttca caaaggcctc cttggggaca ccatgagctc 90420
actgtcatca gattgctcca atcacagctg tggcttgcac acaaccgcca tctctgcccc 90480
agcagatgct gtgtgtaaac agttgtatta attacatctc aaaaacatgg ttcttgccag 90540
atcctcagga tttgggtgca gcctctgagg tgggtgggag gccctcgagg gagaaatgtc 90600
tgcaggaaat tcttccccta cgagaggtct gttttctaag ttatctaaga gctactgcag 90660
ctgtttactg cagagtgacc ctgctcaaag ctgtggtcac ccaaggcttt gaaaggggac 90720
ctccacttcc gccctgggtg gagcaccgtg ctggagaccc acgcctgcca aggcctcatt 90780
gtcatctcca cacgccgtcc ttggggtggg ccactcctgg gacacgcaga caggaagccg 90840
gccacctgag ccactcggag gctctatcca gagtcagctg ccaagcctca cgtcacacat 90900
cactgttagt cttggagggc tggcggggcc ctgaagtcaa ttgaacactt ggatgacagg 90960

gaacttgcca ctgccagagg caatatgctc cattttttg acagttccaa caatttttct 91020
ttaaactgtc ataaaaaatt gctgctgtga ataccagtgt cggcgtccct gcctcacctt 91080
tacctggtgc ttttccacca cacaaaactg tttctcctcg tgctggcctt gggcttgcag 91140
acagctgatt cttctcctcc cgcggctgag cagcctcctc cgagcaaccc tctgacaact 91200
ctgctccttc tgacaacctc tgcaagggct gccagatgtg aacaaggggc ccgggcagaa 91260
ggtatccagg aagactggaa actcgaggaa gcctgccctg tcctgtccac cagactttac 91320
gcttgcgtca ctgggctttg ggacctaagt cctcgtcatt tgttcctttt gcagttccta 91380
ctgttctcag cacttccttc cagcttactg aggtacactc agatgtgata tgccatcggt 91440
acagacacag ttctgctcca gcatttcccc gtgttctttc tgtcgctcta tttactgaat 91500
taccgtgagg atgtggagcg aggctgagtt ctgtatttta acaccatttt aattctcacc 91560
tactgagaaa tccatcctct tatcactgtg ctttttttaa cctgtcacga atccatgaaa 91620
tcctatcagc cagcctgcat acttcctttt aaggtgcagt tgaatcagga gaaacttgcc 91680
gcacatgctg cgtccgggca cagcattggc tgaggctgct gccctgacct gtccgctttg 91740
tagtactgcc cagctatgaa acaggttagc cacacatgac ctgcatttag gagtaacaag 91800
tctgtctgta catgcacata cagcaacttt tttaaactgt ctatattttt tcctgagata 91860
ggtatttata atatctccat cttctttccc attttgaaac ttagaacaag tttgcctgtc 91920
aacagttctc cacagcatac tgtgtattct aggattttct aaggttgagc aacggaggtt 91980
cagcaatttt gacttaattt cttcccatcc cttttccacg cagcccagaa gccttggatc 92040
acgtggtgag gggaagaggt tgtgctatgt cgggaaactc tgtatcgaag ctcggctcag 92100
atcatgacat tctcttgact aaaaccctca gtttccatca aacttgtcac tctggcatta 92160
aagcctgtca ctgtgtggct ctgaaaacct ctctgaacgt gttccctgcc tctgccctgc 92220
aggtccctgt gctccacaga agcccactta tgtgacccac ccccactcat caccaccttc 92280
cctcacccag agcctcagct ccccactccc acctgtaaga cccctactgg aaagattccc 92340
acctgcccct caagattaat ctccaaggac atttccaaat tcctctcccc atctctcagc 92400
cagatggctt tgctccctcc aggaacccca gccaccttcg acctccagca gggcactcca 92460
ctccacattc tcctggtctg tctggctcat cttacctgag ccatgctctc caggtgaagg 92520
actatgtcta actcaactct gctttaaaag cagctaacac attgctcttt gcatattgtt 92580
cactcactaa gttgaactgg acttggacat gcacactgaa ctgcagcgtc tgctgcttct 92640
tggtggccca gctcgtcaaa agaataagat ttcagcaaaa caatgtaaca attttttttta 92700
ccaaaagtaa tgttaacaat atatggtttt cccctgatgt ttgcgtcaaa atgcttttg 92760
gaaaaaacat ttttcaactc tttagggtca gaattaagca atgaaattta tataccacat 92820
gtataatgtg tatgtttatc taagtatctg ttcatttata tatcttaaat agaaatttta 92880
aaaatttttt taaaactcct gataaacatt ctcaggaggc acactatgta actgttggtt 92940
gatataccta gctagatggt gaaatcagat tttgtttaaa gcatggagga gagggaaaaa 93000
ttaaatcttg cagattctgc agtccttaac atctttgaaa gaggaacatt tcagacaatg 93060
taataagaag gccacgtgct ttgacttctg tagattttaa aaatacttct gtatagtttc 93120
ttcttccttt gaagaagttt ggggagtttg ggaagatgga gaaagatata agaatagact 93180
ccccatatgg gtcatgaatt atctttttgc atcagaactc ttagtgcagt ttcagtattt 93240
tcttcctcag gagggtgagc tgcttccgaa tgtcctcccc ttctttgagg catcctctgt 93300
tggtgaactt tgagagcatc catttatgaa gttgatgacc tttcccagtc tctgcaagcc 93360
cttcagtgtg tgtcctctct gagcaaatct gaattgtgtg cttaatacat ggaaagggat 93420
ttgggagggt tgctttttaa actgatttct taattaatat tatggtttag ttaactagac 93480
agtctcattg cagaagtgca taaccataat atgtcttcaa atatatctcc cttcctaaca 93540
ccctgtaata tacttttgta aagataccct tacagaatgt gatccaccat ttatgaacct 93600
gcagcattgc attcagagac taagtgaaaa gctggcagat tttcatttaa agcacaagct 93660
aaggaagaaa gctggtctag aaggagctac agaagggtaa tgcttaggga gggaatgatg 93720
tgcctgtggg tggtggtagt taaatctaac caaagaatga tgtcgtgggt gtttggatat 93780
tggatggtcc acattgggcc acattctttc aaacataaga gtctgtagaa atatgacctg 93840
taaaagactc ttaaatattc tggaaactgt ttcttccttg tcacatcctt atatatactt 93900
gaacctatgc ctaccagaca tgacatgtga ctattcatac agatttcatc atctctggtt 93960
taagaataaa ggatgctgca tagaaggctc acatctttta attcacaaga ctgaaactgt 94020
tctgaaatga cattgtttct aaaaattcat tacttgcatt atattcattt ttattttcc 94080
atgccagaag ggtagaagtt cctgtgctca tattaagaaa cagcaatgtc aatcgaggcc 94140
caactcaaat ccaatttata ggagttataa agggcgtgtg cctgttttgt ctagaagcag 94200
tgttgggcag cactgagtag gatagaccac ctgttgctac cgataaagga gcagcttctc 94260
gaatgctcct gtctggtagg cactatcccg agtgctttgg cccctcatcc acaatctgtg 94320
tggcaaaagg cattgcaggc aattcagtga ggagaccgag gcatggagag caagtgccat 94380
ggaattccct aaggccgtgc agggagcagg ttgccaagct gggttgaaac cgtcctccgt 94440
aggctcccaa ctccgccgtc gctgctactg tgctggatga tgcctggtag atgcagatgt 94500
ggagccccat ggattctgag acaggccggg tttcagtcct gccctagctg cctattggct 94560
ggatgacctt ggcaagttga ctttcgtgag cctcatttgt ctcatctctc aattaagaaa 94620
acctagagcc tatctgtggg ggttatctga aggattccag ggatgcatat ggcactgtct 94680
accgcatgcg gtaactgttt cacaaatgat gaggagcgat ttatgttctt agtggaaata 94740

tgtcggcgtg tgaagtccca aagctctgcc ctgcctggct tgatccagtg cctaggcact 94800
gcccctcttc ccctctctcc caacccactg taagaggcta ggctgcctca gtaactctga 94860
ggggcattga ctcttttcat ccaaaaattc atgttactgc cccacatttt ttctgttgtt 94920
ttacaacgca gtaggaagtg ggcagactgt caggaaaagt gatttatagt catgtattgc 94980
ttgtgctttg gcttcatttg atccaatgca gatcagctgc actcagaaaa ctactcaagt 95040
gaaagagaaa aagtaactga agggggaaat ctggatgagt aagaattcca gggataggaa 95100
tattaatagc aagctttttg cctgatatag tcactttatg ctgcaggggt gcccctttat 95160
aaagtgcttg tacaatggat gtttgctttt gattttggat ttggagtcta atgaatgttc 95220
taaattatta ttagaggagc ttgcggttgt tacatgtctg cctttattgc ttatttttag 95280
ccatctcccc tgatgtcaaa tgctcaggca agaatgatac attcatttat aatgtggctc 95340
cttcagaaat ataccacata ccttttggtg tggtttgtgg ctgagaagag tggggaatgc 95400
acaagtggaa aactgcagaa agattatgcc ttcatcactt caagtatttg agatgaaact 95460
agatcatttg ctgttgcttt ttattctcat tctaagtgct tttcaaagtc agcgctaaga 95520
ttttaaaatg gttttctgtt gttggcagag agggaattac tctattactt tctgataaaa 95580
cagagtcttt catgatcaaa gagaaccagg ctctagtagt tccagtatcc taacgtggac 95640
actaattgtt tccctccttt tcttcatgaa aacagcttct gcacaaatga tagccttgtg 95700
aactagccat gggcacaact ggagaagcat ttagggagct ttagtgcaaa ttgagaccac 95760
ctacacatct gactctacag ggtttgacaa catccagggt gaatcacaaa acatcagtct 95820
aatcagggct tatatagaaa gagtgaaaga actctgattt catcctaaag attatttata 95880
ttaaccattg ttccaaatgc attaactatt ttaatttagt tgttttgatt gttaaaaaaa 95940
acacatctgt ttggtagata agacataatt taagacaaat gttctatttg ataagctttt 96000
agaaacaact tatttttatt ctttcctgtg agataactca gatgtggaga atgtgacaaa 96060
attttaagca taacatgaga agggctgaca cacatagatt tctgtgtgct tacttgaaaa 96120
caacaaaatt taagaatttg gtataggagt tgtatcaggt agtgcagagt ccccaggaga 96180
cctagagacc caggtctggg agcctagcgg caagggctga atgtgggatg acatcagcag 96240
aaactcacag ccactgctat tccaaaaacc cagcagcagc tcagtgcagg gcagtgctga 96300
tagtacagtg cctgcaatcc tggagtggat ttggatgtgt caggtacgca cacgctcact 96360
gctcccccag cagtacgttg aacagtgtgc gtccaggtgt ctgtagggcc cctcgcccta 96420
actcacaaaa ccattctggg tcagaagcca ccaatattgt catcatcctc ccttttctga 96480
gaaccctagt aagtccctcc agtggggcaa gcccaccttt tcccttcatt ctgtggcaat 96540
atgccttcat ttcctaatca gttttgccct gctcattcaa tgcaaaatgg atctgctttc 96600
cttgggcacc aatatgtcca gggattgttt atcaatcttc agttctgttt cctttacata 96660
tccctccaaa aatcaggcct gcactgcctg tgcactccac aatccacagg cctgaaggaa 96720
atgttatctt tgatgtagag acttaaagta aaactcttca aattaattat ttcatgcaaa 96780
aggctagtcc tgactctaat tctaagacat gtctcctaaa ctctggaagt ctgatgtatc 96840
ctattatcaa catttatcct taatgtgatg gtttatcatt tatcctcaaa gctgcattgt 96900
aaaatgtaca ctgtaaagtg tacattttaa agtcggtttt aaaaaatcat atttagagat 96960
cctggtaaaa atctatcaag tcaagacatt accttattac ccatggaatt gtcttcaact 97020
cttacagttc aaatattcct gaattggctt tcacaataaa catcctaaat atgtaagtag 97080
aaacatatat attgccaact ttgtgccttc ccaagcaaaa ttaaaataca ggaaaagtca 97140
gtttgttttg cccataaata aatatatgtg tgtgtgtatg tgtgtgtata cacatacaca 97200
ctcagaaaag atagaagcag cagcatattt tggcagcatc tggtttattg gaactcaaac 97260
gttctgattg tgcatacaga ctagttaatg tggtaacaat tatgtatttc ttccctgctc 97320
cttgccttct ttccctcccc agtttttttc ttcctgatag taggtgtgta ctttttttcct 97380
atttccattg gcaagccaca tgacaagcaa aacgatcact cgaagaatat tgttccctca 97440
atcaagaaaa atgcccattg ggttttgtta tttgatgtta tttgatgaca gagacctatt 97500
gtttttccat ttttcttttt ttgttttccg tggcacctat ggaattaagc aatataaaaa 97560
atctattatt tcagatgttc acgtctaatg aatttcatgt gaaatactgg cagtataacc 97620
ccaaatagag gaaatttgtg aagagtggat gctgcagggc atgagacatc tgcacagagt 97680
tcatctcttc cagcatcttg catgtcccaa gcactgccct gccaggcaga gaatgctgca 97740
gatcacggca gtgaattcca gttgttcaga gcacatttga cttccaaatt ctcaaggcca 97800
cagatttgag gacagaacaa tatttgcatt tgaaattgga agattatttt ttgcacaagt 97860
gcctatatgc tatatagagt ttgcccactc tgcattatct tccccctgtt cccccgttat 97920
ctggcacaag ctattcaaaa gacacgccta cttgtaaaat aaatggtttg caaactaagg 97980
aaaatactta aatctcatgt aaatggtact atactatgta taaaaatgtg aagaaacaca 98040
gaacagctca tgaacacctc cactgctgta taaaagaacc atcttttttc tggctcctat 98100
tggatgcctt agaaaaatct gtatttcctc tttagttatt gtgtttgaaa gatgaagttg 98160
agacaaaagt tctattcttt ttaagttggc agaacttctg aaaggtgatt tttagctgca 98220
gtgtgactca ttccaaatgc agaaatctct gaccctgagt tagtctattt gtcatgcaag 98280
agcctagaaa agccctgagt gataagaaat ggccataggc cattcccaca gaattttcaa 98340
caaaaataga atcatgctta tgttctagtc atgacttaga acttataact catgttcgga 98400
actgtccatg ttcacgcaca ggggccgtat cactccgcca gagctgccct gggtgccggt 98460
gtgcagaggg gtccgagagt gactgtctct tcctctgttg tcgaatgtgt gggttatctc 98520

cataaatggc tgccatgagc atccttgttc acacattttt aggtacttga gtgagtgtct 98580
gtggaataat tttgggaagt gaaatctgtg gtcagaggtt tgtgagtttt acatgctaca 98640
ttttcagaag ttgagaaata gcagtaggct gaaggcaagt cgccatgcct ggaattcatg 98700
aacactagtt gaaagaactg gcgtgagtta gtcatgacag gagagatggg gaagggagtt 98760
gcaggtagga gggccatctt caaattctca aagtatagtc actccaaacc aaaattcgat 98820
ttaatctgta ggactccatt ctcaaagcac agtcactcca aaccgaaatt cgatttaatc 98880
tgtaggactc caggtggcag aataagaggc aatggatggg tggaagcgaa acagggccaa 98940
agtttgactt catgtgcaac ttcctaagga gtgatttgaa ctccacaaac atgaactaag 99000
cacctcaaca caggctgggc aagttgctgt tcttttggag cttacatctt agtggggaaa 99060
gagaaatgcc tatgtaaaca tataaatcag caggatacat tgtgaggacg gtcattgctc 99120
agtgagactg caatagagtg atacgctgga gggggctgca agggagaagg tgggagggac 99180
agcatttagc agaatgagca gcacagtccc ataggaagaa gaatttattg cctccttagg 99240
caaataaatt cccaaacctt gaacatcaga aaggaaatag attaatgtgc acagaggatt 99300
aaattatgtg atctgcaaag tcatttaaaa tctatttcca cataaaacat attaatgcaa 99360
cctaaacaaa aggggtctgg ataccctcat cttcttccca agcatcaagt ctttctatag 99420
ttaaactgag atgcttttat tcttggaaaa ttttaaggac tatctacagc aatggaagaa 99480
tcgggtgttg ggatgtgttc ccaggtaata atgactgcag gctgatttgg cccttgaggt 99540
gtggcctcat ggccctctcc aaaaaaaatc aaggacctgc tacaaagcac aaagccgact 99600
gcaatgcttg ctgcttactg gttagggcag ctcctctttg ccagcgacca agcagaaagc 99660
aagacaagac aggttctgaa gcagtaattc aaagccttcc tcgctttccc atgtgagtca 99720
ttgctagtca gaatattacc tttgcagaga ggcttaattc caaatttgct cttaaaggga 99780
tatcctctcc tggtttaggt ataaactttt gactcacagg acaaattcta tcattccttt 99840
gggcctagga ttgcatttat ttccatgaca aaagggcctg tctggtgttt cagcaaatga 99900
aaacaaaaat ataaagccca tctccttttg aatgagctct aaaacagttc tccactggac 99960
ttcagaacaa gagggagctc tgggctgctg gctggttgtg catttgctgt gggttccctc 100020
cggcaggcga cctctccgcg ctgagaaggt tatccggata accaagtaag aaagtacatg 100080
aggaggcaca gaaagaaaaa tgtgagagat aacagcataa acacacagtg tatgttgtta 100140
tgaggcatca catgatgaga tactgctggg gagggaagaa gtgaggagat tcctaggaat 100200
cttatgagaa tttccagaga caacaagttt tgagcttttt tttaatttag aaaatttacc 100260
ttatttttaa aagaatatgt aacatatccc atgctataaa attctagaca tagtagattt 100320
aaaacagcat aatggaaaat ataaatatct attttctttt cctatttatg tattctgtgc 100380
cagtaggaat gtagccaaaa agagagaaaa ggggtctctg cagacatgga tgtctctgtg 100440
acttgatcac tgctaaccca agaagataat aaagcagaag catgtatcca ggttgctgca 100500
gccaagcctg cccggtctgc ggggcgtcct cacacatggg gcagctctcc caccccacac 100560
actgggaaag gcggacagag gctgggcaaa gcccccaatt ttcgttggca ctgaccccga 100620
tgatttatag gcctttgttt cccatgttaa atgtcttacg atcattaaat tatttatagc 100680
tcaattagca tgtgtccaaa accaggaagt tcataggaga ctgtgtgact gggaattaag 100740
gagcaaagca actttccagt ctgtgattta ctgggtttcc attctgtttc ctgttcggat 100800
ccggaagtag aatttcaaat attgcttttc atgctttatt tgggaccgat tttagccccg 100860
ctctcctttc tcttgccatt cgctggccat tagccaccag cctctgcaca atgaccagct 100920
ggcccctggc agatcttggg cccaggtgtg aagtcgctgg agaagcattt cagggccaag 100980
atgggagtga tttcattttc cattgacact atgcagaaat gaaggggatt caagtgcctt 101040
cagaaaagct tccttccagc gaatggagtt ttgggggttt tccagacttg caactgcttt 101100
tattcttgga agcatcattg ttgcttttc cccccttcca tttatatccc aggaactgat 101160
tcagaaacca tagaaattgg atttggaatc gctgaatgct agcagacagc tgactgcact 101220
cttcccaaga aaccctgcca gctgggttcg ggtatcgcgc ggtgtgtgct ctctctgcct 101280
ggcccgctga gtcctctaac tctaatggat tccttcttac accaaagtgc actagaacta 101340
aagtgttttg cttcattctt tagacatttt gtggtttagg gctcaatcag ccagggtatg 101400
atttgcaatc cacagtaacc ggtttcagag cagctgccca gcgaggcagg tttcatctcg 101460
cttgctagac gttttgtttt ttttttttc taaacctcac acctttttatt tattagactt 101520
ggattccagt ttcctgagcc tgtttgtgcc actgattaga caggcttgaa gcagaaccca 101580
ccaggcttcc tgaataaaat gcagcagtga ttgtattagg gggttttaaa ttgctcaaaa 101640
tactgtctaa aaaacactaa aaatcatgtt actttctaga ttgaataaaa tcctatagaa 101700
atgaattcct ggacttgata tgtagcaagc tggcattggc tcgggagtga gtgggctcag 101760
ttaagtgagc taagatgaga tggtgcacag gcgagcaccc acctgaggag tgtttggatg 101820
ttatgatagc cagctcctct gtaaagacct gtccttctat gtcagcagcc cagcagataa 101880
atgacgtgta aataccacat ttaggagggc ttatgatgat gccaattaat ggagaccttt 101940
ttgaaacagg aaggaggtga aacatattcc tttgcttcta catcactgtg tgccaggcac 102000
tgtttacagc atctcgttta accagcagtc accacctgac ggatggctga tgtggggtgg 102060
ggtcccaggg tgggattgcg tgatgggctt ggggtctctg gctgatgggt gccagagctg 102120
ggactggaac tcctggcgtg actgaggcag acacctgggc tacccagcct cacccacgac 102180
gccctcacta agtgacccac aggactcacc ggaagcaggg cagcaaggtc cccctacaga 102240
ggtccccact gcaaaccgat acccagctta gacagcagtt ctgcagtcgg cgtctcaccc 102300

cttcgggtct cattgtgact cactttgata gccacacgat ttaagggtgg ttcagtagtg 102360
atttgatgag tgctgtggct cagggtcatt cccctgccca agcatttcaa attccagaag 102420
ttcatgccct gcatggtggg tgaaaagtct caggccaacc atgagcacac agcagccagg 102480
cgactgaggc agctgcccgg ggtggcacgt tgctcaaacc catcatttgg agtcaaaaca 102540
aacagatgat tagctggggt ggtcactttc aatcaagagt tttcacatcg cctagacatg 102600
gcctcagaat caggcctggt gtggccaggg gctgatctca cagtagacag gaagtgtggc 102660
ccgagggcca tggctgcccc ctcagaaggc cctgtggagt ggctggccga gcctcagcag 102720
cctcctgtga agcgaggaag ggtcttcctg ccggcctctg gagatcagta tgggaatgca 102780
caagtaggaa acgctggatg ggaatccctc tgccctgtga taccaaggca gtgagtttgt 102840
agactatgga attgctgtcg gagggctctg taaccggcca aggtcacaca ggtagccatt 102900
ggtagagcag ggactggaat cccagacccc caacttccag gactgtgcac ctttctttat 102960
cccatacagc cttacagtca agtgccagtg caacacctga ttcccaggtt ccagcctttg 103020
tcttttataa tgggaatcaa ccttatcttg acgatccaga gatagtcatc aaggaagatt 103080
aaattatccc cttagactca gagtgaccat atcattttcc ctccacacaa ggacactttt 103140
gagaatgaaa aggaggagat gtctgtacca gacgctggat gacaggcacc gacaggctgt 103200
ctgccagggg agcagcgatt cctgtatgtt gtagaaagtt tttcaaaagt caccttggaa 103260
agaggttttg ttccttaacc ttctgttaaa taggaagctc cgtgaatgaa aacaactccc 103320
ttccctaaac attctagtaa tgacccaaca ctgccaagcc tgccagctct gcctcatggt 103380
cgtgttgact gtgtgagact atgtgagtgc ctgctacaca gtacgctttc agtaaacatg 103440
gtattgcctc gataatccca caaaaatgtc ctattcaaat cacctggcac ccaggaaatt 103500
tccttctttt ttttcccagg tgaaatatac agttgaaaac acctgacagc aattcccctc 103560
tcccatgtgt ttgcaggatg gtggttttgg ttcctccatc tttgatgtgt acaagtgtga 103620
tgttttcccc ccacagacaa gtaaaccaca ttctcttcac attcccaatg ttttgtcaat 103680
gtacctcctt caatagagga tcgataagga aaaaaatcat tgacaatctc aattagattc 103740
actatttcat ccaaaagcat agcttagaac tctagttttt gttcaacact cttgccctat 103800
gagtgcacag aactttaatt ctgatacaaa catccctgaa tgtttagctt tgacagagat 103860
tccaaggtga tttgataaga agcagggctg tgtttgggct ctgggagttt ttgatatggt 103920
ttcaagcccc atccaaaacc cacagacctc tagaaagtag gtgcctgcct tcctgcagca 103980
gccctggagc ctgctggggg ctttgagcag ctgctgccaa gccaggcctc acccgacact 104040
ctgatgggca cggccatggt ggcaggggct tggacgctgc caggtgactc taacttgtgg 104100
ccagggtggg aagcactgct ccacagaggt gccaaaacca ggttccttcc tgtgttctca 104160
catttcacag cctcaatgta aaaagtaaga catgggcact ctggaatatt acaaaaatat 104220
agaaaagcat gttatagtaa ataaaaggct cacagaattt tgtcatttag gaacaatgat 104280
tattaatata ttagtgtgtg tttttgctca ttaacagtat atcctgagat atttcctata 104340
ccatttaata ttttaaaaga tgtttacact ggccacagta gctcatacct ataatcccaa 104400
cactttagag ggcaaggcag gaggatcact tgaggcttaa aaattagcca ggtgtagtgg 104460
cacatgcctg tagtcccagc tactcaggaa gctgaggctg gaggatcact tgagcccagg 104520
agttcaaggc tgcagtgagc tataattgca ccattgcact ccagcctagg tgacacagtg 104580
agaccctgtt tctaaaataa ataataaata aattaaaaca tttaaaaata catgatgttt 104640
aattattaga ggactcaatt ttatatctat gtatacaata atttttaagt ttcttaatat 104700
tggactttta gtaccttttt aaaaatacta tttttaaaaa aatctgtatt tctaactttt 104760
tataacaagg aacctttggc tttgagatga ctggggaatc cattctttcc tatagtatcc 104820
atgtccaatg gacttaaagt attaatcaat gtgtttatgt tttgttattt ttctggcatt 104880
acaaaaaatt ctaaatatat tgttaccgcc tgtataaata tcagcttttg agagaaggac 104940
attgtgtaga aataatgaaa cactgcaact tgtatttgta ttattctttt ttttttttt 105000
tttttgaga tggagtctcg ccctgtcacc caggctggag tgcaatggtg cgatctctgc 105060
tcactgcaag ctccgcctcc caggttcaca ccattctcct gcctcagcct cctgagtagc 105120
tgggactaca ggtgcccgcc accgcgccgg gctaattttt tgtattttta gtagagacgg 105180
ggtttcacca tggtctcgat ctcctgacct catgatctgc ccgcctcagc ctcccaatgc 105240
actgggatta caggcattat attattcttt aaattcacat gagaatttag tatggcttca 105300
aaaaatacca taagttaaaa tatcaccaag actctgttca gacaaaagta tcagaaaagt 105360
gagccaggca ctcacatagt ttatagttta taaaagtgag acaggcatga tctcttaacc 105420
tcactatagt cctgtgaata aggtttattt acatttcatt ttacctgcca ggattattgt 105480
aaaaacgcca agcacattgc ctacacaaac taaatattca gtcaatggct gctattttca 105540
tgagttcgtt ttaacatata tttattgtcc tctactggat ttaagaagtt atatttatta 105600
tcatctaaga ttttagctat tccttctctt aaaaatagat tttataatca atggcagtaa 105660
gggagagtaa ctcgcagttc tctgaatctc aaggggttcc tggaagcctt cctgaaggta 105720
tagtgaaatt tcagcttcac attcccatcc atgagctccc tgcaaatatc ccggtctgct 105780
ctcaggaccc agtgacttac ctatgcagag gctgtagata gcacctggag cttcctgtgt 105840
gccctcctca aactcagcca atgccgtcat acagtagcag gcaggtgtct ttgctgggta 105900
gttggactgg atgtccctgg gattgcagaa ctggaatggg gagtgacatc aggaaactat 105960
aatcatcagg acaacatggt ttgccataac tttaagtttt aagcgaccgc agattatgcg 106020
gagagagatg catgcccaca gccatgcttc ccatgtaact ggagaggggt ctgaagtttg 106080

aaacaagtgt tcctaggcac gggttacagt gtttgttatc atcatacttg atttagaatg 106140
gggcacaaca tgtggattca tggtaactgt tacaacctta ctcattttaa tacctgaaaa 106200
catgctttcc ccatgctggg aatcgaaaga ttctcctagg aaaagaaagg cttgacaaca 106260
tcgattcaaa aagggcatgc attttcctca tttaaataac tctaatgtgc aagtagatcc 106320
cctgacctca agctcagaag agtccaggcc ttcacacctt ctctgcttct gctctggggc 106380
cagctattga gattcctgtg cccacgcaat gcgcacatcc cacccctggc cgctgtccac 106440
aagaaatcca gttgcaccaa gcaccccact ttttgcacct ctcatttatg tactcctaag 106500
agcctcacca caactccctt ctaaaaacat gagttcctga ctgggaattc gatgctgccc 106560
aggcagcttt gctcagaggg agcagccttc tagaaatgtt tcaagtaaac tttcaagtat 106620
aactaaattc aaaaaaaaca catacacaca cacacacaca cacaagtcaa aggtgtgtaa 106680
tttggccaat atcacaaacc aattagccct ttgtaagtgg cacccagatc aggacagctg 106740
accataccag caccctagaa gcaccccgtg ctgcctcctg ggacagggct accaccatcc 106800
taaggccagc acgatgggcc agctttgcct gctgttgaat tttgcttaca tagaatcctc 106860
cagtaggtac tcctttgggt caggttcttt cactcaacat tatgtgttga tatttttcca 106920
tgctgtgctg caaaattgta tttcttgcat tccataactg ggcagttcca tcataggaga 106980
ataccacact gcgttcgtcc attctaccgc caatggacat atgggttctt tctcttttct 107040
tgcagttaca agtttatgaa tattgtccca cgtgtccctg gtgaactttt gtttgcattt 107100
ctgttgggta cctcagagtg gcgttgctgg gtcagagggt actggtcgct ttagtagctt 107160
tgaaagatat tgccaaaaca ttttccagcg cagttatagc aaattataca ccaccagcag 107220
tagaaaacat ctcctaattg ctcacagtaa acccccaaag attgccacat acatcttcca 107280
tatcaattac ttaactattc agcaaatttg aagggaaata tatttaatct ttttattcaa 107340
atagtttata aagtggaata gagatgtggg taaaagttgt cttgccacct ttttagatcg 107400
gtaaaagttt gttgaatgca ggcaagaaaa gatgagaaat aatggtaccc aatgaaagac 107460
atagcagtct acaaggaggg gcatttcccg gggtgggggg gacccacact ctgtaactcc 107520
cacattcaat tagcatgtta taggtaagct gcagaaaacg aggcagcttg tcaaagagga 107580
acggctcttg gccatggttg ctgccctagg aggatatttg atactagcag agctggggca 107640
accctggagg aaaccacctg gaatgatggg agaactcctc cagggaacat ggccctttaa 107700
tagatctctg ttataaaaaa taatcccaaa gcagccacca gggcatactg ctgcgatcaa 107760
gtcctaggcg gtattccctt ctgcgccata gaccctgtgc agagtgccct caacgaagga 107820
gcaaggaaga ccaagtctcc cgagggtttg catatgtgta tgtgattctg cagtcatggt 107880
gaatgacaca gtcagggctg cggaaaagca ttggtaaagt gtatatttga ggcttcagaa 107940
gtttgaaaag gctagatttc ctaggccaaa acactgaaaa tttgcaatta gaacttcagt 108000
gctgatgctg ggaagactgg agttagtttg agacatgcac ctgtgcagaa ctgggccccc 108060
agaaaaggag aaggaaggga atccagacca gagtagggcc tgacaccact cagactcggc 108120
gtgtctataa attagaattg cgttacaatt acactttgac attttagtgg tttttaaagt 108180
gcccagcaca agttaatttt tcattaatga atcctttatt cataaaatgc ttagatggag 108240
attacccttt tgagcatttt gccagtgctt ctgaaattaa tggggacctc ctgttggagg 108300
acacagtctg ttgcaatagg tgaccactgc tctgaatcta tgtcacctct ccaggaccac 108360
gggcacaacc atcacctgag gcatgttgga gatgcagatg gtcaggccct cctagaatct 108420
cagaatctgc attttagcaa agtcctgggt aattcctatg tccattggag tttgagaagc 108480
actggtaatc tcaaatactt taaaagatta ctagagtaag ataggctcag taggtacctg 108540
aaggcaccat cccaaagacc agagtggtag aagcaggtgg accagcctct gaacacattt 108600
ctcccccact ccccggctgt gtggaaggtt gccacctttg gggtagtcat tcaacaaaca 108660
cgtgtcaact gtccactatg tgtcaggcca ccactgggca ctggctgtgg ctagctggat 108720
agacaccatt tctgccctcc agaaatgtca tgtccactgg cacatgacaa gtcactaagt 108780
cattcagagc catgggtgac agctccaggg gccgacaaag gagctgtgat ctcacagatc 108840
cacagagaag tgtcccaggg cgggcgggaa ccaggactgc acagggaggg gtgaagtgac 108900
acataagaag tcagcccatc agcctgaaat gctcccccaa atcttcccat tcagtgtttt 108960
ctcagtagca aactcgtggg aaaattggtt attttactta aaaaactcat actagaaagc 109020
tagtttaact ttaaaaataa attttaaaaa catttttatt aacaaatcct acctttcctc 109080
caaagtcaag gagaaaagaa tagaagtgaa caatggacca agtaagccta aaactctgct 109140
ctttcccctg ctcattttac agttcaagtg ccattcaatt tatcctggca agaagaggaa 109200
ggcatcatca agaccttaat tttctaatac atctgatctg agaagaatgt gaaagctata 109260
aaattaattt ttgatcaata actacaggcc ttttgagaga gtgccctcct aatgaattga 109320
gtacctattt ctccatacac agtgtctatc atgacctaca aacccttttc ccatgaggtg 109380
taacagagag agattacagc cttggaactg gatgtcagac tctcctggtt taagacaata 109440
agccatgaca tagagcctga aaccaacaca atcttccgag tggttccaga aacatatagg 109500
ggataatgtt ggctctgatg ctgtacatcc ccaacaacca tcaactattt ggaaactaga 109560
atttcagcat aattggagtt ggtgttaccc tagcaaatgc tgtgggaaga gagtctcact 109620
gtgtatcttc tcctgtttaa agcctgaatt tgttcagaat gtaatatctc tgtttagcca 109680
ctctactgaa actgatctag gaaatgttca aaaaaaggta tcccaaggat ccctttgtag 109740
ctacatctgt gggattcccc tcgctctggc gtggcctggc ccctctgcat ttgacaatac 109800
ggtcctatgc ttttgtcttc ctgggctgcg tgaacccacc ctgccctggt tcacctctcc 109860

tcttgaccca tccttatcag tgtcttgaaa ggtccttcta ttggaggaca cattctgttg 109920
cagcaggtga ccactgcccc aaatctgttt cacctcccca gggccatggg cacaaccatc 109980
cctggagtgt gttagagatg cagttggcca ggtcctccaa aatctcagaa tctgcatttt 110040
tgcaaagtcc tgggtaactc ctatgtccat gagagtttga gaagtactgg tctcatgagt 110100
tcctgacata caaatagtgc tgaggccagt atgctgactg ggtagccaga tacaagtgaa 110160
aaccttcctg ttttttgcaa acctggatgg acccgaggcc gctgacgtgg gccaggacaa 110220
gctactcttt ttcagtgttt ctgttgcatc gctgtgtctc tctgtgatca ggtgctgccc 110280
tccctggcag gaggactgca gacaggatga ccaagagcac tctacacagc ctgctctcca 110340
gtgttggggg acgccaccca ccctcgtggt tcctgttcat ctgcctacac gtggagggcc 110400
caagagggct aatatgtgac tatctccact tcctggtacc ctgtgtgaat aacttcactt 110460
actaaaggga tgttgagcaa ctttattaat aatgaagaaa gcactttggt ttgacaaata 110520
atcactccat tttttcattt gaaagttaac tcttgttagt agagaaagca atgtattaca 110580
accacaagga cgtttacatg gaaatgaacc atctgcaaag catccccccat tttcctttta 110640
aatcagccaa tgggtggtgg tgggagaaat attcaccaga gtatttaaca tctatccccc 110700
ttcctagact gtcagctcca tccgggcgga gactgttggt atctccacag cacacacagg 110760
gcctggcaca catccggggc tcagtgagca cttgctgaat ggtgaacaga ttagctctcc 110820
tgggaacgtt gttgacacat ctcataacac tggtttggag tggagggcat tcatcgggct 110880
gcatattcct atttttaatt gtattctcca ctggttacag cacctacagt tataaagaca 110940
ttgttaacat tgcttatagg aagacatttg atggaaatga gtccaaaggc attacggtta 111000
gaaactggcc aggtgtcatt tttgagagat tagataactg ttttccggta gagtgaattg 111060
cctgtttgtt gcaagttggg actttgctgg gctggtttac agggccaagg ggaaagagat 111120
aagtggatct tctagtgaga ggtcatctgt tttgaaagcc tggaagattc catgaactaa 111180
atccaagtct tacaacacag ggaagtgtgt catactgtgc agggatgaag tctccaattt 111240
agcatgaaaa caagagctcc tcacactgtc ctcttcagaa agcccataca atccaaactt 111300
ctgaatgctt agctgcttac aaccatacat agattgaggg ataaaactct gatatggaag 111360
agaaggtaaa catttttttgg cagacattcc caggaaaagg cggctctctt ctctcattgc 111420
tgctgctctt tcagaatcca tttcaacaga ggaggagtca atgggagccc cgtgcctctg 111480
gcagatatca tatggcgttt cagtggcatt gtgtgttacc cttcttaggt aacagctcag 111540
ccattagaag aatgtcctac acaccttctc attttctgtg atgagaggaa tgtgaggtac 111600
tgcccttcga gagctgtcat ttgtcctagt agccagcagc gtgactgtgc tgtcttctgc 111660
tctgtctccc tgtcagcctt ctgcccagcc accaccacta tagttttgtt ctctccattg 111720
gaactcctgg ttcagagaat taccataaaa aacagacccc tagacataca acactctatc 111780
acataatggt gactttgtct tctatttttgg attactgagc tttcttgggt aacttccact 111840
aaatcgaagt taatattaga agaacttcct cttactagaa tcgaaaagca tttaagtgat 111900
gcagtcaagt ttgtaccata agtaattcag tcatttaaca aatatatatg gcctctgtgc 111960
gacagtgacc ttgactggga atgaagctgt cccatgtggg gcctgttctt caaaggcagt 112020
tccctgctgc ccagttcagt ccagtggatc tgggcatctc tctttaatcc gcattagggg 112080
ctctttactg attcttcact atccaaaaag acttggaggg gagacctgag cccacttctg 112140
gaaggaaatg ataacaattt atttagataa tctttgtgca acaagtcaat tcactgaaga 112200
gatctgctct ctaggagcct ctgtgacccc accataactg ggaaggctct acctctccag 112260
tcttcgggcc acatttctct ctggcctgct gtcttcccag cactctcagc cttgctcatg 112320
gagcactcta gtcctccgtc gaccttggcc tttggtaacg tgatttttca cctggcagct 112380
cccatctggt ctcactccct ctttttgtcc agtctgcatg acacagcctc acatcgttag 112440
tgttccctca ctcccctctt actgcccaac ctgcaaagtc catgcctggg ccagtgcagc 112500
atgtgtcctc aatgggctgc tggtggcagt ggggggaacc gcacagccac gctgtgtgct 112560
gctgaagaaa tgcacagcct cctaccctcg ccctcaagag gcagccatgg ctgcgcattt 112620
ctgcccttct gagctccgct cacttttggc agcagccgtt ccaacctgca tgggatcttc 112680
actctctcac agatgtgctg actcctcctg ctgcctcccc tctctgtgcc ttctcactct 112740
ctgttccctt tgccctttct cccctttctct cctctgccta cctccaagcc atccatcaca 112800
ggacagctca agcatcagat cctctgggac actttcctta gttgttcagt ctgatgaggt 112860
gtccctcatc ctctcttagc tgaaaatcag cagctgcctc aacttctttt ccagcatgtc 112920
tcatgagtat tgccacaaca gcatctgtca caatgtgggg tagtggctga cttgcttttc 112980
tgccattcaa ctgagttccc tcagtgctgg ggccagcgtg cagtgtcttg tattcagtat 113040
atagctgatt aattgatgaa ttgattaatt aatggttcac actagcacag tgcaaccttc 113100
aatgcaaaga tctcatcaaa ataattcaca tggtgggata ttttagaagg atgaccaggc 113160
tagtttgtag taagaaaaaa tcaacaagac taggtcagga attctttttt tgtctacagg 113220
cttgctatag aagatattga aaatcatcta cctaattacc tttattttat caggttgtgt 113280
attaaatatc acgtctgggg gaagaaaatg tgatatgtga ttacagacct ttcctggtac 113340
aacatagtac gtttcagatt aactcaaggt attgtggtga tattgcggtc aaagccaggt 113400
gattaaagag tcattctttg aaacaaatat ctgtgcaatc aattaagaaa ttaatttgca 113460
aattttattt gcttagagta attgatatat cattcctttt acaaacaaat ataaagaaaa 113520
cttaactaaa aatactgcat atctctttca gattatatat cccagaaagg atatattttt 113580
ctcctttctg gtcttccttt ttggtgtagc atctgtagga aatgcatttc ttcatagcta 113640

agtgtacctc cttgtgaaat atcttcagag tctactggtg cacataagca attgctggca 113700
gcagcttgag ggtctccatc tcacatttat catatgcctt attgcatgag gctttgcaag 113760
aggaggtcta gagctacaat atctcatgga tatgaatgtc aattcaaatc ccagtggcag 113820
tttatgaggg ggaaagccta gaagagaaga aacctagagg aatcaagcag gaggggagag 113880
taataaaaga ctagagcagc aggtttttct taactcaaac tagaattaaa tctctgtgtg 113940
tgtgtgcatg tgaatgtgcc cgtatgtgca tgcatgcacg tgtgtaaatg gatgtgtgtg 114000
tgtgtgcatg tgtgtgcaag taagtgtgta tacgtgtgtg ggcatgtatt gtgtacatgt 114060
atgtgtgttt tatgcatctg tttgcaagta tgtgtgtatg cacataaaag tgtgaatgta 114120
catgtgtgct tggtgtatgt gtgtgtatta atgtatgcgt gtagttctag agtctagtta 114180
gagaaagtgc ataaagaaat agggaaatta acaagaaagc tatagcttaa attataggaa 114240
aaactttct ccctatcagt catggtttta aaatgttcag acttgatatg tttcccagtg 114300
ctattgtcag aaaatgtccc tatgacattc catactactt caatcaaatc taaaaccttt 114360
gttccaacat gttttattga tatgagtata tttcaaattt ctaccaggtt tttggagagg 114420
tattttggcc ataaaattga ctaaattatt caaaataaaa aatgaataag cctgggccaa 114480
ggcttggaga cttgcttaac tcagttctta aattttcaga ttttcaaaat tacaaattta 114540
agctctaaaa tcatggtgct gtgtatgata ttctttgatt gcaacttatg gttgaaaaac 114600
tatagagggc tttatgctaa gagttgtgga tcttaggatt ttcatgaaat ctgcattatc 114660
atcatctgca agtttagatg gggcataact gatccaaagg atggatccct cgggggcaat 114720
tcaactggct gattccagcc aagatgacaa cagtcaggat ccgttccctt ctgatcatcc 114780
attgggtgcc ctgatttcct ctacagccct agctgaaaga ccagacacta tctcaggctg 114840
gctgccccac atgccttgct ccacaccaaa ttcacagtct ataaacctga gcctccagtg 114900
ctcctactac catactcact cgaacattcc cgattctgac ctggagatgt caacagctac 114960
ttgatgccac tctcttctat ctttctgtag ctaagccatc cccaagtttg tcgattcacc 115020
ctctttaacc cctgtcgggg tgtccattgt gccccttcac cctgccatct ccctggtgca 115080
ctgttttgca aagttcagca tacatgagcg tcacctggga accttaataa agtgcagatg 115140
ttgattcagc aaatctggga tgccctcggg ctgcatttcc agcaggctcc tggggatgtc 115200
cccgctgctg tgctgcagat gacactctca gtggtgggac tccaggctct gctgtcgcct 115260
cctaggggtt tctccacact ccctggaggc ctaatgggcc cttctccaca tggcagtaag 115320
atctgttttt gtgtttgtgt ttcaagttgg gagaaggaga ttatttaata ctaaaatgtg 115380
caacatggga ttgagaaaac taattattag tcataagttg agtatgcaac attgaaacca 115440
catgctttaa aaaattataa gaaaaaatca tagtatttga aagttacaag ctattatggc 115500
taactccatt tatctcagtt agagaagaag agtcacctgt caccagggca ctgccagaag 115560
ccaggctcat ttccaacagc actgggtgct ccagctttgg ggtgccagct cctcccataa 115620
agcaaacaca tacctaggga tgatatttct ttgcaagggc tctgccctac agcttgtaca 115680
tctcaagaag ttatgtaatt aaactgtctg ttttgagaaa attgtagatt cacacatact 115740
agctgtaaga aatgatgcgg ataaatccag cgtaccagct ttccccacgg agacgtcttg 115800
cagcgtcaca gccaggatga ggcattgacc caggcgaagt ccagagcacc tgtgcgctac 115860
agggcccctt gcactgtgct gtcacagaca cgcccacttc cagatgccat ctaggacccc 115920
ctccaaaaag cagaggcatt cttaaaaaca cacatctgca catgttcctc ttcatttgaa 115980
tctgtcagtg gcttctcagt gcctttcaaa tgaaatctaa agtccttaca agccttgcag 116040
caggaacctc tccatcccac ttcccctcac actctcagct tcatctctgc taggctctgt 116100
tcagccaggc agcctttcac agtccctctc ctcctgccct gccaggaagg tcccctgccc 116160
ccaactcttc cccacatgtg gcggggcccc gcttgtcctt agaagcccag ctgaactgct 116220
tcctgaagga acccctccag aacctctcag accaggtcag gtttctgcac tcttagatca 116280
tccccatggc ataatcacag ttgtgatgtt gtgatgattc agtgaatgtc tgtctcccca 116340
ctggatggta agcttcctga gggcaggaac agcattggtt ccagtcaatg ctatgtccca 116400
ggactgttcg tttttgcaca tactaatcct aaaaggacga tgacaacagc aaccacttac 116460
atgacctaga tgctcttctg ggtgttgtgc aaatattaac aatttaatcc ttgcaacaat 116520
ccacgaggga ggcattcttc tactcccact taacagacaa ggacagtgaa gctagtaaag 116580
agaagtcatt tgcccaaggg gaccccacta ctgttggcag agctgggtgc aaacgcaggc 116640
ttgtgaagcc aggacccatg cattcaaaga ccatgccagg tgcccccact gcacacctca 116700
tccccacata ccagtgaggg ggagagaaat gctcctgcac tgcctctgat taactgcttt 116760
cctagaagtc acacatataa aagggattta attctagtgg gattgaatct caatagtttc 116820
cttattaggt tgatttctgt taatagttta agtactggat atacatgaat tagaaaatct 116880
agattattag caaatgcaaa ctataaagta ttttataaat gttatcttgt ttgtcagggg 116940
atgagtgaga tattcattat acaaaaagta gtgtggattt tgaggtagaa ggtttactaa 117000
ggatcatacc gtagtatgaa atagccacaa acattcagtg aaaccaaaca cccccgctta 117060
acctcaaact aacactaaat aataaggaat agacttgggg gcagtgcaag tgtatttcta 117120
atggtgaaaa ccattcccca gtgaaaacta atgtaccatc tagttaataa gagctcctct 117180
gacccacgca catcaatact tacatcccaa tggtgatgtg acattttggg ttttgtattt 117240
cttttgcaaa ttgagctagc atttttgatg agtggcaggg ctctgctacc caacctttgg 117300
acagtttcca agcataaaat cacaattcca gataattctg tcacaaagat ctgggtctca 117360
ttaggaagga gaggaagctg ggagatgatc cagtccaacc tcccccaaac caaacatcac 117420

ggccttctca gttgtttcac caaccatcta aatgttttag taattctaaa aattgatgcg 117480
ctttttccac gaaaggaagt gttaccacat tttccaagtg ggaggcatct atatccttac 117540
tccttcatcc tctccttccc accccctcac cccccaccac ccacacaaca tctgcaattc 117600
ttaaactaaa gcacaaattg ttacaaaagt taattgcact ttcaaaggaa tgcttgtata 117660
gaaactttct cggcttcaag gaaaaataat acgctttgaa tggctgttca acagcataga 117720
aattagctga gtagaaggca ctcatatagc cattaggacc aatcctttct gccgccaaca 117780
cccccttat aaagacttga cagtgggcca gaataaacaa cttcaggatg aattcagttg 117840
agacacaaag tacacacttc cagtttttcc cttctctggt tactggcctc aataaccagg 117900
cagtcaactt aaaaagaaaa acaaaagctt gcttcagatt acagattgca gacttcttat 117960
aatatgtcca tttcaccagg ccccgctctc agccccggga aaggccactg gaaaccacct 118020
cacatggtag ggccttgcgg gagccagtaa taaccttatc tccgtcaaca tgttctgtca 118080
gattgaatgg ggcagccaga gaagccagag ttggcacagg aaccaaaaca aaggcttccc 118140
atcctcctgg agtgagcggt tgagcctgga ttggtgctta gacctataat gggtgcaagc 118200
agcgttcatt catagtggct ttctagaccc agggacttgg ccccagccct gctgctccac 118260
tcctcttctt gcttcattac cacgagtctc ctagaccacc gaacgatgcc tgcatttgaa 118320
agacacttct gctgatcaaa gcagctgatg tgtccctttg cggttcattt ctaattgtcc 118380
ccaaggagga gaaattcaaa tagtttatta ctgagagtta aagaaatcca ctgaaatatt 118440
ctttggtcta aaattactgt catggcggag cagcttcacc ttagtcattg cccttaaata 118500
tgaaagctat ttaagaaagt ttgcccttaa atatgaaagc tattttaaaa agtttaatga 118560
aagaagagaa tcacaaaaca ttttcaaaaa gcaaaagaaa acctaagaga aaagttgaaa 118620
gtaggaattt tttaaagaat atacgacgtg tgttctgtga ctcacccctg caagttattt 118680
gtgtgtattc ccttgcatag taattaataa tgaagcaaag catggcaatg atatcttttc 118740
ttgtctagta ttctagaaga ctccatgttt ttggaaaata tcactctagt tagatctcaa 118800
atatattcaa tcagaaaatg ggttttctac aagattctat atctgtagtc aatagcaaat 118860
ataattctat taagctagta ggatgtgata ggaaactaaa acctagggga gaccaaagca 118920
aggaaaaata cttcctcatc caaacttgag agcaatttac cgtcaggcct actattaata 118980
gatggaatac agattccatt ttcattactc aactgccata ttcattatta cactgtacag 119040
aaaagggaat cacatctgtt gaaaacttat atatgatgtt catgcatgca ttccagtaat 119100
tcaacaattt ttatttatct ttttattgct tgctaatttt tcaaaataat aagctaaaga 119160
aaacaaaatg tttgtgctgt tctcagatga catgttatct ctttaaagga caaaatgtgc 119220
tgtgaaataa tagaatgctt tcagcactca agtgtgagtg agtgctcata catgagagaa 119280
agccgtgggg actacagaag ccaagaagca gatctagctg gggaggcctt tgcagaggat 119340
gtagttgtgt ggagaggcca cacacgtgga attcccagga gggctgtgga ggcggggaat 119400
ctgcaggaaa gcactggggt gagaaacgtg atgagaaaca attattgtct taaaatatct 119460
gcagggctgt aaggtagaga agcaatacgt tgcatctgtg ttaagtcaaa caaaattatc 119520
aagggactgg tttcagctta acataaggaa caattatgtg atagggttgt caataacaag 119580
agtagactgc ttcttcacac actcctagtc actcagaatg gtccaggagg agtggacaac 119640
catttggtag agtatgggaa ggcaggggcc ctgggtggga gtggtgaggg tagggagtga 119700
gtatcccaat ctagaagtaa attgtgccca gcacggagct gcaacactgc cctgcacaca 119760
aacacacaca aataacaatc cccagcccct gcatttccct ctccggtttc aggaccttgt 119820
atcttacttc aattccttta tttagctgat gatgaaatag gaagagctta gcactaagaa 119880
aatccttttg gagtttggcc ttgggggaaa atgaatcact ccaaccaggt ctgtcttcta 119940
gaaagtatag gatgaaaggg ctcctcatca catacttcct gacctcctgc taggcctttc 120000
cctaaaacag gggctggcaa agcacaacct gtgggtcacg cctagcctgc cacctgtttt 120060
tgcaaataaa gttttattgg agcatgacta tatgtatttg cttacagtct gtggctgcgt 120120
tcacactatc ccagcagagt tgaataattg ggacagggac catatgatgg gtgaagctga 120180
aaacatttac tctctggctg tattcagagg aggtttactg agcccttctc tgagacatgg 120240
caagcgctgc ttcaggctca tgcttcacta gattcaggcc tggggcagta aagagccagc 120300
tcaggatagc actcccgact cactcatttt ttcaggcagg ggagccatct aatgtcaagt 120360
gcctacgtgc aggaactggt ctgttaatta gcagctctcc tcatggaagg gataatatat 120420
tctagaaaca ggagtgcggc cctattgcaa gaatgtcctg agccaaaatt aagattcttc 120480
tatggcagaa acttggctgg ggcttctcct gagttaactt ggtagttgtt agtgattttt 120540
gagtcagttt ttccttgtca acgaccccag gaatgagttt gggattacag ggtagccagg 120600
gaaagggaaa gcttcacgcc cgcccccggg acaaggtctg tcttcacact gctacatccc 120660
ttcacccact ttaaaatgaa acttaaaagg aggatttcag ttgagtagga agtgagaaga 120720
gggctcattt taaaacaagc gttaaatgaa aacccacaca cactcagagc acacaaatcc 120780
aaccacgctt acaaaaccat cacagagggt caggcgaggc ccttttctaa atgaaaaaga 120840
acaggggtgg agactgttct gagagcatgc tgggttccct gaagggaatt ctcagctgta 120900
tgtgccccgc acaggatccc tgctagacac aaggccagct gccttccttt caagccgcag 120960
acgcatccct gtgtccaggc gggctggtca gctgcggtca gcaccagctt ccccgctcca 121020
tggtgaggtc atcacaacat gtgagcagga gggcaggccg gcaacctctg agtgcttaga 121080
gaaagggacg ggattcctcc tgtgcaaccc ctctagtctc actcagactc aagtctgact 121140
aaggggccag gtgctttgac cagggactct cccctctcac ttccctccca ggagtcacag 121200

gtacatgagt ccttgtttta caaatgaaga aaacagaccc aacatgatta agatgttgcc 121260
ttcatagggg tggcaccagg attccaaacc atggactcca ctgagcccag tgcccactga 121320
catgtgccag taacagtgca gctgcctgtg gttctgtcga ctaaactgcc ggcagaggct 121380
ggctttccac cttctttttt tttttttcac tcttcaaaca ctttatgaca tgaacataaa 121440
ctactggctg catcgttctg ctgacaacat gacatgtttc tataacttga aaaaagcaag 121500
cagtggactg ctcattggta aaattgagtc agtaatcttt taggaaggtt atttttcttc 121560
cttttactgc ttctcatctg ttccccgcag taaagaggac aagatgacga cgactcaggg 121620
aacacctcca gcctgaagca gcaccatgcg agcttagacc ttagggtcgg cttagaaacc 121680
acaggcgggg cggcttgggc ccctcggaca ctccctctcg aagctgcttc tccccaagct 121740
accccaaagg cactgagcgc cctctgcccc ccagcaattc aattcactgg ctgtcctgct 121800
cctgtcagta ctgagagttg catgtttgac cctcggggga aaagtccaga ggccctgggg 121860
tgtccagcat gctctgaggt ccctgctgct gaccccttgc gctgtcagca ttcagagaca 121920
ttcacacagc acagcctccc aggctaacag ctgtcatgga acagtggagc agctagacgt 121980
ggccattctg tggcccagtg ctgcagaggt caaagggaca agcgcaggga gcatctttgc 122040
tttcagaaaa aaaaaaaaaa aaaagaagca cactggtgca ctgacctgct cctggtgtct 122100
ttgtgattgc tcttttcttt cgattttggg ttgtcttttt tttttgaaa gaggggcttt 122160
tatgcttttt tcctaatgtt catgggtaaa ccaatgtaaa tgtgtgtatg tttatagaga 122220
tggctttaaa tcgcaattct gcagtagaga ttgatttttt aaaaaacatg ggtaaaaatt 122280
gaagaaaaat tttaaaagaa catttaaacc atcttgggct aggggtggat atgcaccacc 122340
ccacggaagc caaacaaaat ctctctgcag ataaacattt gcaaaaagaa tttccaatcc 122400
caatttttga gtcagagatc ttttatttcc ttgcaaatta catatctgtt tcaggatttt 122460
tgactataag aagaatgaat gaagatgtgt ttcttacaga taactatgaa caaaccagga 122520
aggataataa cttgtatccc ccaattcgaa tccagaggat gggaaggcat aaaaaaaaga 122580
aatggaagaa actttatttt tagtggtaaa tggtgggact atgtatttta cgtatggtga 122640
agtcaccaag cccaacactt ggcacttgta ggcaaggtag tcttctaatc tgaatgtgaa 122700
gtattatgtt ttcatttgct tggtaatgag gaatattggt gctttcgtcc cagttctcga 122760
gctgactgac ttctctttct gacgtgtgtt cctttagcac acctctacac tgcatggctc 122820
tgagatgtcc tgtgactgtt tcatgtgtaa agttgcctcc ccaaaggact cacatattcc 122880
ttcagggcag tgagtacttc tgattcatcc ttagcagcta ccttcgcgct actttactag 122940
atatgttgta gttgaattaa tgaacaaaag aacaagcaac tttggtgcct ggtgtgcatc 123000
tcagagcagg gtggagtgag cctggccaaa gggtcatcat gcaacctctg tggctgactc 123060
catctggcca cggagcttct cagccatgct tggtattcac atgacttcta gggcgacagc 123120
tcaaccagca aataaacagc ttcatatggg aaatattact cagcctttgt catcaaggag 123180
tgagtcacgg gcctgaactg aatagaagat agaggagaaa aggtgtgtgg actgggtgag 123240
acagcgccca gcgaggtgaa ctcccggcag ccctgcctgt ctttacctgc acatcacctt 123300
gctagggtgc cttcggttgt gagggcctgt ctaggaagag aagagttgca ccctggcagg 123360
cagcactgag ctgtctcatg caaagctgag gaagaaagag tgagctgccc agtgagcctg 123420
ctggggtggt ggaggctggg ctgggctgtg cagtctgcag cccccagcag cccttggcac 123480
ctttctactg cctggtgctc accagctctc cagtaacaaa gagggacgtg aagtcagagg 123540
ggaagggagg tagcacaggg cagtcttgac tttgaacaaa gagctggctt cctgaagtca 123600
gctggccggg ttttgaagcc gattttccag cagtgatctt tgatgccaac cccatttagg 123660
aattctgtat ctccccctac cttctaccag atgtctctga gctcaccttt ggtgataatc 123720
atgcaatctc cgtcatcccc acgtccacac tgccccattc tgtcccaccc cgggttctgt 123780
ggtgctgtcg gctccccagc gagccaggaa gggagaggcc agctctgctg gggctcctgc 123840
cgccctggct ctgcactgcc cttctctggc aggtctgagg cgccactgga ggagccacac 123900
ggccctgaag cagcaaggca gatgccctgg acacagtgga ggcacagagt gcaagcaccg 123960
gcctggccca cagactttg gaggggaagt ggtattattc agttcaaaag tatgcctgtg 124020
tgtaaagaga gagcccctga acatgagtaa gcaaaagtct cagcgcagag attagacaag 124080
tagaatgctg gcccgagagg aggcgtttac tcaccctctg tctaggaagg aaagccaggc 124140
ccagcacgct cactgctatc tatcctctca cacagaggga ttttgaatcg aagccagcat 124200
cctgtccttt ctccaatgtc ccctgctcag gagtcaggac tcagcaaggc ccaccccagc 124260
cacacacaga tacagttcca ggactcagaa ctcagcgagg cccaccccag ccacatgcag 124320
gtccagttcc aggattcagg acacagtgag gcccacccga gccacatcca ggtccagttc 124380
caggactcag gattcagtga ggcccacccc agccacacac aggtccagtt ccaggactca 124440
ggactcagcg aggcccaccc cagccacatg caggtccagt tccaggattc aggacacagt 124500
gaggcccacc ccagccatat ccaggttcag ttccaggtaa atcatctgcc ttcctccgtc 124560
caaaagcctt gtttcctgtg tgtccttgtg tttaaaatgg aaacgttatg agaaactgcc 124620
tgccagggca aagggtgctg cccggcacac agtagggact caaaatgaaa ctattgtatt 124680
gaatacataa cagatcaacg ggtattgctt tctgaaatct tttttagccc aattttgttt 124740
cttatagtcc aataacaggt caaattcatt tctgatttac tagccattca gttgcccata 124800
aaaaatggaa agtgatttaa gattattagt ttaaaaacca atgaaggtaa aacagttatc 124860
attgaaggca cataggcaga aatagattgc aatagttgct gccatgtgaa gcctcagtgt 124920
catgctccat atttagagag atctatgatt tctgaggccc tttcatgtcc atgatctcag 124980

tactgctcac aactgccctg tgaaattcgc cgagctggcc ccatgtcaat cagagtacac 125040
tgagcactga gacccagcat gttgagataa ctggctagag atcatcccat aatggtacca 125100
tcacaatctt cacactgtag aagtttgatg atgtcactgg aagcatattc cacagtccct 125160
tgtgaactgg ccttcctgtg atcagaagca tcagtgaact cccaagaggg tgggaactcc 125220
caagaggtat tctcactcta cttagtgtat attttacaaa tcacaagctt ggctttggat 125280
tcttttaatg gctagaagga gaatcatggg gttggaagtc caccagtttg ggtattctgt 125340
tccctaactc aaaataaaga gatgttattt tcaagtcttc tgcttgttaa cttaattaga 125400
gatacatgag tttgcagctg tgctgggcat gccgcagctt ggcatgttta gtccagaagg 125460
catattataa tgtacatgga agattgtcag aaattcaaaa ggacttttg agtatcacat 125520
gtgtattttc aagttccaat atagattcac attcagtttg acaggtatct ttggatgcct 125580
atcagttaag aactatttat tagttgtgga ataaaatagg gtaaaataag gaacaactga 125640
ggaaaaaaca taaaatttgc tttgtgaata aaagttgtct tcaaaattat gacttttttcc 125700
atcccacaaa agttttgatt aaacccacaa tgaaaattta aataagtgta tttactttgg 125760
tttaaccact tatttcatta tgactcacaa ctataggttt tctagtttcc attattacaa 125820
actattgtgt ggtttaaatc aatttcatag actagtctag ttctatagtc acaatttata 125880
aaatttttt atgtggtaaa ttgagtgtct tcatagatgt acatgattat ttctcaattt 125940
ttaaggaatg tatttttaa gatagccttc tttagccttc tttaacactg atttttgtaa 126000
attttttaca gatttttttta aattttggt aattttttag cataaagtaa tacatggtca 126060
ctatggaaaa cataaaaaca caaaaactat gaagagtaaa taagaaaaac acccagaaat 126120
ttaccattca gaaaaggtca ttgttaacaa cacggtgtat cttcctcctg tcatgcttcc 126180
gtgcatttga gcacatttga gatgtgtata catgttcact ttgagatttt agtatagcaa 126240
aagaaatgac cggtcctgat tcaatgaaac ctctggcaaa ctcgctatat tttccttaca 126300
tatttttaag ttcatcctat aaatgaacta tccattcatc ttatttgaga ttttcttaaa 126360
tctttcagca agaaagcggg aaaaaaatcc tcctctggcc tttaaagcct aattaaatat 126420
atgactaagc tagaaatatt ttataatgac caaccagaaa gtggcaagga ctgtcactct 126480
tcccatacag cccacctcct cctctatctc cctcaggcac acggaaacga gaaaggcaga 126540
gaaacccagg acaagtcatc caagactttg gtcacatggc catccattgc tttcacaaca 126600
aaaatataaa tccaacatgt gtgtgtgcat ttcataccag taggtccaat aagctatcta 126660
tatatacaca tatgtgtaca cacacacaca cacatcctta cagacactcc ccagcttact 126720
acagtttgac ttaagatttt ttgactttac gatggtgtga aagcaatgca cattcaatgg 126780
aaaccatact tctaatgttg aattttttat cttttcttgg gttagttgat gtctgatatg 126840
ttactttctt gcgatgccag gcaatggctg ggagccagag ctcccagtca gccatgcaat 126900
caagaggcta aacagctgat actatacagt ggactgtgtc accagcattt tggggatatt 126960
gtgttttgtg tttttgaatc ctatcatgtc tacaaaatgc cattttcgac tgctatttc 127020
aatttagggt gggtttatca ggacataacc ctatggaaag ttgaggacca tctgtatatc 127080
tggtagggaa agatggataa caaattcata ggcaaataat aatttcatga ttattattaa 127140
gttattccta cttaataata agtagtgatc actgccaggg agcagagaat gcaggataat 127200
gtgacagatg taatggtggg tacttaagct aatgtagttg cagaacaggc ttttctagag 127260
ggtaggcctt taagcgtacc tcgaagatgc aaaggaagca aagatgcgaa gatctgggct 127320
ggggatggaa gcagagacaa cttggaggcc aaggggagag actgacaaca gcccagctca 127380
tacctcagca gcctttaatg catagctaag aaaacaacaa attaaaacaa ttatagttta 127440
cttagacgat tctaagtgtc taagtggatt tgggcaaatc tggagaaact tgttctaata 127500
ctgtgtctta ataagtaata tagatttgcc caggcttgtg ggcagagtgg tatacacccc 127560
ataatagcag aggaaggcca cagggcctac cctacaaaac cagaggcatt taaaaactta 127620
aaggaggcag attgctttta ttttcagtta aaataaagtg aggagtttct caagaaaaat 127680
aataacgaga ccaccggccc gccctagatg tccaacaaga atgcacagat aacttcgtat 127740
atccactttc ctgaacctgc ccctgacagc caagtggagc acaacaacag agatgaacct 127800
caaaactact gtgctgtgac ataaggcttg ctcaagagga cagtgtggtg tgagtccatc 127860
tatgttctaa agcaagcaaa gctattctgt agtgaaaatg gatcaggaca gcagttgcct 127920
ctggtgtatg ggggcaggga tcgactggga ggggcatgag ggatgacagt tagggtttcg 127980
atcatgacag gaattcagat tactccagca tgtgcatttg ttaaagctca tcaaatgcta 128040
cacttaagat taatcctctc acagtttgtg gatgttacct taaaaacaac aatgatgact 128100
gcaaactaat attgaactct ggttagtgat ataccaatgt gaagtatagt gatatctcta 128160
ctttacttta aaatgcatcc aaaggcagac tagaggacca tatctgacag acagaaaaat 128220
agatatgtga taaggtgaat gtagtaaaat gctaacataa ggatgtttgc ggtacaattc 128280
tttcagcttt tctatacatt tataaatcat aataaaattt taggacaaaa agttagtgct 128340
ttgaagtcct aagtcatagg gcctgctgct cttgatgcag tagaatttgt cttcagattt 128400
gcaaagggta aggcaaacca ctagcatttt gtatggaact tgatgcaaat acttttaatt 128460
gtctggtttt caaatgtata gacttaaagt aatatcaact ctttctttga atcaactact 128520
gaaataccta gtcttaaata aatattttta tgtaatcctt aaagtactat gtattcattt 128580
ttctttcttc tttcttttct ggtttgataa atattctata aagtaactgt gtttaatggc 128640
caacatttga gtaagtccat atgcagatcc aaacatctca gtttagacaa taacttaaga 128700
caatatagag tggctgacat cccctaacgt gggtccagat gcatgttatg ttatgtttct 128760

gttgcattct caatagttaa ctttaataaa agaaagtcaa aagcttatat attttttcaa 128820
tcttcaaaac atttctggga ggttgtctta gttaatttta tgttgctata cccatatcac 128880
agactgggta atttataaag aaaataaatg tatttggctc atggttctgg tggctgggaa 128940
gtccaagagc atggcattgg catctgcttg gcagctggtg agggccttca tgctgtgtca 129000
atctatggtg gaaggtcaag agagcatgca tgtgaggtgg tggggaagag aaaaagcggg 129060
tttaactcat ccttttatca gggactcact cccgtgatag ctaacccatt cttacatgaa 129120
tggcattaat ccattcctta gggcacagct ctcatgacct aattataata cctcttaaag 129180
tttccacctc tcaacactgt tgcattggtg attaagtttc caataaacgc actttggaaa 129240
acacattcaa accacagcag agatcaacgt tattgtcacc attttcatat ttgaggaaag 129300
catggcacag agagcttgga gaagtacttc aaggtcaccc aatgaggaag tggctaaaca 129360
aaaaccttat cttaaattaa ttaaaaacct cttgctcttt gcagttttgt cttaaatcta 129420
cctaatttgt gactgtaatt tttaagtaat ttactcatat aagtggtctc acattaaatt 129480
ttctcattgc tttatatttc taacatgaga tatttggtat aaggatggaa ccaagatcat 129540
accttgtttt aattagaaaa cctagaccaa gtcattgtga tcctcatcct agatttcagt 129600
taaatgctgc tgtctccttt tgggtatgtg acaggggaaa gcctcagaag aaacaacctt 129660
atgtgttttc ttttgatact ttagtaatta acccaggata gtattcaaga ttgacatgcc 129720
ttatattgaa tcaaatagca tatcaactgc cttcttattc tcaagtatag acatgttggg 129780
taattgggca tttaagtttc tttgcaattt tttccattat taacaaaatt aatgagcaac 129840
attctgcata aggtctgttt cctcagaata cgtttcccaa agtggaatca tcatgacgta 129900
gaatttaagc atacttactt gtttaaacaa attgtccagt tgcttcccaa aatgttttgt 129960
gaattaagat ttacatcaag aatatgtaat gttgttactg tctcccaaat acaggatctt 130020
tttctgaata taaaagttat acatgctaat tgtagacaat gaagggtcat tatcctcata 130080
gataatgaag tgcttctaat acttgtgctt ttattcattt attcaaaaag tgctaaataa 130140
gccctgaagg ggcttttggg gggtcatttg ggcttattt agcacttttt gaataaataa 130200
ataaaagcac aagtacagtt tttttaaaat actgttttct ataatagatt aatcttaaat 130260
ggcatgtttt cctttatttt actgacaaaa gttacttact ctgtgattga ataataaaaa 130320
ttctttggtt cagctgagag aaacttgcaa gctgacgtcc ttgattattt aaaatgaaag 130380
cagctgcctg ttttcatctc tctgcatcct gaggaaactc ttctgcaacg tgttccagcc 130440
ctaggttcta gctgaccctg ttcatctgtt tggcacgagg ggcccaacta acacttgcgg 130500
ctacctggac gacagccaat ctagttggaa tgagagttag aggccatagt ctgtcagctg 130560
ggaaagcagc ttttattcca aggtgtgcca accgaaaggc cacatgttat tgtcacaacc 130620
tggtacctac atcagtgctg acatctttaa gaaccttaga attgggaaat cagtttagcc 130680
ctatctgcat gtgtagccga caaccacaca attgttccaa cttgaggttg cattcagagc 130740
aacctcattt cccccatact cctgaggaaa agcagaccag agacgctggg tcaatccaga 130800
gttatggttg gaaaaatgat ggaataattc tgcccctggt gataggagag agggactcca 130860
tcttgtcaac tgtcatggtt cccatgtgaa agctatcatt atcactgaaa ttgaatgaga 130920
acacagaagg gaagaacagg gaaatcccca cagagttaaa gaggatgtga agattgcttc 130980
atgtttaatg tttgtgtaag tgctttgggt tggttatgtg ctgtctgaac atgtgctcat 131040
ttccatggct cattgagagg gcagacagtc caatgatact ctttagaatc attcccatgg 131100
ggaaggaaca aagaagcctg taaaatagaa atgcacatgt aaaaagcatt gaagaaagtg 131160
ccagtgtatt gattttggcc atggtttgtg ctctaccacc tggttactgt gattgcagaa 131220
gtgcctttgc agatgaggaa gaacctggcc aaggctcaat ccaacatcca aagccagagg 131280
ccatatttct tcactcttaa gataatttgg gttcaaatta tagtcccttt acacactctc 131340
tgcctcaaaa ggcccaagac tctcttttgt tatgcttgcc taaacatgcc tttcaaagaa 131400
ctagttctgt aaatacaact ttattataaa cctctccttt gcttttaaaa atggatcacc 131460
acgtccattt ctatggtcca actttgtccc ttaatttaaa atttttcttt ggattaagtt 131520
tgatgccttg aaacattagg aactcaagca tacaagattg tatgctggtg gtgagggaag 131580
taactgtgcc tccgcctgtg ctgggtggat caacatggag tgtggacgag catagggatg 131640
tgtgggtttc tcactagctg agagtgtttt taaatgttgt attttgatgt ttgttatttt 131700
ctgaatattc tacagttaga cctttgattt attctttgat gcattcattt gaataatatt 131760
tttaatctcc agccagttag gtttttaatt tacactttttg tccctgattt taggtgtagt 131820
gttgtgtaca ctactgccca gtgtatgtta tgtttgtaaa cattcattgc acgcacaaca 131880
atgtgactca caatattttt gagaagtaaa aagttcatta tatagttatt aactcaaccc 131940
tacagttata ttcgtgaaat accttgtgaa atttattttt tgcctactgg agctcttaca 132000
ggttaatcct gtcttcaaga ttttcataga attttcatct accacccacc cctttaaatt 132060
tcaacatttt tttattttgg cattttaatg caattcaatg cattataggg acaagctatc 132120
tcttattatg aattgcacct tatataaact taaagatctt ttatcacaaa tttctttgct 132180
gtgtccttta gtgagaattt gtattatcag tcactaaagc tcactaagtt agtaagcttt 132240
gcgcccagat gacctgggca ggaatgggtg agtctctgtg tggagagagt gaagaaactg 132300
ctacccttaa tacctggacc ttgagggatt gttttatttt agtttttctg catttctcag 132360
tatttcatgt gatatctgtc tttttcttcc agtttgccaa ggcacgagta acaagctcac 132420
gcagttgggc acttttgaag atcattttct cagcctccag aggatgttca ataactgtga 132480
ggtggtcctt gggaatttgg aaattaccta tgtgcagagg aattatgatc tttccttctt 132540

aaaggttggt gactttgatt ttcctacaca aataaaattg gagaaaatct aagtggagaa 132600
aggcctgggc agaattccac ttgaagtgtg tttatttttg ctatggcaat gacaagtctt 132660
acagagctac aaacgagagt tttatgagaa agccatttta ccagctaatg tcaagtaata 132720
actagaaaag gatatcaaat agaaacaggc taatctggag ttccatgtca tcatagacac 132780
tgacgtttat ccctgaccat tacctcagtc atgatgtgct gccatactcg ctcttaaaaa 132840
cttttttttaa aagccctgct ttgcaccatt tgcctattcc cttagtgtaa atactcctac 132900
tatagctgat ttcaaggtac caagtttcac tcagctggtc acagaattct tatttcacga 132960
taggcgctaa tgaccccata ggagccagct ctgaaggctt cagagtttca ctgaattttg 133020
gatggggttt acttagcctt cttctgtttt tcttttacct ttccttttta aataagaaat 133080
aatgcaagac agatacaaag taattctttt taatttccat tttcactgga gagtgttgaa 133140
ccccgtgagg catgagagca cagtgttcca gaacaatgct tactgctcat tatcacaggg 133200
gtcaaaggct aacgtgcagg gattgttgca gatcgtggac atgctgcctc ctgtgtccat 133260
gactgcaatc gtctacctat tttacagttg ttgagcactc gtgtgcatta gggttcaact 133320
gggcgtccta gggctccctg gacccatttt agaccttgag ttcttgagtt cctcaaaaga 133380
gaaatcacgc atttatgttt tctcttctta gaccatccag gaggtggctg gttatgtcct 133440
cattgccctc aacacagtgg agcgaattcc tttggaaaac ctgcagatca tcagaggaaa 133500
tatgtactac gaaaattcct atgccttagc agtcttatct aactatgatg caaataaaac 133560
cggactgaag gagctgccca tgagaaattt acagggtgag aggctgggat gccaaggctg 133620
ggggttcata aatgcagaca gcagttccga tggctcccag cgagcttgtc actcaattcc 133680
acctcggaga aggcttttat ttttacccag tacacgtgca ctgagtgccg gctgtgtgta 133740
agatactgca ggggaagtta ctgagaagat ggcagatact ggaatgggaa gatttaagcg 133800
gggtaccagt gtttacatgg acatgaaaaa atactgagag atagtaagaa atcgtaaaga 133860
ttctgagtaa aagagagtat gaccaaacaa gctgagcagg aatcgtgaat ctatgtgtgt 133920
aggcagtgaa taaactgcca gtcttattac ctggacctca aggataaaag acatacagta 133980
aaaatcaacc cacattgagg acagtttcga gagtcgcgct gctacacaga aagccctgtg 134040
taagttaagg atagagaatg aggtgttcta gaactttgaa tttttgtgag caggactcgt 134100
gaggttcctg tgagaggaaa caatgaagga tgatagaaaa gaagggaaat tgattttaaa 134160
aaactggaga tagcagtgat tgtgcctcac tgtgcagtgg gtttggggcc aggaatgtta 134220
aattggtaac ttcatttaac gcccacaacc tttcttcaaa gtaggcactg tacagatgcc 134280
ccttgactta tgatggcatc ctatctggct ggaccccgcc gagggtgaag gcgtcattag 134340
gtcggatttc agggctaatt gaatgtatat tgccttcaca ccatggcaaa gtcgaaaatc 134400
tgtgttaaat catgctaagc cggggactgg ctgtgctctg ccatcgtaca aataaataaa 134460
tggaagtcaa gtaactccct tgagggcccc agctagtgaa tggagaggcc agctatggcc 134520
accactctct gccccagggc gctcaacgcc cctcctgtgc catgcagttc tgacagggag 134580
gcagtgctgg taggaaaggg gtgtgatgaa aggggtgccc agcagaggga gtcatatccg 134640
gagtgacagg agcccaacag gggtgcagcg ctggaaccca agccagcacc tctggtcatg 134700
gctcctcagt tcaccgccta taaaattgtg tggttccccc acaccccttg ctgctcagag 134760
cagccgcgca catgcttgtg ctgtgcgtgc ctcctgtgag atggcctggt acaccggttc 134820
ctacagtgcg cctcacacgc tgtctcggag ggaggcagcc tgtgcgggtg cctggacctc 134880
cgagccagac cctctgggtt cctgcctggc cccgtccctc agcagccaga tggctcggga 134940
gcacattctc caatccctcc gtgtctctgt ttcgtcatct tcaaaaatgt ggatggcata 135000
gctgctaaaa aatggtgaca tacttcctag gtggtgcaga aaattaagtg actgtaggaa 135060
caggcctcag cagctccttc cacttccttg gtatgattgt tttttaaacc aaggctggga 135120
ttgtatagat gcagattagt taatgtgata ccattaatag ctaacctagt gcctgctgca 135180
gggtgagcct cccctaagcc accgggaagc ggctcctgca gcctccctca cgtgtgctgg 135240
ccctcctctg gcagtcattg cctgtggtgt gctgaaggcc cagctctgac tgtgcctctg 135300
tgctctcctc gccccgcccc ctgctctctc tcaggtcttt ggtctgttgt ccgagctgcc 135360
acagcagcct ggacatccct gttggtgttt ccagccctgt cctctcctga gttccatcca 135420
cctgtgcatg gctttttcat gagtgtttc acggatggtt ctgctgtcat ctccaacctg 135480
ataaacaaag caccacgatt cagcccttat gaccccaagc ttccttcctc agttccttgc 135540
ttctgtgcat ccactgaaga agcctgttcc actgtttccc tgcactgggt ctcctgtctg 135600
caggaagcct tcagccctca cttccacact cctctaagat gtgtgcctgt gcccttctgg 135660
ggaagctcat tttcctagca gcctccagga tcttcagggg tgaatccctc ctttcccacg 135720
ttggtactct gtacacacaa catgcccatt ccctgcctgg ggagctgggc attgcttcat 135780
gaatcagagg tcaatttttt ctctattaaa gtcacagatg ctcattgcac cattgtgaga 135840
atgaatgaag atagtgctta taaatcagcc agcaaggtac ccagcctcac tgtgtcaggg 135900
tctccctggg catgaggtgg ttagagtgtg tgacatgtct gtccccaagc ctgtcagctc 135960
ccagatcgaa gccagtggat ctcattcatc ctcgcagcgc ccacagcact tgcacagggt 136020
tttgtacaca taagtcattc tgtcaatgtt catgtttaat gtcatcagtg gaacactccc 136080
actttgtaaa gacttgaatg tgttcatccc tgacttttcc acatcttgtt agttcttctt 136140
tggaaacagc tgtacagttt caccatcctg tgcatccctg gagtctacct gtctctgtca 136200
tacattcaga ttcttcttgt ttcgtgtcac tctcatatcc ttttctctaa tgaaaagctc 136260
cgcctgggca tgcaaggtgg agccctggat gccagcccct cacctggcat ccagggctgt 136320

agcactcagg aactgcctcc ctgccctgcc taccccctac atcatgcgac cattccagtc 136380
cagccaatca gccccttggg acccagctta ccacatgcat atcatttatg ctgtgaccac 136440
tgactaaacc attctcttcc ttcctcccca tatttctaaa tttctaatca ttgctcaaag 136500
cccaattcag agaaaaccct agctcctcca tggcaccatc attaacaatt ttatctggcc 136560
gccccccggg aagttcactg ggctaattgc gggactcttg ttcgcaccat ggcatctctt 136620
tagcagaaca taaatgcgaa gagcacatgc atccttcatg ggaatttaaa ggagctggaa 136680
agagtgctca ccgcagttcc attctcccgc agaaatcctg catggcgccg tgcggttcag 136740
caacaaccct gccctgtgca acgtggagag catccagtgg cgggacatag tcagcagtga 136800
ctttctcagc aacatgtcga tggacttcca gaaccacctg ggcagctgta agtgtcgcat 136860
acacactatc tctgcctcca gctcctatgg gggacagctc tacagcactg gggcagggga 136920
gagaagccat gtttagtaag tcacattaat cagaaacaaa aagtagtaag caaaatatct 136980
gaccactaga aaagcatgta tttaccacgg acatagagat cgtttttttg tggcgggtgg 137040
cagcccagct ggttggcagt gcaggccacc ggaggcagat cccctgcagg gacagcagag 137100
cacttgtgtc ctgagaagag ctgctgttca tggggctggc agcaccaggg cctctcctag 137160
cctgccctgc tgacactggc cagactccta catgcttctg agtctccaga ggctacccgg 137220
ccctcctgaa gcaccagggc tgaatccacc cccagctgag ggcatgaaca ctgccacatg 137280
gagtcacaca cacagctggg cactgccatg gagaggaagt ctgtccatgt ttccttgaat 137340
actggtggcc tggtccctgt cccattcccc agtgaggcag cctgtgggga agcctggcag 137400
ggaaccaggc gcaggtcagc gtggcgccct gactcaggcc agcactgatg ggggactctg 137460
agacgcaagc tcacactcac ccagctcccc tgggctgcgc ccgttcctga tcgcttggac 137520
tttctgttct ttagagtaag aagtgatcac catttcctgc ttctttgttt ctccacaact 137580
gtgcagtgga tgcctgtttg ttttctgccc tcagaacaaa aaaaaaaaaa aatagagctg 137640
acgtgaatct tcaaaatcat caactacagg gctttggatt tttgtgtatt tgttttattt 137700
tcattttatg gatggattgt gatgaaatgc ccgtaataca agattttcca tcttaaccat 137760
tgtaagttac aatgtcagtg gcattataca tccacatggg tgtgtggcca tcaccaccgt 137820
ccacacacag aactctttta tcttgcaaag ctgaaactct acccattaga cagtaactct 137880
ctgctctccc ttccttccca gcctctggcc ctggcaggca acagtccact tgatgtctct 137940
atgaatttga ctgctctggg gctctcatac aggtggaatc atgtagtatc tgtccttttg 138000
tgtctggctt atttcaccta gcaaaatgtc ccgaaggttt atccatgctg tagcacgtgt 138060
taagaatgtc cttcctcttc atggctgaat aatattccat tgtatgttga cactacattt 138120
tgtttgtcca ttcacctatc tacagacact ggggttgctt ccatcttttg actgtttgaa 138180
taatgctgct gtgaacatgg gtattgaggc tctttgtttt atagacatat tattccacca 138240
gatacccatc ctgacaccta ctatgtttgc aagaaactga aagctttatt ttacattgca 138300
aaatttcata ttatgagatc aaggttagca tttcctcagc tgtctggtgg acaatgggga 138360
ggttaaactg tgcacatttt attttttttt aatgaacctg gaacggttat ggggccagtg 138420
tttgccatgg atcaggtcag gcagcccaca atggcaggtc tccatgttct gtacaacaac 138480
tgtgggaaag acccacagag aaagtgctgg aaaggggaat gatgggtagg ttcatgcagt 138540
aaaaagattc aaatactaca gggcattgaa ctataggcca atatagcatt gctttaagaa 138600
taaacaaaaa ataagacagt aagaataagc ctagcaaaat caaaagtcta taaagaactg 138660
acatttcaag ccaataagag aataattcct tattcaataa attgtctgga atgacttaac 138720
tattaggggt gaaaatatca aagtgagaga actataaagg gtttttaaaa aggaattagg 138780
tatgttgggt tagtcgcatt ggagagtgca aattcaccat cgacctgata cctgaaattt 138840
cctccttacc atctagaggc aagttgggaa tgctgccagg ctcctgtggt aaaggaagct 138900
cctctcttga ctggtgcttt atggctacac gttcctgctc agaatggatc tcatttagtc 138960
ttcaccaaaa aaaaaaatct catgagatga tttaagtgtt ttatggacaa gatgtctaaa 139020
actcagaaaa atttcacagt gtgcctagct tttatgttta tgttgaagtt gggcattaga 139080
agttagaatg aatgggttta cttcagagaa aattaaatcc atcacccact ccttgtacta 139140
tgaattccaa atacatatta aatacatata ataaaatatt taatatatat gtaagtgcca 139200
gaaggaaaca taaatatgaa tattttgtaa tatcaagttg aagaaaagcc aaaatctgac 139260
atcataaaag aaaactttca agtaaaatat gttaatggct accaggaaaa tattgtgcaa 139320
tgtctgattg ccatgaagag ggttaatatc cttgctatat cactctgtga agtcatcttt 139380
aaaagactaa gaaaaagatg aatctcttaa taaaaacctg gcccagaaca tgagcagcct 139440
ctctctctca ctctcactgt ctctctttct gtcacacaca cacacgcaca catacacaca 139500
cacacacaaa tatggccaag aaataaagta aaatgttatt tctaatgtaa taagtaggtc 139560
aaaatagaaa aagaaagcat cacaccttcc tttgcaaagt atttgggttc cttttgcttt 139620
taaacacctg ggtcagctgg ggtgtcgaga aacagaaatt ctcacgttct gcttgtgggc 139680
atatatgtta ataaaaccaa gcttggcaat atgcctgcaa tatgtatcta aagcttcaaa 139740
gtatgtatag ctttgaccaa tcaatatcac atttcggaat aagagaaaaa gaaataatga 139800
aagtgaaaat cataagagat gtagaaacat attcttatac aagaattcct tgcagcctta 139860
tttataataa attttgtgaa caaattatat atctaaaaat aagagattgg ttgaaaaaat 139920
tatgcagcag ccatgctatt gataatcatg ttagatagaa gcatatttaa aggcatggaa 139980
aaattgccat gttttatatg ggtttttaag gttataacac aatgtatagt gggattccaa 140040
ttcctgtata tacatagact tatatgtcta tattgattaa ctctggatga gtctcatgtc 140100

ttctttttgc tttcttctat tatccatatt ttatacgatg tgcctgcatt tctttttgt 140160
aacagatggt caatactaga atcataaaca gatcttgttt gtttattggc aaatgtttcc 140220
cgttagaaaa agatgcattt ttcttttaaa tatttttatt ttatacaatg attacaagct 140280
tataatagaa atttgaaaat tatatgtgag tacagggtaa aaagttgaaa gaatgggatt 140340
gcacgctaca gatctagctg cttttagcac gcctgcgtag gaccttgctt tctctagacc 140400
tctgttgcag tctctctgcc tacctcctca caacgtccat cccccgcggt cactgtcgtg 140460
atgccagcct ccccggcctt catgtctcta aggagcacca gcgcggcaat tagcgccctt 140520
tgccttggtg gtattctggc ttcacagtca catgggagat caatcgtcag cttttctgtt 140580
tgaaatctaa attcttcctg actgcagggg acctcgggac ccatgaacac ctctagttta 140640
ctatgtcttc acagtaaaag atatctgcat gactggactc tttaacaaat ttggtggtta 140700
acctactctt tctatataga tatagcactt cgaccttcag acttctcaat actgataaaa 140760
agaaaacacg acagatgaca ggaaaacctt tgcagctata atttgtaatc ggccaattat 140820
aaaaactgca aaaattgacc agatagctaa ggttttacac agtcatgaaa gtgatctgca 140880
ctgttaacat ttcaccctct gtgcaccatt ctgtgcttct ctctggtttg gagtctagaa 140940
ggttttattt acaggctatg acttaacaat cccagaacgg ctgacacatg cagtcactca 141000
agactggaca cagcaaggaa gtagtgggtc catgccaaag gctcagccag acgagacact 141060
ctagctgtgg caggagatgc cagggaatgc tccaagccta agcagattgt aaacaaggaa 141120
cctcaaattc atgaaaaatt cttgcttatg tggcccatgt cagtaattac tctctgcctc 141180
agtttccgca gctgacatgt aaataaaagc agttcatggt tcatcttctt ttcttatcgg 141240
ggtctcaagt gattctacaa accagccagc caaacaatca gagaataagt tgaaaagatt 141300
gtcttcattt attgaatgtg cttaactcag gcccgggaaa gggcgtcatc agtttctcat 141360
catttcactg agatatgcat ctattacttt tacatttcag gccaaaagtg tgatccaagc 141420
tgtcccaatg ggagctgctg gggtgcagga gaggagaact gccagaaacg taagtcagtg 141480
aacagcctca gacccatgtg tgaccgcccc tctcttcctt cacttgctta ggtgattgga 141540
tttgttttcc ctctgaagac tccaaagagt tactttatta cagggtcaga tgtgaaccag 141600
taggtgaagg acagtcttgc aaatctcacc gcatgcagtt aatccagggt gggctatttt 141660
gggagcttca gcctatcaca aataagtgaa catcagcagg ggctgggcgc ggtggctcac 141720
ccctataatc ccagcacttt gggaggcgga ggcggtcgga tcacgaggtc aggagatcga 141780
gccattctgg ttaacacagt gaaacctcgt ctctactaaa aatacaaaaa attagccggg 141840
cgtggtggcg ggcgcctgta gtcccagcta ctcgggaggc tgaggcagga gaatggcatg 141900
aacctgggag gcggagcttg cagtgagccg agattgtgcc actgcattcc agcctgggcg 141960
acagagcgag actccgtctc aaaacaacaa caacaacaac aacaacaata agtgaacatc 142020
agcaagtacc ccagccctgt cctctgaaca cagcacactt tcccaggaat ggaagacttg 142080
ctcctgttga cagcagtcac cagacttctt gtttcctctc cctccctggc tttctttggt 142140
acccacctac acagaagcct gagcacgggt tctcatgggg acttttccat gtggaccctg 142200
ctttacgatg gagagggcca ttctcctagg tatggttgtc tggctcagcc tctcagtggc 142260
caaggaacct ggggacatga gctcaaaaac ggacactatg tccttaagct gaattgtggg 142320
ggggctgtta ggcccttcta aacactactt cccagcaggt atttttgttc tttgtatgtg 142380
ctttctgcat tgcccaagat gcatctaatt atttagcagg tctcaaagtc tagacttgat 142440
ctcatgagtt ctcttaagtg attaaaaata aatcaggaga aaaaagaggc aatcagaaaa 142500
gggcatggtt tgacttagtt tgaatgtggt ttcgttggaa gcaaatgtgt cttcactttt 142560
tcatgaaaaa gtctgcaagt gctctgcgac atccctggga aatgatccta ccctcactct 142620
tcagctcaca gggaaccttt gctctttttc agtgaccaaa atcatctgtg cccagcagtg 142680
ctccgggcgc tgccgtggca agtcccccag tgactgctgc cacaaccagt gtgctgcagg 142740
ctgcacaggc ccccgggaga gcgactgcct ggtaagatgc ccctccagca gcctccctgg 142800
agcaggctgg ggctgcaccc gccccaccca caccaggaca gaagacttcc tgtgggggag 142860
ctgtcaatta gcatttgtca taacagacag gatattgccc tctgcctggt gacaaagtat 142920
ctttagtatc ctgcctccac cactcactga gaccttggga aaatgatggg actaccatgc 142980
ctccatttcc ttacctgaca atgatgcata acaaagtctc tcccagttga atgcttaaat 143040
gatgagatgc ctgtgatgtc cgtcattagg acctgggcac agaacaagca ctaaatacta 143100
catgcaagta tttgtcatga atgtgccttg ttgccagcag cacactctct ttattgtttg 143160
acttcggcta tacctctaga gacttgacac tgtgaggtcc ctaagagacc catggagagc 143220
cacacaggtc ttgctggctg gggctgggtt agggcctcct gacacggatc cctcggctcc 143280
tccaccactg ctcaggcacc tcctgagctg caccctgccc tcaaggggtc ctgaagtact 143340
cactgtcgcc ccattgctcc agaaagtgcc agcagaagcc ttgctgcccc agcgggctct 143400
gagcagcact ggagggtaca ggtcagaagc gtcttggaag tcctggagac gccaaggctg 143460
gtggatgtga ctcctggagt gggagctggt gtgacgaagc ccttcctaag actaaatcca 143520
gagcactctg tggtttcaga gaagattcct aaattccaga gtttggaccc agacccagga 143580
attgtgactt ggttggcctg agctgtttct aatgtgagcc ccagggagaa gactgtgcgt 143640
ggggttggtc ctaggaaaag ccctcgctgt attgggtctg gctcctttac acggcattgt 143700
tctagcaagg ctttctgcca ttcagcaata cattataaaa tataccctca attgtacttt 143760
ataagggaag cccaatgtcc tttataaggg aaattaaaca taatttcatt ccatagtcac 143820
cgctataatg tgtgaactcc atcatctata cgttagtaaa cagacgtatt tttatcataa 143880

tccataaatt atgataggtg ggacagtgca cctaagaaaa aaatggactt tttagagaag 143940
ggtctttctg actctgcaga gggcgccagc tgggttttcc cacactagtg gaacactagg 144000
ctgcaaagac agtaacttgg gctttctgac gggagtcaac accgtgctgc gcttcctccg 144060
tgtgtggcgc tgagtgtact tacctcactt gcccagcgtg tcctctctcc tccataggtc 144120
tgccgcaaat tccgagacga agccacgtgc aaggacacct gccccccact catgctctac 144180
aaccccacca cgtaccagat ggatgtgaac cccgagggca aatacagctt tggtgccacc 144240
tgcgtgaaga agtgtccccg tgagtcctcc tctgtgggcc ctctaactgg tcaggcatcc 144300
ttgtcccgct ctgtctcctg ctgagccctg gagtatccca tcttggagag tctttgggtg 144360
gatgtgtttg ccttgcttgg aggaggcgac cctgtgcccg tccaggcaca caggcgaggg 144420
gaggggctgg cttgctaccg aggagcgggc aggtggtggc catctccacc catggggggct 144480
gctcagtgca cagggcagat ctgggtggcc aggccacctc acaggagaaa cacctgctgc 144540
tcagccctca ccactcatcc agcagccaca gccgtgggta ttcagttgtc tgctgggcac 144600
aaagccgtgg gcatgccact gtttagtgct tgtgccaagc aggtatttaa tacaccgaaa 144660
tcagagagtc tatcagaaga cctgccttct tgagtggtta aaattctagt gaaagttatg 144720
cctcttagga gtattgcaga ggttttgttt ttgtttttat tttgttttgt tttaatggtt 144780
tgggtttgag ttttgcttgt ttgtacttac atttgtactg gtggctccag ggtttaggga 144840
aattgtgaca taaaataatt cctgacagag aaagcaaaac tttgtctaat gaaagagttt 144900
tagaagccac tcttgatctc tagaagggga gattaactga gaaaaaaaat tgaaagaaca 144960
attatgaggg ggagatttta ccctgccaga tttgtgtaca tgaaaaattt tacattccgt 145020
atggaaaaaa aaaacacaaa ataataagcc attataaggt aaatgacaaa caaagctaaa 145080
gaaaaatgtg ccacagtgat gacacagata tatctttgag atagggctta acagagcttt 145140
aaaatccata ggaaaacact tcgagcctga gataccaaga gcagatggtt cacagaagaa 145200
tcatcaatgt cctataaata tttttgagga tcttcttggg gaacttaaaa caggaacagg 145260
ccaggcacag tggctcattg gctcatgcct ttaatcccag cactttggga gactgaaggg 145320
gctggattgt ctgaggtcag gagtttggga ccagcctggc caacagggtg aaacctcgtc 145380
tctactaaaa atacaaaaat tagccgggcg tggtggcgca cgcctgtaat cacagccgct 145440
caggaggctg aggcaggaga attgctttaa cccaggaggc ggaggttgca gtgagctgag 145500
atcacaccac tgcactccag cctgggtgac agagcaagac tccatctcag acaaacaaaa 145560
aaggaagaca tagagctcct aaaaataacg cagaagtctg ctattaatac aaatgaatta 145620
ctttaaaggt gagagcaggt ggaggagagg gctgaggtgc ctgctgggac gcaaaacagc 145680
tggcccctca agggacccag tgtttcctgc catgatgaaa cacctgtatt gtccacattg 145740
cggcctagaa tgttattaaa ctcttgaacg ggattccttc tctatttgca acctttcatt 145800
ctttgtcctt aaagtaaata aagccaaagg aggatggagc ctttccatca cccctcaaga 145860
ggacctggac cgcctgtgtg aggcccgagc acctggtgcc accgtcatca ccttcctttc 145920
atgctctctt ccccaggtaa ttatgtggtg acagatcacg gctcgtgcgt ccgagcctgt 145980
ggggccgaca gctatgagat ggaggaagac ggcgtccgca agtgtaagaa gtgcgaaggg 146040
ccttgccgca aaggtaggaa gcccgccggt gtgcggacga ggcttgttct cggctgctga 146100
ggctgggctc tcatgccacc tccaaaggaa cacatcttcc tcttctcatt aaaaaacaac 146160
tatacatatc gtttctttaa aacagaagat aaagctgtaa agctaggtta ggcaatggga 146220
aggcactgaa ggttgtgacg gggtgggggg ctctgatgag aacagtcaca gagccagccc 146280
cgctcagcag ctgccaggtg cccagccctg gggagaatcc agggaaggca gagctggaag 146340
cagtgcagct ccaagcggcc catgggaaat aatgaggaga acgcaaggtc agtgtgaggt 146400
gacagggatg gcatctccta caccgccgta gccccaaagt gtactatagg tcctggtgtc 146460
ccccttccc gcctgcactc tccccagccc cttcagtgtt tgttgagtga atgaaggatg 146520
atgtggcagt ggcggttccg gtgaccggaa ttccttcctg cttccctctg cctgtggatc 146580
cctagctatt cttaatccaa caaatgtgaa cggaatacac gtctctctta tctctgcagt 146640
gtgtaacgga ataggtattg gtgaatttaa agactcactc tccataaatg ctacgaatat 146700
taaacacttc aaaaactgca cctccatcag tggcgatctc cacatcctgc cggtggcatt 146760
taggggtga gtcacaggtt cagttgcttg tataaagaaa aacaaaatct gccttttaa 146820
ctggtagaga ttggtgatca ataatcaccc tgttgtttgt ttcagtgact ccttcacaca 146880
tactcctcct ctggatccac aggaactgga tattctgaaa accgtaaagg aaatcacagg 146940
tttgagctga attatcacat gaatataaat gggaaatcag tgttttagag agagaacttt 147000
tcgacatatt tcctgttccc ttggaataaa aacatttctt ctgaaatttt accgttaatg 147060
gctgatgttt tgatattttt caaaagtgca gtttctcctg caggcaaaag gggacacgtt 147120
aagtccaggc ttgggtcatt cactgcggtg taaacacgct ttctccctcc cgcccggccc 147180
cagccagctg ccttggtggc ccataacccc tgagggtaga gggaggggac aggggtaggt 147240
gacaggcagc ctgggcctca ggcttttgaa actggacgcc agagccttgt ggggccacgg 147300
gcaagcctcg ggtctatgac tgccgcctga gctccgcttc cttcctctct aaaatgggaa 147360
gattagacca aaataacaag actgttttaa ggttggaatc aaataaggaa aatttgtaaa 147420
gctccttgta tgtgatacca gatccacaat tggcagataa tcgcagcagg agcctcttcg 147480
gggtaatcag atacgcggcg cagcaggggt ctcagggcca cagccagggg ggcggcggga 147540
gacatgcgga atcgcagcgg aaggcgggag gcagctgtga actgtggctc ggcctgcgtc 147600
cgccctgcgc atgtacactc agagaagatg ataatgaaaa agaaagcaaa tccaatttc 147660

ccacttactg ttcatataat acagagtccc tgagagtcta gagtaatgtc tcatacaaaa 147720
aagaaactcc tacgtggtgt gtgtctgaag tctttcatct gccttacagg gtttttgctg 147780
attcaggctt ggcctgaaaa caggacggac ctccatgcct ttgagaacct agaaatcata 147840
cgcggcagga ccaagcaaca gtaagttgac cacagccaaa gcctggtaga ttacatttgc 147900
ctttttagtt ggaaattagg cttaacagga gagttgctaa gatagggcac agagctcctg 147960
catctctcgc cggcattccc aaatgctatc tcacatgagc aggcacaggg agcaagactg 148020
cacgaccact ggcacaggct gtccgctaaa ccacagactt ctcagcgctc gccagtgctt 148080
ctgcttctgt gtccactcca gatcccacat tgcacttagt tgtcaaatct tttcagtcca 148140
tttctaacct atattagctc ctgtgtcttt ccttgtcttt cacggccttg acacttacaa 148200
aacgtgtggg tcaggtactt tgcacactgt ctaaccatgt ctgttcagct ggtgttttct 148260
caggatgcaa ttgaggttat gcacatctta tcacagggac cagagagact ttttagcacc 148320
actcttcaag aatttccact ttttcagctt tgacagtgga atagacatgc aggtgctcac 148380
acacaagcat ctttaatatg gtaatggtaa tcatcagttt agtggtgtgg aggaggagat 148440
gggaatctct tagtgaaacc cgccttggaa gcagcctcgt tatgagaact gctgccccta 148500
cttgactctt aaagcactag ataatactgt gcaacattaa agagaataag agtgcgtgaa 148560
atatgcattg cctcccataa actcccttgg ctctgaatct ctgatactaa atatgtggct 148620
accgttgctt cccagaaagg ccttttttgct ctgaattctc tggaatgctt tctttgacca 148680
agattcttat aaaaataaga gatttagagc aattttcttg gatggctggt atgagccagt 148740
tggcttagtt gtagggattt aaacaagata agggttactt acttttcaca tttaatgaga 148800
agtctggtga ttccagctcc tactgagaca gggtggccac acgttccagg gtgtgactca 148860
ctgaggcccc agacctgccc tgcaaggaaa acctggctct gccctggtgt cctggcctcc 148920
ctgggcatat gtgggggaga attcctaatg gtattggtta caggctccta tgcgagacca 148980
ctcatctgtg taggagaaag gaaaaagatg ggggaaagaa gagcagcagg gagaggagaa 149040
gcctctggat gatactctaa cccccctgcca tccaacacct gaacatcagt ctcttcatcc 149100
agtgctctca gctggcccag cccccagcct ggggtcagat gagagcttcc tgcaaatgca 149160
gatctctttc ctgtggctcc ttctcaatta cagacagctc ctccacaagg tgcactctgg 149220
ccttgtgctc cctccccaaa ccagcccagc cctcccagcc tgcatcatcg tggtcctgta 149280
ggggctagag gttctcacac ccatcgtggt ctggcagagg ctggtggttc tcacacccat 149340
cgtggtccgg caggggctta gtggttctta tacccatcgt ggttcaggag gggctagtgg 149400
ttctcacacc catcgtggtc tggctggggc tagtggttct catgtccacc gcgtgctttc 149460
ctgctcctcc aggtggctga ggacatcccc ccttcggtct gaatgacttc catccagtca 149520
tctgatatac acattggacc acccaatagc atcctagtgt catgttggat ggtgaagaaa 149580
atgccacagt tactgctttc agggcctcac aaccttgggc atagcttttt ggaggaaggc 149640
cccacttccc aggcatccct cccagacctg gtcagaggcc cctgctcttt gcttccatgt 149700
tgcccacact cactgtgctc ttcacaccgg ctcaaaatga tctgcttacg gggttgtgtc 149760
accaccagat caagcgtcct ggagaggagg aaacatattt aacctgcaca gaatttggga 149820
cagagaacct ctagtgtttg ttcaataaat atatgaatgg atagagggac aggttgggtg 149880
gtggatagat ggatgaaccc acacctttga agtgtatttg gctgtttgag aggttagaat 149940
atgttctcaa tttccaggca aaatgaaaat ggagaaaata taatgacatt aaggcatttt 150000
attcatcctc cccatctgcc actgggttaa agatactaaa taaacaagga actatctttt 150060
gcctggagga actttaaaaa cacctgcagt tttcaaaagg tgcagtgtgt gcctcccaca 150120
gcatgaccta ccatcattgg aaagcagttt gtagtcaatc aaaggtggtc tggagaaaca 150180
aagttttcag ggatacattg tttttataat ttttcaccac atgatttttc ttctctccaa 150240
tgtagtggtc agttttctct tgcagtcgtc agcctgaaca taacatcctt gggattacgc 150300
tccctcaagg agataagtga tggagatgtg ataatttcag gaaacaaaaa tttgtgctat 150360
gcaaatacaa taaactggaa aaaactgttt gggacctccg gtcagaaaac caaaattata 150420
agcaacagag gtgaaaacag ctgcagtaag tcaccgcttt ctgtttagtt tatggagttg 150480
gttctaatgg gtcctttatt tgtatttaga atattgaagg gctattccca tttaaattac 150540
ttttttcagt tccttaagaa gcaaattaaa atcttaagat tcctaactgt gaaattacca 150600
tgtgaattcc attaaaactt tttccagatc attaccattc aatgggatga atttaccctg 150660
aggtttaggc taccaattat ttgtaatgta agtaactaaa tttagtatta gttatattac 150720
cttttagttg taggtcactc tctgctcatt tcagcctgta aagactacag ctacacacat 150780
acacacacag aggaatggaa tgagcacttt acatcaacac ttcctgttct ggctctagag 150840
cctcagcttt tgaagctggt gagagcctgg cctgtgctgg gccttggcca cgggcagcgt 150900
cagctttgag tcaagtgctg gtctggcctc cctagctttg agcctctgtc aattccctta 150960
atctgtttag gctttggctt cctcatccat agaatggaga tatgaatgat tcctacgccg 151020
tagtgctttg agagaattca gtgaaattcc tgtgtgtaaa acccttccat ggtgcctagc 151080
acacagcaca cagccaatgg cccaatggct cctatcagct gtgggatttg tcatcagaac 151140
accaccagct ctgctccagg ctgccctggg taccatcaaa acacaccctg tgcccagcag 151200
cacctgctcc tctgcacacc tggttccttc agcaggggca gtggccgtgg gagcacagaa 151260
aacatggagt cccatctggt ttaattgatg ccattgccaa aggggaggac tcacggcacc 151320
ccctctcggg tgccagggtg cctggctccc accaggagga agacctgtcc tccactgtca 151380
ggcacatttc agtcttccca gcagccagca caactacttt gtccttccag tcacggtcgg 151440

cctctgggaa gcccagtctg tgtcctcctc cttcaggggt agccagcatg tctgtgtcac 151500
ccaaggtcat ggagcacagg gcccctcccg ggaaggtgcc gtctcctccg gcccctcggg 151560
tccctgctct gtcactgact gctgtgaccc actctgtctc cgcagaggcc acaggccagg 151620
tctgccatgc cttgtgctcc cccgagggct gctggggccc ggagcccagg gactgcgtct 151680
cttgccggaa tgtcagccga ggcagggaat gcgtggacaa gtgcaacctt ctggaggggt 151740
aggaggttat ttctttaatc cccttgcgtt gatcaaaaat aaggctccag gttgttgtta 151800
tagctttaca ggcattctgt ttgattttct cttcctttta ttctttgccc ttggcttttg 151860
gaggttttgg gttttctgtg gggagacggg aagttgtttg attgcgttat ttttggcaaa 151920
tttaagcaca ataggaaata agcaagtatt attgcctaat ataatccaat aatttataga 151980
atctcttttc ctggaagtat cttaaatttt tctaagctac aaaaagttcc taagacaaat 152040
gagacagtca tcaatggttc atctagccaa caccgtggcc atttgggctt ttctttgtag 152100
tgcccgattc ctggtgtgtg aaaataaatt aacacaaatt atattgccaa gttaatatct 152160
gttttatgtg cccccagcat gtgttgaaca tcaaacagta ccagggactt taaatatacc 152220
cacggacaaa gaaataattc ataatgatgt ttgttgaatt tagttgcaat caataaaaag 152280
tgcagtttgt gaatgctctg aggttcttga tattgatgta aggctttgaa cgacaaatga 152340
ggacaaaaca taaataggaa agtaaaactg aaggatagag gccaaggcca tgttttagaa 152400
gatttaaaga aaaagggaaa tttggtgagc accataggaa ttacagatgg ctgtaggaat 152460
tcttcctgtt ttactctctg ggcatggacc acagcttgga tccagaaata tttaggagca 152520
ggataagagg accaagttca attctatagg aatcctttag ctgataggct cagaacaaat 152580
cacataattg atagtgctgc ttcaacttca agtaaggaat attgatgcaa tccttacagc 152640
tacaaatgga cagtggtctc atgttttcag ttttcaagtg tttcttaaga ggcaaggtga 152700
tgaaaacgcc cacgtgggga gccccatgtc cttccattag tgtagagaaa cctggtgtcc 152760
agcagcacct gctccctctg caagcccagc cccettcagc aagggcagtg acccagagaa 152820
gaagcacaga agacacaacc ctgtatcaca ttttgtttaa tggtgccatt gaccaaaggg 152880
gaggatgaaa ggcacacact tttttgttgt tttttgagac agagtctcac gccatcaccc 152940
aggctggagt gcagtgatgt gatctcaact cactgcaacc tctgccccct gagttcaggt 153000
gattctcctg cctcagcctc ccaactagct ggaattacag gtgtgcacca ccatgtccag 153060
ctaatttttt gtagttttag tagagacggg gtttcaccac gttggccagg ctggtctcaa 153120
actcctgacc tcaagtgatc tgcccgcctc ggcctcccaa agtgttggga ttataggcat 153180
aagccactgc acctagccaa ggcacacact ttggagaata aacactcctt gttcgctgct 153240
ggagggtaga actatgcttg actactaggc agagtccagt cttactgaca aacagccgta 153300
catctgttct gtcttttcaa tcaaacatca gcttcttgct taacattgat gtgtacatct 153360
tgagggatgt caaaatattg taagctaagt ttttcatacc tgtgttccac actcaccatt 153420
tttagtaata accattgagc gagttcattc tccctccttc ctttttctat cacttaatct 153480
aaaattatca tttttccagc ttaattttga taaccatgaa tctggtatta gaggcaggga 153540
acacctcctc aggactatct tttcttttat catttggctt gcttacccaa tatgcaaaaa 153600
ctatgctgta gaaaaagcag aaaagatatc ttgattatga atgaagctcc tgtgtttact 153660
cagagagaag atgacccagg attcagttaa caaaatcagc tgattatatt actatatagt 153720
cctggagtcc caactccttg accattacct caagttattt ggaattttga agaggtgatt 153780
tgtgttcctg caataatgtc tcaggggtgg gctgacgggt ttcctcttcc tcctctcagt 153840
gagccaaggg agtttgtgga gaactctgag tgcatacagt gccacccaga gtgcctgcct 153900
caggccatga acatcacctg cacaggacgg gtaagagccc cttgctgcta tccacgtcca 153960
tttcatggga agggccttca cagaagccga acagtgatga tggcccaggg catcctgtgt 154020
gggcaggacg gccatcagag ccacttccca gaggagacgg caggcgctga cagcgctgtc 154080
cgggcagggt gtcggtgaca ttagcacaca cattagcctg cgatgaacat tcactctttc 154140
tgctgacacc cccaacctta tctaagctta tcaaatcctc acatttaacg gaggctgttt 154200
tcacctggtt tcccccatcc ctgacctagt cagcattgct ttatcgcttt catcaaacat 154260
cctcaaattc ttaacattag cttgtaatta attgaagaat ttttaaagaa attgctagca 154320
aaactttta aactgcacaa ctttgtatct atatgttcaa taacatatag atacaatatt 154380
ctttacaata atcttttaaa gaatatgagt gagaattcgg gcccctctca caccaaatgt 154440
cctgatgttg ttaattctca atgttattat atagggagct ctgtttctt gtgagcttca 154500
acagccagtt ctaaatctac taactgaaaa catttttag acattctcta aattgggcag 154560
aagatgacag gactgtgttt tgagggatag gctgccagcg tggctgctta caaagtaaag 154620
acttggttta taggtttgca tggtgttggg ttaaatttct gtcattaaaa taattggcga 154680
tattgacata gtcatctaat tatgctggct ctgggcacac acagcccttg agtggacaaa 154740
accaacatga gagaacttag ccaaggggaa agcctttccc tgctggtttt atttctgcta 154800
cttctgaagt gtggggcaca caacctgagc agtgctttta tttgagtccc aatgctttta 154860
tttgagtttt gcaaggttat tccaagtttt acaaatagaa ggtagcgtat gactcagtcc 154920
ttgatatgcc aaccactgca cagagacttg ccaccttcct gtcactggag aaacactcat 154980
gtgggttttc ttaaatttgc ctccctctga gcttcccttt aacttcaact ataatatgca 155040
agaaagacta tctgaccata aatacacatt tgggccaatc aagatggttt tgccaaggaa 155100
agatgcccac aatggttaag cagaatgcaa taatgtagag aatatcattt ctttcatgct 155160
ggtgtatatc atatgcattc aaaaacaggg agaacttcta agcaactaac agtgaccata 155220

tcaagcaggt gcaatcacag aataactggt tttctccttt aagaattttt ctatcatttg 155280
gctttcccca ctcacacaca ctaaatattt taagtaaaaa gttacttcca ttttgaaaga 155340
gaaaagaaag agacatgcat gaacattttt ctccaccttg gtgcagggac cagacaactg 155400
tatccagtgt gcccactaca ttgacggccc ccactgcgtc aagacctgcc cggcaggagt 155460
catgggagaa aacaacaccc tggtctggaa gtacgcagac gccggccatg tgtgccacct 155520
gtgccatcca aactgcacct acgggtgagt ggaaagtgaa ggagaacaga acatttcctc 155580
tcttgcaaat tcagagatca aaaatgtctc ccaagttttc cggcaacaaa ttgccgaggt 155640
ttgtatttga gtcagttact taaggtgttt tggtccccac agccatgcca gtagcaactt 155700
gcttgtgagc aggcctcagt gcagtgggaa tgactctgcc atgcaccgtg tccccggccg 155760
ggcctgtgtt gtgcaatgct gcacatcaca acaggagggt agggggacaa aagagcacag 155820
gtcctggcag ctgccacagt ctccaggggc ttttgcgttt ctctccagat ttctaaggtt 155880
aacatgggga ttagctgttt tgcaatgaat aaaaggtaac attgcctgga atgttgctta 155940
aagacacttt tttaaagcta gttgattgtt aagctgttgc tacttaaatt aaaactactt 156000
tgggccagac gcagtggctc acgcctgtaa ttccagcact ttgggattcc aaggcaggca 156060
gatcacttga ggtcaggagc ttgagaccag gctggccaac atggtgaaac cccacctcta 156120
ctaaaaatac acctgtagtc ccagctactc aggaggctga ggcaggagaa ttgcttgaac 156180
ccgggaggca gaggttgcag tgagccaaga tctcgccact gcactccagc ctgagcacca 156240
agagcgaaac tctgtcgcaa aaaacaaaaa caaaaaaaaa agctactttg actggaatta 156300
gcagaagcac tctgattgtg tgtatcttat ttactggaat aataaagctg tcaatcaaac 156360
tggatcccac tcaacaatca gaaagagaag ttgagctgtc atatagtagt tcacacttac 156420
ttctgtttct caaaatcctc agctttgttt ggaactgtta ctcattcttt ctctgaatcc 156480
atctgtatga gttgtgtgcc cttgggcaag ggtcttacct tctctgtgcc tcactttctt 156540
ttctgtaaat tgggataata atgctgcata gctcacagga tttttatgac catgagttaa 156600
gatatgtcat atacttaaaa tggtgcctgg aaaatggtga atactgagtc aatgatagca 156660
tcattgatgg tgggatggtg atgaggaggt gggagtcaca atggtggtgt tgatggtggt 156720
gatggtggtg aggaggtggg agtcacagtg gtggtggtgt tgatggtggt gaggaggtgg 156780
gagtcacaat ggtggtggtg atggtgttga tggtggtgag gaggtgggag tcacaatggt 156840
ggtagtgatg atggtgttga tggtggtgag gaggtgagag tcacaatgtt ggtggtgttg 156900
gtggtggtgg tggtgaggag gtgggagtca caatggtggc agtgttggtg gtgaggaggt 156960
gggagtcaca atggtggtag tgatgatggt gttgatggtg gtgaggaggt gagagtcaca 157020
atgttggtgg tgttgatggt ggtgatggtg atgaggaggt gggagtcaca atggtggtga 157080
tgagggtggt gatgatgatg aggaggtggg agtcacaatg gtgtcagtgt tgatggtccg 157140
atggtgatga ggaggtggga gtcacaatgt tggtggtgtt gatggtggtg atgatgatga 157200
ggaggtggga gtcacaatgg tgtcagtgtt gatggtggcg atggtgatga ggaggtggga 157260
gtcacaatgg tggtggtgat gacggtgttg acagtggtga cgaggcggga gtcacaatgg 157320
tgtcggtggt gatggtggtg aggaggtggg agtcacaatg gtggtggtgg tgatggtggt 157380
gatggtggtg aggaggtggg agtcacaatg gtggtggtgt tgatggtggt gatggtggtg 157440
aggaggtggg agtcacaatg gtggtggtgt tgatggtggt gatggtggtg aggaggtggg 157500
agtcacagtg gtggtggtga tgagggtggt gatggtgatg aggaggtggg agtcacaacg 157560
ttggtggtga tgatggtgtt actggtggtg acgaggtggg agtcacaatg gtggtggtgg 157620
tgatggtggt gaggaggtgg gagtcacagt ggtggtggtg ttgatggtgg tgatggtggt 157680
gaggaggtgg gagtcacagt ggtggtggtg ttgatggtgg tgatggtggt gaggaggtga 157740
gagtcacaat ggtagtggcg atgatggtgt tggtggtgag gaggtggaag tcacggtggt 157800
ggcgatgatg gtggtgagga cgtgggagta acaacagtgg cagtgacggt gattgagaca 157860
tgatgatgat ttgtcaactt tctaggaaaa caatcatata atctccaaca gtgatatctt 157920
aatatctttt ccaaaagtat cagatcatat tataagggcc aagtttccag aataatatca 157980
gacataatga cagtggacat cagagcttgg catctaaagg taatgggaat agctctaatg 158040
tctcagcgtg aaaaacaaca tttgctatta gtctgagata ctaattatct agttaaggaa 158100
gtactcacct atacctagtt tttaactgtt ttttaaaatc tggaattgat tttgaatttt 158160
aacaaatatt tccctgggaa caatgtaaga ttcttcatat tttcgccttt gggtatacca 158220
acatgccagc tctgttggcc actttgtgag ctcgatgaag catggtataa aagatgcttt 158280
gctagtgttt cacgtaatct atttctataa gcaattttgg agctaagcct ctgaaacaga 158340
attatattat ctgtatagaa taaatgtttt atcttccccc ttttctttct tctggaatag 158400
atgtgcatca gtatctctgc atcaatatct ctatatcagt atctctgtgt cagtgagcat 158460
atgttgctgg gcttagggga ggtccagaaa gtgattgggt tttggcattt tcaatacact 158520
tactttgtat aagaaatagt ttgccaaata tagaaagagg ggatttagtc aagatttaaa 158580
ttaaaaatgt tagtggtcat ttttctaatg tctttctatt ttttcccagg tcctaataaa 158640
tcttcactgt ctgactttag tctcccacta aaactgcatt tcctttctac aatttcaatt 158700
tctccctttg cttcaaataa agtcctgaca ctattcattt gacatatgga attttataaa 158760
tattttcttt agtatgtgtg attacattcc tgattctgag ccttttttaga tgagtatata 158820
gtttgatata atcttgttat tgccacctgt gtcttctccc aaagccatta attatatagg 158880
aattacacga tagaaatggg tttaattttt aaaatacggc caagtgttga tgagagggaa 158940
aattttttta atttctttca ctgagtattt atgacgtgca caacattcct gaatatattg 159000

tctctctcat ttctcagatg ggatgtattg ccttctccat ttctattgtt aaagaaacac 159060
ttacaggggt ttctttaaca acttgtgaac agcagcatca gagcccagac tacagcataa 159120
gcagctgctg attccaaaag ccctaccttc caaccgggca ggtgcagcca cccagacgag 159180
ggggaggaac cctggaggaa tagctatttc tttttttttt ttgtcgagac ggagtcttgt 159240
tctgtcaccc tggctggagt gcagtgccgt gatcttggct cactgcaacc tccacctccc 159300
aggttcaagc aattctcctg cttcagcctc ccgagtagct gggattacag acacctgcca 159360
ccacgcctgg ctaatttttg tatttttagt acagacaggg tttcaccatg ttggccaggc 159420
ttgtcttgat ctcctgacaa gtgatccaca caccttggcc tcccaaagtg ctgagattac 159480
aggcgtgagc cactgcgccc agcaggaata tctattttta aatggaactg tgttttcata 159540
gtacacggtg aggagaaagt tgctttgaaa tctttatcct aataaaccaa ataatatgaa 159600
aatttgccta ttttaattat atgtaacaaa gtttagttac tgctataatt gcaaatatgt 159660
ataaattcct taccaaaaaa aaaagaatca agtgggagcc agagaataat ttttctgaca 159720
gaattaaata acatgctata gctgcttgag ttcatactca atagtcattt ctgcagagtt 159780
accgagggcc tcatcagcgt cagcaggagc ccctcgcctt ctgacgctct cacatccttc 159840
tctcctgcag ccccgtcctg ccactgtcct tgtccagctt ctcttcaagg gtcaactggt 159900
ctacctttcc ctacaagtct gtcacagctt cttgttagca atccctatgg ttgcccaaaa 159960
gcattttcag agcctgcata agactgcatc ttgtagaaaa tttgcagttt caatctgccc 160020
tccctctgcc gggtgttccc attgtattgc attcagcagg cagggagaga ctgctattag 160080
gtctgttcct gagtgactgc tttctgtctc agactgtttg gtgtctgtag gaggtagtgg 160140
ggtgggcagt aacgaggtct cctgtatatt ccacccctac gaagcctgtg tgtttggttt 160200
atgaactaag ctcaaaagca ccacaggggt aagactgcag tacatgacac catggaaaag 160260
agggagcacc cagaccccca aattaagaag agcagtgtag agaacagaga cctggagagc 160320
agagatagaa actgttagga tcagattata gtgttacacc agggctcccc aggcctctca 160380
catattgaaa tgtacttgtc catctttctc caggccagga aatgagagtc tcaaagccat 160440
gttattctgc ctttttaaac tatcatcctg taatcaaagt aatgatggca gcgtgtccca 160500
ccagagcggg agcccagctg ctcaggagtc atgcttagga tggatccctt ctcttctgcc 160560
gtcagagttt cagctgggtt ggggtggatg cagccacctc catgcctggc cttctgcatc 160620
tgtgatcatc acggcctcct cctgccactg agcctcatgc cttcacgtgt ctgttccccc 160680
cgcttttcct ttctgccacc cctgcacgtg ggccgccagg ttcccaagag tatcctaccc 160740
atttccttcc ttccactccc tttgccagtg cctctcaccc caactagtag ctaaccatca 160800
cccccaggac tgacctcttc ctcctcgctg ccagatgatt gttcaaagca cagaatttgt 160860
cagaaacctg cagggactcc atgctgccag ccttctccgt aattagcatg gccccagtcc 160920
atgcttctag ccttggttcc ttctgcccct ctgtttgaaa ttctagagcc agctgtggga 160980
caattatctg tgtcaaaagc cagatgtgaa aacatctcaa taacaaactg gctgctttgt 161040
tcaatgctag aacaacgcct gtcacagagt agaaactcaa aaatatttgc tgagtgaatg 161100
aacaaatgaa taaatgcata ataaataatt aaccaccaat ccaacatcca gacacatagt 161160
gattttaatt atttaagagt agtttagcat atattgcttt atgatttaat taaaaatctc 161220
caaaatatat gccaaagaag tagaatgaga aaaatgtata tttctctttc acttcctaca 161280
gatgcactgg gccaggtctt gaaggctgtc caacgaatgg gtaagtgttc acagctctgt 161340
gtcacatgga cctcgtcaag aatgaccaca ctgctgtggg tgaagatgct ttcctgcatt 161400
tctgactgtc ctctgtcctg atcaagtttc tatggctctg ggccagccta ccctcagcca 161460
gggtttctgc agagactgcc cagctggttc cacgtggctc cacgtgccaa ctttgtcctc 161520
agtggaggga aagttggaca cacagtgctg gggctgctcc ctgctccgcc gttgctcgat 161580
gcatggcctg cctctgaatt ccttggttcc actggttttg ctgggtcctt ctgtgcctct 161640
agctcctctt tttttctgtc cacttacccc attggtccca tcacaagcct gtgtgtgagt 161700
ggcctttctg ttcgatgaca acctccagca taggggagtg tttctccttg ctttctttcc 161760
cagacacact gcccagcaaa ggcaaaaggg cttccttcaa catcagctct ggccagtttg 161820
ccagagcaaa gccctgagaa aagcaaggtt gaaaagtctt attcaaactc accaggaaag 161880
agtggtgtta ctctcgatgg cgtctagcca ggaatcatgg aattatacac cgagcacctg 161940
tttgccattt tggatgtttc caaacatgaa ccaaacttcc aggcccctct gccatctctg 162000
gtaacattta caaagtccct tcctcaccac tgcccttcct tcattttggc atgctcctcc 162060
gcccccgagt tgacagccat agctctctct cctgccacca gtgtcacatg atcgaggaag 162120
aaggcaactt caaaaagact gggtcccctt ccactcccat ctcttcagtg agctgctagg 162180
acacccagca gaacttcccc actccacact gcaatctcag ggatcttagt cacggggctt 162240
tccaccatgt ctccacctgg aaaccagtca tggccattcc ttcttacatc tgctcttttc 162300
catctttttc ttctcctcct gttcacccgc ccttactctt gtggcgccct atggatatgc 162360
gctccatagc aaatgattct ttatatctta cggtattcta gtgagctggc acatgtggct 162420
tctggtttcc tctctctgga actagacatg acctctgtgg gagggaggat taaatgcacc 162480
ctacagtctg aggctgcatg atgacatcac tcatcacaat gatgctttct atgtctgaat 162540
cctattcctt tataacccct ttcaagctcg ttcagagagt atttcacaca atccatgtgc 162600
tcatcttaaa agccaaggac ccagaggagt ctcagcattg ccaaaaagtc ccttcaccca 162660
gcctggccag aggcagtgcc tggtccatgt gtatggacta tggcacttca attgcatgga 162720
aatactcttg gaatgaacaa aataccaatc catgaaaaag cattattgaa gtctaagtta 162780

ttttttgaat catatttgt taatcaacaa attgaaaaat actcattata tggagaggtc 162840
cagataaagc ctcaatttta aaaaatgagg aaaagtgtgc ctggtagggg actggggaga 162900
gcttgagaaa gttggaaacg ttgccttaga agcctgtttt ttctcctttt agaagctaca 162960
tagtgtctca ctttccaaga tcattctaca agatgtcagt gcactgaaac atgcaggggc 163020
gtgttgagtg ccaaggccat ggaatctgtc agcaacctca cccttccttg ttcctccacc 163080
tcattccagg cctaagatcc cgtccatcgc cactgggatg gtgggggccc tcctcttgct 163140
gctggtggtg gccctgggga tcggcctctt catgcgaagg cgccacatcg ttcggaagcg 163200
cacgctgcgg aggctgctgc aggagaggga ggtgagtgcc agtcctgggt gggctcagga 163260
gccctcgcac cccgacagga acaagggcca gccccgagaa cgggccatta gcagttgtgt 163320
atgttagata cataattgta ttatgatgca gaaagaatct ctgaatgtgc agttataccc 163380
agttggtgac atgttggtac atccatccga ggaaatggca atgtttctag gctgcaccct 163440
tcaatgtcca caaagctgtg tggcatctgc ttaggacccg gtgcctgtgt gtgcatagga 163500
gggaggccag gaagcctggc tgttgatccc atgctggcac tgtggcgaag gcgagagatt 163560
cctgctttgg aaaacaccat tgtccacaca gtggctttgt ccatgatgga cttcgccaca 163620
gcccagtcct gtgctggaag ccatgttctc tggaaagagc aacccagcgg ctcataagca 163680
taagcgcgtg tgatgtgccc caaccaaacg accgccatgc acaacttccc taccggagtt 163740
ttcaatccag ttaataggcg tggaaacaga catagaaatt gtgtttgttg aaaggtagct 163800
gttcagttaa agaacacctg tatcagagcc tgtgtttcta ccaacttctg tcaagctctg 163860
tagagaaggc gtacatttgt ccttccaaat gagctggcaa gtgccgtgtc ctggcaccca 163920
agcccatgcc gtggctgctg gtccccctgc tgggccatgt ctggcactgc tttccagcat 163980
ggtgagggct gaggtgaccc ttgtctctgt gttcttgtcc cccccagctt gtggagcctc 164040
ttacacccag tggagaagct cccaaccaag ctctcttgag gatcttgaag gaaactgaat 164100
tcaaaaagat caaagtgctg ggctccggtg cgttcggcac ggtgtataag gtaaggtccc 164160
tggcacaggc ctctgggctg ggccgcaggg cctctcatgg tctggtgggg agcccagagt 164220
ccttgcaagc tgtatatttc catcatctac tttactcttt gtttcactga gtgtttggga 164280
aactccagtg tttttcccaa gttattgaga ggaaatcttt tataaccaca gtaatcagtg 164340
gtcctgtgag accaattcac agaccaaagg catttttatg aaaggggcca ttgaccttgc 164400
catggggtgc agcacagggc gggaggaggg ccgcctctca ccgcacggca tcagaatgca 164460
gcccagctga aatgggctca tcttcgtttg cttcttctag atcctctttg catgaaatct 164520
gatttcagtt aggcctagac gcagcatcat taaattctgg atgaaatgat ccacacggac 164580
tttataacag gctttacaag cttgagattc ttttatctaa ataatcagtg tgattcgtgg 164640
agcccaacag ctgcagggct gcgggggcgt cacagccccc agcaatatca gccttaggtg 164700
cggctccaca gccccagtgt ccctcacctt cggggtgcat cgctggtaac atccacccag 164760
atcactgggc agcatgtggc accatctcac aattgccagt taacgtcttc cttctctctc 164820
tgtcataggg actctggatc ccagaaggtg agaaagttaa aattcccgtc gctatcaagg 164880
aattaagaga agcaacatct ccgaaagcca acaaggaaat cctcgatgtg agtttctgct 164940
ttgctgtgtg ggggtccatg gctctgaacc tcaggcccac cttttctcat gtctggcagc 165000
tgctctgctc tagaccctgc tcatctccac atcctaaatg ttcactttct atgtctttcc 165060
ctttctagct ctagtgggta taactccctc cccttagaga cagcactggc ctctcccatg 165120
ctggtatcca ccccaaaagg ctggaaacag gcaattactg gcatctaccc agcactagtt 165180
tcttgacacg catgatgagt gagtgctctt ggtgagcctg gagcatgggt attgtttttg 165240
gtatttttttg gatgaagaaa tggaggcata aagaaattgg ctgaccctta tatggctggg 165300
atagggttta agccccttgt tatttctgac tctgaaactt gcattcaatt cactccacca 165360
agttatctca tctttgaaat ggcttttttt aaaggtgcct agaatatgat ggcgtgcagt 165420
ctataaactg ttgcccacct tctgtacttt ctctcagaat aattcacatt cttctccagt 165480
gtctgttgat tgttactttg tggaataagt tcttggaaaa ttccacaaga ttattgttat 165540
cttcttacta ccaattctat tgaactttct ccaccttctc tgggccttcc ccagccagtg 165600
gtgggaagat gctggctgga gtctgacaga gcctcttcta cactggcctg ggcttgctgt 165660
gagttggtgg aaacctttgc tcttgtccca acacagagca agtgaaagag gaggtcaagg 165720
ggctcaggca gcggactagg gaagcagaat cgaggaaaag gaaaaatggc tgacttatta 165780
cctcaaaact ctagagaatt tagttgatct tacagccaag aaggacaaaa gccagagagt 165840
aatatcctcc gcctcatgtc taacccacag aatacatagc aagtaaagag aacatgggcc 165900
tttataaaaa tgtcttaaga tacaattttt taattggagg aaatctacag tttaatttc 165960
tctgggcagc ttttcttcct tttattatag taggggaaat cccatgttga tatacttcta 166020
aatgaaagat gatgaattga tataatacaa taaaaaatct gtaaaattga tgatatactt 166080
atcaagaaaa attagctttc attttaacgg tttacaaatt gagtcaagtc ctagtaacaa 166140
aatgttaagt ctattaacat aaccacaaga aatacaggaa gacgggcaat ctgtgaagcc 166200
tttcacttac aatctctggc ccctcacctg tgctgtgtag gaaaatcttt gtgcacaatt 166260
tgcttcctta attcattttt tattcattca acacattcta ataaattata caaaatcatg 166320
ttgaaatgtg aatttcagtg gtatttataa atgcagtgtg aggagggttt ggatgtattc 166380
taagacaata gttgtgcttt gggaaggaag cagtgttcac tgaaaagtgc ccccaggacc 166440
ttttaattgg aggaaatatg cttctgtgga gttggaaatg gggtagaaga tagataaggt 166500
caaggcttaa aagttaagtg cacccaacat ctgaagcgtc catgggcctg gcatggtggc 166560

tttcgcctgt aatcccagca ctttgggagg ctgaggcagg aggatccctt gagcttagga 166620
gtttgagacc agcctgggca acatactgag acccagtctc tacaaaaaat aaaaaattag 166680
ctgggtgtgg tgtctcatgc ctgtagtccc agccactcag gagatgggaa gatggcttga 166740
gtccaggaga tctaggctgc agtgagctaa aatctcacca ctgcactcca gcctgggtga 166800
caaagcaaga ccctgctcaa aaaaatagtt agatataaat attaatatag atacctatat 166860
atatctgaat atagatatct atatatactc tgtatatagt tatttagata tataaatata 166920
tatgatatat atttagagag atatatattt agagagatat atatttagag atttatatat 166980
attttatata tatttagaga tatatatctc taaatatata tctctctcta aatatatata 167040
tatctctctc taaatatata tatatcccta aatatattaa ataaataaaa gaaataaaag 167100
aaagctcagt ttggcctcct gcttgtcctg tctcctcatc ccctcttccc cctccatcat 167160
tttatttcct tgccccatgt ttcttcactg cggccatgtc ccccctcctc tccaatgatg 167220
gatgtcatgt ctgctgcagt cagagggcga caagcctgga gtgttccctg aagcctgtgg 167280
tttgtggttt gtcctgcagc tcaggctgcc caggcctcac cagcaatcct ggcgggcagg 167340
gcaccacact gggatggaga gggggaagct ggaggaggca ctttctggta aagaaagcaa 167400
aagccagcag tgcccaggcc aatttcaaca gggagttaaa tagcacctta atcctgtggc 167460
aggacagctc atggggccat gtgtgctctt agaaagactc acatgcacgc atgcacggca 167520
gcaatgactc catactcacg ttccccctgca gacaccaggc ccccacagcc ggcacacaca 167580
ctgcagcccc agttccatgt tgctagcagt ggcttagtga atgagtaaag ttcttaaaat 167640
gcaggggaca cctgcccttc attcataagg ctggacgtac acctctcctt aaggagttca 167700
agagctagtg gaatcccaat tcatacggta gagccattca cagatgagag agacaagcca 167760
gaaggaagga accaaaagtc atgtcagcag ttaggacaaa ataacaggct ttcaaggtca 167820
caaagcctca gggacactcc tgcggtggga ctgggctagg agccatgggg gctccaactg 167880
tgcgctctgc ctgccagcct gtgggtgctg gggctccacg aagattgttg tggaatacca 167940
agcatgcttg ctgtaggtca cggtgcacgt ttactacttc caagacaaac agccgagaac 168000
aaagctcgct ttagcttctg cgtacaccga acgggacaca cgactgaaca gcgttcccat 168060
tgtgcctgct gggtggggag gaagtgatgg cccagtgggt ctatcagatg ttagtaggat 168120
ggggcctggc ggggctccag gctctgtgtg gccgacaccc acgccccccg ctctgctccc 168180
cattcccagc cccaggtcag ccctgcgagg ccctgcagca gatgggctgc tcaaactgct 168240
ctggtttgca gatttttctt ccctctcaaa tgaatacaat atgttttcaa gtctcaacca 168300
gatcttgaga aaataggaag agccagaggg tttctttggt gttatggttg tacagcttcc 168360
cagactccgg gggagagatg tgatttgtgc tttctggcaa tcccatggcg tattaaattt 168420
tcataggctt tccagtttaa atttagggta ggcaatggaa gggaacgcaa aacagatttc 168480
taggtgtact gtgtgtgtgt ctcccacgtc taaagtctgt taactggagc acccaacagg 168540
ccccacaggc tgccttcaca cagaggacct ggggcgcctc cgacccattg gggtgagcag 168600
tgggccatgg agggagccag ggtcaggaga cctggttgtg ggcctgacct gaccctgctc 168660
agggtggcct caggtgggcc gttcacctcg tcagcctcag cttaccctct gactacagtg 168720
acctcagaca aaatacgctt cctggccctg tccagttctg actttttata aacaagcact 168780
tatccaagtt aaagggatat tttcaatatc tactgagtcc acagatatta aatatctcct 168840
ctcttcttta aaattgtggc attatcttta gaatataaaa ggaaaataac acacactctc 168900
cttgaaaata gagagcctaa acactctgca ggaaatattt aaagctatag tttttgtttg 168960
tttgtcttga atgcaagtgg cctggacttt gacttgcttt gagtctttga ccttcatgac 169020
ttcagtacag ttcaaccctg acagttttga agtaggtatg tgcctagatc tgccctagtc 169080
cctgctggaa tgttgaagaa gcaaaggtcc aggccctcag agcacttgcc acgtacttgc 169140
caacagatac ggggcggaga cttgagtcaa cgtaagagca agtgtgtgcc gggtgatccg 169200
acactgcaga gcgccagcta gaccctaagc gtgtgctagg ggctgaccaa gccgttcttt 169260
cctcaaaaac ttggtgggga gggtattttt aaaatcacac aaatatttaa gtacagatta 169320
tgatgactgc ctcaaagcag tggctcttca gcttcatcaa gcttcagagt ccagagggtt 169380
tgttcatatg gaaggctagg cctgtctcct gcatttcacc ctcttggcct gggggcggga 169440
cccaagaatg tgtggctcta aaaggttccc aggcaatgct gaggctgctt tctgaaggaa 169500
aaactgcaag ataccaggag agtttcattt agattgaaga gtcgaggaag gctcctctga 169560
gaaagagtct gctaaggaag gaggaggtgg gttctgggga cagaggttct cccgtgggta 169620
agggtggagg gaagctctcc tggggagaag gtgggcagga ggaccagagg ctggagggag 169680
gagggcagtc agcctcgggg cttcccagga acagggacgg ccagggcagg gtttagggca 169740
aggaaagcgt gtgagcatat ttgtatttta gtaaatattt acagtttgcc ctccatgtct 169800
gcagtttcat atccatggat tcaatcaacc acaatgaaaa acgttgggga aaaaaattgc 169860
atcggtactg aacatatacg gacttttttt cttgtcatta ttccctaaac aatacagcat 169920
aacaattatt cacatagcat ttgcactgta ttaggtacta taggtaatca ggagatgctg 169980
tagatgggag gatgtctgta ggttacacac aaatgctgtg ccactttata tcaggggctt 170040
gagcatcctc acattttgat atttaaggga ggtcctggaa ccaattcccc agatactgag 170100
ggtccactgt ctgtgtcccc tcgccccacc ttgcctttgt ctcctgtctc ctatctccac 170160
cctgcctccc gccagcctgt tgctcctgac ctgcccgggc accctggagc agcaccctat 170220
ctcagagcct ggctcagtgt gttcacttct gcagagaaac taacttgccc aagtccacac 170280
tcaaaacata ggcattgctg agatgtgaaa agcagctgtg gatgctttct gctacagtct 170340

gtgtgttctt ttccatatct gaataaaagg tcaccaccat ttgtatttta aagagaaaga 170400
gaatttatgg gtggaaattg gggattccct cattctcagt cagacagaaa agagggcccc 170460
attgtgtgcc tgattgcaaa taaatttagc ttcctcagcc caagaatagc agaagggtta 170520
aaataaagtc tgtatttatg gctctgtcaa aggaaggccc ctgccttggc agccagccgg 170580
aattagcagg gcagcagatg cctgactcag tgcagcatgg atttcccata gggagcctgg 170640
gggcacagca cagagagacc acttctcttt agaaatgggt cccgggcagc caggcagcct 170700
ttagtcactg tagattgaat gctctgtcca tttcaaaacc tgggactggt ctattgaaag 170760
agcttatcca gctactcttt gcagaggtgc tgtgggcagg gtccccagcc caaatgccca 170820
cccatttccc agagcacagt cagggccaag cctggcctgt ggggaaggga ggcctttctc 170880
cctgctggct cggtgctccc cggatgcctt ctccatcgct tgtcctctgc agcacccaca 170940
gccagcgttc ctgatgtgca gggtcagtca ttacccaggg tgttccggac cccacacaga 171000
ttcctacagg ccctcatgat attttaaaac acagcatcct caaccttgag gcggaggtct 171060
tcataacaaa gatactatca gttcccaaac tcagagatca ggtgactccg actcctcctt 171120
tatccaatgt gctcctcatg gccactgttg cctgggcctc tctgtcatgg ggaatcccca 171180
gatgcaccca ggagggcccc tctcccactg catctgtcac ttcacagccc tgcgtaaacg 171240
tccctgtgct aggtcttttg caggcacagc ttttcctcca tgagtacgta ttttgaaact 171300
caagatcgca ttcatgcgtc ttcacctgga aggggtccat gtgcccctcc ttctggccac 171360
catgcgaagc cacactgacg tgcctctccc tccctccagg aagcctacgt gatggccagc 171420
gtggacaacc cccacgtgtg ccgcctgctg ggcatctgcc tcacctccac cgtgcagctc 171480
atcacgcagc tcatgccctt cggctgcctc ctggactatg tccgggaaca caaagacaat 171540
attggctccc agtacctgct caactggtgt gtgcagatcg caaaggtaat cagggaaggg 171600
agatacgggg aggggagata aggagccagg atcctcacat gcggtctgcg ctcctgggat 171660
agcaagagtt tgccatgggg atatgtgtgt gcgtgcatgc agcacacaca cattccttta 171720
ttttggattc aatcaagttg atcttcttgt gcacaaatca gtgcctgtcc catctgcatg 171780
tggaaactct catcaatcag ctacctttga agaattttct ctttattgag tgctcagtgt 171840
ggtctgatgt ctctgttctt atttctctgg aattctttgt gaatactgtg gtgatttgta 171900
gtggagaagg aatattgctt cccccattca ggacttgata acaaggtaag caagccaggc 171960
caaggccagg aggacccagg tgatagtggt ggagtggagc aggtgccttg caggaggccc 172020
agtgaggagg tgcaaggagc tgacagaggg cgcagctgct gctgctatgt ggctggggcc 172080
ttggctaagt gtccccccttt ccacaggctc gctccagagc cagggcgggg ctgagagagc 172140
agagtggtca ggtagccctg cctgggtgct ggagacaggc acagaacaac aagccaggta 172200
tttcacagct ggtgcggacc cagaaagact tctgcttttg ccccaaaccc ctcccatctc 172260
catcccagtc ttgcatcagt tatttgcact caacttgcta agtcctattt ttttctaaca 172320
atgggtatac atttcatccc attgacttta aaggatttgc aggcaggccc tgtctctgag 172380
aatacgccgt tgcccgtcat ctctctccga cagcagggca gggggtccag agatgtgcca 172440
gggaccagag ggagggagca gacacccacc cggcctgggc aggtcctcct cattgcttgc 172500
atccgcctgg ttagcagtgg cagtcagtcc tgccgagtca ttcgtgaggc gctcacccaa 172560
ctccaggcag atgtaaaagg tgacctacaa gaagacaaac aaaaacatct ggagcgctct 172620
tatgccagca tctgcccttg acaccaccag gcaggctgtt gctgggagcc gtggtgcttg 172680
ggtaagctcc ttcccatggc agagctcctg ggacgcattg tagaagcagg gaccacctcc 172740
caggataacc agatagcagc acaccctgca cagccccttt tactccagca tcatcgggca 172800
ttgatatctc agctgcagcc acaggcggcc cccagcaccc caggaagtgg ggagcgctca 172860
tgcttctctg agcacaaaaa tcactgaata tttttgccat tctcatggtc ataacccggg 172920
ccacagagta gaacactcct atcactgttg ttagacagtg gtcctgggag agggtcttgt 172980
gtgcctcgga tgccagggcc tctttttatt gggaggtgct tgttatttct gtgtgtggct 173040
gcatttgttt cccaagactg ccacaacaaa tcatcaccaa cttggtagct caacatagca 173100
cagctttatt ccctcctggc tctggaggcc aggtgtctaa aaggccatgc tcccacaatg 173160
gttctgagga ggatccttcc tgcctctctg gcttctggtg gctccagcat ccctgggctg 173220
tggctgcacc tccccatgtc aacctccgtc ttcacaaggc cttttcctgt gtctctgcaa 173280
ccacaggccc ctctcctttc tcttaataaa gataccagtc attgagtttg aaaattgcta 173340
agagagtctg ttgtaaatct tcttagcaca aaaaaaaatg acagatatgt gaagtggtag 173400
atatattaat tagtttgatt tgatcactcc gctatgtgta taaatgtcaa aacaaacatt 173460
gcactccata aatatatata ttaaaaaaga tcccagtcat tgcatttagg acccacccta 173520
aatccaggat gatttcattt caagactttt aactagattt gcaaaacccc atttccaaat 173580
aaggtcacat tctgcagttt tgggtagacg tgaaatgtgg agacactgtg caacccactg 173640
tcttggggag ggggtggtca gcctggggca gatgttgctg ggtgtggagc tacatccact 173700
catgccctga cctggaaccc agacctgctt ccccagctct cctcctggtt atctgaagca 173760
gggaatggag agcactgccc tccttgccca ggcagtctct atcacctggt tttagtttct 173820
tcttagcaca tattgcccca gaatatctgg ttggtttatg gcttacttga gtttgtgcct 173880
acctgtccca accgggaggt gagccctggc tattccccaa acccggccct gcatgtggga 173940
gctgcccttc ctccgttcat cagaggggc caacagtcca cagctgttct taatcatctc 174000
ccagtaaccc ccagctccac aaaggtgact ccttacatgg tggagaggtg gtcgggccat 174060
ccgtgtgaaa tgtgtatgtg accgttttcc ttaagggca cgtagtcttg gcaggtttcg 174120

ctcaatatag gatgagctca ggactccagt ggactgtgga ttcagatctg gattctggcg 174180
cattcgccgt gtgaacgggg gcacgttgct ggcctgtctg cgcctcgtct cccgactgtg 174240
gagtgtgttc tgccccttgt ctttctggga ggtagggagg gcagtgagcc ccttcgcatc 174300
gcccaccaca ggcccagcac atggctgatc cccactgagt gttcttttcc tcctttgatc 174360
ccctttggct gacctaggtt ggagcagcca ctaaaatata cccagaaaca tcttcctaat 174420
ctacatctgt gccaaccctc attccctggc gcagcatgac catcacatgc ccgccattgt 174480
tcctgatctc tgctgctcat gacctgctct ccagcgctcc ttctcatgct cacattccag 174540
ttggcctgac ctagataagt ggaggtttat ttgaccccaa aaattagcct tctacaaacg 174600
aatataatag tgtccattac agagaataaa cttagtgcgt gtcccattta agcagaagtt 174660
actgaaagcc tgagtttaag tttccagggc ctgaaagttt tccatgacag ttttctgcat 174720
aatattacct acaatttcaa tctgttattt aaagccattc ttgtgtttgt tgtactttga 174780
ttagctttat tttgatttga agtcctttta cattacgggc agttaacgct ttgtctctgt 174840
tagatttgct ttttagttca caagagaaac ctcattcctc tgtatttgaa tagttgcaat 174900
gatggaacag ctgtccctgg agggaaatga aaacagtgat tccccaaatt gtgacaatag 174960
aaatttgctc ttgggttact tacaatgtat ctgagtatta aaaaattttc tttttaaacg 175020
tttgaagtaa aactacccag aaacacttag tggctgacca gaaactaaac tcctggcatc 175080
ctcaaaatgg gatttattgg cttataaatg tcctgtgttg actcacaaag gcacaaacta 175140
tctaggtaag ttttcttcta aatgttgatg ggagagctgg ccactgttat gcaagtttca 175200
ttgtcctgac taaactgcca aagagattac ataaaattat atcaactaga caaaaggaaa 175260
aaggaaaaaa aacagaggtg tcttgggagg aatccatatg agaccagtag accatgagag 175320
agacatccct tgccatctac aaggaaaatg gattttgttc tccatatgca aaaccatctc 175380
aggagcttgc ggagacacca cttgcttact agccagaaag agcaggtgcc tcctaaattc 175440
cccacacagg agctcacagt ggctttcatg cactgggatt aagttagact taagaaagcc 175500
tgtctactct tcctgggatt tacaagccag ctagtaaatc ccagaataaa tcacacggca 175560
cagtcatcca aagatcccgt catccgtgcc gtttggaaag ccctgctcct gtgccacccct 175620
ctccccgtgg agcctcccat gcccaggact gcagagtcct gccattcaga ctgcaactca 175680
tctcacattc ttccaaacta tttggacaac agagctttct catcacctaa tgcagattac 175740
agtctcacag aattgagtgt tcaggcagac actgatgtgg ttctgtagta cagcaaacaa 175800
tatcagttta cagtcctgag gccaggcctg gtgaacaacg cacggtagcg gtggggcagg 175860
gttctcagaa tgaaactggc ttacacatgg cactctctga ccacaactgt ataagcacca 175920
aactacactt agttccatct atgaggtaaa atttaatgca gatgaacatc aaagaaaacg 175980
tcaaaggctc ctttttacaa gtacgtgggc tacttaattt ggtccaagtc cattttaaaa 176040
agccctaggt gctttcacgg ctctgctact gacaagaagc cccagtgcct gtgagctgct 176100
aatgggaggg agaggaagat gagctgagtg ggccgggcta tcccgtccac accgggagac 176160
agggaaggag actccaagct ggtggtgcca gcacattcca ggccactcag gcctattcct 176220
aggtgccagg tcacgaaaac cacgctgaca gatcgtgctg tgtgcgtgtc atagcacaca 176280
agcaggactg tgagagagtg aaagtgacac tgggtggagc actgaggaag ggccacagtg 176340
tgttggtgga gataggctgt catggagaag agaccctggc ttgctctaca ttgcttccaa 176400
tgcaactgca aggcaggtcc cagagggctc cggccttcgt catccaggtt tgctccctcc 176460
cctcatggct ttcccatcct cagatgagga ctcggcagag cctacccctg ctgactaact 176520
gtggccccag ggtggtgact cagccctgca cctcctgatc ccgtctgcac tgggccagag 176580
aggatgactt acccagcacg ttcacatcac acagctttgt ggattcctag gtccaaggac 176640
cagagatttc agttatgtga gttatttttt ttatttgttc ttgcgtattc cacaaagggt 176700
cgcagctaaa cttaacctaa tgatcacttt agtatatcac taaaaagaca aagctcacag 176760
tgctgttgaa gcacattcat catctttaga cattttgact agttatttct taagcattta 176820
cctgctagtg ttaagcatca catgaaatac atatagaagt aagacaaaat ttcttatctc 176880
cccaagtttg ccaacaaata cagagcagga agggaagcag gtcagagcag gaggcgcagc 176940
tatagtgagg ccaccatgca aggcacaggg agggtgagct ccaagtttga atggaatggg 177000
tctgtcagcc aagccccctg gctctgggaa gatagcagtg aacaagccag atggcccctc 177060
accctccaga gccgtgagtc ctgcagacca aacagcgtga caggtccttt ccctgtccag 177120
gaggcctctg tgggtgagag ttggctgcgg acagggcgtg aaggcacttg agggtgggga 177180
agtgactctg actgggagat gctgaggaca gggaggaaac caccagataa gggacactgg 177240
ggaggagggg tggacccctc agggccaagc acatggagcc tcatcacaaa ggcaagatgg 177300
tggccaaatt caaggtcgct gcaaaaggaa tggagaagag agaatagatt tggcatttgg 177360
aggaaatggt gacaatcatg agcacctacc cgggactctc catgggtgct atctctacat 177420
aaactcattc caccctctga ttaatccatt ctacatatgg ggaaacaaag gcatgcggtg 177480
tttacgtcac ttgccaagat ctcaggattt gatccaggtg gcctggttcc atggtgcagc 177540
ctctcagcct gcatggatgc cccagctcag agcatgactc tcaggacagg ggtcccagca 177600
gccctccctc cctgagcagc agggtgcccg tgctgcacca cttctgtcta ggaataggac 177660
attctgacac tttcctgcct cttccgaggt ctagcactta ctctatgcct gcctgggaag 177720
gtggcaagct ggcctgagga acagactctt ccattttta gggagctcaa ggccacagat 177780
gctctgagat ctggagtcca gagacaggag cggaggcttc tcctggtgac cactctgctt 177840
aaaaacttca tcagatccgt agtttcagag ccccctgaa ccccatccct tacctctacc 177900

agttgcaggt gggtctctgg ggtggggctg ccctccccac cagcacccca agggctaaaa 177960
ggttgagggg agaacaccat catttgtaca gggggatcct ggaagatgag gcctgagaaa 178020
gccctgcggg gcccctcacc ttctccctag ctgtggccaa gagtgtctgg ccttgcctgc 178080
ctcaggacca gcccaaagtg gaggtgagag gtgagcccca gcccccaggg gaagggtgat 178140
ggtggtcttg gtctcagcat ggttctggta gaggtgggtt attttgaaga tgatgaacct 178200
taagcctctt tctgatcttg ctttaaataa atacttctga acaacagcaa caacagaata 178260
gtgttgatag gaaagccctc cactccacca gaaccacgcg gccttctcgt cctcccctcc 178320
tccacttcct tcctaagtca ctgctccatg agctcttcca caggagattt acaaaacaga 178380
acacaaacaa tccagttcct gcctctcact ctgaactcct cccaagactc gtggggtgcg 178440
gcagcccctg ggaacaccca gcccttcaag gtcaaacaca gcccccgccc ctcactctgg 178500
ggtaccctgc cagaataagc cccgacagcc atgtggagca gagccttctt ttttgtaagt 178560
ggaagttcca ggctggcttt tcaaatcccc ttttaacctc agtgctgtat ttcaaaattc 178620
attccagttt tcctgtagta attaacaaaa ataaatattt taatttcaat taaagtgagg 178680
gtctcggaga agaagcagga actgagtttc ctgagaggcc ccgctgaggc tttgttgata 178740
tttcttcctg cgacctctgc tcggaccctg ggagctcaca ggccgtatcg cagctcttat 178800
ctttggggac cagttaaagc ataactgcgc caggcacaga gttgtccttt caaatgtgcc 178860
ggcagtggga cggagaccca tgcgtcaagt ctcctctaag ttcacatggg attctctcct 178920
tgtcccaaag ctgtctctga cttaaaaccc tccaactgat tacctgaatt ccagaatatg 178980
tcctgtgctc tctgcccttt cccacgcctt tggtgaagac cggtgttctg aggaaacaga 179040
cactgtgtag aaatggctca ggtcctttaa agccctggtg tgaggagtgg ggaagggctg 179100
ggccagaggt cagctggatt tgttagattg acagagtgac gcggacttcc ccagaggcac 179160
gggaccaagg tgcatgctca cgctgtctca tgctctcaca cataatgtgt gtgtgtgtgt 179220
gtatatatat atacacatat acatatatat atatacacac atatgcatat atataaaacc 179280
ccaagcagcc tctggcttag caggtgcatt tcccagcagg gcaattaaag ccatggtccc 179340
agtagtggtc ttggggtctc agggtatttg gtctgtgcag ccacatgctt cagtctctgg 179400
accccaggtc atctaacgag gtggtcgtgt ggggactggg atagaaaagg tgtctgcacg 179460
gacgtgtgtg aaagggctgg cacatcgcca gtgctcagca ctgtcagctg ctatcaccag 179520
tcattcaatc attcattcat tcattcagtt gttcattctt caacaggccg ttttaaaaat 179580
gtgcccagta taccaaaatc tccgctaagc atttaaagag gcagaatgaa agttagcagt 179640
ggtggtgaaa cgaagctggg aatgtgctct gagggcctcc ttgtgggctt aatgaatatg 179700
tagaaaccac gcattttaaa tagagaggga gaaagggaga ggttcctggt cctctgcatg 179760
gggacttgtg tgtggctctt tactgtaggc ctgtgccact cctgctcaac agctaccaca 179820
gaggacgcct tcaacaaatg tgaagaacga acaaaaggta caaatgtgaa gaacgaacag 179880
ggtagaaaga aaggagaaag caagggtgag ggtgagaaat caagggacag agaagagaga 179940
agaggagata gcctgggagt tcacacagcc aagaaggtag acactcagtt gaaccagcaa 180000
gaggctgagc ctaactctcc ctttcgaatg ggcaggagtt catgatattt aataaacaga 180060
ggccttgctc tgtaagagac agggtaccag gcagagagca agtcagcatc gcaggagtca 180120
aacgaggcag acagcggggg cagggagctt gcctctgaag gagacccagg ctgccagagt 180180
agcagggagt ctgggccagt cctcttttgg gaagcgcttc ctcggcttct gcccccccctc 180240
tcctctccct ttccacccac catcctgaca taatacttcc taatctggaa gtgttgtcca 180300
gagaagaacc tgctcatttc ctcttaagta ggcagggaag cactaacgtc cagcagcatc 180360
ggaaacccgt aggagcgctc tcggcagtgc agggtgaggg gacagtccat gtagtcatga 180420
gacgtgggtg tcaggcaagc gtctcttttc caaaagagaa aaacattaaa ggcctcacaa 180480
acggcgccca aagactaatt ctgcatagca tctttgcgag accctaggtt cttatgatga 180540
ctggttttgc ctgagaaaga aaaaatttta attttgcttt gacatgccaa ttcaacaaat 180600
cattttcaca taatattcat gcaaaaaaaa aacaatttgc cagaaaactt gggaatccat 180660
ccacatctac agcttttccc tgcagtcaca ctacagtggg atccctccat acaggagcgg 180720
cagagtggag caggctagag atgcctgttt gtttctgttt gctgcaccgc agcaagcatt 180780
tctgtcgtgc ccactctgta ctagaaagta catgaacatc agccataaag ggaactagaa 180840
aggtggccca ccctcttggt ggagagagaa gagagtgtgg tagaaacaat aataagaagt 180900
ctgcagaact tgacccctcc cagcctctcc cacctgccag cctggccctt gcagagagat 180960
gcaggctgcc attcttaggc caaagcctgg gacagttggg ctcagcaagg taggcatccg 181020
tcaagcaagg aggagcaggg gtcagcagtg accccagcag ccagcaggga gaaaggtgca 181080
tgtgacaagg acaccagagg ccgtgggtca ggatcagcca gggtcagggt agcatttcta 181140
ggaattcact ctgttgggcg ctgtgctggc tgcttctcac atattattcc tttcttactc 181200
tcagagcaga gatttcaatt gcagcgagat tgtggaggca gccagggagg tggggagggt 181260
ggtgtcttct aaaagcattt tcagtatcca tgtggtttca gtaataataa taataataaa 181320
ccagtgaaaa gtaaaacagg acaaaaatct tcataggcag tgaaccatat cagagagtcc 181380
aagaaagcac aatgagagtg tggcttaaaa accctgaacg acattccttt gcaccagctt 181440
ggtgaggagg gcatggtccc cgccaccccc cacccccact ttgcagataa accacatgca 181500
ggaaggtcag cctggcaagt ccagtaagtt caagcccagg tctcaactgg gcagcagagc 181560
tcctgctctt ctttgtcctc atatacgagc acctctggac ttaaaacttg aggaactgga 181620
tggagaaaag ttaatggtca gcagcgggtt acatcttctt tcatgcgcct ttccattctt 181680

tggatcagta gtcactaacg ttcgccagcc ataagtcctc gacgtggaga ggctcagagc 181740
ctggcatgaa catgaccctg aattcggatg cagagcttct tcccatgatg atctgtccct 181800
cacagcaggg tcttctctgt ttcagggcat gaactacttg gaggaccgtc gcttggtgca 181860
ccgcgacctg gcagccagga acgtactggt gaaaacaccg cagcatgtca agatcacaga 181920
ttttgggctg gccaaactgc tgggtgcgga agagaaagaa taccatgcag aaggaggcaa 181980
agtaaggagg tggctttagg tcagccagca ttttcctgac accagggacc aggctgcctt 182040
cccactagct gtattgttta acacatgcag gggaggatgc tctccagaca ttctgggtga 182100
gctcgcagca gctgctgctg gcagctgggt ccagccaggg tctcctggta gtgtgagcca 182160
gagctgcttt gggaacagta cttgctggga cagtgaatga ggatgttatc cccaggtgat 182220
cattagcaaa tgttaggttt cagtctctcc ctgcaggata tataagtccc cttcaatagc 182280
gcaattggga aaggtcacag ctgccttggt ggtccactgc tgtcaaggac acctaaggaa 182340
caggaaaggc cccatgcgga cccgagctcc cagggctgtc tgtggctcgt ggctgggaca 182400
ggcagcaatg gagtccttct ctcccttcac tggctcggtt tctcttaggg accctcacag 182460
cactaagggg tgcgcgtccc ctgtcaggcc ctcgaatgcc ctcccacagc caggcccctc 182520
tgaggtttca ctctggcctg ctgggctcct agcagccacc aacccatgat gctgggccct 182580
gaaaacacac gcagacctgg atgagtgagg ccactgggca caaccagggc tcccagctca 182640
ccagagcagc ctgggacaca gagggtgctc agaaacctac cagagcagcc ctgaactccg 182700
tcagactgaa atcccctgtt gccgggagga ggcgccgggc ctgggggacg ggtcctgggg 182760
tgatctggct cgtctgtgtg tgtcactcgt aattaggtcc agagtgagtt aacttttttcc 182820
aacagaggga aactaatagt tgtctcactg cctcatctct caccatccca aggtgcctat 182880
caagtggatg gcattggaat caattttaca cagaatctat acccaccaga gtgatgtctg 182940
gagctacggt gagtcataat cctgatgcta atgagtttgt actgaggcca agctggcttt 183000
tattgttagt taatttacat tatatcctct gacatgcaag tattttcttt cgagataatg 183060
actaatgata atgtaatcat tgctgtctat ctattgtact gagaaaacac ggcagaggaa 183120
atcgagtcca gctgccgtcc aaaagtcact ggagattgca atgagctcgt ctggcagggt 183180
gggggtatg ggagggaaag agcttaggaa acggctctcc ctgcaaagtc caaccaaact 183240
ttaacgttaa ccaaaccatt aatgttgcca tgaatttgaa gtgaaccaga gggaggtggc 183300
agaagaagct taatggggaa tagttccggt agagaaatga ggcttaagat gaactaccct 183360
ggcccttatg tgtcagagag aacggcttga caaacacaca ctgaggatgt ctgcagggat 183420
aaaagaagaa agggagatga cccttgcttc tcgctctcgg gaggaccatc tggtccggcc 183480
ctggggattc tctgtttcct cttctgaatc ccagtgttgc ccagcactgg cctgtaccca 183540
tcctcacgag ggccgctctc ctcacccggc cctaggtccc tgccctgtcc tgagcctaca 183600
ggggcctccc atgttgagaa agtgttgctg acacattgtc tctgaccgct gtgccaggca 183660
ttttctgctg aattaccgca cttggtcctt gaatttcacc cagcaactta ctgaaaggct 183720
ggaacccatg aacctacccc ttcactgagg aaaatcagtt accccagcca tctacagcga 183780
caggagcaag ggaggagtcg cctcacctct ctagaaatgt gtatttgagg agaacactat 183840
tgaaatgaat ttccaagaat aatctagtca gtattacaaa agcaaaatta tttgggatat 183900
cgtccttttt tacttagtat tttttctttt tcctatagca ttattaactt tctgattttc 183960
caaatacata cacattttta aatttcctga gtctttatct cttctgttaa aatgtaagat 184020
ttatgataca aaggcagaga tttgtgtcca tgaataagtg aagtttggtg tgcacctgtg 184080
agctgagcca cctcaattaa tggaacagat aaggaaataa aggtctgctg atgcattgtt 184140
atttacagcc attttcagaa tgtatctcct ctccacgagg gaactgcagg gtcctgcccc 184200
aagccattta ttttgtcctc aagcagcccg cccctcccac tccaggcaca gcccggtctc 184260
ctgctggtct cccctcttcc cacttgctcc ccctcatcta tgctccagac agaggccaca 184320
tatatttttt aacttttttt tttttttttt tgagacagag tcttgccctg tcacccaggc 184380
tggagtgcag tggtgcagtc tcggctcact gcaacctcca cctcccgggt tcaagtgatt 184440
ctcctgcctc agcctcctga gtagctggga ttacaggcgc acaccaccat gcccagctaa 184500
tttttgtat ctctagttga gacagggttt cactatgttg gccaggctgg tctcgaactc 184560
ctgacctcat gatctgcccg cctcggcctc ccaaagtgca tattttttaa ctttatcaga 184620
cttttcattc tctgctcaac atctttcttt ggtcctccag gtatgttcag ataaaacctg 184680
agcacctggc catgactgat gggttgctgg gccatctggc cctggcaact ctcccgtcca 184740
ccaggtcccc ctcccgtcac gctccaggca tagcctgtgt gtgccagcgc aatgcccaca 184800
ctccatgcac aagtggaagc cctctcaaag tcagtggctt agtgccttga tgtggtcaca 184860
cccattctca ggaagtccgt tcccactgaa aacattgtgt gttttcaaca tcattgaggc 184920
tgccacggca gattataatc actggcctag gcagcccact ggaactacca gaccatgagc 184980
ctgaattttt tgtttaaaaa tcatatcctg ttttctctac tctctagtct ctagtcaagg 185040
tgaattattc aatttaataa attagggggcc tagtgtgttg taccaaggag ctaaaaagag 185100
agaactcgca acaccttcca gcccattctc cacctaacac tggctatact ggctctcctc 185160
tctctcgctg tttgttccaa aatctaataa cctgtcttcc cactagaatt catcatacat 185220
gtttaaaaac ctagttaaat agtagttaaa ctgactgcat agatctggaa atgagacagt 185280
ctttctttta caaatccata tagactatga gttgggggca ggggatgaca caagaatcta 185340
ttttcttgcc cccaaaccat tgctttcctt ccaatgttaa gcttgtattc tgtgtattaa 185400
ttcaggtggt tccgtttggg aatggcctct gttacccaga gatgggaggg ccatcagaac 185460

tcggggttgt ctgaaaaaac actggttcta aaattatcac tgctttcact tgtttttaac 185520
catcatagtt gtttgatttt gaaggaaaaa catgagggtt tttattctat gcttgttata 185580
tctatattgt ggtttcgtat tttttagatt ttagtacctg acattttttt aacttttatt 185640
ttaggttcag gggtacatgt gcaggtttgt tatataggta aatttgtgtc atgggggttt 185700
gttacacaga ttattttatc acccagggat taagcctagt acccattagt tatttttcct 185760
gatcctctcc ctcctcccat cctccaccgt cctatagacc ccagtgtgtg ttgttcccct 185820
ctaagtgtcc atgtgttctc atcatttagc tcccacttat aagtaagaac atgcggtatt 185880
tgattttctg ttcctgcatt agtttgctag ggatgatggc ctctagctcc atccatgttc 185940
ttgcaaagta catgatctca ttctcttttg tggctgccta gtgttccatg gtgtatatgt 186000
accacatttt ctttatccag tctgtcattg atgggcattt aggttgattc catgtctttg 186060
ctattgtaaa tagtgctgca gtgaaaatac gcatgcatat gtctttatgg tagaatgatt 186120
tatattcctt tgagtaatgg gattgccggg tcaaatggta gttctgtttt tagctatctg 186180
agaaattgcc acactctttt ccacaataat tgaactaatt tacattccca ccaacagtgt 186240
aaaagcattc ctttttctcc acaacctcac cagcatgtgt tgggattttt tttttttttt 186300
acttttcaat aatagccatc tgactggtat gagatggtat ctcagtgtgg ttttgatttt 186360
tatttcttta atgatcagtg atgttaagct ctttttcata tacttgttgg ctgcatgtat 186420
gtcttcttct aaaaagtgtc tgctcatgtc ctttgcccac tttttaatgg gattgtttaa 186480
tttttttcttg tgaatttact taagttcctt atagatgctg gttattagac ccttctcaga 186540
tttgtagctt gcaaaaatgt tcacccattc tgtgggttgt cttcactctg atgatagttt 186600
cttttgctgt gcagaagatc ttcagtttag ttagatccca tttgtcaatt tttgcttttg 186660
ttgcaattgc ttgatgtgtt ttcatcatga aatcttagcc cattcctata tccagaatgg 186720
tattacctag gttgtcttcc agggttttta tagtttgggg ttttacattt aagtctttaa 186780
tccatgttga gtttattttt gtgtatggtg taaggaagga gtccagtttc aatcttcttc 186840
atggctagct agtcatcatt tattgagtag ggagtccttt attcattgct tttttttttt 186900
tgtcaacttt gtcaacgatc acatggttgt aggtgtgcag ccttatttct gggctctcta 186960
ttctgtttca ttggtctgta tgtctgtttc tgtactagta ccatgctgtt ttggttactg 187020
tatccctgta gtttaaagtc aggtagcatc atgcttccag ctttgttctt tttgcttagg 187080
attgccttgg caattcaggc tctttttttgg ttccatgtga atttttaaat tgtattttct 187140
agttctgtga agaatctcat tggtagtgtg ataggagtaa cattgaatct ataaaatact 187200
ttgggcagta tagtcatttt aatgatattg attctttcta tccatgagca tggaatgttt 187260
ttccatttgt ttgtgtcatc tctgatttct ttaagcagtg ttttgtggtt cttattgtag 187320
agatctttca ctttcctggt ttactgtatt tctaggtatt ttattctttt tgtggcaatt 187380
gtgaattgaa ttgcattcct gatttggttc tcagcttgac tgttgttggc atattggaat 187440
gctaattatt tttgtacatt gattttgtac aactgagtct tcactgaagt tgtttatcag 187500
cttaaggggt tttgggcaag actatggggt tttctagata taggatcatg tcatctgcaa 187560
acagagatag ctgttttcct ctcttcctgt ttggatgtcc attatttctt tctctcacct 187620
gatttatctg gccaggactt ccaatactat gttaaatagg agtgttgaga gagggaatcc 187680
ttgtcttgtg tcaattttca aggggaatgt tttcaacttt tgcccattca atatgatgtt 187740
ggctgtgggt ttgccataga tggctattat gttgaggttt gttctttaaa tacctagttt 187800
attgagaatt ttaaacatgt tgaattttat tgagagcctt ttctgcatct attgagatga 187860
tcatgtggct tttgtcctta gttctgtttg tgtggtgaat cacatttatt gatttgcata 187920
tgttgaacca atcttgcatc ccagggatga agccgacttg attgtggtgg cttaagcttt 187980
ttgatgtgct gctggattcg atttgccagt attttgttga ggatttttat gtctatgttc 188040
atcagagata ttggcctgaa gttttctttt tttgtggatc tctgccaagc tttggtatca 188100
ggatgacatt ggcctcatag aatgagttaa ggaagagtcc ctccttctca attttttttgg 188160
aatagtttca gtaggaatgg taccagcttt ttttgtacat cttgtagaat ttggctatga 188220
atccatctag tcttaggctt tgttttggtt ggtaggctat ttattactga ttcaattttg 188280
gagctcatta ttggtctgtt cagggattca gtttcttcct gaggttttta tttttatcaa 188340
atggaactta accttttttca tttccaattt ttttatgatc taaaaatgtg cagtttacag 188400
ccctgttcag aatctgcatc ttcctcattc tgcagataca ggtccctcag agcaggtgac 188460
tgagtgtgta tcctgtctgg agcataatac ttatgctagt agagttactg ttgtctttat 188520
tgttaattac caaagtttac cacttatcag tcacttacta cttgctgggc attgcactaa 188580
gcatttcagt tgtattatct tgttgggtcc ttacagcaat cctgtgaaac agatactgct 188640
attaccccac tttatagaga ggtagactga ggcttccagc attgaagcaa attgcccaag 188700
actacagaaa tgtaggtttc taaacatcaa gaaacagtaa ccagtaatga tgactaaagc 188760
aagggattgt gattgttcat tcatgatccc actgccttct tttcttgctt catcctctca 188820
ggggtgaccg tttgggagtt gatgaccttt ggatccaagc catatgacgg aatccctgcc 188880
agcgagatct cctccatcct ggagaaagga gaacgcctcc ctcagccacc catatgtacc 188940
atcgatgtct acatgatcat ggtcaagtgt gagtgactgg tgggtctgtc cacactgcct 189000
agctgagcct tggtggctgc tcttagccaa acagctgagg cctttgcatc cctggagaaa 189060
tgtcatcaca ttacttaagg caggcacaca aatccagaaa catctgtaaa taccccttca 189120
agcattcttt taaagacact tcttgactca ttgggcagta tgacctgaca tttgcccatg 189180
tttgcaagca aataaataaa actaaagtct tccgcaagcc attacaccaa aatattctat 189240

tcgctgagtt actcaatgaa ataccgagtt gccctatatt ttgaagcctg ttaccagaga 189300
gactgaatgt ttttaaatgc atggcagtga gtaacaacat aaggctaata gagtcaacat 189360
ttctgctttg acttaaacct tttaaaccag tggatttatg tgaagtctct gcagtgtggc 189420
atttaaacat ttcaatctaa ataagagtgt gtaatttgat tgatgctatt attctaccag 189480
attcacgagt gcagtgggct ctggaggtag cattacatgc atgggatgag catttgcaaa 189540
agaaagttgt atagggaata tgacagagcc aagttaatgt aaatattaat gcctttctga 189600
actctaggcc acagagttga tcttttttaa cttccttggt ttgggctaag gaagctgtga 189660
tccagagaag ccacgtgatt tgtctaaggt cacatagcag tctggcctaa aatagcttga 189720
tatgctgtgg atggaaaata aatgtgatcc ctcaagaggc atgaggattt ccaggcagta 189780
gccatacctc caaattgttt aatctggatt tagattgttg ggtagtcaca tgcagcagca 189840
cagttaacag tgtgtcctcc tgtggaagtg gccagcacag ccagccctct cacttgcatg 189900
catgcccacc agccttctca cttgcatgca tgcccactgg gtatgtgctg tactggagac 189960
gccgggggta ggggcccagt cccaacccca aattctttaa agcctatttt tctaagttgc 190020
atctggtttc ctacctgaag gaatgctaag ggtggatgtt gagtgaggac cttggtgcag 190080
ggcaccctgc agtcaggata gttcatggag agcaattgta cagacccaca ctgctccatc 190140
ccctcaggcg taacacagga tgctgacccc aggaagagtg ggcgtagaaa aactagaggg 190200
cattattgtt attctgattc aaatgtacag tgctggcatg gtctttaaac agtaaccagt 190260
actagctggc caagacagaa aagtctacca caaagacttg gttctttcat cacttatttg 190320
actggaagtg tcgcatcacc aatgccttct ttaagcaatg ccatctttat catttcttcc 190380
agtgttctaa ttgcactgtt ttttctcatt ccttccccag gctggatgat agacgcagat 190440
agtcgcccaa agttccgtga gttgatcatc gaattctcca aaatggcccg agacccccag 190500
cgctaccttg tcattcaggt acaaattgca gtctgcgctt ccattgggaa gagtccctct 190560
aatgagcatc tcatgtcact gtgttctgtc acatgccagc ctggcctccc tgtgtcccag 190620
atcgcattat taaaccctcc agcgcattag agcaagcctc agtaaggcgc aggccacatc 190680
gtgaactaag cagcatccgt gagtggggcc cacccaactc catctccccc tccccgtctg 190740
aactctcctc tggtgctcgt cctcactgtc cggctagcca aagcctcagc tgggtctaag 190800
agagaagcat ggtctattgg gctttggtgt caggcagacg tggcttcaca cccctgactc 190860
tccacttctt cgcatcaccc aggcagccga tccacctatc tccttccata acacaggaat 190920
accaaaacca agctcacagg attgtctcaa agattcaata aaatatgttg caaaatacgc 190980
tccctaacac ctcacagcaa ggtgcacaaa tcgatgaatg ctgcagcttc ttccctttct 191040
gtttcctcag aagctatttg aatctcatgt aggggctttc aagcatcaaa ggatggttca 191100
tgttttattt taaggcaccc acatcatgtc atgaggggag gcagctataa tttagagaac 191160
caagggggat ttcattataa caaaattggc aaacacacag gcacctgctg gcaatagacc 191220
cctgctccta tagccaagaa gtggaatagc atctctacgg gccattctaa tagcctcaaa 191280
atctctgcac caggggggatg aaagaatgca tttgccaagt cctacagact ccaacttcta 191340
ccgtgccctg atggatgaag aagacatgga cgacgtggtg gatgccgacg agtacctcat 191400
cccacagcag ggcttcttca gcagcccctc cacgtcacgg actcccctcc tgagctctct 191460
ggtatgaaat ctctgtctct ctctctctca agctgtgtct actcatttga acaaattgaa 191520
ttttagggaa aataaccatc tagtgaaact cacatggaaa tgaagtcaat tttaaccaaa 191580
tggtaaaatc aaaatcaaaa taaattaagt gtattaatta ttttgttgca ttgcaacaac 191640
ttgattgtaa gccttttagg tccactatgg aatgtaatta aatcaaaact aaacctagtt 191700
gctctaaaac taacgattaa gacaaaaatt aaacaccttc acaatatacc ctccatgagg 191760
cacaccacct gcattcagga aaagtggatg agatgtggta caagcattcc atgggcaatt 191820
ctctgtttct ttttccagag tgcaaccagc aacaattcca ccgtggcttg cattgataga 191880
aatggggtat gtatgaacac cttataagcc agaatttaca gctctccact atggctctat 191940
tttacatgga aaatgcctta acctaaataa ttttaaccca gataatcttg agttttcttc 192000
ctgtgtgggt ttttccctgc acggctgtca cgcctcacag tgccgttcaa agcgtgactc 192060
ctggaccagt agtagcatcg cctggccttg ttagaaacgc catttttcag gccactgccc 192120
cagtttgacc aaatcaggac ctctgggggt ggcacccagt agtctatgtt tgagccactt 192180
tccaggtgat gctgatgtct gttgaagtgt gaggccgtgg tctagaccgc actgtgccat 192240
gcagaaacca ctagccacat gtggctactt caacttaaat gttaatgagt taaaatgaaa 192300
taaaatataa aattcagttt ctcacacatg tgaagtgtcc agtagccaca cgtggctagt 192360
ggtgaccgta ttgaagagca ccgctcatag cacacctccc tcactgcgga aagttctgct 192420
gtacagcacc cagcaacagc cctgctgccc aaccctgcag cctgtggccc aagtagcacc 192480
agcacccacc agggtgcaag actctcaagg cctgcccaac ctactaatca gaaccagcat 192540
ctcaaggaga tctcgggtga tttttgcaaa cactgaagtt ggggcagccc tgaccggagt 192600
aaccttccct catttcctcc tgcagctgca aagctgtccc atcaaggaag acagcttctt 192660
gcagcgatac agctcagacc ccacaggcgc cttgactgag gacagcatag acgacacctt 192720
cctcccagtg cctggtgagt ggcttgtctg gaaacagtcc tgctcctcaa cctcctcgac 192780
ccactcagca gcagccagtc tccagtgtcc aagccaggtg ctccctccag catctccaga 192840
gggggaaaca gtggcagatt tgcagacaca gtgaagggcg taaggagcag ataaacacat 192900
gaccgagcct gcacaagctc tttgttgtgt ctggttgttt gctgtacctc tgttgtaaga 192960
atgaatctgc aaaatttcta gcttatgaag caaatcacgg acatacacat ctgtgtgtgt 193020

gagtgttcat gatgtgtgta catctgtgta tgtgtgtgtg tgtatgtgtg tgtttgtgac 193080
agatttgatc cctgttctct ctgctggctc tatcttgacc tgtgaaacgt atatttaact 193140
aattaaatat tagttaatat taataaattt taagctttat ccagatactc ataacctgct 193200
aacacacaca catatacaca cacatacaca tacacacata tacacacacc acacacatac 193260
acagacacca cacacatacc atacacagac acatacacat gcacacacat atacacacac 193320
acctcaaata catacacacc acacacacat acatgtatac acacatacac acaccacaca 193380
tacaccacaa aaaccccaca cacatacaca tatacacacc acacacacca catacacaca 193440
cgtatacaca catatataca cacatacacc atgcatacat acacaccaca catacataca 193500
gacacaccac acacacgtac acacaacaca caacacagac acacacgtac acacactaca 193560
gacatgtatg cacacataca cacacaccac acatacatac acacagacac atatacacta 193620
cacacaccat tacatacaca cgtacacata caccacacac accacacata cacacaccac 193680
acacacatac acacgccaca cacacaccac aaaaaccgca cacacataca aacatataca 193740
cactacacca cacatacaca cacacaccac acaccacaca cacacataca cacaccacac 193800
acaccacaca tacacgcacc acacatacac acacgtagac acaccacaca caccacagaa 193860
acacacatta acacaccaca tacacatatg tatgtgcata tacacaccca caccccacac 193920
acacatgtat aaagatttag atatatataa aacatacgtt atatatatgt tgatgtaata 193980
tctaatatct atatatctaa tatgtagttt attagctatc taatatctat gtcatatata 194040
tcaaatcttt atatataaaa atatgtagaa atctttatac atatgttata tgtatataaa 194100
gatttagata tataacatat gtaagttata tatatgttag tgtaatatct aatatatagt 194160
ttattggcta tctaatataa tataaacaga ttatcaatat tataagctat tagaaaaatg 194220
caagttaagg cagatgatat acctctttca caccaactca cacaccaact acacacacac 194280
atacacacag acacacacga cacacaccat acacatgtac acacacacca catatacaca 194340
aacgtacaca cacaccacac acacatacac accacacaca caacacacat acatacacat 194400
ccacacacca cacatgtaca cacaccacac acacacatac acaccacata cacatatgta 194460
tgcacacata cacaccaaca ccacacagac accacacatg cataaacata tagacatata 194520
cacaccacac accatatgta cacatgtaca cacacaccac atatacacac aacacacaca 194580
aatacacaca ccacacacac accacaaaaa ccccacacac acaaacatat acaccccaca 194640
catacgcata tatacacaca cacatacaca ccacacacat acacaccaca cacacaccac 194700
acatacacac acgtacacac accacacaca caccacagac acacaccaca catacataca 194760
catacacaca ccacacacac gtacacacac cacacacaca cagacacaca tagacacacc 194820
acatacacac ccacaccaca cacacacaac tcataccaca catacataca caatagacac 194880
atacacacca cacacaccat acatacacac gtatacacac accacatata cacacacgta 194940
cacacacacc acacacaccc acatgcacac accacacaca catacaaata tacaccacac 195000
acacatacac cacacacacg gtgcacatac acacacatat acacacacca gacacacata 195060
ccacatacac atcacacata tatgtataca tgcatacaca tacacacaca catacacaca 195120
ctctcctcaa ggcagtttat cctctgagaa ctttaaattt acaaaagaca catatgtcca 195180
ttactttgag aaggacagga aagaacccac tttcttttgc agcaacagca agagggccct 195240
cccaaggctc ctgctccctg tcataagtct ccttgttgag gacattcaca gggttcagaa 195300
cccagggatc ctgcatggga tggtgctttg ctgattactt cacctctgat ttctttccac 195360
tttcagaata cataaaccag tccgttccca aaaggcccgc tggctctgtg cagaatcctg 195420
tctatcacaa tcagcctctg aaccccgcgc ccagcagaga cccacactac caggaccccc 195480
acagcactgc agtgggcaac cccgagtatc tcaacactgt ccagcccacc tgtgtcaaca 195540
gcacattcga cagccctgcc cactgggccc agaaaggcag ccaccaaatt agcctggaca 195600
accctgacta ccagcaggac ttctttccca aggaagccaa gccaaatggc atctttaagg 195660
gctccacagc tgaaaatgca gaatacctaa gggtcgcgcc acaaagcagt gaatttattg 195720
gagcatgacc acggaggata gtatgagccc taaaaatcca gactctttcg atacccagga 195780
ccaagccaca gcaggtcctc catcccaaca gccatgcccg cattagctct tagacccaca 195840
gactggtttt gcaacgttta caccgactag ccaggaagta cttccacctc gggcacattt 195900
tgggaagttg cattcctttg tcttcaaact gtgaagcatt tacagaaacg catccagcaa 195960
gaatattgtc cctttgagca gaaatttatc tttcaaagag gtatatttga aaaaaaaaaa 196020
aaaagtatat gtgaggattt ttattgattg gggatcttgg agtttttcat tgtcgctatt 196080
gatttttact tcaatgggct cttccaacaa ggaagaagct tgctggtagc acttgctacc 196140
ctgagttcat ccaggcccaa ctgtgagcaa ggagcacaag ccacaagtct tccagaggat 196200
gcttgattcc agtggttctg cttcaaggct tccactgcaa aacactaaag atccaagaag 196260
gccttcatgg ccccagcagg ccggatcggt actgtatcaa gtcatggcag gtacagtagg 196320
ataagccact ctgtcccttc ctgggcaaag aagaaacgga ggggatgaat tcttccttag 196380
acttactttt gtaaaaatgt ccccacggta cttactcccc actgatggac cagtggtttc 196440
cagtcatgag cgttagactg acttgtttgt cttccattcc attgttttga aactcagtat 196500
gccgcccctg tcttgctgtc atgaaatcag caagagagga tgacacatca aataataact 196560
cggattccag cccacattgg attcatcagc atttggacca atagcccaca gctgagaatg 196620
tggaatacct aaggataaca ccgcttttgt tctcgcaaaa acgtatctcc taatttgagg 196680
ctcagatgaa atgcatcagg tcctttgggg catagatcag aagactacaa aaatgaagct 196740
gctctgaaat ctcctttagc catcacccca accccccaaa attagtttgt gttacttatg 196800

```
gaagatagtt ttctcctttt acttcacttc aaaagctttt tactcaaaga gtatatgttc 196860
cctccaggtc agctgccccc aaaccccctc cttacgcttt gtcacacaaa aagtgtctct 196920
gccttgagtc atctattcaa gcacttacag ctctggccac aacagggcat tttacaggtg 196980
cgaatgacag tagcattatg agtagtgtga attcaggtag taaatatgaa actagggttt 197040
gaaattgata atgctttcac aacatttgca gatgttttag aaggaaaaaa gttccttcct 197100
aaaataattt ctctacaatt ggaagattgg aagattcagc tagttaggag cccatttttt 197160
cctaatctgt gtgtgccctg taacctgact ggttaacagc agtcctttgt aaacagtgtt 197220
ttaaactctc ctagtcaata tccaccccat ccaatttatc aaggaagaaa tggttcagaa 197280
aatattttca gcctacagtt atgttcagtc acacacacat acaaaatgtt ccttttgctt 197340
ttaaagtaat ttttgactcc cagatcagtc agagccccta cagcattgtt aagaaagtat 197400
ttgatttttg tctcaatgaa aataaaacta tattcatttc cactctatta tgctctcaaa 197460
taccccctaag catctatact agcctggtat gggtat                          197496
```

<210> 11
<211> 4530
<212> DNA
<213> Homo sapiens

<400> 11

```
aattctcgag ctcgtcgacc ggtcgacgag ctcgagggtc gacgagctcg agggcgcgcg 60
cccggccccc acccctcgca gcaccccgcg ccccgcgccc tcccagccgg gtccagccgg 120
agccatgggg ccggagccgc agtgagcacc atggagctgg cggccttgtg ccgctggggg 180
ctcctcctcg ccctcttgcc ccccggagcc gcgagcaccc aagtgtgcac cggcacagac 240
atgaagctgc ggctccctgc cagtcccgag acccacctgg acatgctccg ccacctctac 300
cagggctgcc aggtggtgca gggaaacctg gaactcacct acctgcccac caatgccagc 360
ctgtccttcc tgcaggatat ccaggaggtg cagggctacg tgctcatcgc tcacaaccaa 420
gtgaggcagg tcccactgca gaggctgcgg attgtgcgag gcacccagct ctttgaggac 480
aactatgccc tggccgtgct agacaatgga gacccgctga acaataccac ccctgtcaca 540
ggggcctccc caggaggcct gcgggagctg cagcttcgaa gcctcacaga gatcttgaaa 600
ggaggggtct tgatccagcg gaacccccag ctctgctacc aggacacgat tttgtggaag 660
gacatcttcc acaagaacaa ccagctggct ctcacactga tagacaccaa ccgctctcgg 720
gcctgccacc cctgttctcc gatgtgtaag ggctcccgct gctggggaga gagttctgag 780
gattgtcaga gcctgacgcg cactgtctgt gccggtggct gtgcccgctg caagggccca 840
ctgcccactg actgctgcca tgagcagtgt gctgccggct gcacgggccc caagcactct 900
gactgcctgg cctgcctcca cttcaaccac agtggcatct gtgagctgca ctgcccagcc 960
ctggtcacct acaacacaga cacgtttgag tccatgccca atcccgaggg ccggtataca 1020
ttcggcgcca gctgtgtgac tgcctgtccc tacaactacc tttctacgga cgtgggatcc 1080
tgcaccctcg tctgccccct gcacaaccaa gaggtgacag cagaggatgg aacacagcgg 1140
tgtgagaagt gcagcaagcc ctgtgcccga gtgtgctatg gtctgggcat ggagcacttg 1200
cgagaggtga gggcagttac cagtgccaat atccaggagt ttgctggctg caagaagatc 1260
tttgggagcc tggcatttct gccggagagc tttgatgggg acccagcctc caacactgcc 1320
ccgctccagc cagagcagct ccaagtgttt gagactctgg aagagatcac aggttaccta 1380
tacatctcag catggccgga cagcctgcct gacctcagcg tcttccagaa cctgcaagta 1440
atccggggac gaattctgca caatggcgcc tactcgctga ccctgcaagg gctgggcatc 1500
agctggctgg ggctgcgctc actgagggaa ctgggcagtg gactggccct catccaccat 1560
aacacccacc tctgcttcgt gcacacggtg ccctgggacc agctctttcg gaacccgcac 1620
caagctctgc tccacactgc caaccggcca gaggacgagt gtgtgggcga gggcctggcc 1680
tgccaccagc tgtgcgcccg agggcactgc tggggtccag ggcccaccca gtgtgtcaac 1740
tgcagccagt tccttcgggg ccaggagtgc gtggaggaat gccgagtact gcagggggctc 1800
cccagggagt atgtgaatgc caggcactgt ttgccgtgcc accctgagtg tcagccccag 1860
aatggctcag tgacctgttt tggaccggag gctgaccagt gtgtggcctg tgcccactat 1920
aaggaccctc ccttctgcgt ggcccgctgc cccagcggtg tgaaacctga cctctcctac 1980
atgcccatct ggaagtttcc agatgaggag ggcgcatgcc agccttgccc catcaactgc 2040
acccactcct gtgtggacct ggatgacaag ggctgccccg ccgagcagag agccagccct 2100
ctgacgtcca tcgtctctgc ggtggttggc attctgctgg tcgtggtctt gggggtggtc 2160
tttgggatcc tcatcaagcg acggcagcag aagatccgga agtacacgat gcggagactg 2220
ctgcaggaaa cggagctggt ggagccgctg acacctagcg gagcgatgcc caaccaggcg 2280
cagatgcgga tcctgaaaga gacggagctg aggaaggtga aggtgcttgg atctggcgct 2340
tttggcacag tctacaaggg catctggatc cctgatgggg agaatgtgaa aattccagtg 2400
gccatcaaag tgttgaggga aaacacatcc cccaaagcca acaaagaaat cttagacgaa 2460
gcatacgtga tggctggtgt gggctcccca tatgtctccc gccttctggg catctgcctg 2520
```

acatccacgg tgcagctggt gacacagctt atgccctatg gctgcctctt agaccatgtc 2580
cgggaaaacc gcggacgcct gggctcccag gacctgctga actggtgtat gcagattgcc 2640
aaggggatga gctacctgga ggatgtgcgg ctcgtacaca gggacttggc cgctcggaac 2700
gtgctggtca agagtcccaa ccatgtcaaa attacagact tcgggctggc tcggctgctg 2760
gacattgacg agacagagta ccatgcagat gggggcaagg tgcccatcaa gtggatggcg 2820
ctggagtcca ttctccgccg gcggttcacc caccagagtg atgtgtggag ttatggtgtg 2880
actgtgtggg agctgatgac ttttggggcc aaaccttacg atgggatccc agcccgggag 2940
atccctgacc tgctggaaaa gggggagcgg ctgccccagc cccccatctg caccattgat 3000
gtctacatga tcatggtcaa atgttggatg attgactctg aatgtcggcc aagattccgg 3060
gagttggtgt ctgaattctc ccgcatggcc agggaccccc agcgctttgt ggtcatccag 3120
aatgaggact tgggcccagc cagtcccttg gacagcacct tctaccgctc actgctggag 3180
gacgatgaca tgggggacct ggtggatgct gaggagtatc tggtacccca gcagggcttc 3240
ttctgtccag accctgcccc gggcgctggg ggcatggtcc accacaggca ccgcagctca 3300
tctaccagga gtggcggtgg ggacctgaca ctagggctgg agccctctga agaggaggcc 3360
cccaggtctc cactggcacc ctccgaaggg gctggctccg atgtatttga tggtgacctg 3420
ggaatggggg cagccaaggg gctgcaaagc ctccccacac atgaccccag ccctctacag 3480
cggtacagtg aggaccccac agtacccctg ccctctgaga ctgatggcta cgttgccccc 3540
ctgacctgca gcccccagcc tgaatatgtg aaccagccag atgttcggcc ccagccccct 3600
tcgccccgag agggccctct gcctgctgcc cgacctgctg gtgccactct ggaaagggcc 3660
aagactctct ccccagggaa gaatggggtc gtcaaagacg tttttgcctt tgggggtgcc 3720
gtggagaacc ccgagtactt gacaccccag ggaggagctg cccctcagcc ccaccctcct 3780
cctgccttca gcccagcctt cgacaacctc tattactggg accaggaccc accagagcgg 3840
ggggctccac ccagcacctt caaagggaca cctacggcag agaacccaga gtacctgggt 3900
ctggacgtgc cagtgtgaac cagaaggcca agtccgcaga agccctgatg tgtcctcagg 3960
gagcagggaa ggcctgactt ctgctggcat caagaggtgg gagggccctc cgaccacttc 4020
caggggaacc tgccatgcca ggaacctgtc ctaaggaacc ttccttcctg cttgagttcc 4080
cagatggctg gaaggggtcc agcctcgttg gaagaggaac agcactgggg agtctttgtg 4140
gattctgagg ccctgcccaa tgagactcta gggtccagtg gatgccacag cccagcttgg 4200
ccctttcctt ccagatcctg ggtactgaaa gccttaggga agctggcctg agaggggaag 4260
cggccctaag ggagtgtcta agaacaaaag cgacccattc agagactgtc cctgaaacct 4320
agtactgccc cccatgagga aggaacagca atggtgtcag tatccaggct ttgtacagag 4380
tgcttttctg tttagttttt actttttttg ttttgttttt ttaaagacga aataaagacc 4440
caggggagaa tgggtgttgt atggggaggc aagtgtgggg ggtccttctc cacacccact 4500
ttgtccattt gcaaatatat tttggaaaac 4530

## Description

## FIELD OF THE INVENTION

The present invention relates to prognostic methods which are useful in medicine, particularly cancer chemotherapy. More particularly, the invention relates to assessment of surviviability of a patient whose tumor cell gene expression is analyzed. Additionally, the sensitivity of tumor cells to receptor tyrosine kinase targeted chemotherapeutic regimen is assayed by examining the mRNA expression of the *EGFR* and *Her2-neu* genes in humans.

## BACKGROUND OF THE INVENTION

Lung cancer is the leading cause of cancer-related deaths among both males and females in western countries. In the United States, approximately 171,000 new cases of lung cancer are diagnosed and 160,000 individuals die from this disease each year. Despite improvements in the detection and treatment of lung cancer in the past two decades, the overall 5-year survival remains less than 15%. Ginsberg, et al., In: DeVita, et al., Cancer. Principles in Practice of Oncology, Ed. 5, pp. 858-910. Philadelphia: Lipincott-Raven Publishers, 1997. To further improve the survival rate in patients with Non-Small Cell Lung Carcinoma (NSCLC), their prognostic classification based on molecular alterations is crucial. Such classification will provide more accurate and useful diagnostic tools and, eventually, more effective therapeutic options.

Cancer arises when a normal cell undergoes neoplastic transformation and becomes a malignant cell. Transformed (malignant) cells escape normal physiologic controls specifying cell phenotype and restraining cell proliferation. Transformed cells in an individual's body thus proliferate, forming a tumor. When a tumor is found, the clinical objective is to destroy malignant cells selectively while mitigating any harm caused to normal cells in the individual undergoing treatment

Chemotherapy is based on the use of drugs that are selectively toxic (cytotoxic) to cancer cells. Several general classes of chemotherapeutic drugs have been developed, including drugs that interfere with nucleic acid synthesis, protein synthesis, and other vital metabolic processes. These generally are referred to as anti-metabolite drugs. Other classes of chemotherapeutic drugs inflict damage on cellular DNA. Drugs of these classes generally are referred to as genotoxic. Additionally, a class of chemotherapeutic agents specifically inhibit mitogenic signaling through receptor tyrosine kinases (RTKs), in cells where RTKs are over active. (Drugs of the Future, 1992, 17, 119).

Susceptibility of an individual neoplasm to a desired chemotherapeutic drug or combination of drugs often, however, can be accurately assessed only after a trial period of treatment. The time invested in an unsuccessful trial period poses a significant risk in the clinical management of aggressive malignancies. Therefore, it is of importance to assess the expression status of genetic determinants targeted by specific chemotherapeutic agents. For example, if a tumor expresses high levels of DNA repair genes, it is likely that the tumor will not respond well to low doses of DNA-damaging genotoxic agents. Thus, the expression status of genetic determinants of a tumor will help the clinician develop an appropriate chemotherapeutic regimen specific to the genetic repertoire of the tumor.

Receptor tyrosine kinases (RTKs) are important in the transduction of mitogenic signals. RTKs are large membrane spanning proteins which possess an extracellular ligand binding domain for growth factors such as epidermal growth factor (EGF) and an intracellular portion which functions as a kinase to phosphorylate tyrosine amino acid residues on cytosol proteins thereby mediating cell proliferation. Various classes of receptor tyrosine kinases are known based on families of growth factors which bind to different receptor tyrosine kinases. (Wilks, Advances in Cancer Research, 1993, 60, 43-73)

Class I kinases such as the EGF-R family of receptor tyrosine kinases include the EGF, HER2-neu, erbB, Xmrk, DER and let23 receptors. These receptors are frequently present in common human cancers such as breast cancers (Sainsbury et al., Brit. J. Cancer, 1988, 58, 458; Guerin et al., Oncogene Res., 1988, 3, 21), squamous cell cancer of the lung (Hendler et al., Cancer Cells, 1989, 7, 347), bladder cancer (Neal et al., Lancet, 1985, 366), oesophageal cancer (Mukaida et al, Cancer, 1991, 68, 142), gastrointestinal cancer such as colon, rectal or stomach cancer (Bolen et al., Oncogene Res., 1987, 1, 149), leukaemia (Konaka et al., Cell, 1984,37, 1035) and ovarian, bronchial or pancreatic cancer (European Patent Specification No. 0400586). As further human tumor tissues are tested for the EGF family of receptor tyrosine kinases it is expected that its widespread prevalence will be established in other cancers such as thyroid and uterine cancer.

Specifically, EGFR tyrosine kinase activity is rarely detected in normal cells whereas it is more frequently detectable in malignant cells (Hunter, Cell, 1987, 50, 823). It has been more recently shown that *EGFR* is overexpressed in many human cancers such as brain, lung squamous cell, bladder, gastric, breast, head and neck, oesophageal, gynaecological and thyroid tumours. (W J Gullick, Brit. Med. Bull., 1991, 47, 87). Receptor tyrosine kinases are also important in other cell-proliferation diseases such as psoriasis. EGFR disorders are those characterized by *EGFR* expression by cells normally not expressing *EGFR,* or increased EGFR activation leading to unwanted cell proliferation, and/or the existence of inappropriate *EGFR* levels. The EGFR is known to be activated by its ligand EGF as well as transforming growth factor-alpha (TGF-$\alpha$).

The Her2-neu protein is also a member of the class I receptor tyrosine kinase (RTK) family. Yarden and Ullrich, Annu. Rev. Biochem. 57:443, 1988; Ullrich and Schlessinger, Cell 61:203, 1990. Her2-neu protein is structurally related to EGFR. Carraway, et al., Cell 78:5, 1994; Carraway, et al., J. Biol. Chem. 269:14303, 1994. These receptors share a common molecular architecture and contain two cysteine-rich regions within their cytoplasmic domains and structurally related enzymatic regions within their cytoplasmic domains.

Ligand-dependent activation of Her2-neu protein is thought to be mediated by neuactivating factor (NAF) which can directly bind to p165(Her2-neu) and stimulate enzymatic activity. Dougall et al., Oncogene 9:2109, 1994; Samata et al., Proc. Natl. Acad. Sci. USA 91:1711,1994. Ligand-independent homodimerization of Her2-neu protein and resulting receptor activation is facilitated by over-expression of Her2-neu protein. An activated Her2-neu complex acts as a phosphokinase and phosphorylates different cytoplasmic proteins. HER2-neu disorders are characterized by inappropriate activity or over-activity of HER2-neu have increased HER2-neu expression leading to unwanted cell proliferation such as cancer.

Inhibitors of receptor tyrosine kinases EGFR and HER2-neu are employed as selective inhibitors of the growth of mammalian cancer cells (Yaish et al. Science, 1988, 242, 933). For example, erbstatin, an EGF receptor tyrosine kinase inhibitor, reduced the growth of EGFR expressing human mammary carcinoma cells injected into athymic nude mice, yet had no effect on the growth of tumors not expressing EGFR (Toi et al., Eur. J. Cancer Clin. Oncol., 1990, 26, 722.) Various derivatives of styrene are also stated to possess tyrosine kinase inhibitory properties (European Patent Application Nos. 0211363, 0304493 and 0322738) and to be of use as anti-tumour agents. Two such styrene derivatives are Class I RTK inhibitors whose effectiveness has been demonstrated by attenuating the growth of human squamous cell carcinoma injected into nude mice (Yoneda et al., Cancer Research, 1991, 51, 4430). It is also known from European Patent Applications Nos. 0520722 and 0566226 that certain 4-anilinoquinazoline derivatives are useful as inhibitors of receptor tyrosine kinases. The very tight structure-activity relationships shown by these compounds suggests a clearly-defined binding mode, where the quinazoline ring binds in the adenine pocket and the anilino ring binds in an adjacent, unique lipophilic pocket. Three 4-anilinoquinazoline analogues (two reversible and one irreversible inhibitor) have been evaluated clinically as anticancer drugs. Denny, Farmaco 2001 Jan-Feb;56(1-2):51-6. Recently, the U.S. FDA approved the use of the monoclonal antibody trastazumab (Herceptin®) for the treatment of HER2-neu overexpressing metastatic breast cancers. Scheurle, et al., Anticancer Res 20:2091-2096,2000.

Because effective chemotherapy against tumors often requires a combination of agents, the identification and quantification of determinants of resistance or sensitivity to each single drug has become an important tool to design individual combination chemotherapy. Studies have unsuccessfully attempted to reliably correlate the relative levels of expression of *EGFR* and/or *HER2 neu* in malignant cells from cancer patients with survivability.

The prognostic importance of *EGFR* and in NSCLC has heretofore remained controversial. Studies using binding assays correlated increased *EGFR* expression with advanced stage NSCLC and shortened overall survival" whereas studies using semi-quantitative techniques for measuring *EGFR* mRNA or protein expression failed to show a consistent correlation with clinical outcome. Veale et al., Br. J. Caner 68:162-165, 1993; Fujino et al., Eur. Cancer 32:2070-2074, 1996; Rusch, et al., Cancer Res 53:2379-2385, 1993; Pfeiffer, et al., Br J Cancer 74:86-91, 1996; Pastorino, et al.,. J Clin Oncol 15:2858-2865, 1997. Studies of EGFR expression in NSCLC tumors using immunohistochemical methods have shown frequencies for EGFR overexpression between 32% and 47% in NSCLC tumors. Veale et al., Br. J. Caner 55:513-516, 1987; Veale et al., Br. J. Caner 68:162-165, 1993; Fujino et al., Eur. Cancer 32:2070-2074, 1996; Rusch,et al., Cancer Res 53:2379-2385, 1993; Pastorino et al., J.Cin.Onc. 15:2858-2865, 1997; Tateishi, et al., Eur J Cancer 27:1372-75, 1991; Rachwal, et al., Br J Cancer 72:56-64,1995; Rusch, et al., Cancer Res 15:2379-85,1993; Pfeiffer, et al., Br J Cancer 78:96-9, 1998; Ohsaki, et al., Oncol Rep 7:603-7,2000. Moreover, significant differences in *EGFR* expression has been reported among histological subtypes, generally with higher *EGFR* expression in SCC compared to AC and LC. Fujino et al., Eur. Cancer 32:2070-2074, 1996; Veale et al., Br. J. Caner 55:513-516, 1987; Pastorino et al., J.Clin.Onc. 15:2858-2865, 1997; Pfeiffer, et al., Br J Cancer 78:96-9, 1998; Ohsaki et al., Oncol. Rep. &:603-7, 2000. However, these studies reported no consistent correlation of *FGFR* overexpression with lung cancer patient survival.

Observations of a purported correlation of *EGFR* overexpression with a decrease in patient survival were made in some inconclusive studies. Veale et al., 1987; Ohsaki et al., 2000. However, Veale et al., analyzed a population of only nineteen NSCLC patients. Ohsaki et al., correlated *EGFR* protein expression with poor prognosis in NSCLC patients with p53 overexpression ($P$=0.024).

As with *EGFR,* the prognostic importance of *HER2-neu* and in NSCLC has heretofore remained controversial. HER2-neu protein overexpression has been demonstrated in NSCLC, including squamous cell carcinoma, adenocarcinoma, and large cell carcinoma. Veale et al., 1987; Schneider, et al., Cancer Res 49:4968-4971, 1989; Kern et al., Cancer Res. 50:5184-5191, 1990; Weiner, et al., Cancer Res 50:421-425, 1990; Scheurle, et al., Anticancer Res. 20: 2091-2096, 2000. Earlier studies, using protein assays, reported an association of HER2-neu protein overexpression and inferior overall survival in pulmonary adenocarcinomas (AC). Kern, et al., Cancer Res 50:5184-5191, 1990; Kern et al., J Clin Invest 93:516-20, 1994. However, contradictory studies reported no correlation of HER2-neu protein overexpression with inferior overall survival in pulmonary adenocarcinomas (AC). Pfeiffer et al., Br. J. Cancer 74:86-91, 1996.

Another critical question is the evaluation of interrelationships between HER2-neu and EGFR co-overexpression as prognosticators of cancer. Tateishi et al., (Eur. J. Cancer 27:1372-75, 1991), measured EGFR and HER2-neu protein co-expression, in 13% of AC analysed, and found that co-overexpression of these two genes correlated with inferior 5-year survival. However, as with HER2-neu overexpression alone, association between HER2-neu and EGFR co-expression and survival in squamous cell carcinoma (SCC) and large cell carcinoma (LCC) of the lung has not been reported.

inconsistent methodologies for the determination of *EGFR* and *HER2-neu* expression levels has been at the root of the problem in determining to what extent expression of these genes may be used to prognosticate cancer patient survivability. Heretofore investigations of *HER2-neu* and *EGFR* expression in NSCLC has resulted in enormous variations in frequencies of NSCLC tumors scored positive for both *EGFR* and *HER2-neu* expression. Overexpression of *HER2-neu,* defined as positive protein staining in adenocarcinomas (AC), was reported in 13-80%, in 2-45% in squamous cell carcinomas (SCC), and in 0-20% in large cell carcinomas (LC) by using paraffin embedded tissue on light microscope slides and HER2-neu antisera. Pfeiffer et al., 1996; Kern et al., 1990; Kern et al., 1994; Tateishi et al., 1991; Shi, et al., Mol Carcing 5:213-8, 1992; Bongiorno, et al., J Thorac Cardiovasc Surg 107:590-5,1994; Harpole, et al., Clin Cancer Res 1:659-64, 1995; Volm et al., Anticancer Res 12:11-20,1992. Moroever, a recent report illustrates the non-specificity of current protocols designed to assess HER2-neu expression levels. The HercepTest® for measurement of HER2-neu expression in invasive breast cancers was shown to have very high false positivity. Jacobs et al., J Clin Oncol 17: 1983-1987, 1999.

If a precise, accurate, and consistent method for determining the expression levels of *EGFR* and *HER2-neu* existed, one could ascertain what expression levels correlate to patient survivability and whether or not a receptor tyrosine kinase targeted chemotherapy is appropriate. Consistent demonstration of *EGFR* and/or *HER2-neu* overexpression in NSCLC, using a standardized method, is desirable in establishing clinical trials for current and future receptor tyrosine kinase targeted chemotherapies, e.g., chemotherapeutic agents, antibody-based drugs, to treat cancers overexpressing these receptors.

The current protocols for measuring *EGFR* and/or *HER2-neu* gene expression, aside from being insufficiently accurate for tumor prognostication, suffer from a second limitation in that they require a significant amount of fresh tissue that contains non-degraded mRNA. Most patient derived pathological samples are routinely fixed and paraffin-embedded (FPE) to allow for histological analysis and subsequent archival storage. Thus, most biopsy tissue samples are not useful for analysis of gene expression because such studies require a high integrity of RNA so that an accurate measure of gene expression can be made. Currently, gene expression levels can be only qualitatively monitored in such fixed and embedded samples by using immunohistochemical staining to monitor protein expression levels.

The use of frozen tissue by health care professionals poses substantial inconveniences. Rapid biopsy delivery to avoid tissue and subsequent mRNA degradation is the primary concern when planning any RNA-based quantitative genetic marker assay. The health care professional performing the biopsy, must hastily deliver the tissue sample to a facility equipped to perform an RNA extraction protocol immediately upon tissue sample receipt. If no such facility is available, the clinician must promptly freeze the sample in order to prevent mRNA degradation. In order for the diagnostic facility to perform a useful RNA extraction protocol prior to tissue and RNA degradation, the tissue sample must remain frozen until it reaches the diagnostic facility, however far away that may be. Maintaining frozen tissue integrity during transport using specialized couriers equipped with liquid nitrogen and dry ice, comes only at a great expense.

Routine biopsies generally comprise a heterogenous mix of stromal and tumorous tissue. Unlike with fresh or frozen tissue, FPE biopsy tissue samples are readily microdissected and separated into stromal and tumor tissue and therefore, offer andvantage over the use of fresh or frozen tissue. However, isolation of RNA from fixed tissue, and especially fixed and paraffin embedded tissue, results in highly degraded RNA, which is generally not thought to be applicable to gene expression studies.

A number of techniques exist for the purification of RNA from biological samples, but none is reliable for isolation of RNA from FPE samples. For example, Chomczynski (U.S. Pat. No. 5,346,994) describes a method for purifying RNA from tissues based on a liquid phase separation using phenol and guanidine isothiocyanate. A biological sample is homogenized in an aqueous solution of phenol and guanidine isothiocyanate and the homogenate thereafter mixed with chloroform. Following centrifugation, the homogenate separates into an organic phase, an interphase and an aqueous phase. Proteins are sequestered in the organic phase, DNA in the interphase, and RNA in the aqueous phase. RNA can be precipitated from the aqueous phase. Unfortunately, this method is not applicable to fixed and paraffin-embedded (FPE) tissue samples.

Other known techniques for isolating RNA typically utilize either guanidine salts or phenol extraction, as described for example in Sambrook, J. *et al.*, (1989) at pp. 7.3-7.24, and in Ausubel, F. M. *et al.*, (1994) at pp. 4.0.3-4.4.7. Koopmans et al., describes methods for isolating RNA from paraffin-embedded tissue sample which are time consuming or using Proteinase K. Again, none of the know methods provides reproducible quantitative results in the isolation of RNA from paraffin-embedded tissue samples.

Techniques for the isolation of RNA from paraffin-embedded tissues are thus particularly needed for the study of gene expression in tumor tissues, since expression levels of certain receptors or enzymes can then be used to determine the likelihood of success or appropriateness of a particular treatment.

We report here a significant association between high levels of the intratumoral *EGFR* mRNA and high levels of intratumoral *HERZ-neu* mRNA with an inferior survivability. Accordingly, it is the object of the invention to provide a method of quantifying *EGFR* and/or *HER2-neu* mRNA from tumor tissue in order to provide an early prognosis for receptor tyrosine kinase targeted chemotherapies. It is also the object of the invention to provide a method for assessing *EGFR* and/or *HER2-neu* levels in tissues fixed and paraffin-embedded (FPE) and predicting the probable sensitivity of a patient's tumor to treatment with receptor tyrosine kinase targeted chemotherapy by examining the amount *EGFR* and/or *HFRZ-neu* mRNA in a patient's tumor cells and comparing it to a predetermined threshold expression level.

**SUMMARY OF THE INVENTION**

In one aspect of the invention there is provided a method for assessing levels of expression of *EGFR* mRNA obtained from fresh, frozen, fixed or fixed and paraffin-embedded (FPE) tumor cells.

In another aspect of the invention there is provided a method for assessing levels of expression of *HER2-neu* mRNA obtained from fresh, frozen, fixed or fixed and paraffin-embedded (FPE) tumor cells.

In another aspect of the invention there is provided a method of quantifying the amount of *EGFR* mRNA expression relative to an internal control from a fresh, frozen, fixed or fixed and paraffin-embedded (FPE) tissue sample. This method includes isolation of total mRNA from said sample and determining the quantity of *EGFR* mRNA relative to the quantity of an internal control gene's RNA.

In another aspect of the invention there is provided a method of quantifying the amount of *HER2-neu* mRNA expression relative to an internal control from a fresh, frozen, fixed or fixed and paraffin-embedded (FPE) tissue sample. This method includes isolation of total mRNA from said sample and determining the quantity of *HER2-neu* mRNA relative to the quantity of an internal control gene's mRNA.

Furthermore there are provided oligonucleotide primers having the sequence of EGFR-1753F (SEQ ID NO: 1) or EGFR-1823R (SEQ ID NO:2) and sequences substantially identical thereto.

Oligonucleotide primers having a sequence that hybridizes to SEQ ID NO: 1 or SEQ ID NO:2 or their complements under stringent conditions are provided.

Fürthermore there are provided oligonucleotide primers having the sequence of HER2-neu 267-1F (SEQ ID NO: 4) or HER2-neu 2699R (SEQ ID NO: 5) and sequences substantially identical thereto.

Oligonucleotide primers having a sequence that hybridizes to SEQ ID NO: 4 or SEQ ID NO: 5 or their complements under stringent conditions are provided.

In yet another aspect of the invention there is provided a method for determining a receptor tyrosine kinase targeted chemotherapeutic regimen for a patient, comprising isolating RNA from a fresh, frozen, fixed or fixed and paraffin-embedded (FPE) tumor sample; isolating RNA from a fresh, frozen, fixed or fixed and paraffin-embedded (FPE) matching non-malignant tissue sample; determining a gene expression level *of EGFR* in both samples; dividing the level of *EGFR* expression in the tumor sample with the *EGFR* expression level in the matching non-malignant tissue sample to determine a differential expression level; comparing the differential *EGFR* gene expression level with a predeterimined threshold level for the *EGFR* gene; and determining a chemotherapeutic regimen based on results of the comparison of the differential *EGFR* gene expression level with the predetermined threshold level.

In yet another aspect of the invention there is provided a method for determining a receptor tyrosine kinase targeted chemotherapeutic regimen for a patient, comprising isolating RNA from a fresh, frozen, fixed or fixed and paraffin-embedded (FPE) tumor sample; isolating RNA from a fresh, frozen, fixed or fixed and paraffin-embedded (FPE) matching non-malignant tissue sample; determining a gene expression level of *HER2-neu* in both samples; divining the level of *HER2-neu* expression in the tumor sample with the *HER2-neu* expression level in the matching non-malignant tissue sample to determine a differential expression level; comparing the differential *HER2-neu* gene expression levels with a predetermined threshold level for the *HER2-neu* gene; and determining a chemotherapeutic regimen based on results of the comparison of the differential *HER2-neu* gene expression level with the predetermined threshold level.

In yet another aspect of the invention there is provided a method for determining a receptor tyrosine kinase targeted chemotherapeutic regimen for a patient, comprising isolating RNA from a fresh, frozen, fixed or fixed and paraffin-embedded (FPE) tumor sample; isolating; RNA from a fresh, frozen, fixed or fixed and paraffin-embedded (FPE) matching non-malignant tissue sample; determining gene expression levels of *HER2-neu* and *EGFR* in both of the samples; dividing the level of *EGFR* expression in the tumor sample with the *EGFR* expression level in the matching non-malignant tissue sample to determine a *EGFR* differential expression level; dividing the level of *HER2-neu* expression in the tumor sample with the *HER2-neu* expression level in the matching non-malignant tissue sample to determine a differential *HER2-neu* expression level; comparing the differential *HER2-neu* and *EGFR* gene expression levels with a predeterimined threshold level for each of the *HER2-neu* and *EGFR* genes; and determining a chemotherapeutic regimen based on results of the comparison of the differential *HER2-neu* and *EGFR* gene expression levels with the predetermined threshold levels.

In yet another aspect of the invention there is provided a method for determining the survivability of a patient, comprising isolating RNA from a fresh, frozen, fixed or fixed and paraffin-embedded (FPE) tumor sample; isolating RNA from a fresh, frozen, fixed or fixed and paraffin-embedded (FPE) matching non-malignant tissue sample; determining a gene expression level of *EGFR* in both samples; dividing the level of *EGFR* expression in the tumor sample with the *EGFR* expression level in the matching non-malignant tissue sample to determine a differential expression level; comparing the differential *EGFR* gene expression level with a predeterimined threshold level for the *EGFR* gene; and determining the survivability of a patient based on results of the comparison of the differential *EGFR* gene expression levels with the predetermined threshold level.

In yet another aspect of the invention there is provided a method for determining the survivability of a patient, comprising isolating RNA from a fresh, frozen, fixed or fixed and paraffin-embedded (FPE) tumor sample; isolating RNA from a fresh, frozen, fixed or fixed and paraffin-embedded (FPE) matching non-malignant tissue sample; determining a gene expression level of *HER2-neu* in both samples; dividing the level *of HER2-neu* expression in the tumor sample with the *EGFR* expression level in the matching non-malignant tissue sample to determine a differential expression level; comparing the differential *HER2*-neu gene expression levels with a predeterimined threshold level for the *HER2*-neu gene; and determining the survivability of a patient based on result of the comparison of the differential *HER2-neu* gene expression level with the predetermined threshold level.

In yet another aspect of the invention there is provided a method for determining the survivability of a patient, comprising isolating RNA from a fresh, frozen, fixed or fixed and paraffin-embedded (FPE) tumor sample; isolating RNA from a fresh, frozen, fixed or fixed and paraffin-embedded (FPE) matching non-malignant tissue sample; determining gene expression levels of *HFR2-neu* and *EGFR* in both of the samples; dividing the level of *EGFR* expression in the tumor sample with the *EGFR* expression level in the matching non-malignant tissue sample to determine a *EGFR* differential expression level; dividing the level of *HER2-neu* expression in the tumor sample with the *HER2 neu* expression level in the matching non-malignant tissue sample to determine a *HER2 neu* differential expression level; comparing the differential *HER2-neu* and *EGFR* gene expression levels with a predetermined threshold level for each of the *HERZ-neu* and *EGFR* genes; and determining the survivability of a patient based on results of the comparison of the *EGFR* and *HER2-neu* gene expression levels with the predetermined threshold levels.

A method of normalizing the uncorrected gene expression (UGE) of *EGFR* and *HER2-neu* relative to an internal control gene in a tissue sample analyzed using TaqMan® technology to known *EGFR*and *HER2-neu* expression levels relative to an internal control from samples analyzed by pre-TaqMan® technology is described.

## DESCRIPTION OF THE DRAWINGS

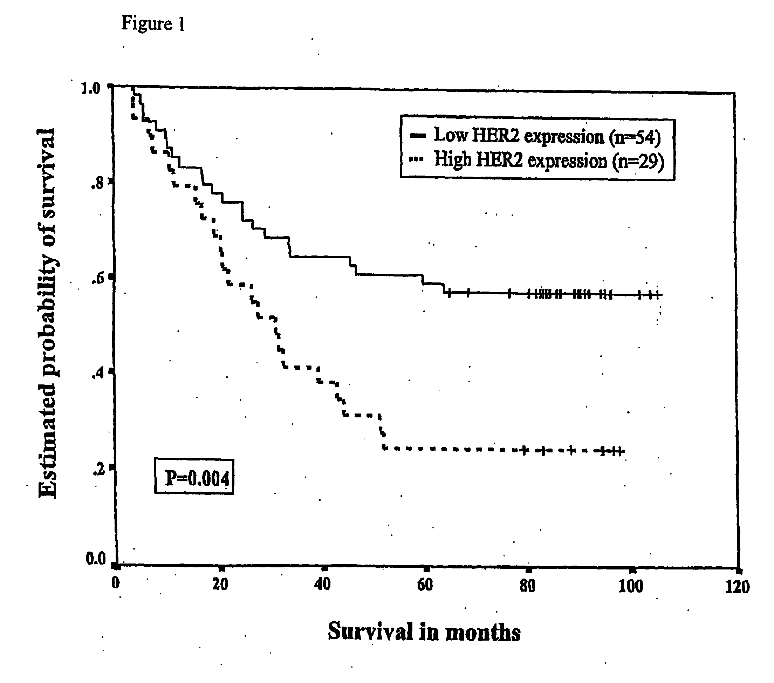

<u>Figure 1</u>. Estimated probability of survival of curatively resected non-small cell lung cancer patients versus the *HER2-neu* mRNA expression status. The median survival was not reached in the low *HER2-neu* expression group compared to 31.1 months (95% C.I: 21.96- 40.24) in the high *HER2-neu* expression group( *P*=0.004).

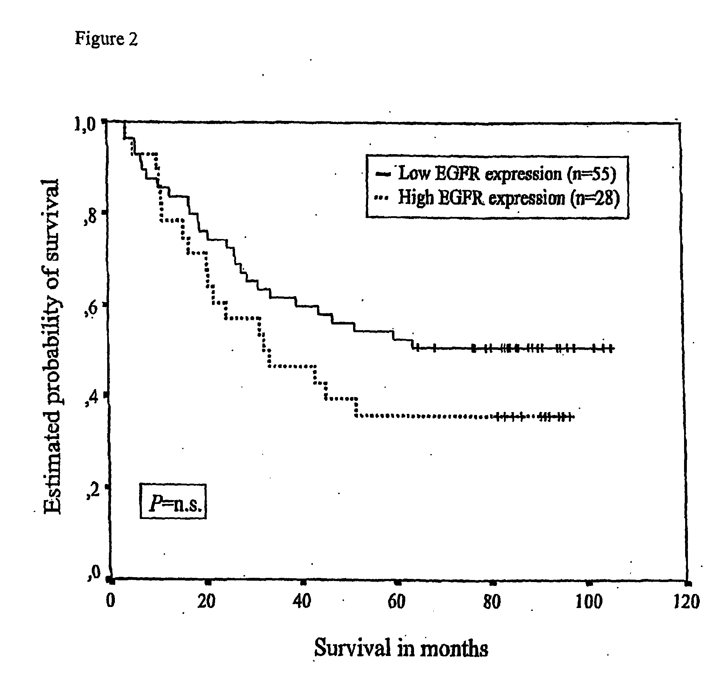

<u>Figure 2</u>. Estimated probability of survival of curatively resected non-small cell lung cancer patients versus the *EGFR* mRNA expression status. A trend towards inferior overall survival was observable for the high *EGFR* expression group, but did not reach statistical significance. The median survival was not reached in the low *EGFR* expression group compared to 32.37 months (95% C.I: 8.43-56.31) in the high *EGFR* expressor group (*P*=0.176).

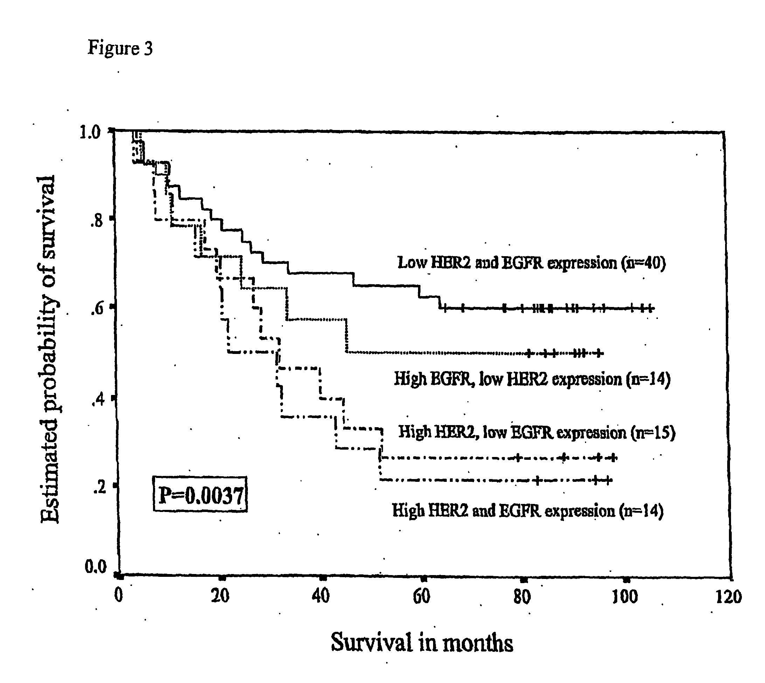

<u>Figure 3</u>. Estimated probability of survival of curatively resected non-small cell lung cancer patients versus combined patterns of *EGFR* and *HER2-neu* co-expression in NSCLC. The median survival was not reached in the group that showed low *HER2-neu* and *EGFR* expression, compared to 45.47 months in the high *EGFR* expression group, 31.10 months (95% C.I.: 14,77-47.43) in the high *HER2-neu* expression group, and 22.03 months (95% C.I: 2.30-41.76; P=0.003) in the high *HER2-neu* and *EGFR* expression group.

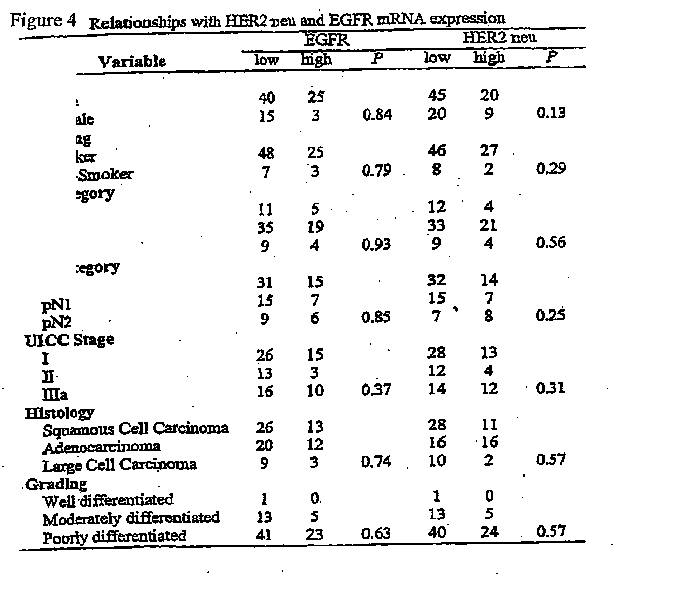

<u>Figure 4</u>. Table showing high and low *EGFR* and *HER2-neu* expression in patients and tumors.

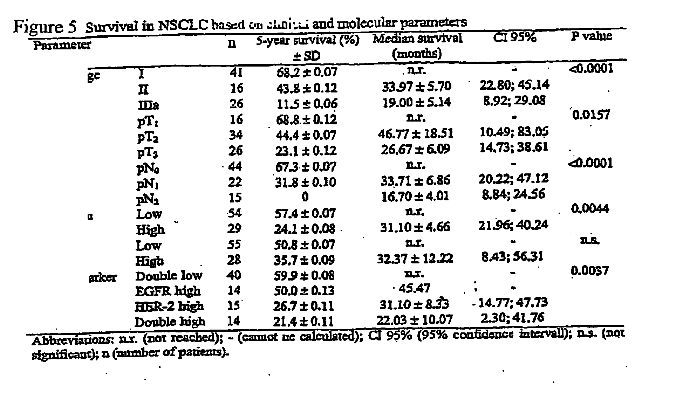

<u>Figure5</u>. Table showing the survival of patients based on clinical and molecular parameters.

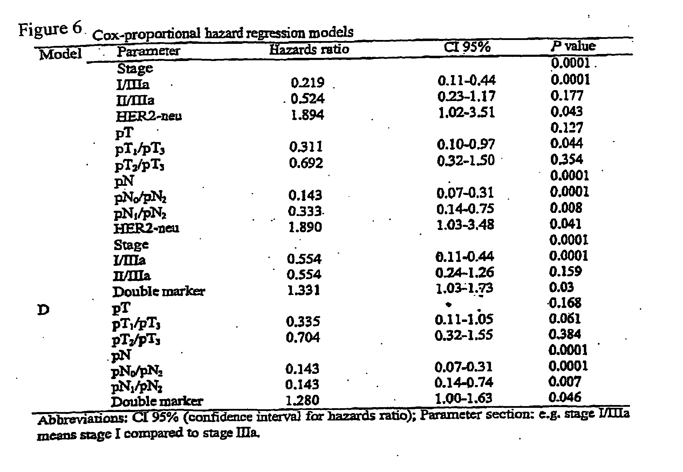

<u>Figure 6</u>. Table showing Cox-proportional hazard regression models. Double marker refers to both *EGFR* and *HER2-neu* expression.

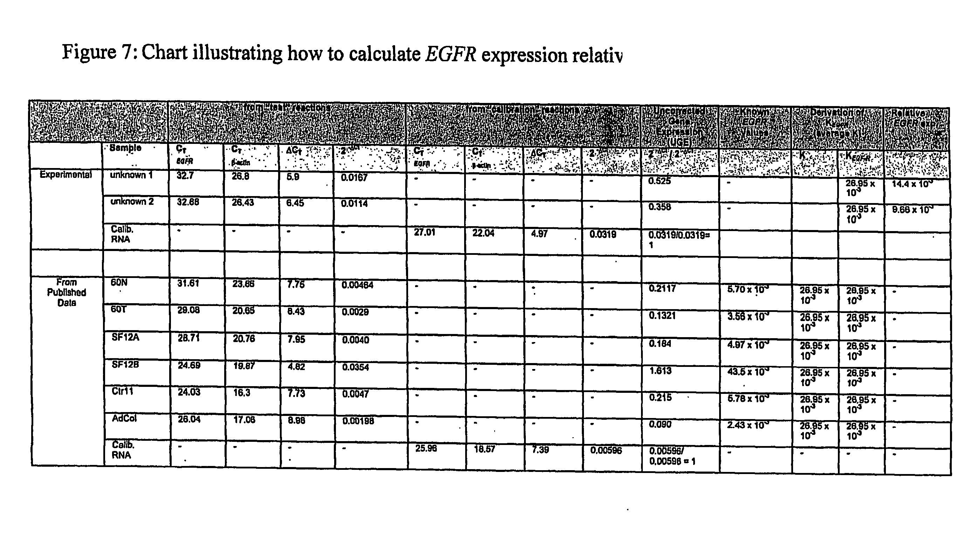

<u>Figure 7</u> is a chart illustrating how to calculated *EGFR* expression relative to an internal control gene. The chart contains data obtained with two test samples, (unknowns 1 and 2), and illustrates how to determine the uncorrected gene expression data (UGE). The chart also illustrates how to normalize UGE generated by the TaqMan® instrument with known relative *EGFR* values determined by pre-TaqMan® technology. This is accomplished by multiplying UGE to a correction factor $K_{EGFR}$. The internal control gene in the figure is β-actin and the calibrator RNA is Human Liver Total RNA (Stratagene, Cat #735017).

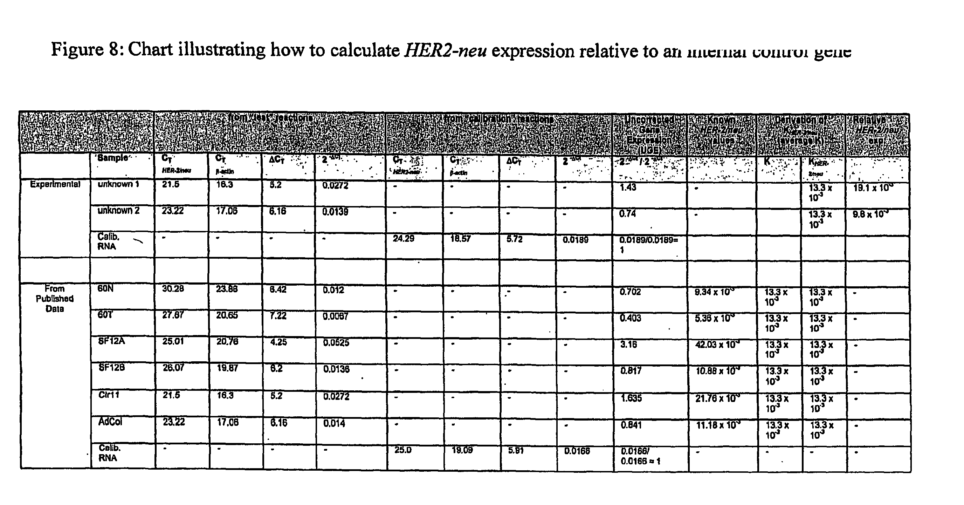

Figure 8 is a chart illustrating how to calculate *HER2-neu* expression relative to an internal control gene. The chart contains data obtained with two test samples, (unknowns 1 and 2), and illustrates how to determine the uncorrected gene expression data (UGE). The chart also illustrates how to normalize UGE generated by the TaqMan® instrument with previously published *HER2-neu* values. This is accomplished by multiplying UGE to a correction factor $K_{HER2\text{-}neu}$. The internal control gene in the figure is β-actin and the calibrator RNA is Human Liver Total RNA (Stratagene, Cat #735017).

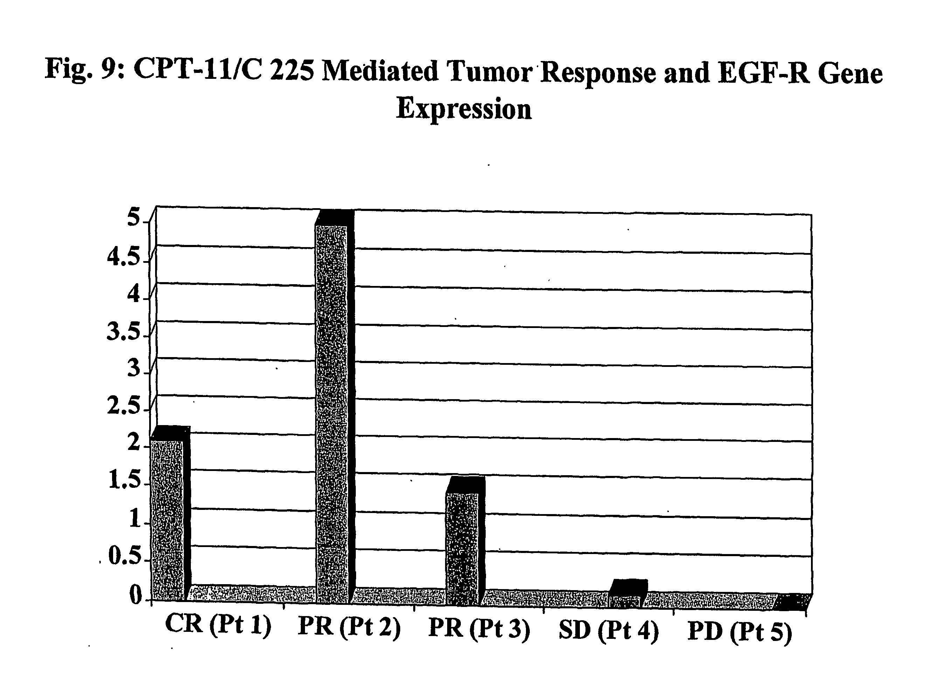

Figure 9 is a graph showing the Corrected EGFR expression values of 5 different colon cancer patients' tumors. The patients were on a CPT-11/C225 receptor tyrosine kinase targeted treatment regimen. Patient 1 was determined to have a corrected EGFR expression level of 2.08 x $10^{-3}$ and had a completed response (CR). Patient 2 had a corrected EGFR expression level of 8.04 x $10^{-3}$ and had a partial response (PR). Patient 3 had a corrected EGFR expression level of 1.47 x $10^{-3}$ and also showed a partial response (PR). Patient 4 had a corrected EGFR expression level of 0.16 x x $10^{-3}$ and had stable disease (SD) showing no response. Patient 5 had a no EGFR expression (0.0 x $10^{-3}$) and had progressive disease (PR).

## DETAILED DESCRIPTION OF THE INVENTION

Tumors expressing high levels of *HER2-neu* and/or *EGFR* mRNA are considered likely to be sensitive to receptor tyrosine kinase targeted chemotherapy. Conversely, those tumors expressing low amounts of *HER2-eu* and *EGFR* mRNA are not likely to be sensitive to receptor tyrosine kinase targeted chemotherapy. A patient's differential *HER2-neu* and *EGFR* mRNA expression status is judged by comparing it to a predetermined threshold expression level.

The invention provides a method of quantifying the amount of *HERZ-neu* and/or *EGFR* mRNA expression in fresh, frozen, fixed or fixed and paraffin-embedded (FPE) tissue relative to gene expression of an internal control. The present inventors have developed oligonucleotide primers that allow accurate assessment of *HER2-neu* and *EGFR* gene expression in fresh, frozen, fixed or fixed and embedded tissues. The oligonucleotide primers, EGFR-1753F (SEQ ID NO: 1), EGFR-1823R (SEQ ID NO: 2), or oligonucleotide primers substantially identical thereto, preferably are used together with RNA extracted from fresh, frozen, fixed or fixed and paraffin embedded (FPE) tumor samples. Provided are oligonucleotide primers, HER2-neu 2671F (SEQ ID NO: 4), HER2-neu 2699R (SEQ ID NO: 5), or oligonucleotide primers substantially identical thereto, preferably are used together with RNA extracted from fresh, frozen, fixed or fixed and paraffin embedded (FPE) tumor samples. This measurement of *HER2-neu* and/or *EGFR* gene expression may then be used for prognosis of receptor tyrosine kinase targeted chemotherapy

This embodiment of the invention involves, a method for reliable extraction of RNA from fresh, frozen, fixed or FPE samples, determination of the content of *EGFR* mRNA in the sample by using a pair of oligonucleotide primers, preferably oligionucleotide primer pair EGFR-1753F (SEQ ID NO: 1) and EGFR-1823R (SEQ ID NO: 2), or oligonucleotides substantially identical thereto, for carrying out reverse transcriptase polymerase chain reaction.

Another embodiment of the invention involves a method for reliable extraction of RNA from fresh, frozen, fixed or FPE samples, and determination of the content of *HER2-neu* mRNA in the sample by using a pair of oligonucleotide primers oligonucleotide primers, HER2-neu 2671F (SEQ ID NO: 4), HER2-neu 2699R (SEQ ID NO: 5), or oligonucleotide primers substantially identical thereto.

"Substantially identical" in the nucleic acid context as used herein, means hybridization to a target under stringent conditions, and also that the nucleic acid segments, or their complementary strands, when compared, are the same when properly aligned, with the appropriate nucleotide insertions and deletions, in at least about 60% of the nucleotides, typically, at least about 70%, more typically, at least about 80%, usually, at least about 90%, and more usually, at least, about 95-98% of the nucleotides. Selective hybridization exists when the hybridization is more selective than total lack of specificity. See, Kanehisa, Nucleic Acids Res., 12:203-213 (1984).

The methods of the present invention can be applied over a wide range of tumor types. This allows for the preparation of individual "tumor expression profile whereby expression levels *of HER2-neu* and/or *EGFR* are determined in individual patient samples and response to various chemotherapeutics is predicted. Preferably, the methods of the invention are applied to solid tumors, most preferably NSCLC tumors.

A "differential expression level" as defined herein refers to the difference in the level of expression of either *EGFR* or *HER2-neu* in a tumor with respect to the level of expression of either *EGFR* or *HER2-neu* in a matching non-malignant tissue sample, respectively. The differential expression level is determined by dividing the UGE of a particular gene from the tumor sample with the UGE of the same gene from a matching non-malignant tissue sample.

A "predetermined threshold level", as defined herein relating to *EGFR* expression, is a level of differential *EGFR* expression above which (i.e., high), tumors are likely to be sensitive to a receptor tyrosine kinase targeted chemotherapeutic regimen. A high differential *EGFR* expression level is prognostic of lower patient survivability. Tumors with expression levels below this threshold level are not likely to be affected by a receptor tyrosine kinase targeted chemotherapeutic regimen. A low differential *EGFR* expression level is prognostic of higher patient survivability. Wether or not differential expression is above or below a "predetermined threshold level" is determined by the method used by Mafune et al., who calculated individual differential tumor/normal (T/N) expression ratios in matching non-malignant tissues obtained from patients with squamous cell carcinoma of the esophagus. Mafune et al., Clin Cancer Res 5:4073-4078, 1999. This method of analysis leads to a precise expression value for each patient, being based on the individual background expression obtained from matching non-malignant tissue. The differential expression of *EGFR* is considered "high" and indicative of low survivability if the UGE *of EGFR :* β-actin in a tumor sample divided by the UGE *of EGFR :* β-actin in a matching non-malignant tissue sample, is above the predetermined threshold value of about 1.8. The differential expression of *EGFR* is considered "low" and indicative of high survivability if the UGE of *EGFR :* β-actin in a tumor sample divided by the UG*E of EGFR :* β-actin in a matching n+on-malignant tissue sample, is below the predetermined threshold value of about 1.8.

A "predetermined threshold level", as defined herein relating to differential *HER2-neu* expression, is a level of *HER2-neu* expression above which (i.e., high), tumors are likely to be sensitive to a receptor tyrosine kinase targeted chemotherapeutic regimen. A high differential *HER2-neu* expression level is prognostic of lower patient survivability. Tumors with expression levels below this threshold level are not likely to be affected by a receptor tyrosine kinase targeted chemotherapeutic regimen. A low differential *HER2-neu* expression level is prognostic of higher patient survivability. The differential expression of *HER2-neu* is considered "high" and indicative of low survivability if the UGE of *HER2-neu* : β-actin in a tumor sample divided by the UGE of *HER2-neu* : β-action in a matching non-malignant tissue sample, is above the predetermined threshold value of about 1.8. The differential expression *of HER2-neu* is considered "low" and indicative of high survivability if the UGE of *HER2-neu* : β-actin in a tumor sample divided by the UGE of *HER2-neu* : β-actin in a matching non-malignant tissue sample, is below the predetermined threshold value of about 1.8.

A "threshold level" for *HER2-neu* was determined using the following results and method. The corrected *HER2-neu* mRNA expression, expressed as the ratio between *HER2-neu* and β-Actin PCR product, was 4.17 x $10^{-3}$ (range 0.28-23.86 x $10^{-3}$ in normal lung and 4.35 x $10^{-3}$ (range: 0.21-68.11 x $10^{-3}$) in tumor tissue (*P*=0.019 Wilcoxon test). The maximal chi-square method by Miller and Siegmund (Miller et al., Biometrics 38:1011-1016, 1982) and Halpern (Biometrics 38:1017-1023, 1982) determined a threshold value of 1.8 to segregate patients into low and high differential *HER2-neu* expressors. By this criterion, 29 (34.9%) patients had a high differential *HER2-neu* expression and 54 (65.1 %) had a low differential *HER2-neu* expression.

A "threshold level" for *EGFR* was determined using the following results and method. The median corrected *EGFR* mRNA expression was 8.17 x $10^{-3}$(range: 0.31-46.26 x $10^{-3}$) in normal lung and 7.22 x $10^{-3}$ (range: 0.27-97.49 x $10^{-3}$) in tumor tissue (*P*=n.s.). The maximal chi-square method (Miller (1982); Halpern (1982)) determined a threshold value of 1.8 to segregate patients into low and high differential *EGFR* expressors. By this criterion, 28 (33.7%) patients had a high differential *EGFR* expression and 55 (66.3%) had a low differential *EGFR* expression status.

In performing the method of the present invention either differential EGFR expression levels or differential *HER2-neu* expression levels are assayed in a patient to prognosticate the efficacy of a receptor tyrosine kinase targeted chemotherapeutic regimen. Moreover, in the method of the present invention differential *HER2-neu* expression levels are assayed in a patient prognosticate the efficacy of a receptor tyrosine kinase targeted chemotherapeutic regimen. Additionally, in the method of the present invention differential *EGFR* expression levels are assayed in a patient to prognosticate the efficacy of a receptor tyrosine kinase targeted chemotherapeutic regimen. Alternatively, both differential *EGFR* expression levels and differential *HER2-neu* expression levels are assayed in a patient to prognosticate the efficace of a receptor tyrosine kinase targeted chemotherapeutic regimen.

"Matching non-malignant sample" as defined herein refers to a sample of non-cancerous tissue derived from the same individual as the tumor sample to be analyzed for differential *EGFR* and/or differential *HER2-neu* expression. Preferably a matching non-malignant sample is derived from the same organ as the organ from which the tumor sample is derived. Most preferably, the matching non-malignant tumor sample is derived from the same organ tissue layer from which the tumor sample is derived. Also, it is preferable to take a matching non-malignant tissue sample at the same time a tumor sample is biopsied. In a preferred embodiment tissues from the following two locations are analyzed: lung tumor and non-malignant lung tissue taken from the greatest distance form the tumor or colon tumor and non-malignant colon tissue taken from the greatest distance form the tumor as possible under the circumstances.

In performing the method of this embodiment of the present invention, tumor cells are preferably isolated from the patient. Solid or lymphoid tumors or portions thereof are surgically resected from the patient or obtained by routine biopsy. RNA isolated from frozen or fresh tumor samples is extracted from the cells by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

However, tissue obtained from the patient after biopsy is often fixed, usually by formalin (formaldehyde) or gluteradehyde, for example, or by alcohol immersion. Fixed biological samples are often dehydrated and embedded in paraffin or other solid supports known to those of skill in the art. See Plenat et al., Ann Pathol 2001 Jan;21(1):29-47. Non-embedded, fixed tissue as well as fixed and embedded tissue may also be used in the present methods. Solid supports for embedding fixed tissue are envisioned to be removable with organic solvents for example, allowing for subsequent rehydration of preserved tissue.

RNA is extracted from paraffin-embedded (FPE) tissue cells by any of the methods as described in US 6248535, filed December 20, 1999. As used herein, FPE tissue means tissue that has been fixed and embedded in ansolid removable support, such as storable or archival tissue samples. RNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. RNA is then extracted from the sample.

For RNA extraction, the fixed or fixed and deparaffinized samples can be homogenized using mechanical, sonic or other means of homogenization. Rehydrated samples may be homogenized in a solution comprising a chaotropic agent, such as guanidinium thiocyanate (also sold as guanidinium isothiocyanate). Homogenized samples are heated to a temperature in the range of about 50 to about 100°C in a chaotropic solution, which contains an effective amount of a chaotropic agent, such as a guanidinium compound. A preferred chaotropic agent is guanidinium thiocyanate.

An "effective concentration of chaotropic agent" is chosen such that RNA is purified from a paraffin-embedded sample in an amount of greater than about 10-fold that isolated in the absence of a chaotropic agent. Chaotropic agents include, for example: guanidinium compounds, urea, formamide, potassium iodiode, potassium thiocyantate and similar compounds. The preferred chaotropic agent for the methods of the invention is a guanidinium compound, such as guanidinium isothiocyanate (also sold as guanidinium thiocyanate) and guanidinium hydrochloride. Many anionic counterions are useful, and one of skill in the art can prepare many guanidinium salts with such appropriate anions. The effective concentration of guanidinium solution used in the invention generally has a concentration in the range of about 1 to about 5M with a preferred value of about 4M. If RNA is already in solution, the guanidinium solution may be of higher concentration such that the final concentration achieved in the sample is in the range of about 1 to about 5M. The guanidinium solution also is preferably buffered to a pH of about 3 to about 6, more preferably about 4, with a suitable biochemical buffer such as Tris-Cl. The chaotropic solution may also contain reducing agents, such as dithiothreitol (DTT) and β-mercaptoethanol (BME). The chaotropic solution may also contain RNAse inhibitors.

RNA is then recovered from the chaotropic solution by, for example, phenol chloroform extraction, ion exchange chromatography or size-exclusion chromatography. RNA may then be further purified using the techniques of extraction, electrophoresis, chromatography, precipitation or other suitable techniques.

The quantification of *HER2-neu* or *EGFR* mRNA from purified total mRNA from fresh, frozen or fixed is preferably carried out using reverse-transcriptase polymerase chain reaction (RT-PCR) methods common in the art, for example. Other methods of quantifying *of HER2-neu* or *EGFR* mRNA include for example, the use of molecular beacons and other labeled probes useful in multiplex PCR. Additionally, the present invention envisages the quantification *of HER2-neu* and/or *EGFR* mRNA via use of a PCR-free systems employing, for example fluorescent labeled probes similar to those of the Invader® Assay (Third Wave Technologies, Inc.). Most preferably, quantification of *HER2-neu* and/or *EGFR* cDNA and an internal control or house keeping gene (e.g. β-actin) is done using a fluorescence based real-time detection method (ABI PRISM 7700 or 7900 Sequence Detection System [TaqMan®], Applied Biosystems, Foster City, CA.) or similar system as described by Heid et al., (Genome Res 1996;6:986-994) and Gibson et al.(Genome Res 1996;6: 995-1001). The output of the ABI 7700 (TaqMan® Instrument) is expressed in Ct's or "cycle thresholds". With the TaqMan® system, a highly expressed gene having a higher number of target molecules in a sample generates a signal with fewer PCR cycles (lower Ct) than a gene of lower relative expression with fewer target molecules (higher Ct).

As used herein, a "house keeping" gene or "internal control" is any constitutively or globally expressed gene whose presence enables an assessment of *HER2-neu* and/or *EGFR* mRNA levels. Such an assessment comprises a determination of the overall constitutive level of gene transcription and a control for variations in RNA recovery. "Housekeeping" genes or "internal controls" can include, but are not limited to the cyclophilin gene, β-actin gene, the transferrin receptor gene, GAPDH gene, and the like. Most preferably, the internal control gene is β-actin gene as described by Eads et al., Cancer Research 1999; 59:2302-2306.

A control for variations in RNA recovery requires the use of "calibrator RNA." The "calibrator RNA" is intended to be any available source of accurately pre-quantified control RNA. Preferably,Human Liver Total RNA (Stratagene, Cat #735017) is used.

"Uncorrected Gene Expression (UGE)" as used herein refers to the numeric output *of HER2-neu* and/or *EGFR* expression relative to an internal control gene generated by the TaqMan® instrument. The equation used to determine UGE is shown in Examples 3 and 4, and illustrated with sample calculations in Figures 7 and 8.

These numerical values allow the determination of whether or not the differential gene expression (i.e., "UGE" or of a particular tumor sample divided by the "UGE" of a matching non-tumor sample) falls above or below the "predetermined threshold" level. The predetermined threshold level for *EGFR* and *HER2-neu* is about 1.8.

A further aspect of this invention provides a method to normalize uncorrected gene expression (UGE) values acquired from the TaqMan® instrument with "known relative gene expression" values derived from non-TaqMan® technology. Preferably, TaqMan® derived HER2-*neu* and/or EGFR UGE values from a tissue sample are normalized to samples with known non-TaqMan® derived relative *HER2-neu* and/or *EGFR* : β-actin expression values.

"Corrected Relative *EGFR* Expression" as used herein refers to normalized *EGFR* expression whereby UGE is multiplied with a *EGFR* specific correction factor ($K_{EGFR}$), resulting in a value that can be compared to a known range of *EGFR* expression levels relative to an internal control gene. Example 3 and Figure 7 illustrate these calculations in detail. $K_{EGFR}$ specific for *EGFR,* the internal control β-actin and calibrator Human Liver Total RNA (Stratagene, Cat #735017), is $26.95 \times 10^{-3}$. These numerical values also allow the determination of whether or not the "Corrected Relative Expression" of a particular tumor sample divided by the "Corrected Relative Expression" of a matching non-tumor sample (i.e., differential expression) falls above or below the "predetermined threshold" level. The predetermined threshold level for *HER2-neu* or *EGFR* is about 1.8. In determining whether the differential expression of either *EGFR* or *HER2-neu* in a tumor sample is 1.8 times greater than in a matching non-tumor sample, one will readily recognize that either UGE values or Corrected Relative Expression values can be used. For example, if one divides the Corrected Relative Expression level of the tumor with that of the matching non-tumor sample, the K-factor cancels out and one is left with same ratio as if one had used UGE values.

"Known relative gene expression" values are derived from previously analyzed tissue samples and are based on the ratio of the RT-PCR signal of a target gene to a constitutively expressed internal control gene (e.g. β-Actin, GAPDH, etc.). Preferably such tissue samples are formalin fixed and paraffin-embedded (FPE) samples and RNA is extracted from them according to the protocol described in Example 1. To quantify gene expression relative to an internal control standard quantitative RT-PCR technology known in the art is used. Pre-TaqMan® technology PCR reactions are run for a fixed number of cycles (i.e., 30) and endpoint values are reported for each sample. These values are then reported as a ratio *of EGFR* expression to β-actin expression.

$K_{EGFR}$ may be determined for an internal control gene other than β-actin and/or a calibrator RNA different than Human Liver Total RNA (Stratagene, Cat #735017). To do so, one must calibrate both the internal control gene and the calibrator RNA to tissue samples for which *EGFR* expression levels relative to that particular internal control gene have already been determined (i.e., "known relative gene expression"). Preferably such tissue samples are formalin fixed and paraffin-embedded (FPE) samples and RNA is extracted from them according to the protocol described in Example 1. Such a determination can be made using standard pre-TaqMan®, quantitative RT-PCR techniques well known in the art. Upon such a determination, such samples have "known relative gene expression" levels of *EGFR* useful in the determining a new $K_{EGFR}$ specific for the new internal control and/or calibrator RNA as described in Example 3.

"Corrected Relative *HER2-neu* Expression" as used herein refers to normalized *HER2-neu* expression whereby UGE is multiplied with a *HER2-neu* specific correction factor ($K_{HER2\text{-}neu}$), resulting in a value that can be compared to a known range *of HER2-neu* expression levels relative to an internal control gene. Example 4 and Figure 8 illustrate these calculations in detail. $K_{HER2\text{-}neu}$ specific for *HER2-neu,* the internal control β-actin and calibrator Human Liver Total RNA (Stratagene, Cat #735017), is $13.3 \times 10^{-3}$.

$K_{HER2\text{-}neu}$ may be determined for an internal control gene other than β-actin and/or a calibrator RNA different than Human Liver Total RNA (Stratagene, Cat #735017). To do so, one must calibrate both the internal control gene and the calibrator RNA to tissue samples for which *HER2-neu* expression levels relative to that particular internal control gene have already been determined (i.e., "known relative gene expression"). Preferably such tissue samples are formalin fixed and paraffin-embedded (FPE) samples and RNA is extracted from them according to the protocol described in herein. Such a determination can be made using standard pre-TaqMan®, quantitative RT-PCR techniques well known in the art, for example. Upon such a determination, such samples have "known relative gene expression" levels of *HER2-neu* useful in the determining a new $K_{HER2\text{-}neu}$ specific for the new internal control and/or calibrator RNA as described in Example 4.

The methods of the invention are applicable to a wide range of tissue and tumor types and so can be used for assessment of clinical treatment of a patient and as a diagnostic or prognostic tool for a range of cancers including breast, head and neck, lung, esophageal, colorectal, and others. In a preferred embodiment, the present methods are applied to prognosis of NSCLC tumors.

Pre-chemotherapy treatment tumor biopsies are usually available only as fixed paraffin embedded (FPE) tissues, generally containing only a very small amount of heterogeneous tissue. Such FPE samples are readily amenable to microdissection, so that *HER2-neu* and/or *EGFR* gene expression may be determined in tumor tissue uncontaminated with non-malignant stromal tissue. Additionally, comparisons can be made between non-malignant stromal and tumor tissue within a biopsy tissue sample, since such samples often contain both types of tissues.

Generally, any oligonucleotide pairs that flank a region *of EGFR* gene, as shown in SEQ ID NO: 10, may be used to carry out the methods of the invention. Primers hybridizing under stringent conditions to a region of the *EGFR* gene for use in the present invention will amplify a product between 20-1000 base pairs, preferably 50-100 base pairs, most preferably less than 100 base pairs.

Specific oligonucleotide primer pairs and oligonucleotide primers substantially identical thereto, that allow particularly accurate assessment *of EGFR* expression using fresh, frozen, fixed or FPE tissues are provided. Preferable are oligonucleotide primers, EGFR-1753F (SEQ ID NO: 1) and EGFR-1823R (SEQ ID NO: 2), (also referred to herein as the oligonucleotide primer pair EGFR) and oligonucleotide primers substantially identical thereto. The oliogonucleotide primers EGFR-1753F (SEQ ID NO: 1) and EGFR-1823R, (SEQ ID NO: 2) have been shown to be particularly effective for measuring *EGFR* mRNA levels using RNA extracted from fresh, frozen, fixed or FPE cells by any of the methods for mRNA isolation, for example as described Example 1.

Furthermore, any oligonucleotide pairs that flank a region of *HER2-neu* gene, as shown in SEQ ID NO: 11, may be used to carry out the methods of the invention. Primers hybridizing under stringent conditions to a region of the *HER2-neu* gene for use in the present invention will amplify a product between about 20-1000 base pairs, preferably about 50-100 base pairs, most preferably less than about 100 base pairs.

Specific oligonucleotide primers pairs and oligonucleotide primers substantially identical thereto, that allow particularly accurate assessment of *HER2-neu* expression in fresh, frozen, fixed or FPE tissues are provided. Preferable are oligonucleotide primers, HER2-neu 2671F (SEQ ID NO: 4) and HER2-neu 2699R (SEQ ID NO: 5), (also referred to herein as the oligonucleotide primer pair HER2-neu) and oligonucleotide primers substantially identical thereto. The oliogonucleotide primers HER2-neu 2671F (SEQ ID NO: 4) and HER2-neu 2699R (SEQ ID NO: 5) have been shown to be particularly effective for measuring *HER2-neu* mRNA levels using RNA extracted from fresh, frozen, fixed or FPE cells by any of the methods for mRNA isolation, for example as described herein.

Identical oligonucleotides that hybridize under stringent conditions (as defined herein) to all or a portion of the oligonucleotide primer sequence of EGFR-1753F (SEQ ID NO: 1), its complement or EGFR-1823R (SEQ ID NO: 2), or its complement or oligonucleotide primer sequence of HER2-neu 2671F (SEQ ID NO: 4), its complement or HER2-neu 2699R (SEQ ID NO: 5), or its complement are provided.

Under stringent hybridization conditions, only highly complementary, i.e., substantially similar nucleic acid sequences as defined herein hybridize. Preferably, such conditions prevent hybridization of nucleic acids having 4 or more mismatches out of 20 contiguous nucleotides, more preferably 2 or more mismatches out of 20 contiguous nucleotides, most preferably one or more mismatch out of 20 contiguous nucleotides.

The hybridizing portion of the nucleic acids is typically at least about 10 (e.g., 15) nucleotides in length. The hybridizing portion of the hybridizing nucleic acid is at least about 80%, preferably at least about 95%, or most preferably about at least 98%, identical to the sequence of a portion or all of oligonucleotide primer 5 EGFR-1753F (SEQ ID NO: 1), its complement or EGFR-1823R (SEQ ID NO: 2), or its complement or oligonucleotide primer HER2-neu 2671F (SEQ ID NO: 4), its complement or HER2-neu 2699R (SEQ ID NO: 5), or its complement.

Hybridization of the oligonucleotide primer to a nucleic acid sample under stringent conditions is defined below. Nucleic acid duplex or hybrid stability is expressed as a melting temperature ($T_m$), which is the temperture at which the probe dissociates from the target DNA. This melting temperature is used to define the required stringency conditions. If sequences are to be identified that are substantially identical to the probe, rather than identical, then it is useful to first establish the lowest temperature at which only holmologous hybridization occurs with a particular concentration of salt (e.g. SSC or SSPE). Then assuming that 1% mismatching results in a 1°C decrease in $T_m$, the temperatre of the final wash in the hybridization reaction is reduced accordingly (for example, if sequences having >95% identity with the probe are sought, the final wash temperature is decrease by 5°C). In practice, the change in $T_m$ can be between 0.5°C and 1.5°C per 1% mismatch.

Stringent conditions involve hybridizing at about 68°C in 5x SSC/5x Denhart's solution/1.0% SDS, and washing in 0.2x SSC/0.1% SDS at room temperature. Moderately stringent conditions include washing in 3x SSC at about 42°C. The parameters of salt concentration and temperature be varied to achieve optimal level of identity between the primer and the target nucleic acid. Additional guidance regarding such conditions is readily available in the art, for example, Sambrook, Fischer and Maniatis, Molecular Cloning; a laboratory manual, (2nd ed.), Cold Spring Harbor Laboratory Press, New York, (1989) and F. M. Ausubel et al eds., Current Protocols in Molecular Biology, John Wiley and Sons (1994).

Oligonucleotide primers disclosed herein are capable of allowing accurate assessment *of HER2-neu* and/or *EGFR* gene expression in a fixed or fixed and paraffin embedded tissue, as well as frozen or fresh tissue. This is despite the fact that RNA derived from FPE samples is more fragmented relative to that of fresh or frozen tissue. Thus, the methods of the invention are suitable for use in assaying *HER2-neu* and/or *EGFR* gene expression levels in all tissues where previously there existed no accurate and consistent way to *assay HER2-neu* and/or *EGFR* gene in fresh and frozen tissues and no way at all to assay *HER2-neu* and/or *EGFR* gene expression using fixed tissues.

Over-activity *of HFR2-neu* refers to either an amplification of the gene encoding HER2-neu or the production of a level of HER2-neu activity which can be correlated with a cell proliferative disorder (i.e., as the level of HER2-neu increases the severity of one or more of the symptoms of the cell proliferative disorder increases).

A "receptor tyrosine kinase targeted" chemotherapy or chemotherapeutic regimen in the context of the present invention refers a chemotherapy comprising agents that specifically interfere with Class I receptor tyrosine kinase function. Preferably, such agents will inhibit EGFR and/or HER2-neu receptor tyrosine kinase signaling activity. Such agents include 4-anilinoquinazolines such as 6-acrylamido-4-anilinoquinazoline Bonvini et al., Cancer Res. 2001 Feb 15;61(4): 1671-7 and derivatives, erbstatin (Toi et al., Eur. J. Cancer Clin. Oncol., 1990, 26, 722.), Geldanamycin, bis monocyclic, bicyclic or heterocyclic aryl compounds (PCT WO 92/20642), vinylene-azaindole derivatives (PCT WO 94/14808) and 1-cycloproppyl-4-pyridyl-quinolones (U.S. Pat. No. 5,330,992) which have been described generally as tyrosine kinase inhibitors. Also, Styryl compounds (U.S. Pat. No. 5,217,999), styryl-substituted pyridyl compounds (U.S. Pat. No. 5,302,606), certain quinazoline derivatives (EP Application No. 0 566 266 Al), seleoindoles and selenides (PCT WO 94/03427), tricyclic polyhydroxylic compounds (PCT WO 92/21660) and benzylphosphonic acid compounds (PCT WO 91/15495) have been described as compounds for use as tyrosine kinase inhibitors for use in the treatment of cancer.

Other agents targeting EGFR and/or HER2-neu receptor tyrosine kinase signaling activity include antibodies that inhibit growth factor receptor biological function indirectly by mediating cytotoxicity via a targeting function

Antibodies complexing with the receptor activates serum complement and/or mediate antibody-dependent cellular cytotoxicity. The antibodies which bind the receptor can also be conjugated to a toxin (immunotoxins). Advantageously antibodies are selected which greatly inhibit the receptor function by binding the steric vicinity of the ligand binding site of the receptor (blocking the receptor), and/or which bind the growth factor in such a way as to prevent (block) the ligand from binding to the receptor. These antibodies are selected using conventional in vitro assays for selecting antibodies which neutralize receptor function. Antibodies that act as ligand agonists by mimicking the ligand are discarded by conducting suitable assays as will be apparent to those skilled in the art. For certain tumor cells, the antibodies inhibit an autocrine growth cycle (i.e. where a cell secretes a growth factor which then binds to a receptor of the same cell). Since some ligands, e.g. TGF-$\alpha$, are found lodged in cell membranes, the antibodies serving a targeting function are directed against the ligand and/or the receptor

The cytotoxic moiety of the immunotoxin may be a cytotoxic drug or an enzymatically active toxin of bacterial or plant origin, or an enzymatically active fragment of such a toxin. Enzymatically active toxins and fragments thereof used are diphtheria, nonbinding active fragments of diphtheria toxin, exotoxin (from Pseudomonas aeruginosa), ricin, abrin, modeccin, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin. In another embodiment, the antibodies are conjugated to small molecule anticancer drugs. Conjugates of the monoclonal antibody and such cytotoxic moieties are made using a variety of bifunctional protein coupling agents. Examples of such reagents are SPDP, IT, bifunctional derivatives of imidoesters such a dimethyl adipimidate HCl, active esters such as disuccinimidyl suberate, aldehydes such as glutaraldehyde, bis-azido compounds such as bis (p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as 1,5-difluoro-2,4-dinitrobenzene. The lysing portion of a toxin may be joined to the Fab fragment of the antibodies.

Cytotoxic radiopharmaceuticals for treating cancer may be made by conjugating radioactive isotopes to the antibodies. The term "cytotoxic moiety" as used herein is intended to include such isotopes.

In another embodiment, liposomes are filled with a cytotoxic drug and the liposomes are coated with antibodies specifically binding a growth factor receptor. Since there are many receptor sites, this method permits delivery of large amounts of drug to the appropriate cell type. The exact formulation, route of administration and dosage can be chosen by the individual physician in view of the patient's condition. (See e.g. Fingl et al, in The Pharmacological Basis of Therapeutics, 1975, Ch. 1 p. 1). It should be noted that the attending physician would know how and when to terminate, interrupt, or adjust administration due to toxicity, or organ dysfunctions. Conversely, the attending physician would also know to adjust treatment to higher levels if the clinical response were not adequate (precluding toxicity). The magnitude of an administrated dose in the management of the oncogenic disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient.

Depending on the specific conditions being treated, such agents may be formulated and administered systemically or locally. Techniques for formulation and administration may be found in Remington's Pharmaceutical Sciences, 18th ed., Mack Publishing Co., Easton, Pa. (1990). Suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections, just to name a few. For injection, the agents of the invention may be formulated in aqueous solutions, preferably in physiologically compatible buffers such as Hanks's solution, Ringer's solution, or physiological saline buffer. For such transmucosal administration, penetrants appropriate to the barrier to be permeated are used in the formulation. Such penetrants are generally known in the art.

The invention being thus described, practice of the invention is illustrated by the experimental examples provided below. The skilled practitioner will realize that the materials and methods used in the illustrative examples can be modified in various ways. Such modifications are considered to fall within the scope of the present invention.

## EXAMPLES

EXAMPLE 1

*RNA Isolation from FPE Tissue*

RNA is extracted from paraffin-embedded tissue by the following general procedure.

**A. Deparaffinization and hydration of sections:**

(1) A portion of an approximately 10 μM section is placed in a 1.5 mL plastic centrifuge tube.
(2) 600 μL, of xylene are added and the mixture is shaken vigorously for about 10 minutes at room temperature (roughly 20 to 25 °C).
(3) The sample is centrifuged for about 7 minutes at room temperature at the maximum speed of the bench top centrifuge (about 10-20,000 x g).
(4) Steps 2 and 3 are repeated until the majority of paraffin has been dissolved. Two or more times are normally required depending on the amount of paraffin included in the original sample portion.
(5) The xylene solution is removed by vigorously shaking with a lower alcohol, preferably with 100% ethanol (about 600 μL) for about 3 minutes.
(6) The tube is centrifuged for about 7 minutes as in step (3). The supernatant is decanted and discarded. The pellet becomes white.
(7) Steps 5 and 6 are repeated with successively more dilute ethanol solutions: first with about 95% ethanol, then with about 80% and finally with about 70% ethanol.
(8) The sample is centrifuged for 7 minutes at room temperature as in step.
(9) The supernatant is discarded and the pellet is allowed to dry at room temperature for about 5 minutes.

**B. RNA Isolation with Phenol-Chloroform**

(1) 400 μL guanidine isothiocyanate solution including 0.5% sarcosine and 8 μL dithiothreitol is added.
(2) The sample is then homogenized with a tissue homogenizer (Ultra-Turrax, IKA-Works, Inc., Wilmington, NC) for about 2 to 3 minutes while gradually increasing the speed from low speed (speed 1) to high speed (speed 5).
(3) The sample is then heated at about 95 °C for about 5-20 minutes. It is preferable to pierce the cap of the tube containing the sample with a fine gauge needle before heating to 95 °C. Alternatively, the cap may be affixed with a plastic clamp or with laboratory film.
(4) The sample is then extracted with 50 μL 2M sodium acetate at pH 4.0 and 600 μL, of phenol/chloroform/isoamyl alcohol (10:1.93:0.036), prepared fresh by mixing 18 mL phenol with 3.6 mL of a 1:49 isoamyl alcohol:chloroform solution. The solution is shaken vigorously for about 10 seconds then cooled on ice for about 15 minutes.
(5) The solution is centrifuged for about 7 minutes at maximum speed. The upper (aqueous) phase is transferred to a new tube.
(6) The RNA is precipitated with about 10 μL glycogen and with 400 μL isopropanol for 30 minutes at -20°C.
(7) The RNA is pelleted by centrifugation for about 7 minutes in a benchtop centrifuge at maximum speed; the supernatant is decanted and discarded; and the pellet washed with approximately 500 μL of about 70 to 75% ethanol.
(8) The sample is centrifuged again for 7 minutes at maximum speed. The supernatant is decanted and the pellet air dried. The pellet is then dissolved in an appropriate buffer for further experiments (e.g., 50 pL. 5mM Tris chloride, pH 8.0).

EXAMPLE 2

*mRNA Reverse Transcription and PCR*

**Reverse Transcription:** RNA was isolated from microdissected or non-microdissected formalin fixed paraffin embedded (FPE) tissue as illustrated in Example I, or from fresh or frozen tissue by a single step guanidinium isocyanate method using the QuickPrep™ *Micro* mRNA purification kit (Amersham Pharmacia Biotech Inc., Piscataway, N.J.) according to the manufacturer's instructions. After precipitation with ethanol and centrifugation, the RNA pellet was dissolved in 50 ul of 5 mM Tris/Cl at pH 8.0. M-MLV Reverse Transcriptase will extend an oligonucleotide primer hybridized to a single-stranded RNA or DNA template in the presence of deoxynucleotides, producing a complementary strand. The resulting RNA was reverse transcribed with random hexamers and M-MLV Reverse Transcriptase from Life Technologies. The reverse transcription was accomplished by mixing 25 μl of the RNA solution with 25.5 μl of "reverse transcription mix" (see below). The reaction was placed in a thermocycler for 8 min at 26°C (for binding the random hexamers to RNA), 45 min at 42°C (for the M-MLV reverse transcription enzymatic reaction) and 5 min at 95°C (for heat inactivation of DNAse).

"Reverse transcription mix" consists of 10 ul 5X buffer (250 mM Tris-HCl, pH 8.3, 3 75 mM KCl, 15 mM MgCl2), 0.5 ul random hexamers (50 O.D. dissolved in 550 ul of 10 mM Tris-HCl pH 7.5) 5 ul 10 mM dNTPs (dATP, dGTP, dCTP and dTTP), 5 ul 0.1 M DTT, 1.25 ul BSA (3mg/ml in 10 mM Tris-HCL, pH 7.5), 1.25 ul RNA Guard 24,800U/ml (RNAse inhibitor) (Porcine #27-0816, Amersham Pharmacia) and 2.5 ul MMLV 200U/ul (Life Tech Cat #28025-02).

Final concentrations of reaction components are: 50 mM Tris-HCl, pH 8.3, 75 mM KCl, 3 mM MgCl2, 1.0 mM dNTP, 1.0 mM DTT, 0.00375. mg/ml BSA, 0.62 U/ul RNA Guard and 10 U/μl MMLV.

**PCR Quantification of mRNA expression.** Quantification of *EGFR* cDNA and an internal control or house keeping gene (e.g., β-actin) cDNA was done using a fluorescence based real-time detection method (ABI PRISM 7700 or 7900. Sequence Detection System [TaqMan®], Applied Biosystems, Foster City, CA.) as described by Heid et al., (Genome Res 1996;6:986-994); Gibson et al., (Genome Res 1996;6:995-1001). In brief, this method uses a dual labelled fluorogenic Taqman® oligonucleotide probe, (EGFR-1773 (SEQ ID NO: 3), $T_m$ = 70° C; HER2-neu 2657 (SEQ ID NO: 6), β-actin-611 (SEQ ID NO: 7) that anneals specifically within the forward and reverse primers. Laser stimulation within the capped wells containing the reaction mixture causes emission of a 3'quencher dye (TAMRA) until the probe is cleaved by the 5' to 3'nuclease activity of the DNA polymerase during PCR extension, causing release of a 5' reporter dye (6FAM). Production of an amplicon thus causes emission of a fluorescent signal that is detected by the TaqMan®'s CCD (charge-coupled device) detection camera, and the amount of signal produced at a threshold cycle within the purely exponential phase of the PCR reaction reflects the starting copy number of the sequence of interest Comparison of the starting copy number of the sequence of interest with the starting copy number of the internal control gene provides a relative gene expression level. TaqMan® analyses yield levels that are expressed as ratios between two absolute measurements (gene of interest/internal control gene).

The PCR reaction mixture consisted 0.5 μl of the reverse transcription reaction containing the cDNA prepared as described above 600 nM of each oligonucleoride primers EGFR-1753F (SEQ ID NO:1, $T_m$ = 59° C) and EGFR-1823R (SEQ ID NO: 2, $T_m$ = 58° C) or oligonucleotide primers HER2-neu 2671F (SEQ ID NO:4) and HER2-neu 2699R (SEQ ID NO: 5) 200 nM TaqMan® probe (SEQ ID NO:3 or SEQ ID NO: 6), 5 U AmpliTaq Gold Polymerase, 200 μM each dATP, dCTP, dGTP, 400 μM dTTP, 5.5 mM MgCl2, and 1 x Taqman Buffer A containing a reference dye, to a final volume of less than or equal to 25 μl (all reagents Applied Biosystems, Foster City, CA). Cycling conditions were, 95 °C for 10 min, followed by 45 cycles at 95 °C for 15s and 60 °C for I min. Oligonucleotides used to quantity internal control gene (β-Actin were (β-Actin-592F (SEQ ID NO: 8) and β-Actin-651R (SEQ ID NO: 9).

EXAMPLE 3

*Determining the Uncorrected Gene Expression (UGE) for EGFR*

Two pairs of parallel reactions are carried out. The "test" reactions and the "calibration" reactions. Figure 7. The *EGFR* amplification reaction and the (β-actin internal control amplification reaction are the test reactions. Separate *EGFR* and (β-actin amplification reactions are performed on the calibrator RNA template and are referred to as the calibration reactions. The TaqMan® instrument will yield four different cycle threshold (Ct) values: $Ct_{EGFR}$ and $Ct_{\beta\text{-actin}}$ from the test reactions and $Ct_{EGFR}$ and $Ct_{\beta\text{-actin}}$ from the calibration reactions. The differences in Ct values for the two reactions are determined according to the following equation:

$$\Delta Ct_{test} = Ct_{EGFR} - Ct_{\beta\text{-actin}} \qquad \text{(From the "test" reaction)}$$

$$\Delta Ct_{calibrator} = Ct_{EGFR} - Ct_{\beta\text{-actin}} \qquad \text{(From the "calibration" reaction)}$$

Next the step involves raising the number 2 to the negative ΔCt, according to the following equations.

$$2^{-\Delta Ct}{}_{test} \quad \text{(From the "test" reaction)}$$

$$2^{-\Delta Ct}{}_{calibrator} \quad \text{(From the "calibration" reaction)}$$

In order to then obtain an uncorrected gene expression for *EGFR* from the Tasman® instrument the following calculation is carried out:

$$\text{Uncorrected gene expression (UGE) for } EGFR = 2^{-\Delta Ct}{}_{test} \; / \; 2^{-\Delta Ct}{}_{calibrator}$$

*Normalizing UGE with known relative EGFR expression levels*

The normalization calculation entails a multiplication of the UGE with a correction factor ($K_{EGFR}$) specific to *EGFR* and a particular calibrator RNA. A correction factor $K_{EGFR}$ can also be determined for any internal control gene and any accurately pre-quantified calibrator RNA. Preferably, the internal control gene β-actin and the accurately pre-quantified calibrator RNA,Human Liver Total RNA (Stratagene, Cat #735017), are used. Given these reagents correction factor $K_{EGFR}$ equals 1.54.

Normalization is accomplished using a modification of the ΔCt method described by Applied Biosystems, the TaqMan® manufacturer, in User Bulletin #2 and described above. To carry out this procedure, the UGE of 6 different FPE test tissues were analyzed for *EGFR* expression using the TaqMan® methodology described above. The internal control gene β-actin and the calibrator RNA,Human Liver Total RNA (Stratagene, Cat #735017) was used.

The already known relative *EGFR* expression level of each sample AG221, AG222, AG252, Adult Lung, PC3, AdCol was divided by its corresponding TaqMan® derived UGE to yield an unaveraged correction factor K.

$$K_{unaveraged} = \text{Known Values / UGE}$$

Next, all of the K values are averaged to determine a single $K_{EGFR}$ correction factor specific for *EGFR*, Stratgene Human Liver Total RNA (Stratagene, Cat #735017) from calibrator RNA and β-actin.

Therefore, to determine the Corrected Relative *EGFR* Expression in an unknown tissue sample on a scale that is consistent with pre-TaqMan® *EGF*R expression studies, one merely multiplies the uncorrected gene expression data (UGE) derived from the TaqMan® apparatus with the $K_{EGFR}$ specific correction factor, given the use of the same internal control gene and calibrator RNA.

$$\text{Corrected Relative } EGFR \text{ Expression} = \text{UGE x } K_{EGFR}$$

A $K_{EGFR}$ may be determined using any accurately pre-quantified calibrator RNA or internal control gene. Future sources of accurately pre-quantified RNA can be calibrated to samples with known relative *EGFR* expression levels as described in the method above or may now be calibrated against a previously calibrated calibrator RNA such as Human Liver Total RNA (Stratagene, Cat #735017) described above.

For example, if a subsequent $K_{EGFR}$ is determined for a different internal control gene and/or a different calibrator RNA, one must calibrate both the internal control gene and the calibrator RNA to tissue samples for which *EGFR* expression levels relative to that particular internal control gene have already been determined. Such a determination can be made using standard pre-TaqMan®, quantitative RT-PCR techniques well known in the art. The known expression levels for these samples will be divided by their corresponding UGE levels to determine a K for that sample. K values are then averaged depending on the number of known samples to determine a new $K_{EGFR}$ specific to the different internal control gene and/or calibrator RNA.

EXAMPLE 4

*Determining the Uncorrected Gene Expression (UGE) for HER2-neu*

Two pairs of parallel reactions are carried out. The "test" reactions and the "calibration" reactions. Figure 8. The *HER2-neu* amplification reaction and the β-actin internal control amplification reaction are the test reactions. Separate *HER2-neu* and (β-actin amplification reactions are performed on the calibrator RNA template and are referred to as the calibration reactions. The TaqMan® instrument will yield four different cycle threshold (Ct) values: $Ct_{HER2\text{-}neu}$ and $Ct_{\beta\text{-}actin}$ from the test reactions and $Ct_{Her2\text{-}neu}$ and $Ct_{\beta\text{-}actin}$ from the calibration reactions. The differences in Ct values for the two reactions are determined according to the following equation:

$$\Delta Ct_{test} = Ct_{HER2\text{-}neu} - Ct_{\beta\text{-}actin} \qquad \text{(From the "test" reaction)}$$

$$\Delta Ct_{calibrator} = Ct_{Her2\text{-}neu} - Ct_{\beta\text{-}actin} \qquad \text{(From the "calibration" reaction)}$$

Next the step involves raising the number 2 to the negative ΔCt, according to the following equations.

$$2^{-\Delta Ct_{test}} \qquad \text{(From the "test" reaction)}$$

$$2^{-\Delta Ct_{calibrator}} \qquad \text{(From the "calibration" reaction)}$$

In order to then obtain an uncorrected gene expression for *HER2-neu* from the TaqMan® instrument the following calculation is carried out:

$$\text{Uncorrected gene expression (UGE) for } HER2\text{-}neu = 2^{-\Delta Ct_{test}} / 2^{-\Delta Ct_{calibrator}}$$

*Normalizing UGE with known relative HER2-neu expression levels*

The normalization calculation entails a multiplication of the UGE with a correction factor ($K_{HER2\text{-}neu}$) specific to *HER2-neu* and a particular calibrator RNA. A correction factor $K_{HER2\text{-}neu}$ can also be determined for any internal control gene and any accurately pre-quantified calibrator RNA. Preferably, the internal control gene (β-actin and the accurately pre-quantified calibrator RNA,Human Liver Total RNA (Stratagene, Cat #735017) are used. Using β-actin and the accurately pre-quantified calibrator RNA,Human Liver Total RNA (Stratagene, Cat #735017) the correction factor $K_{HER2\text{-}neu}$ equals $12.6 \times 10^{-3}$.

Normalization is accomplished using a modification of the ΔCt method described by Applied Biosystems, the TaqMan® manufacturer, in User Bulletin #2 and described above. To carry out this procedure, the UGE of 6 different FPE test tissues were analyzed for *HER2-neu* expression using the TaqMan® methodology described above. The internal control gene β-actin and the calibrator RNA,Human Liver Total RNA (Stratagene, Cat #735017) was used.

The already known relative *HER2-neu* expression level of each sample AG221, AG222, AG252, Adult Lung, PC3, AdCol is divided by its corresponding TaqMan® derived UGE to yield an unaveraged correction factor K.

$$K_{unaveraged} = \text{Known Values / UGE}$$

Next, all of the K values are averaged to determine a single $K_{EGFR}$ correction factor specific for *HER2-neu*, Human Liver Total RNA (Stratagene, Cat #735017) calibrator, and β-actin.

Therefore, to determine the Corrected Relative *HER2-neu* Expression in an unknown tissue sample on a scale that is consistent with pre-TaqMan® *Her2-neu* expression studies, one merely multiplies the uncorrected gene expression data (UGE) derived from the TaqMan® apparatus with the $K_{HER2-neu}$ specific correction factor, given the use of the same internal control gene and calibrator RNA.

$$\text{Corrected Relative } \textit{HER2-neu} \text{ Expression} = \text{UGE} \times K_{HER2-neu}$$

A $K_{HER2-neu}$ may be determined using any accurately pre-quantified calibrator RNA or internal control gene. Future sources of accurately pre-quantified RNA can be calibrated to samples with known relative *EGFR* expression levels as described in the method above or may now be calibrated against a previously calibrated calibrator RNA such as Human Liver Total RNA (Stratagene, Cat #735017) described above.

For example, if a subsequent $K_{HER2-neu}$ is determined for a different internal control gene and/or a different calibrator RNA, one should calibrate both the internal control gene and the calibrator RNA to tissue samples for which *HER2-neu* expression levels relative to that particular internal control gene have already been determined or published. Such a determination can be made using standard pre-TaqMan®, quantitative RT-PCR techniques well known in the art. The known expression levels for these samples will be divided by their corresponding UGE levels to determine a K for that sample. K values are then averaged depending on the number of known samples to determine a new $K_{HER2-neu}$ specific to the different internal control gene and/or calibrator RNA.

<u>EXAMPLE 5</u>

*Patient Population and Tissue Acquisition*

**Patients.** Eighty-three patients suffering from NSCLC consisting of sixtyfive (78.3%) men and 18 (21.7%) women, with a median age of 63.5 years (range, 34-82) were studied. Thirty-nine (47%) patients had squamous cell carcinomas, 32 (38.6%) had adenocarcinoma, and 12 (14.5%) had large cell carcinomas. The primary tumors were graded histopathologically as well-differentiated (G1, one patient), moderately-differentiated (G2, 18 patients), and poorly-differentiated (G3, 64 patients). Tumor staging was performed according to the International Union Against Cancer (UICC) TNM classification: Forty-one (49.4%) had stage I tumors, 16 (19.3%) had stage II tumors, and 26 (31.3%) had stage IIIa tumors. All tumors were completely resected (R0 category), by at least a lobectomy as quality control. Patients with histopathological stage IIIa tumors received postoperative radiotherapy. The median follow-up was 85.9 months (min. 63.3; mar. 105.2 months) and no patient was lost to follow-up.

**Tissue Acquisition.** Tissue for gene expression analysis was obtained immediately after lung resection before starting mediastinal lymphadenectomy and was immediately frozen in liquid nitrogen. Tissues were analyzed from the following 2 locations: tumor and uninvolved lung tissue taken from the greatest distance to the tumor. 6 μm frozen sections were taken from blocks of tumor tissue and starting with the first section every fifth was routinely stained with HE and histopathologically evaluated. Sections were pooled for analysis from areas of estimated 75% malignant cells. RNA was isolated from tissue samples according to the methods in Example 2.

<u>EXAMPLE 6</u>

*Statistical Analysis*

Tasman® analyses yield values that are expressed as UGE. The ratio between UGE in tumor tissue and UGE in matching non-malignant lung tissue was used to determine differential gene expression. Associations between the two UGE variables were tested by using Wilcox on signed rank test The Chi-Square test was used to analyze the associations between categorial clinicopathological variables. Hazards ratios were used to calculate the relative risks of death. These calculations were based on the Pike estimate, with the use of the observed and expected number of events as calculated in the log-rank test statistic. Pike, J R Stat Soc Series A 135:201-203; 1972. The maximal chi-square method of Miller and Sigmund (Miller et al., Biometrics 38:1011-1016, 1982) and Halpern (Biometrics 38: 1017-1023, 1982) was adapted to determine which expression value best segregated patients into poor- and good prognosis subgroups (in terms of likelihood of surviving), with the log-rank test as the statistics used to measure the strength of the grouping. To determine a P value that would be interpreted as a measure of the strength of the association based on the maximal chi-square analysis, 1000 boot-strap-like simulations were used to estimate the distribution of the maximal chi-square statistics under the hypothesis of no association. Halpern, Biometrics 38:1017-1023, 1982. Cox's proportional hazards modeling of factors that were significant in anivariate analysis was performed to identify which factors might have a significant influence on survival. The level of significance was set to $p < 0.05$.

*HER2-neu* mRNA expression was detectable by quantitative real-time RT-PCR in 83 of 83 (100%) normal lung and 83 of 83 (100%) tumor samples. The corrected *HER2-neu* mRNA expression, expressed as the ratio between *HER2-neu* and β-Actin PCR product, was 4.17 x $10^{-3}$ (range 0.28-23.86 x $10^{-3}$) in normal lung and 4.35 x $10^{-3}$ (range: 0.21-68.11 x $10^{-3}$) in tumor tissue (*P*=0.019 Wilcoxon test). The maximal chi-square method by Miller and Siegmund (Miller et al., Biometrics 38:1011-1016, 1982) and Halpern (Biometrics 38:1017-1023, 1982) determined a threshold value of 1.8 to segregate patients into low and high differential *HER2-neu* expressors. By this criterion, 29 (34.9%) patients had a high differential *HER2-neu* expression and 54 (65.1 %) had a low differential *HER2-neu* expression. Figure 4 shows associations between clinicopathological data and differential *HER2-neu* gene expression status. There were no statistically significant differences detectable. Figure 1 displays a Kaplan Meier plot of the estimated probability of survival versus the differential *HER2-neu* mRNA expression status. The median survival was not reached in the low differential *HER2-neu* expression group compared to 31.1 months (95% C.I.: 21.96- 40.24) in the high differential *HER2-neu* expression group. To determine a P value, bootstrap-like simulations were used to estimate the distribution of a maximal chi-square statistic, since the threshold value of 1.8 had been chosen after examining the data. The resulting adjusted *P* value was .004 (Log-rank test).

The accuracy of *HER2-neu* as a prognostic factor was next determined by the Cox's proportional hazards model analysis. In univariate analysis of potential prognostic factors, high differential *HER2-neu* expression as well as advanced pT (tumor stage) classification, pN (lymph node stage) classification, and tumor stage were significant unfavorable prognostic factors (Figure 5). In a multivariate analysis of prognostic factors (Figure 6), high differential *HER2-neu* expression was a significant and independent unfavorable prognostic factor, as well as advanced pN classification and tumor stage.

*EGFR* mRNA expression was detectable by quantitative real-time RT-PCR in 83 of 83 (100%) normal lung and 83 of 83 (100%) tumor samples. The median corrected *EGFR* mRNA expression was 8.17 x $10^{-3}$(range: 0.31-46.26 x $10^{-3}$ in normal lung and 7.22 x $10^{-3}$ (range: 0.27-97.49 x $10^{-3}$) in tumor tissue (*P*=n.s.). The maximal chi-square method (Miller (1982); Halpern (1982)) determined a threshold value of 1.8 to segregate patients into low and high differential *EGFR* expressors. By this criterion, 28 (33.7%) patients had a high differential *EGFR* expression and 55 (66.3%) had a low differential *EGFR* expression status. There were no statistical significant differences between clinicopathological variables and differential *EGFR* mRNA expression status detectable (Figure 4). A trend towards inferior overall survival was observable for the high differential *EGFR* expression group, but did not reach statistical significance (Figure 2). The median survival was not reached in the low differential *EGFR* expression group compared to 32.37 months (95% C.I.: 8.43-56.31) in the high differential *EGFR* expressor group (*P*=0.176).

High expression levels (above 1.8) of differential *HER2-neu* and *EGFR* were found in 14 of 83 (16.9%) patients. Forty of 83 (48.2%) patients showed a low differential expression status (below 1.8) for *HER2-neu* and *EGFR,* whereas 14 of 83 (16.9%) showed a high differential expression for EGFR only, and 15 of 83 (18.1%) patients displayed a high differential expression for *HER2-neu.* The median survival was not reached in the group that showed low differential *HER2-neu* and *EGFR* expression, compared to 45.47 months in the high differential *EGFR* expression group, 31.10 months (95% C.I.: 14.77-47.43) in the high differential *HER2-neu* expression group, and 22.03 months (95% C.I: 2.30; 41.76; *P* =0.003; log-rank test; Figures 3 and 5) in the high differential *HER2-neu* and *EGFR* expression group. Univariate analysis displayed high differential *HER2-neu* and *EGFR* coexpression as a significant unfavorable prognostic factor (Figure 5). In a multivariate analysis of prognostic factors (Figure 6), high differential *HER2-neu* and high differential *EGFR* coexpression was a significant and independent unfavorable prognostic factor, as was advanced pN classification and tumor stage.

EXAMPLE 7

*Tumor response to a receptor tyrosine kinase targeted chemotherapy*

Five colon cancer patients' tumors were initially identified as expressing EGFR by immunohistochemistry. Patients were treated with Imclone IMC-C225, 400 mg/$m^2$ loading dose followed by 250 mg/$m^2$ weekly, plus CPT-11 at the same dose and schedule that the patient had previously progressed on. Previous CPT-11 dose attenuations were maintained.

Using the methodology described in Examples 1-4, Patient 1 was determined to have a corrected EGFR expression level of 2.08 x $10^{-3}$ and had a completed response (CR) to a a receptor tyrosine kinase targeted chemotherapy comprising CPT-11 (7-ethyl-10-[4-(1-piperidino)-1-piperidino] carboxycamptothecin) / C225 (an anti- EGFR monoclonal antibody effective in anti-cancer therapy; Mendelsohn, Endocr Relat Cancer 2001 Mar;8(1):3-9). Patient 2 had a corrected EGFR expression level of 8.04 x $10^{-3}$ and had a partial response (PR) to the receptor tyrosine kinase targeted chemotherapy. Patient 3 had a corrected EGFR expression level of 1.47 x $10^{-3}$ and also showed a partial response (PR) to the receptor tyrosine kinase targeted chemotherapy. Patient 4 had a corrected EGFR expression level of 0.16 x x $10^{-3}$ and had stable disease (SD) showing no response to the receptor tyrosine kinase targeted chemotherapy. Patient 5 had a no EGFR expression (0.0 x $10^{-3}$) and had progressive disease (PR) showing no response to the receptor tyrosine kinase targeted chemotherapy. See figure 9.

## Claims

1. A method for determining the level of *EGFR* expression in a fixed paraffin-embedded tissue sample, wherein said method can be used for determining a chemotherapeutic regimen comprising:

   (a) deparaffinizing the tissue sample to obtain a deparaffinized sample;
   (b) isolating mRNA from the deparaffinized sample by heating in the presence of an effective amount of a chaotropic agent to a temperature in the range of 75 to about 100°C for a time period of about 5 to about 120 minutes and recovering said mRNA from the chaotropic solution;
   (c) subjecting the mRNA to amplification using a pair of oligonucleotide primers consisting of an oligonucleotide primer having the sequence of SEQ ID NO: or a sequence at least 80% identical thereto, and an oligonucleotide primer having the sequence of SEQ ID NO:2 or a sequence at least 80% identical thereto to obtain an amplified sample; and
   (d) determining the quantity of *EGFR* mRNA relative to the quantity of mRNA of an internal control gene.

2. The method of claim 1, wherein the pair of oligonucleotide primers consists of an oligonucleotide having the sequence of SEQ ID NO:1 and an oligonucleotide primer having the sequence of SEQ ID NO:2.

3. A method for determining the level of *HER2-neu* expression in a fixed paraffin-embedded tissue sample, wherein said method can be used for determining a chemotherapeutic regimen comprising:

   (a) deparaffinizing the tissue sample to obtain a deparaffinized sample;
   (b) isolating mRNA from the deparaffinized sample by heating in the presence of an effective amount of a chaotropic agent to a temperature in the range of 75 to about 100°C for a time period of about 5 to about 120 minutes and recovering said mRNA from the chaotropic solution;
(c) subjecting the mRNA to amplification using a pair of oligonucleotide primers that hybridize under stringent conditions to a region of the *HER2-neu* gene consisting of an oligonucleotide primer having the sequence of SEQ ID NO:4 or a sequence at least 80% identical thereto, and an oligonucteotide primer having the sequence of SEQ ID NO:5 or a sequence at least 80% identical thereto to obtain an amplified sample; and
(d) determining the quantity of *HER2-neu* mRNA relative to the quantity of mRNA of an internal control gene.

**4.** The method of claim 3, wherein the pair of oligonucleotide primers consists of an oligonucleotide having the sequence of SEQ ID NO:4 and an oligonucleotide primer having the sequence of SEQ ID No:5.

**5.** The method of claim 1 or 3, wherein the internal control gene is a (β-actin gene.

### Patentansprüche

**1.** Verfahren zur Bestimmung des Niveaus der *EGFR*-Expression in einer fixierten, in Paraffin eingebetteten Gewebeprobe, wobei das Verfahren zur Bestimmung eines Chemotherapieregimes verwendet werden kann und Folgendes umfasst:

(a) Entparaffinieren der Gewebeprobe, um eine entparaffinierte Probe zu erhalten;
(b) Isolieren von mRNA aus der entparaffinierten Probe durch Erhitzen auf eine Temperatur im Bereich von 75 bis etwa 100 °C für einen Zeitraum von etwa 5 bis etwa 120 Minuten in Gegenwart einer wirksamen Menge einer chaotropen Substanz und Gewinnen der mRNA aus der chaotropen Lösung;
(c) Unterziehen der mRNA einer Amplifikation unter Verwendung eines Paars von Oligonukleotidprimern, das aus einem Oligonukleotidprimer mit der Sequenz von SEQ ID NO: 1 oder einer Sequenz, die zu dieser zu mindestens 80 % identisch ist, und einem Oligonukleotidprimer mit der Sequenz von SEQ ID NO: 2 oder einer Sequenz, die zu dieser zu mindestens 80 % identisch ist, besteht, um eine amplifizierte Probe zu erhalten; und
(d) Bestimmen der Menge von *EGFR*-mRNA in Bezug auf die Menge von mRNA eines internen Kontrollgens.

**2.** Verfahren nach Anspruch 1, wobei das Paar von Oligonukleotidprimern aus einem Oligonukleotid mit der Sequenz von SEQ ID NO: 1 und einem Oligonukleotidprimer mit der Sequenz von SEQ ID NO: 2 besteht.

**3.** Verfahren zur Bestimmung des Niveaus der *HER2-neu*-Expression in einer fixierten, in Paraffin eingebetteten Gewebeprobe, wobei das Verfahren zur Bestimmung eines Chemotherapieregimes verwendet werden kann und Folgendes umfasst:

(a) Entparaffinieren der Gewebeprobe, um eine entparaffinierte Probe zu erhalten;
(b) Isolieren von mRNA aus der entparaffinierten Probe durch Erhitzen auf eine Temperatur im Bereich von 75 bis etwa 100 °C für einen Zeitraum von etwa 5 bis etwa 120 Minuten in Gegenwart einer wirksamen Menge einer chaotropen Substanz und Gewinnen der mRNA aus der chaotropen Lösung;
(c) Unterziehen der mRNA einer Amplifikation unter Verwendung eines Paars von Oligonukleotidprimern, die unter stringenten Bedingungen an eine Region des *HER2-neu*-Gens hybridisieren, wobei das Paar von Oligonukleotidprimern aus einem Oligonukleotidprimer mit der Sequenz von SEQ ID NO: 4 oder einer Sequenz, die zu dieser zu mindestens 80 % identisch ist, und einem Oligonukleotidprimer mit der Sequenz von SEQ ID NO: 5 oder einer Sequenz, die zu dieser zu mindestens 80 % identisch ist, besteht, um eine amplifizierte Probe zu erhalten; und
(d) Bestimmen der Menge von *HER2-neu*-mRNA in Bezug auf die Menge von mRNA eines internen Kontrollgens.

**4.** Verfahren nach Anspruch 3, wobei das Paar von Oligonukleotidprimern aus einem Oligonukleotid mit der Sequenz von SEQ ID NO: 4 und einem Oligonukleotidprimer mit der Sequenz von SEQ ID NO: 5 besteht.

**5.** Verfahren nach Anspruch 1 oder 3, wobei das interne Kontrollgen ein β-Actin-Gen ist.

### Revendications

**1.** Procédé de détermination du niveau d'expression de l'EGFR dans un échantillon de tissu fixé, noyé dans la paraffine, ledit procédé pouvant être utilisé pour déterminer un schéma posologique de chimiothérapie comprenant :

(a) déparaffiner l'échantillon de tissu pour obtenir un échantillon déparaffiné ;
(b) isoler de l'ARNm à partir de l'échantillon déparaffiné par chauffage en présence d'une quantité efficace d'un agent chaotropique à une température se situant dans la plage de 75 à environ 100°C pendant une période de temps d'environ 5 à environ 120 minutes et récupérer ledit ARNm à partir de la solution chaotropique ;
(c) soumettre l'ARNm à une amplification à l'aide d'une paire d'amorces oligonucléotidiques consistant en une amorce oligonucléotidique ayant la séquence de SEQ ID NO:1 ou une séquence au moins 80% identique à celle-ci, et une amorce oligonucléotidique ayant la séquence de SEQ ID NO:2 ou une séquence au moins 80% identique à celle-ci pour obtenir un échantillon amplifié ; et
(d) déterminer la quantité d'ARNm d'EGFR par rapport à la quantité d'ARNm d'un gène témoin interne.

**2.** Procédé selon la revendication 1, dans lequel la paire d'amorces oligonucléotidiques consiste en un oligonucléotide ayant la séquence de SEQ ID NO:1 et une amorce oligonucléotidique ayant la séquence de SEQ ID NO:2.

**3.** Procédé de détermination du niveau d'expression de HER2-*neu* dans un échantillon de tissu fixé, noyé dans la paraffine, ledit procédé pouvant être utilisé pour déterminer un schéma posologique de chimiothérapie, comprenant :

(a) déparaffiner l'échantillon de tissu pour obtenir un échantillon déparaffiné ;
(b) isoler de l'ARNm à partir de l'échantillon déparaffiné par chauffage en présence d'une quantité efficace d'un agent chaotropique à une température se situant dans la plage de 75 à environ 100°C pendant une période de temps d'environ 5 à environ 120 minutes et récupérer ledit ARNm à partir de la solution chaotropique ;
(c) soumettre l'ARNm à une amplification à l'aide d'une paire d'amorces oligonucléotidiques qui s'hybrident dans des conditions stringentes à une région du gène HER2-*neu* consistant en une amorce oligonucléotidique ayant la séquence de SEQ ID NO:4 ou une séquence au moins 80 % identique à celle-ci, et une amorce oligonucléotidique ayant la séquence de SEQ ID NO:5 ou une séquence au moins 80 % identique à celle-ci pour obtenir un échantillon amplifié ; et
(d) déterminer la quantité d'ARNm d'HER2-*neu* par rapport à la quantité d'ARNm d'un gène témoin interne.

**4.** Procédé selon la revendication 3, dans lequel la paire d'amorces oligonucléotidiques consiste en un oligonucléotide ayant la séquence de SEQ ID NO:4 et une amorce oligonucléotidique ayant la séquence de SEQ ID NO:5.

**5.** Procédé selon l'une des revendications 1 ou 3, dans lequel le gène témoin interne est un gène de la β-actine,

Figure 1

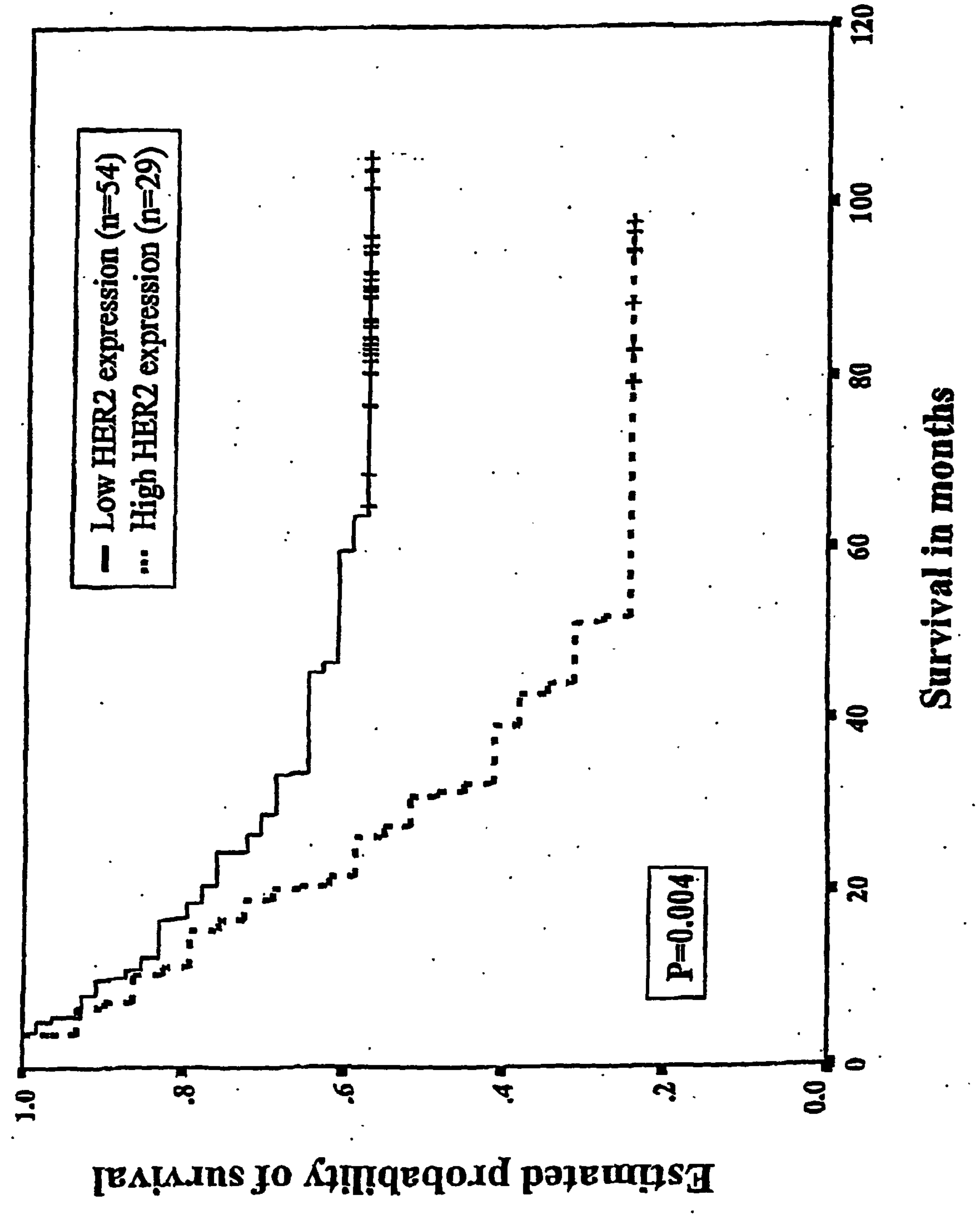
Low HER2 expression (n=54)
High HER2 expression (n=29)
P=0.004
Estimated probability of survival
1.0
.8
.6
.4
.2
0.0
Survival in months
0
20
40
60
80
100
120

Figure 2

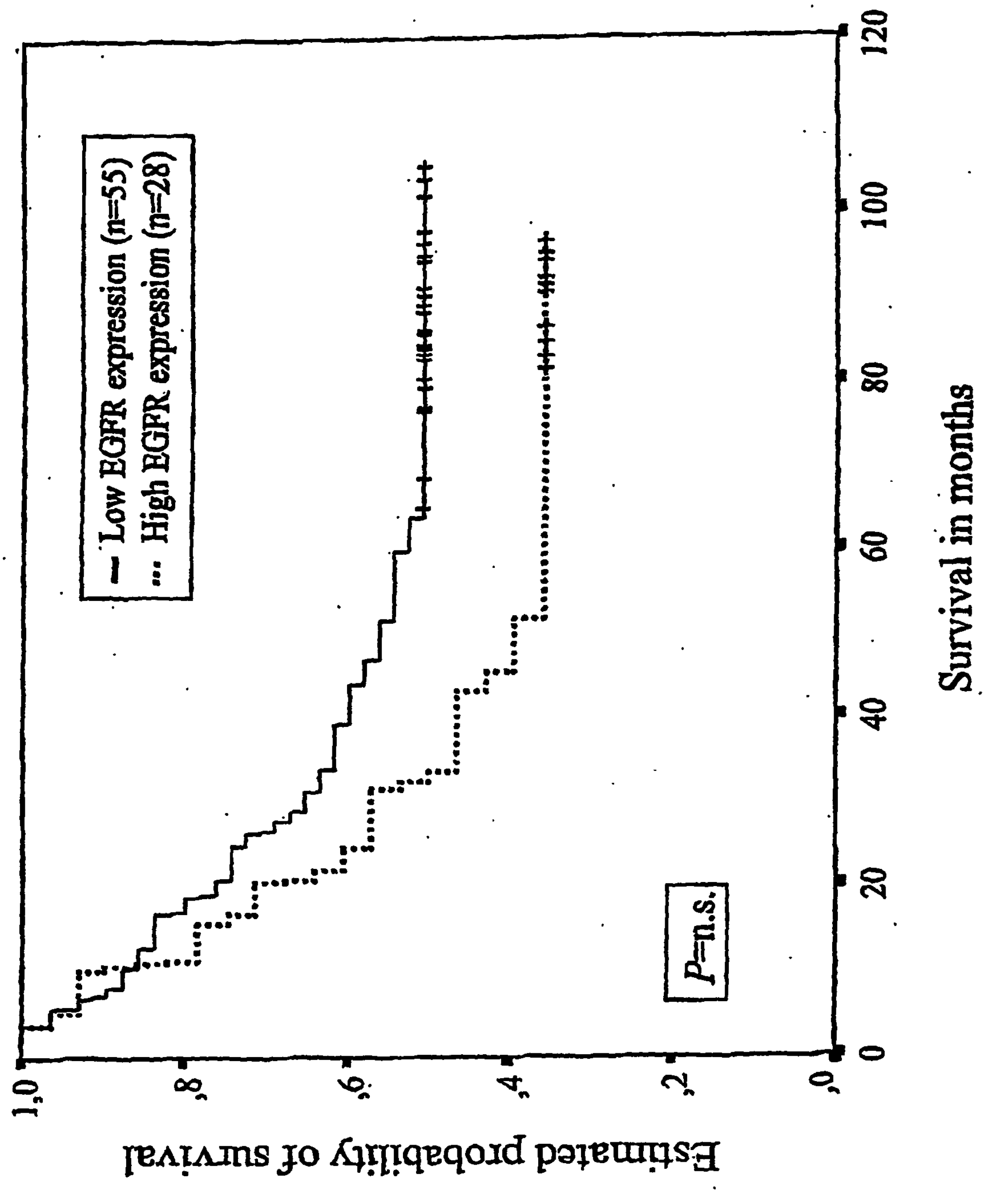
Low EGFR expression (n=55)
High EGFR expression (n=28)
P=n.s.
Estimated probability of survival
1,0
,8
,6
,4
,2
0,0
0
20
40
60
80
100
120
Survival in months

Figure 3

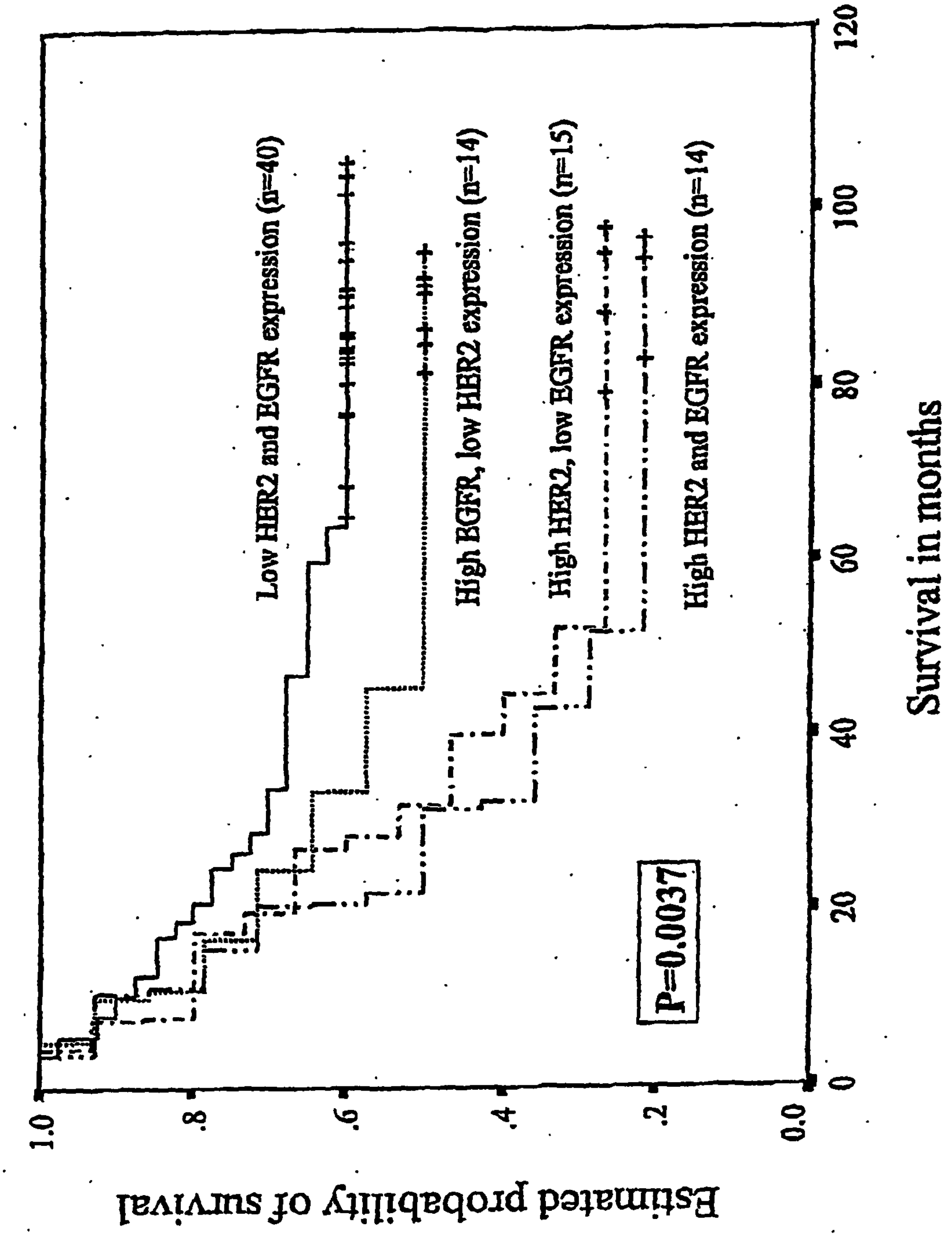

Estimated probability of survival
1.0
.8
.6
.4
.2
0.0
0
20
40
60
80
100
120
Survival in months
Low HER2 and EGFR expression (n=40)
High EGFR, low HER2 expression (n=14)
High HER2, low EGFR expression (n=15)
High HER2 and EGFR expression (n=14)
P=0.0037

Figure 4 Relationships with HER2 neu and EGFR mRNA expression

| Variable | EGFR low | EGFR high | EGFR P | HER2 neu low | HER2 neu high | HER2 neu P |
|---|---|---|---|---|---|---|
| : | 40 | 25 | | 45 | 20 | |
| ale | 15 | 3 | 0.84 | 20 | 9 | 0.13 |
| ng | | | | | | |
| ker | 48 | 25 | | 46 | 27 | |
| Smoker | 7 | 3 | 0.79 | 8 | 2 | 0.29 |
| gory | | | | | | |
| | 11 | 5 | | 12 | 4 | |
| | 35 | 19 | | 33 | 21 | |
| | 9 | 4 | 0.93 | 9 | 4 | 0.56 |
| egory | | | | | | |
| | 31 | 15 | | 32 | 14 | |
| pN1 | 15 | 7 | | 15 | 7 | |
| pN2 | 9 | 6 | 0.85 | 7 | 8 | 0.25 |
| UICC Stage | | | | | | |
| I | 26 | 15 | | 28 | 13 | |
| II | 13 | 3 | | 12 | 4 | |
| IIIa | 16 | 10 | 0.37 | 14 | 12 | 0.31 |
| Histology | | | | | | |
| Squamous Cell Carcinoma | 26 | 13 | | 28 | 11 | |
| Adenocarcinoma | 20 | 12 | | 16 | 16 | |
| Large Cell Carcinoma | 9 | 3 | 0.74 | 10 | 2 | 0.57 |
| Grading | | | | | | |
| Well differentiated | 1 | 0 | | 1 | 0 | |
| Moderately differentiated | 13 | 5 | | 13 | 5 | |
| Poorly differentiated | 41 | 23 | 0.63 | 40 | 24 | 0.57 |

Figure 5 Survival in NSCLC based on clinical and molecular parameters

| Parameter | | n | 5-year survival (%) ± SD | Median survival (months) | CI 95% | P value |
|---|---|---|---|---|---|---|
| ge | I | 41 | 68.2 ± 0.07 | n.r. | - | <0.0001 |
| | II | 16 | 43.8 ± 0.12 | 33.97 ± 5.70 | 22.80; 45.14 | |
| | IIIa | 26 | 11.5 ± 0.06 | 19.00 ± 5.14 | 8.92; 29.08 | |
| | $pT_1$ | 16 | 68.8 ± 0.12 | n.r. | - | 0.0157 |
| | $pT_2$ | 34 | 44.4 ± 0.07 | 46.77 ± 18.51 | 10.49; 83.05 | |
| | $pT_3$ | 26 | 23.1 ± 0.12 | 26.67 ± 6.09 | 14.73; 38.61 | |
| | $pN_0$ | 44 | 67.3 ± 0.07 | n.r. | - | <0.0001 |
| | $pN_1$ | 22 | 31.8 ± 0.10 | 33.71 ± 6.86 | 20.22; 47.12 | |
| | $pN_2$ | 15 | 0 | 16.70 ± 4.01 | 8.84; 24.56 | |
| a | Low | 54 | 57.4 ± 0.07 | n.r. | - | 0.0044 |
| | High | 29 | 24.1 ± 0.08 | 31.10 ± 4.66 | 21.96; 40.24 | |
| | Low | 55 | 50.8 ± 0.07 | n.r. | - | n.s. |
| | High | 28 | 35.7 ± 0.09 | 32.37 ± 12.22 | 8.43; 56.31 | |
| arker | Double low | 40 | 59.9 ± 0.08 | n.r. | - | 0.0037 |
| | EGFR high | 14 | 50.0 ± 0.13 | 45.47 | - | |
| | HER-2 high | 15 | 26.7 ± 0.11 | 31.10 ± 8.33 | 14.77; 47.73 | |
| | Double high | 14 | 21.4 ± 0.11 | 22.03 ± 10.07 | 2.30; 41.76 | |

Abbreviations: n.r. (not reached); - (cannot ne calculated); CI 95% (95% confidence intervall); n.s. (not significant); n (number of patients).

Figure 6. Cox-proportional hazard regression models

| Model | Parameter | Hazards ratio | CI 95% | *P* value |
|---|---|---|---|---|
| | Stage | | | 0.0001 |
| | I/IIIa | 0.219 | 0.11-0.44 | 0.0001 |
| | II/IIIa | 0.524 | 0.23-1.17 | 0.177 |
| | HER2-neu | 1.894 | 1.02-3.51 | 0.043 |
| | pT | | | 0.127 |
| | $pT_1/pT_3$ | 0.311 | 0.10-0.97 | 0.044 |
| | $pT_2/pT_3$ | 0.692 | 0.32-1.50 | 0.354 |
| | pN | | | 0.0001 |
| | $pN_0/pN_2$ | 0.143 | 0.07-0.31 | 0.0001 |
| | $pN_1/pN_2$ | 0.333 | 0.14-0.75 | 0.008 |
| | HER2-neu | 1.890 | 1.03-3.48 | 0.041 |
| | Stage | | | 0.0001 |
| | I/IIIa | 0.554 | 0.11-0.44 | 0.0001 |
| | II/IIIa | 0.554 | 0.24-1.26 | 0.159 |
| | Double marker | 1.331 | 1.03-1.73 | 0.03 |
| D | pT | | | 0.168 |
| | $pT_1/pT_3$ | 0.335 | 0.11-1.05 | 0.061 |
| | $pT_2/pT_3$ | 0.704 | 0.32-1.55 | 0.384 |
| | pN | | | 0.0001 |
| | $pN_0/pN_2$ | 0.143 | 0.07-0.31 | 0.0001 |
| | $pN_1/pN_2$ | 0.143 | 0.14-0.74 | 0.007 |
| | Double marker | 1.280 | 1.00-1.63 | 0.046 |

Abbreviations: CI 95% (confidence interval for hazards ratio); Parameter section: e.g. stage I/IIIa means stage I compared to stage IIIa.

Figure 7: Chart illustrating how to calculate *EGFR* expression relativ

| | | from "test" reactions | | | | from calibration reactions [illegible] | | | | Uncorrected Gene Expression (UGE) | Known *EGFR* K values | Derivation of $K_{EGFR}$ (average K) | | Relative *EGFR* exp |
|---|---|---|---|---|---|---|---|---|---|---|---|---|---|---|
| | Sample | $C_T$ *EGFR* | $C_T$ β-actin | $\Delta C_T$ | $2^{-\Delta Ct}$ | $C_T$ *EGFR* | $C_T$ β-actin | $\Delta C_T$ | $2^{-\Delta Ct}$ | $2^{-\Delta Ct}/2^{-\Delta Ct}$ | | K | $K_{EGFR}$ | |
| Experimental | unknown 1 | 32.7 | 26.8 | 5.9 | 0.0167 | - | - | - | - | 0.525 | - | | 26.95 x $10^{-3}$ | 14.4 x $10^{-3}$ |
| | unknown 2 | 32.88 | 26.43 | 6.45 | 0.0114 | - | - | - | - | 0.358 | - | | 26.95 x $10^{-3}$ | 9.66 x $10^{-3}$ |
| | Calib. RNA | - | - | - | - | 27.01 | 22.04 | 4.97 | 0.0319 | 0.0319/0.0319= 1 | | | | |
| | | | | | | | | | | | | | | |
| From Published Data | 60N | 31.61 | 23.86 | 7.76 | 0.00464 | - | - | - | - | 0.2117 | 5.70 x $10^{-3}$ | 26.95 x $10^{-3}$ | 26.95 x $10^{-3}$ | - |
| | 60T | 29.08 | 20.65 | 8.43 | 0.0029 | - | - | - | - | 0.1321 | 3.56 x $10^{-3}$ | 26.95 x $10^{-3}$ | 26.95 x $10^{-3}$ | - |
| | SF12A | 28.71 | 20.76 | 7.95 | 0.0040 | - | - | - | - | 0.184 | 4.97 x $10^{-3}$ | 26.95 x $10^{-3}$ | 26.95 x $10^{-3}$ | - |
| | SF12B | 24.69 | 19.87 | 4.82 | 0.0354 | - | - | - | - | 1.613 | 43.5 x $10^{-3}$ | 26.95 x $10^{-3}$ | 26.95 x $10^{-3}$ | - |
| | Clr11 | 24.03 | 16.3 | 7.73 | 0.0047 | - | - | - | - | 0.215 | 5.78 x $10^{-3}$ | 26.95 x $10^{-3}$ | 26.95 x $10^{-3}$ | - |
| | AdCol | 26.04 | 17.06 | 8.98 | 0.00198 | - | - | - | - | 0.090 | 2.43 x $10^{-3}$ | 26.95 x $10^{-3}$ | 26.95 x $10^{-3}$ | - |
| | Calib. RNA | - | - | - | - | 25.96 | 18.57 | 7.39 | 0.00596 | 0.00596/ 0.00596 = 1 | - | - | - | - |

Figure 8: Chart illustrating how to calculate *HER2-neu* expression relative to an internal control gene

| | | from "test" reactions | | | | from "calibration" reactions | | | | Uncorrected Gene Expression (UGE) | Known *HER-2/neu* values | Derivation of $K_{HER-2/neu}$ (average K) | | Relative *HER-2/neu* exp. |
|---|---|---|---|---|---|---|---|---|---|---|---|---|---|---|
| | Sample | $C_T$ *HER-2/neu* | $C_T$ β-actin | $\Delta C_T$ | $2^{-\Delta C_T}$ | $C_T$ *HER2-neu* | $C_T$ β-actin | $\Delta C_T$ | $2^{-\Delta C_T}$ | $2^{-\Delta C_T} / 2^{-\Delta C_T}$ | | K | $K_{HER-2/neu}$ | |
| Experimental | unknown 1 | 21.5 | 16.3 | 5.2 | 0.0272 | - | - | - | - | 1.43 | - | | $13.3 \times 10^{-3}$ | $19.1 \times 10^{-3}$ |
| | unknown 2 | 23.22 | 17.06 | 6.16 | 0.0139 | - | - | - | - | 0.74 | - | | $13.3 \times 10^{-3}$ | $9.8 \times 10^{-3}$ |
| | Calib. RNA | - | - | - | - | 24.29 | 18.57 | 5.72 | 0.0189 | 0.0189/0.0189= 1 | | | | |
| | | | | | | | | | | | | | | |
| From Published Data | 60N | 30.28 | 23.86 | 6.42 | 0.012 | - | - | - | - | 0.702 | $9.34 \times 10^{-3}$ | $13.3 \times 10^{-3}$ | $13.3 \times 10^{-3}$ | - |
| | 60T | 27.87 | 20.65 | 7.22 | 0.0067 | - | - | - | - | 0.403 | $5.36 \times 10^{-3}$ | $13.3 \times 10^{-3}$ | $13.3 \times 10^{-3}$ | - |
| | SF12A | 25.01 | 20.76 | 4.25 | 0.0525 | - | - | - | - | 3.16 | $42.03 \times 10^{-3}$ | $13.3 \times 10^{-3}$ | $13.3 \times 10^{-3}$ | - |
| | SF12B | 26.07 | 19.87 | 6.2 | 0.0136 | - | - | - | - | 0.817 | $10.88 \times 10^{-3}$ | $13.3 \times 10^{-3}$ | $13.3 \times 10^{-3}$ | - |
| | Clr11 | 21.5 | 16.3 | 5.2 | 0.0272 | - | - | - | - | 1.635 | $21.76 \times 10^{-3}$ | $13.3 \times 10^{-3}$ | $13.3 \times 10^{-3}$ | - |
| | AdCol | 23.22 | 17.06 | 6.16 | 0.014 | - | - | - | - | 0.841 | $11.18 \times 10^{-3}$ | $13.3 \times 10^{-3}$ | $13.3 \times 10^{-3}$ | - |
| | Calib. RNA | - | - | - | - | 25.0 | 19.09 | 5.91 | 0.0166 | 0.0166/ 0.0166 = 1 | - | - | - | - |

Fig. 9: CPT-11/C 225 Mediated Tumor Response and EGF-R Gene Expression

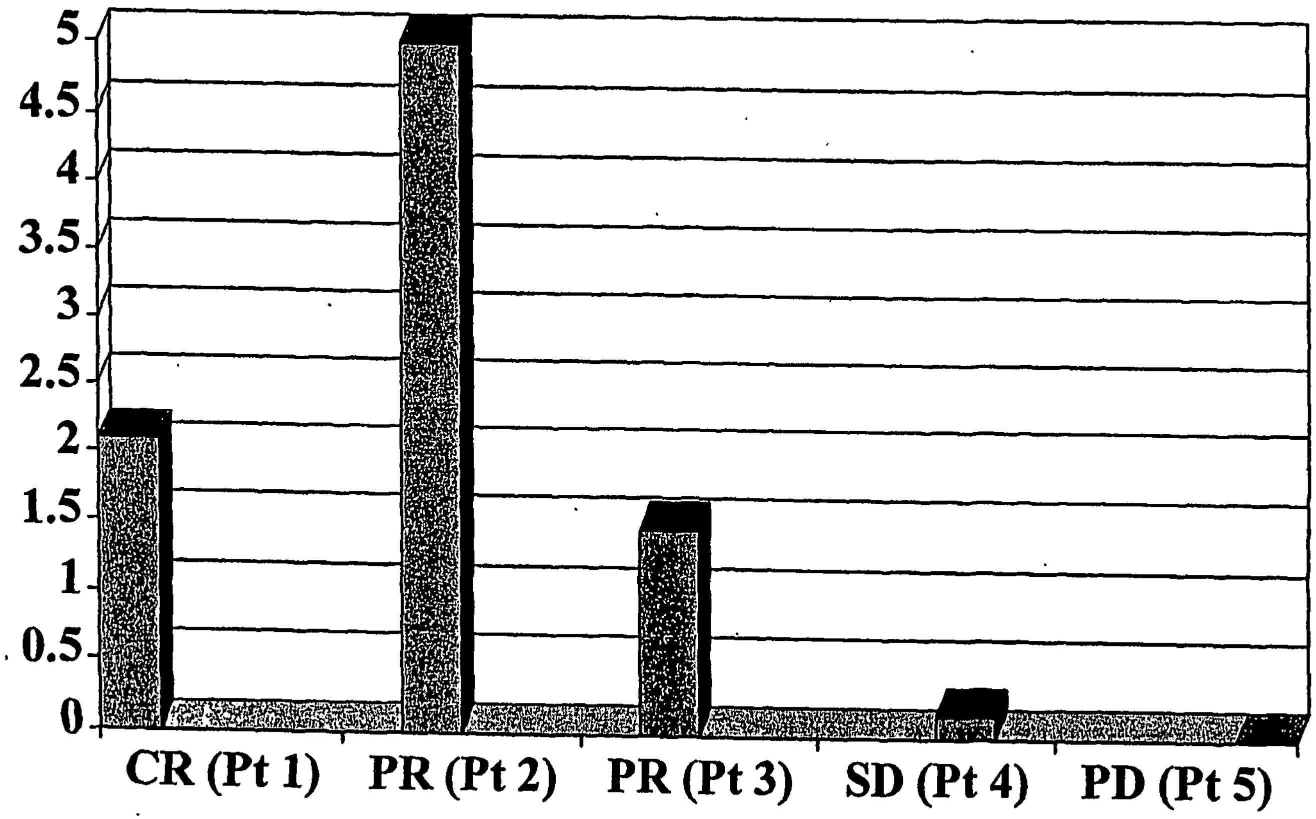

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0400586 A
- EP 0211363 A
- EP 0304493 A
- EP 0322738 A
- EP 0520722 A
- EP 0566226 A
- US 5346994 A
- US 6248535 B
- WO 9220642 A
- WO 9414808 A
- US 5330992 A
- US 5217999 A
- US 5302606 A
- EP 0566266 A
- WO 9403427 A
- WO 9221660 A
- WO 9115495 A


### Non-patent literature cited in the description


- **Ginsberg et al.** Cancer. Principles in Practice of Oncology. Lipincott-Raven Publishers, 1997, 858-910
- *Drugs of the Future,* 1992, vol. 17, 119
- **Wilks.** *Advances in Cancer Research,* 1993, vol. 60, 43-73
- **Sainsbury et al.** *Brit. J. Cancer,* 1988, vol. 58, 458
- **Guerin et al.** *Oncogene Res.,* 1988, vol. 3, 21
- **Hendler et al.** *Cancer Cells,* 1989, vol. 7, 347
- **Neal et al.** *Lancet,* 1985, 366
- **Mukaida et al.** *Cancer,* 1991, vol. 68, 142
- **Bolen et al.** *Oncogene Res.,* 1987, vol. 1, 149
- **Konaka et al.** *Cell,* 1984, vol. 37, 1035
- **Hunter.** *Cell,* 1987, vol. 50, 823
- **W J Gullick.** *Brit. Med. Bull.,* 1991, vol. 47, 87
- **Yarden ; Ullrich.** *Annu. Rev. Biochem.,* 1988, vol. 57, 443
- **Ullrich ; Schlessinger.** *Cell,* 1990, vol. 61, 203
- **Carraway et al.** *Cell,* 1994, vol. 78, 5
- **Carraway et al.** *J. Biol. Chem.,* 1994, vol. 269, 14303
- **Dougall et al.** *Oncogene,* 1994, vol. 9, 2109
- **Samata et al.** *Proc. Natl. Acad. Sci. USA,* 1994, vol. 91, 1711
- **Yaish et al.** *Science,* 1988, vol. 242, 933
- **Toi et al.** *Eur. J. Cancer Clin. Oncol.,* 1990, vol. 26, 722
- **Yoneda et al.** *Cancer Research,* 1991, vol. 51, 4430
- **Denny.** *Farmaco,* January 2001, vol. 56 (1-2), 51-6
- **Scheurle et al.** *Anticancer Res,* 2000, vol. 20, 2091-2096
- **Veale et al.** *Br. J. Caner,* 1993, vol. 68, 162-165
- **Fujino et al.** *Eur. Cancer,* 1996, vol. 32, 2070-2074
- **Rusch et al.** *Cancer Res,* 1993, vol. 53, 2379-2385
- **Pfeiffer et al.** *Br J Cancer,* 1996, vol. 74, 86-91
- **Pastorino et al.** *J Clin Oncol,* 1997, vol. 15, 2858-2865
- **Veale et al.** *Br. J. Caner,* 1987, vol. 55, 513-516
- **Rusch.** *Cancer Res,* 1993, vol. 53, 2379-2385
- **Pastorino et al.** *J.Cin.Onc.,* 1997, vol. 15, 2858-2865
- **Tateishi et al.** *Eur J Cancer,* 1991, vol. 27, 1372-75
- **Rachwal et al.** *Br J Cancer,* 1995, vol. 72, 56-64
- **Rusch et al.** *Cancer Res,* 1993, vol. 15, 2379-85
- **Pfeiffer et al.** *Br J Cancer,* 1998, vol. 78, 96-9
- **Ohsaki et al.** *Oncol Rep,* 2000, vol. 7, 603-7
- **Pastorino et al.** *J.Clin.Onc.,* 1997, vol. 15, 2858-2865
- **Ohsaki et al.** *Oncol. Rep.,* 2000, 603-7
- **Schneider et al.** *Cancer Res,* 1989, vol. 49, 4968-4971
- **Kern et al.** *Cancer Res.,* 1990, vol. 50, 5184-5191
- **Weiner et al.** *Cancer Res,* 1990, vol. 50, 421-425
- **Scheurle et al.** *Anticancer Res.,* 2000, vol. 20, 2091-2096
- **Kern et al.** *Cancer Res,* 1990, vol. 50, 5184-5191

- **Kern et al.** *J Clin Invest,* 1994, vol. 93, 516-20
- **Pfeiffer et al.** *Br. J. Cancer,* 1996, vol. 74, 86-91
- **Tateishi et al.** *Eur. J. Cancer,* 1991, vol. 27, 1372-75
- **Shi et al.** *Mol Carcing,* 1992, vol. 5, 213-8
- **Bongiorno et al.** *J Thorac Cardiovasc Surg,* 1994, vol. 107, 590-5
- **Harpole et al.** *Clin Cancer Res,* 1995, vol. 1, 659-64
- **Volm et al.** *Anticancer Res,* 1992, vol. 12, 11-20
- **Jacobs et al.** *J Clin Oncol,* 1999, vol. 17, 1983-1987
- **Kanehisa.** *Nucleic Acids Res.,* 1984, vol. 12, 203-213
- **Mafune et al.** *Clin Cancer Res,* 1999, vol. 5, 4073-4078
- **Miller et al.** *Biometrics,* 1982, vol. 38, 1011-1016
- **Halpern.** *Biometrics,* 1982, vol. 38, 1017-1023
- **Sambrook ; Fischer ; Maniatis.** Molecular Cloning, a laboratory manual. Cold Spring Harbor Laboratory Press, 1989
- **Plenat et al.** *Ann Pathol,* January 2001, vol. 21 (1), 29-47
- **Heid et al.** *Genome Res,* 1996, vol. 6, 986-994
- **Gibson et al.** *Genome Res,* 1996, vol. 6, 995-1001
- **Eads et al.** *Cancer Research,* 1999, vol. 59, 2302-2306
- **Sambrook ; Fischer ; Maniatis.** Molecular Cloning; a laboratory manual. Cold Spring Harbor Laboratory Press, 1989
- Current Protocols in Molecular Biology. John Wiley and Sons, 1994
- **Bonvini et al.** *Cancer Res.,* 15 February 2001, vol. 61 (4), 1671-7
- **Fingl et al.** The Pharmacological Basis of Therapeutics. 1975, 1
- Remington’s Pharmaceutical Sciences. Mack Publishing Co, 1990
- **Pike.** *J R Stat Soc Series A,* 1972, vol. 135, 201-203
- **Mendelsohn.** *Endocr Relat Cancer,* March 2001, vol. 8 (1), 3-9